# EUROPEAN PATENT SPECIFICATION

(11) **EP 1 917 013 B1**
(45) Date of publication and mention of the grant of the patent: **09.03.2011**
(21) Application number: 06777058.6
(22) Date of filing: 21.08.2006
(51) Int. Cl.: A61K 31/403

(54) **USE OF AZABICYCLO HEXANE DERIVATIVES**
VERWENDUNG VON AZABICYCLOHEXAN-DERIVATEN
UTILISATION DE DERIVES AZABICYCLO

(30) Priority: 22.08.2005 GB 0517193
(43) Date of publication of application: 07.05.2008
(73) Proprietor: GLAXO GROUP LIMITED, Greenford, Middlesex UB6 0NN (GB)
(72) Inventor: HAMPRECHT, Dieter, I-37135 Verona (IT); HEIDBREDER, Christian, I-37135 Verona (IT); MELOTTO, Sergio, I-37135 Verona (IT); MICHELI, Fabrizio, I-37135 Verona (IT); YAMADA, Tadataka, Philadelphia, Pennsylvania 19102-1225 (US)
(74) Representative: Dolton, Peter Irving Ernest
(86) International application number: PCT/EP2006/008314
(87) International publication number: WO 2007/022980

(56) References cited:
- WO-A-2005/080832

## Description

The present invention provides a new use of a D3 antagonist of formula (I), as disclosed in the International Patent Application WO 2005/08032, in the manufacture of a medicament for the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and in the manufacture of a medicament for the treatment of premature ejaculation.

The DSM-IV sets forth two indicia of compulsion. First, the person has repetitive behaviors or mental acts that the person feels driven to perform in response to an obsession or according to rules that must be applied rigidly. Repetitive behaviors include hand washing, ordering and checking, while mental acts include praying, counting and repeating words silently. Second, the behaviors or mental acts are aimed at preventing some dreaded event or situation; however, these behaviors or mental acts either are not connected in a realistic way to what they are designed to neutralize or prevent, or are clearly excessive.

Individuals who meet the DSM-IV criteria for OCD can be scored using the Yale-Brown Obsessive-Compulsive Scale (Y-BOCS): Y-BOCS scores range from 0 to 40. Generally, 0 to 7 is considered a subclinical syndrome, 8- 15 is considered mild, 16-23 is considered moderate, 24-31 is considered severe, and 32-40 is considered extremely severe.

A wide range of psychiatric and neuropsychiatric disorders appear to be related to OCD and form a family of related disorders referred to as obsessive-compulsive (OC) spectrum disorders. Obsessive-compulsive spectrum disorders include somatoform disorders including body dysmorphic disorder and hyperchondriasis, bulimia nervosa, anorexia nervosa, binge eating, paraphilia and nonparaphilic sexual addictions, Sydeham's chorea, torticollis, autism, and movement disorders, including Tourette's syndrome.

Somatoform disorders include body dysmorphic disorder (BDD) and hyperchondriasis. Body dysmorphic disorder (BDD) is a preoccupation with an imagined slight defect in appearance that causes significant distress or impairment in functioning. Individuals suffering from BDD have preoccupations similar to OCD obsessions in that they have repetitive intrusive thoughts, often perform time-consuming, repetitive and sometimes ritualistic behaviours. Hypochondriasis is a preoccupation with the fear of having, or the idea that one has, a serious disease based on the person's misinterpretation of bodily signs or symptoms. Hypochondriacal preoccupations resemble OCD obsessions in that they are often experienced as intrusive and persistent, and the individuals often display repetitive checking behaviours.

The DSM-IV defines anorexia nervosa as a refusal to maintain a minimally normal body weight; intensive fear of gaining weight or becoming fat even though underweight; significant disturbance in perception of body shape or size; and, in females, amenorrhea. The DSM- IV defines bulimia nervosa as recurrent episodes of binge eating followed by inappropriate compensatory behaviours designed to prevent a weight gain. BED is characterized by recurrent episodes of binge eating in the absence of regular use of inappropriate compensatory behaviours. There is some overlap among anorexia nervosa, bulimia nervosa, and BED. However, all three disorders are characterized by a core preoccupation with food and body weight. Individuals suffering from these disorders often perform specific rituals, and have an abnormal preoccupation with food and weight.

Individuals suffering from paraphilias and nonparaphilic sexual addictions (NPSAs) experience similar increasing senses of tension or arousal before committing the act, then pleasure, gratification or relief at the time of committing the act.

Tourette's syndrome is a chronic neuropsychiatric disorder characterized by motor tics and one or more vocal tics beginning before the age of 18 years. The DSM-IV defines a tic as a sudden, rapid, recurrent, nonrhythmic, stereotyped motor movement or vocalization. Tourette's syndrome patients may be able to suppress tics for varying lengths of time, but eventually experience them as irresistible and perform them. Tourette's patients exhibit obsessions resembling OCD obsessions, for example, they often feel the need to perform tics until they are felt to be "just right."

Autism is characterized by difficulties with social interaction, speech and communication, and by a compulsive core. Autistic individuals often display compulsive, repetitive behaviors.

Thus there is a need for a therapeutic agent for the treatment of patients suffering from the somatoform disorders as defined above.

The present invention provides a new use of a D3 antagonist in the manufacture of a medicament for the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, and in the manufacture of a medicament for the treatment of premature ejaculation.

Also provided is a D3 antagonist for use in the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and in the treatment of premature ejaculation.

In another aspect this invention provides a method of treating a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; or premature ejaculation, comprising administering to a mammal in need thereof an effective amount of a D3 antagonist.

The present invention provides a new use of a D3 antagonist in the manufacture of a medicament for the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and in the manufacture of a medicament for the treatment of premature ejaculation.

Also provided is a D3 antagonist for use in the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and in the treatment of premature ejaculation.

In another aspect, this invention provides a method of treating a somatoform disorder such as body dysmorphic disorder or hyperchondriasis, bulimia nervosa, anorexia nervosa, binge eating, paraphilia and nonparaphilic sexual addictions, Sydeham's chorea, torticollis, and a method of treating premature ejaculation, comprising administering to a mammal in need thereof an effective amount of a D3 antagonist.

"Treatment" includes prophylaxis, where this is appropriate for the relevant condition(s).

In one embodiment, the present invention provides a new use of compounds of formula (I) or a pharmaceutically acceptable salt or solvate thereof: 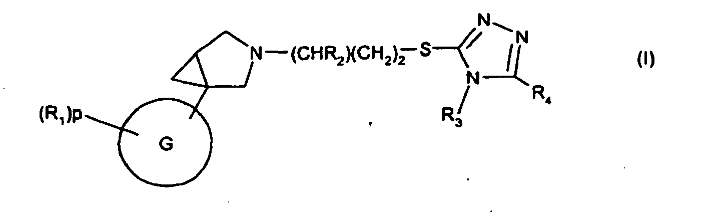 wherein:
- G: is selected from a group consisting of: phenyl, pyridyl, benzothiazolyl, indazolyl;
- p: is an integer ranging from 0 to 5;
- R₁: is independently selected from a group consisting of: halogen, hydroxy, cyano, C₁₋₄alkyl, haloC₁₋₄alkyl, C₁₋₄alkoxy, haloC₁₋₄alkoxy, C₁₋₄alkanoyl; or corresponds to a group R₅;
- R₂: is hydrogen or C₁₋₄alkyl;
- R₃: is C₁₋₄alkyl;
- R₄: is hydrogen, or a phenyl group, a heterocyclyl group, a 5- or 6-membered heteroaromatic group, or a 8- to 11-membered bicyclic group, any of which groups is optionally substituted by 1, 2, 3 or 4 substituents selected from the group consisting of: halogen, cyano, C₁₋₄alkyl, haloC₁₋₄alkyl, C₁₋₄alkoxy, C₁₋₄alkanoyl;
- R₅: is a moiety selected from the group consisting of: isoxazolyl, -CH₂-N- pyrrolyl, 1,1-dioxido-2-isothiazolidinyl, thienyl, thiazolyl, pyridyl, 2- pyrrolidinonyl, and such a group is optionally substituted by one or two substituents selected from: halogen, cyano, C₁₋₄alkyl, haloC₁₋₄alkyl, C₁₋ ₄alkoxy, C₁₋₄alkanoyl; and when R₁ is chlorine and p is 1, such R₁ is not present in the ortho position with respect to the linking bond to the rest of the molecule; and when R₁ corresponds to R₅, p is 1;
in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and in the treatment of premature ejaculation in a mammal.

Thus, a still further aspect of the invention provides a method of treating a somatoform disorder as defined above or premature ejaculation, which comprises administering to a mammal (e.g. human) in need thereof an effective amount of a compound of formula (I) as herein defined or a salt thereof.

Also provided is a compound of formula (I) or a pharmaceutically acceptable salt or solvate thereof for use in the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis; and in the treatment of premature ejaculation.

In one embodiment, the somatoform disorder is binge eating.

"Treatment" includes prophylaxis, where this is appropriate for the relevant condition(s).

Because of the presence of the fused cyclopropane compounds of formula (I) are believed to have a "*cis*" disposition of the substituents (both groups linked to the bicyclic ring system are on the same face of this bicyclic ring system).

In another embodiment of the present invention a new use is provided for compounds of formula (I)' and salts thereof which correspond to the compounds of formula (I) having "cis" disposition, represented by the bold highlight of the bonds: 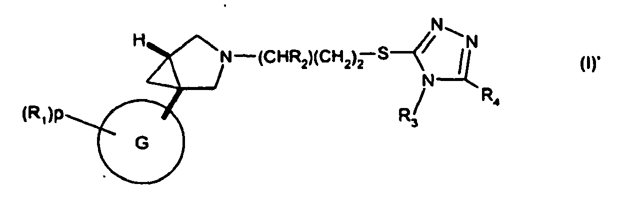 wherein G, p, R₁, R₂, R₃, R₄, and R₅ are defined as above for compounds of formula (I), in the manufacture of a medicament for the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder as defined above; and premature ejaculation, which comprises administering to a mammal (e.g. human) in need thereof an effective amount of a compound of formula (I)' as herein defined or a salt thereof.

Also provided is a compound of formula (I)' or a salt thereof for use in the treatment of a somatoform disorder and premature ejaculation.

In one embodiment, the somatoform disorder is binge eating.

In a further embodiment of the present invention a new use is provided for compounds of formula (IA) that correspond to stereochemical isomers of compounds of formula (I)', enriched in configuration (1S,5R) (or (1 R,5R) when G is 2-pyridyl); 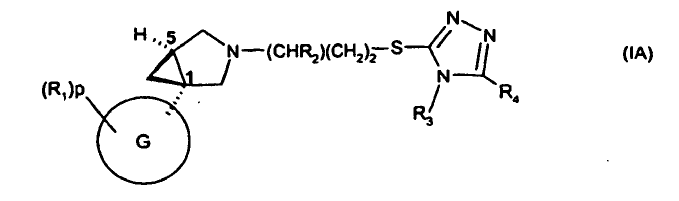 wherein G, p, R₁, R₂, R₃, R₄, and R₅ are defined as above for compounds of formula (I)' or a salt thereof, in the manufacture of a medicament for the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IA) as herein defined or a salt thereof.

Also provided is a compound of formula (IA) or a salt thereof for use in the treatment of a somatoform disorder as defined above and premature ejaculaiton.

In one embodiment, the somatoform disorder is binge eating.

It is intended in the context of the present invention that stereochemical isomers enriched in configuration (1S,5R) or (1R,5R) of formula (IA) correspond in one embodiment to at least 90% e.e. In another embodiment the isomers correspond to at least 95% e.e. In another embodiment the isomers correspond to at least 99% e.e.

In another embodiment of the present invention a new use is provided of the following stereochemical isomers enriched in configuration (1 R,5S):
5-[5-({3-[(1R,5S)-1-(4-Methoxyphenyl)-3-azabicyclo[3.1.0]hex-3-yl]propyl}thio)-4-methyl-4H-1,2,4-triazol-3-yl]-2-methylquinoline, Enantiomer 2;
5-[5-({3-[(1*R*,5*S*)-1-(4-Bromophenyl)-3-azabicyclo[3.1.0]hex-3-yl]propyl}thio)-4-methyl-4H-1,2,4-triazol-3-yl]-2-methylquinoline, Enantiomer 1;
5-[5-({3-[(1*R*,5*S*)-1-(4-tert-Butylphenyl)-3-azabicyclo[3.1.0]hex-3-yl]propyl}thio)-4-methyl-4H-1,2,4-triazol-3-yl]-2-methylquinoline, Enantiomer 1;
(1*R*,5*S*)-3-(3-{[4-Methyl-5-(4-methyl-1,3-oxazol-5-yl)-4*H*-1,2,4-triazol-3-yl]thio}propyl)-1-[3-(trifluoromethyl)phenyl]-3-azabicyclo[3.1.0]hexane, Enantiomer 2;
(1R,5S)-1-(3-Chlorophenyl)-5-methyl-3-(3-{[4-methyl-5-(4-methyl-1,3-oxazol-5-yl)-4*H-*1,2,4-triazol-3-yl]thio}propyl)-3-azabicyclo[3.1.0]hexane, Enantiomer 2:
1-[5-[(1*R*,5*S*)-3-(3-{[4-Methyl-5-(4-methyl-1,3-oxazol-5-yl)-4*H*-1,2,4-triazol-3-yl]thio}-propyl)-3-azabicyclo[3.1.0]hex-1-yl]-2-(methyloxy)phenyl]-1-propanone, Enantiomer 2;
2-Methyl-5-[(1*R*,5*S*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]-1,3-benzothiazole, Enantiomer 2;
   or a salt thereof in the manufacture of a medicament for the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, and premature ejaculation in a mammal.

Thus, a still further aspect of the invention provides a method of treating a somatoform disoder as defined above and premature ejaculation, which comprises administering to a mammal (e.g. human) in need thereof an effective amount of a compound from the list cited above or a salt thereof.

Also provided is a compound from the list cited above or a salt thereof for use in the treatment of a somatoform disorder as defined above and premature ejaculation.

In one embodiment, the somatoform disorder is binge eating.

The term "5- or 6-membered heteroaromatic group" refers to a monocyclic 5- or 6-membered heterocyclic group containing 1, 2, 3 or 4 heteroatoms, for example from 1 to 3 heteroatoms, selected from O, N and S. When .the group contains 2-4 heteroatoms, one may be selected from O, N and S and the remaining heteroatoms may be N. Examples of 5 and 6-membered heteroaromatic groups include pyrrolyl, imidazolyl, pyrazolyl, oxazolyl, isoxazolyl, oxadiazolyl, isothiazolyl, thiazolyl, furyl, thienyl, thiadiazolyl, pyridyl, triazolyl, triazinyl, pyridazinyl, pyrimidinyl and pyrazinyl.

The term "C₁₋₄alkyl" refers to an alkyl group having from one to four carbon atoms, in all isomeric forms, such as methyl, ethyl, propyl, isopropyl, butyl, isobutyl, sec-butyl and tert-butyl. The term "n-C₁₋₄alkyl" refers to the unbranched alkyls as defined above.

The term "C₁₋₄alkoxy" refers to a straight chain or branched chain alkoxy (or "alkyloxy") group having from one to four carbon atoms, such as methoxy, ethoxy, propoxy, isopropoxy, butoxy, isobutoxy, sec-butoxy and tert-butoxy.

The term "halogen" and its abbreviation "halo" refer to fluorine (F), chlorine (Cl), bromine (Br) or iodine (I). Where the term "halo" is used before another group, it indicates that the group is substituted by one, two or three halogen atoms. For example, "haloC₁₋₄alkyl" refers to groups such as trifluoromethyl, bromoethyl, trifluoropropyl, and other groups derived from C₁₋₄alkyl groups as defined above; and the term "haloC₁₋₄alkoxy" refers to groups such as trifluoromethoxy, bromoethoxy, trifluoropropoxy, and other groups derived from C₁₋₄alkoxy groups as defined above.

The term "8- to 11-membered bicyclic group" refers to a bicyclic ring system containing a total of 8, 9, 10 or 11 carbon atoms, wherein 1, 2, 3 or 4 or 5 of the carbon atoms are optionally replaced by a heteroatom independently selected from O, S and N. The term includes bicyclic systems wherein both rings are aromatic, as well as bicyclic ring systems wherein one of the rings is partially or fully saturated. Examples of 8- to 11- membered bicyclic groups wherein both rings are aromatic include indenyl, naphthyl and azulenyl. Examples of 8- to 11-membered bicyclic groups having 1, 2, 3, 4 or 5 heteroatoms, in which both rings are aromatic, include: 6H-thieno[2,3-b]pyrrolyl, imidazo[2,1-b][1,3]thiazolyl, imidazo[5,1-b][1,3]thiazolyl, [1,3]thiazolo[3,2-b][1,2,4]triazolyl, indolyl, isoindolyl, indazolyl, benzimidazolyl e.g. benzimidazol-2-yl, benzoxazolyl e.g. benzoxazol-2-yl, benzisoxazolyl, benzothiazolyl, benzisothiazolyl, benzothienyl, benzofuranyl, naphthridinyl, quinolyl, quinoxalinyl, quinazolinyl, cinnolinyl and isoquinolyl. Examples of 8- to 11-membered bicyclic groups having 1, 2, 3 , 4 or 5 heteroatoms, in which one of the rings is partially or fully saturated includes dihydrobenzofuranyl, indanyl, tetrahydronaphthyl, indolinyl, isoindolinyl, tetrahydroisoquinolinyl, tetrahydroquinolyl, benzoxazinyl and benzoazepinyl.

The term "heterocyclyl" refers to a 5 or 6-membered monocyclic or 8 to 11-membered bicyclic group wherein 1, 2, 3, 4 or 5 of the carbon atoms are replaced by a heteroatom independently selected from O, S and N and which is partially or fully saturated. Examples of "heterocyclyl" which are fully saturated 5 or 6-membered monocyclic rings include pyrrolidinyl, imidazolidinyl, pyrazolidinyl, isothiazolyl, thiazolyl, tetrahydrofuranyl, dioxolanyl, piperidinyl, piperazinyl, morpholinyl, thiomorpholinyl, tetrahydrothienyl, dioxanyl, tetrahydro-2*H-*pyranyl and dithianyl. Examples of "heterocyclyl" groups which are partially saturated 5 or 6-membered monocyclic rings include oxazolinyl, isoaxazolinyl, imidazolinyl, pyrazolinyl, 1,2,3,6-tetrahydropyridyl and 3,6-dihydro-2*H-*pyranyl. Examples of "heterocyclyl" groups which are fully saturated 8 to 11-membered bicyclic rings include decahydroquinolinyl, octahydro-2*H-*1,4-benzoxazinyl and octahydro-1*H*-cyclopenta-[b]pyridinyl. Examples of "heterocyclyl" groups which are partially saturated 8 to 11-membered bicyclic rings include 2,3-dihydro-1*H*-indolyl, 1,2,3,4-tetrahydroquinolinyl, 1,2,3,4-tetrahydroisoquinolinyl and 2,3,4,5-tetrahydro-1*H*-3-benzazepinyl.

Any of these groups may be attached to the rest of the molecule at any suitable position.

As used herein, the term "salt" refers to any salt of a compound according to the present invention prepared from an inorganic or organic acid or base, quaternary ammonium salts and internally formed salts. Physiologically acceptable salts are particularly suitable for medical applications because of their greater aqueous solubility relative to the parent compounds. Such salts must clearly have a physiologically acceptable anion or cation. Suitably physiologically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Salts having a non-physiologically acceptable anion or cation are within the scope of the invention as useful intermediates for the preparation of physiologically acceptable salts and/or for use in non-therapeutic, for example, *in vitro,* situations.

In one embodiment, R₁ is halogen, cyano, acetyl, trifluoromethyl, trifluoromethoxy.

In one embodiment, R₂ is hydrogen. In another embodiment R₂ is C₁₋₄ alkyl (e.g. methyl).

In one embodiment, R₅ is a group selected from: isoxazolyl, 2-pyrrolidinonyl, 1,1-dioxido-2-isothiazolidinyl which is optionally substituted by one or two substituents selected from: halogen, cyano, C₁₋₂alkyl (e.g. methyl), haloC₁₋₂alkyl (e.g. trifluoromethyl), C₁₋₂alkoxy (e.g. methoxy). C₁₋₃alkanoyl (e.g. acetyl).

Suitably, R₁ is bromo, fluoro, trifluoromethoxy, cyano, hydroxy, chloro, methoxy, tert-butyl, trifluoromethyl.

Suitably, R₅ is isoxazolyl, 2-pyrrolidinonyl, -CH₂-N-pyrrolyl, 1,1-dioxido-2-isothiazolidinyl, 2-thienyl, 2-pyridyl, 2-thiazolyl.

In one embodiment, p is 1 or 2.

In another embodiment p is 0.

In one embodiment, R₄ may be optionally substituted phenyl (e.g. phenyl, 4-trifluoromethyl-phenyl, 3,4-difluorophenyl), an optionally substituted bicyclic group such as quinolinyl (e.g. 2-methylquinoline, 8-fluoro-2-methylquinoline), an optionally substituted pyranyl (e.g. 4-tetrahydro-2*H-*pyranyl), an optionally substituted pyridinyl (e.g. 3-methyl-2-pyridinyl, 2-methyl-3-pyridinyl, 3-pyridinyl, 2-methyl-6-trifluoromethyl-3-pyridinyl), an optionally substituted pyrazolyl (e.g. 5-chloro-1-methyl-1H-pyrazol-4-yl, 1-methyl-3-trifluoromethyl-1H-pyrazol-4-yl 1,5-dimethyl-1H-pyrazoly-4-yl), an optionally substituted pyrimidyl (e.g. 5-pyrimidinyl), an optionally substituted pyridazinyl (e.g. 4-pyridazinyl), an optionally substituted pyrazinyl (e.g. 5-methyl-2-pyrazinyl), an optionally substituted furanyl (e.g. 3-methyl-2-furanyl, 2,5-dimethyl-3-furanyl), an optionally substituted thienyl (e.g. 5-chloro-2-thienyl), an optionally substituted oxazolyl (e.g. 4-methyl-1,3-oxazol-5-yl, 2-methyl-5-trifluoromethyl-1,3-oxazol-4-yl), an optionally substituted isoxazolyl (e.g. 3-methyl-5-isoxazolyl), an optionally substituted thiazolyl (e.g. 2,4-dimethyl-1,3-thiazol-5-yl), an optionally substituted triazolyl (e.g. 1-methyl-1H-1,2,3-triazol-4-yl).

In one embodiment, R₃ is methyl.

In one embodiment a new use of a compound of formula (IB) or a salt thereof is provided, wherein R₁, p, R₃ and R₄ are as defined for formula (I):

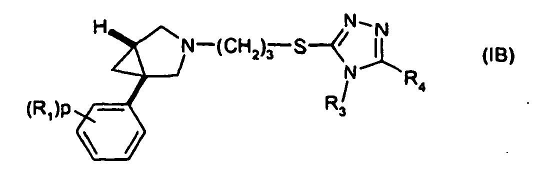

in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IB) as herein defined or a salt thereof.

Also provided is a compound of formula (IB) or a salt thereof for use in the treatment of a somatoform disorder as defined above and premature ejaculation.

In Formula (IB), in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule.

Examples of R₄ include an optionally substituted phenyl (e.g. phenyl, 4-trifluoromethyl-phenyl, 3,4-difluorophenyl), an optionally substituted bicyclic group such as quinolinyl (e.g. 2-methylquinoline, 8-fluoro-2-methylquinoline), an optionally substituted pyranyl (e.g. 4-tetrahydro-2*H-*pyranyl), an optionally substituted pyridinyl (e.g. 3-methyl-2-pyridinyl, 2-methyl-3-pyridinyl, 3-pyridinyl, 2-methyl-6-trifluoromethyl-3-pyridinyl), an optionally substituted pyrazolyl (e.g. 5-chloro-1-methyl-1H-pyrazol-4-yl, 1-methyl-3-trifluoromethyl-1H-pyrazol-4-yl 1,5-dimethyl-1H-pyrazoly-4-yl), an optionally substituted pyrimidyl(e.g. 5-pyrimidinyl), an optionally substituted pyridazinyl (e.g. 4-pyridazinyl), an optionally substituted pyrazinyl (e.g. 5-methyl-2-pyrazinyl), an optionally substituted furanyl (e.g. 3-methyl-2-furanyl, 2,5-dimethyl-3-furanyl), an optionally substituted thienyl (e.g. 5-chloro-2-thienyl), an optionally substituted oxazolyl (e.g. 4-methyl-1,3-oxazol-5-yl, 2-methyl-5-trifluoromethyl-1,3-oxazol-4-yl), an optionally substituted isoxazolyl (e.g. 3-methyl-5-isoxazolyl), an optionally substituted thiazolyl (e.g. 2,4-dimethyl-1,3-thiazol-5-yl), an optionally substituted triazolyl (e.g. 1-methyl-1H-1 ,2,3-triazol-4-yl).

In another embodiment, a new use of a compound of formula (IC) or a salt thereof is provided, wherein R₁, p, R₃ and R₄ are as defined for formula (I): 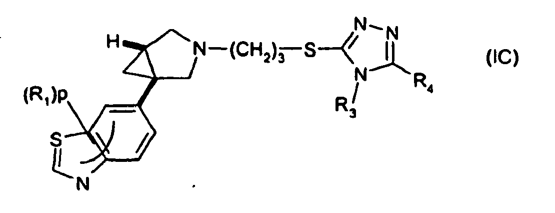 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IC) as herein defined or a salt thereof.

Also provided is a compound of formula (IC) or a salt thereof for use in the treatment of a somatoform disorder as defined above and premature ejaculation.

In Formula (IC), in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule. Examples of R₄ include those defined previously for compounds (IB).

In another embodiment, a new use of a compound of formula (ID) or a salt thereof is provided, wherein R₁, p, R₃ and R₄ are as defined for formula (I): 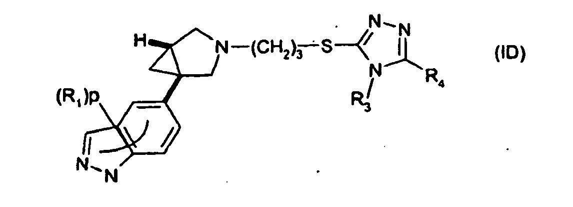 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (ID) as herein defined or a salt thereof.

Also provided is a compound of formula (ID) or a salt thereof for use in the treatment of a somatoform disorder as defined above and premature ejaculation.

In Formula (ID), in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule. Examples of R₄ include those defined previously for compounds (IB).

In another embodiment, a new use of a compound of formula (IE) or a salt thereof is provided, wherein G is 2-pyridyl or 3-pyridyl and R₁, p, R₃ and R₄ are as defined for formula (I): 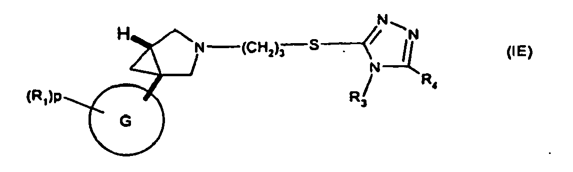 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IE) as herein defined or a salt thereof.

Also provided is a compound of formula (IE) or a salt thereof for use in the treatment of a somatoform disorder and premature ejaculation.

In Formula (IE), in one embodiment, G corresponds to 2-pyridyl (Compounds (IE₁)) and in another embodiment to 3-pyridyl (Compounds (IE₂)), as illustrated below:

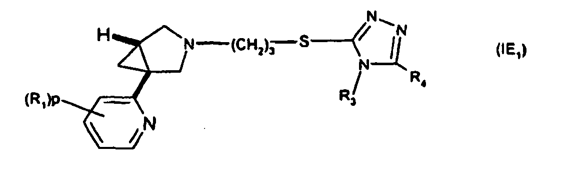

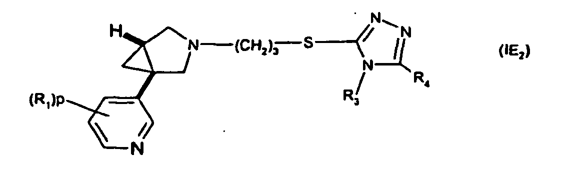

In Formulae (IE), (IE₁) and (IE₂), in one embodiment, R₃ is methyl. R₄ may be phenyl, heterocyclyl, 5- or 6- membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁-₄alkoxy, fluoroC₁₋₄alkoxy, C₁₋₄alkanoyl; and when R₁ is chlorine and p is 1, such R₁ is not present in the ortho position with respect to the linking bond to the rest of the molecule.

Examples of R₄ include those defined previously for compounds (IB).

In another embodiment, a new use of a compound of formula (IF) or a salt thereof is provided, wherein R₁, p, R₃ and R₄ are as defined for formula (I): 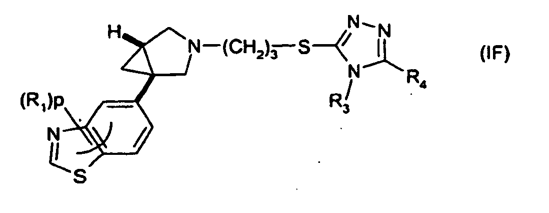 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IF) as herein defined or a salt thereof.

Also provided is a compound of formula (IF) or a salt thereof for use in the treatment of a somatoform disorder as defined above and premature ejaculation.

In Formula (IF), in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule.

Examples of R₄ include those defined previously for compounds (IB).

Further embodiment of the present invention is the new use of compounds of formula (IB)', (IC)', (ID)', and (IF)' which, respectively, correspond to the stereochemical isomers of compounds of formula (IB), (IC), (ID) and (IF) as defined above enriched in configuration (1 S, 5R).

Compounds of formula (IE)' correspond to the stereochemical isomers of compounds of formula (IE) as above defined, enriched in configuration (1R, 5R) or (1R, 5S) depending on the presence of a 2-pyridine ring.

In one embodiment, a new use of stereochemical isomer enriched in the (1S,5R) configuration of formula (IB)' or a salt thereof is provided, wherein R₁, p, R₃ and R₄ are as defined for formula (I): 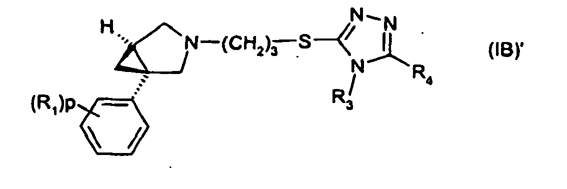 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder and premature ejaculation, which comprises administering to a mammal (e.g. human) in need thereof an effective amount of a compound of formula (IB)' as herein defined or a salt thereof.

Also provided is a compound of formula (IB)' or a salt thereof for use in the treatment of a somatoform disorder and premature ejaculation.

In Formula (IB)', in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule.

Examples of R₄ include optionally substituted phenyl (e.g. phenyl, 4-trifluoromethyl-phenyl, 3,4-difluorophenyl), an optionally substituted bicyclic group such as quinolinyl (e.g. 2-methylquinoline, 8-fluoro-2-methylquinoline), an optionally substituted pyranyl (e.g. 4-tetrahydro-2*H-*pyranyl), an optionally substituted pyridinyl (e.g. 3-methyl-2-pyridinyl, 2-methyl-3-pyridinyl, 3-pyridinyl, 2-methyl-6-trifluoromethyl-3-pyridinyl), an optionally substituted pyrazolyl (e.g. 5-chloro-1-methyl-1H-pyrazol-4-yl, 1-methyl-3-trifluoromethyl-1H-pyrazol-4-yl 1,5-dimethyl-1H-pyrazoly-4-yl), an optionally substituted pyrimidyl (e.g. 5-pyrimidinyl), an optionally substituted pyridazinyl (e.g. 4-pyridazinyl), an optionally substituted pyrazinyl (e.g. 5-methyl-2-pyrazinyl), an optionally substituted furanyl (e.g. 3-methyl-2-furanyl, 2,5-dimethyl-3-furanyl), an optionally substituted thienyl (e.g. 5-chloro-2-thienyl), an optionally substituted oxazolyl (e.g. 4-methyl-1,3-oxazol-5-yl, 2-methyl-5-trifluoromethyl-1,3-oxazol-4-yl), an optionally substituted isoxazolyl (e.g. 3-methyl-5-isoxazolyl), an optionally substituted thiazolyl (e.g. 2,4-dimethyl-1,3-thiazol-5-yl), an optionally substituted triazolyl (e.g. 1-methyl-1H-1,2,3-triazol-4-yl).

In another embodiment, a new use of a stereochemical isomer enriched in the (1S,5R) configuration of formula (IC)' or a salt thereof is provided, wherein R₁, p, R₃ and R₄ are as defined for formula (I): 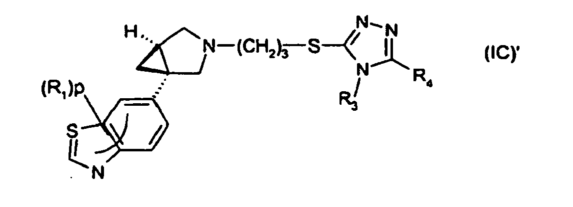 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IC)' as herein defined or a salt thereof.

Also provided is a compound of formula (IC)' or a salt thereof for use in the treatment of a somatoform disorder as defined above and premature ejaculation.

In Formula (IC)', in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule. Examples of R₄ include those defined previously for compounds (IB)'.

In another embodiment, a new use of a stereochemical isomer enriched in the (1S,SR) configuration of formula (ID)' or a salt thereof is provided, wherein R₁, p, R₃ and R₄ are as defined for formula (I): 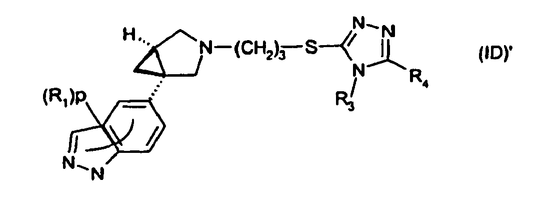 in the manufacture of a medicament for the treatment of of,a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (ID)' as herein defined or a salt thereof.

Also provided is a compound of formula (ID)' or a salt thereof for use in the treatment of a somatoform disorder as defined above and premature ejaculation.

In Formula (ID)', in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule. Examples of R₄ include those defined previously for compounds (IB)'.

In another embodiment, a new use of a stereochemical isomer enriched in the (1S,5R) configuration or (1R,5R) configuration of formula (IE)' or a salt thereof is provided, wherein G is 2-pyridyl or 3-pyridyl and R₁, p, R₃ and R₄ are as defined for formula (I): 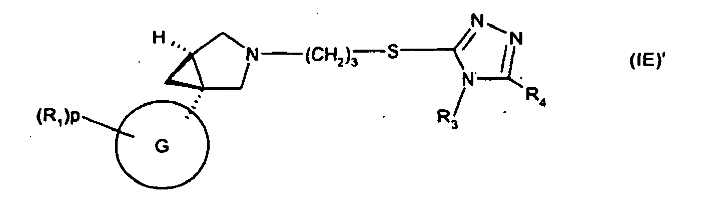 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IE)' as herein defined or a salt thereof.

Also provided is a compound of formula (IE)' or a salt thereof for use in the treatment of a somatoform disorder as defined above and premature ejaculation.

In Formula (IE)', in one embodiment, G corresponds to 2-pyridyl (Compounds (IE₁)') and in another embodiment to 3-pyridyl (Compounds (IE₂)'), as illustrated below:

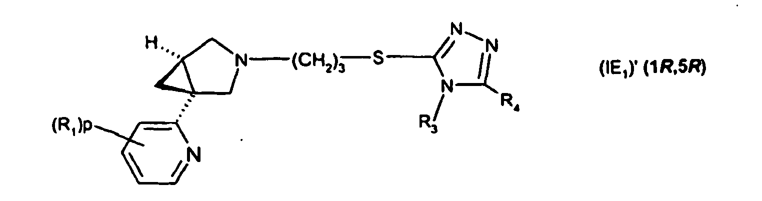

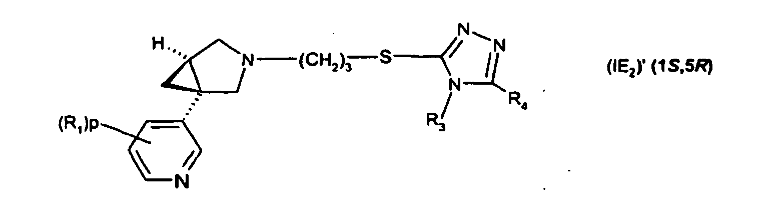

The configuration will then change depending on the type of pyridine ring, as mentioned above.

In Formulae (IE)', (IE₁)' and (IE₂)', in one embodiment, R₃ is methyl. R₄ may be phenyl, heterocyclyl, 5- or 6- membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁-₄alkoxy, fluoroC₁₋₄alkoxy, C₁₋₄alkanoyl; and when R₁ is chlorine and p is 1, such R₁ is not present in the ortho position with respect to the linking bond to the rest of the molecule. Examples of R₄ include those defined previously for compounds (IB)'.

In another embodiment, a new use of a stereochemical isomer enriched in the (1 S,5R) configuration of formula (IF)' or a salt thereof is provided, wherein R₁, p, R₃ and R₄ are as defined for formula (I): 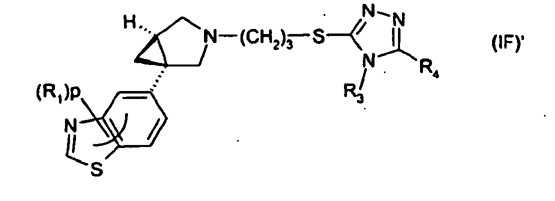 in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder as defined above and premature ejaculation, which comprises administering to a mammal (e.g. human) in need thereof an effective amount of a compound of formula (IF)' as herein defined or a salt thereof.

Also provided is a compound of formula (IF)' or a salt thereof for use in the treatment of a somatoform disorder as defined above and premature ejaculation.

In Formula (IF)', in one embodiment, R₃ is methyl. R₄ may be phenyl, heterocyclyl, 5- or 6-membered heteroaromatic group or a 9- to 11-membered bicyclic group, any of which is optionally substituted by 1, 2, 3 or 4 substituents selected from the group consisting of: halogen, hydroxy, oxo, cyano, nitro, C₁₋₄alkyl, fluoroC₁₋₄alkyl, C₁₋₄alkoxy, fluoroC₁₋₄alkoxy, C₁₋₄alkanoyl; and when R₁ is chlorine and p is 1, such R₁ is not present in the ortho position with respect to the linking bond to the rest of the molecule. Examples of R₄ include those defined previously for compounds (IB)'.

Certain of the compounds of the invention may be used as acid addition salts with less than one, or one or more equivalents of the acid. The present invention includes within its scope the use of all possible stoichiometric and non-stoichiometric forms.

Salts may also be prepared from other salts of the compound of formula (I) using conventional methods.

When administered, the formulations used in the invention are applied in pharmaceutically acceptable amounts and in pharmaceutically acceptable compositions. Such preparations may routinely contain salts, buffering agents, preservatives, compatible carriers, and optionally other therapeutic ingredients.

In the present invention, compounds of formula (I) are administered in safe and effective amounts. An effective amount means that amount necessary to delay the onset of, inhibit the progression of, halt altogether the onset or progression of or diagnose the particular condition being treated. In general, an effective amount for treating an obsessive compulsive spectrum disorder will be that amount necessary to inhibit mammalian symptoms of the particular obsessive compulsive spectrum disorder in-situ. When administered to a subject, effective amounts will depend, of course, on the particular condition being treated; the severity of the condition; individual patient parameters including age, physical condition, size and weight; concurrent treatment; frequency of treatment; and the mode of administration. These factors are well known to those of ordinary skill in the art and can be addressed with no more than routine experimentation. It is preferred generally that a minimum dose be used, that is, the lowest safe dosage that provides appropriate relief of symptoms.

Dosage may be adjusted appropriately to achieve desired drug levels, locally or systemically.

A variety of administration routes are available. The particular mode selected will depend of course, upon the particular drug selected, the severity of the disease state(s) being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, sublingual, topical, nasal, transdermal or parenteral routes. The term "parenteral" includes subcutaneous, intravenous, intramuscular, or infusion. Intravenous routes are preferred.

The compositions may conveniently be presented in unit dosage form and may be prepared by any of the methods well known in the art of pharmacy. In general, the compositions are prepared by uniformly and intimately bringing the compounds into association with a liquid carrier, a finely divided solid carrier, or both, and then, if necessary, shaping the product.

Compositions suitable for oral administration may be presented as discrete units such as capsules, cachets, tablets, or lozenges, each containing a predetermined amount of the active compound. Other compositions include suspensions in aqueous liquors or nonaqueous liquids such as a syrup, an elixir, or an emulsion.

Other delivery systems can include time- release, delayed release or sustained release delivery systems. Such systems can avoid repeated administrations of the active compounds of the invention, increasing convenience to the subject and the physician. Many types of release delivery systems are available and known to those of ordinary skill in the art.

In one embodiment the present invention provides a new use of a compound of formula (I) selected from the following group consisting of:
5-[5-({3-[(*1R*,*5S*/*1S*,*5R*)-1-(4-Methoxyphenyl)-3-azabicyclo[3.1.0]hex-3-yl]propyl}thio)-4-methyl-4H-1,2,4-triazol-3-yl]-2-methylquinoline;
5-[5-({3-[(1*S*,5*R*)-1-(4-Methoxyphenyl)-3-azabicyclo[3.1.0]hex-3-yl]propyl}thio)-4-methyl-4H-1,2,4-triazol-3-yl]-2-methylquinoline, Enantiomer1;
5-[5-({3-[(*1R*,*5S*/*1S*,*5R*)-1-(4-Bromophenyl)-3-azabicyclo[3.1.0]hex-3-yl]propyl}thio)-4-methyl-4H-1,2,4-triazol-3-yl]-2-methylquinoline;
5-[5-({3-[(*1S*,*5R*)-1-(4-Bromophenyl)-3-azabicyclo[3.1.0]hex-3-yl]propyl}thio)-4-methyl-4H-1,2,4-triazol-3-yl]-2-methylquinoline, Enantiomer 2;
2-Methyl-5-[4-methyl-5-({3-[(1*R*,5*S*/1*S*,5*R*)-1-phenyl-3-azabicyclo[3.1.0]hex-3-yl]propyl}thio)-4H-1,2,4-triazol-3-yl]quinoline;
2-Methyl-5-[4-methyl-5-({3-[(*1S*,*5R*)-1-phenyl-3-azabicyclo[3.1.0]hex-3-yl]propyl}thio)-4H-1,2,4-triazol-3-yl]quinoline, Enantiomer 2:
5-[5-({3-[(*1R*,*5S*/*1S*,*5R*)-1-(3,4-Dichlorophenyl)-3-azabicyclo[3.1.0]hex-3-yl]propyl}thio)-4-methyl-4H-1,2,4-triazol-3-yl]-2-methylquinoline;
5-[5-({3-[(*1S*,*5R*)-1-(3,4-Dichlorophenyl)-3-azabicyclo[3.1.0]hex-3-yl]propyl}thio-4-methyl-4H-1,2,4-triazol-3-yl]-2-methylquinoline, Enantiomer 1;
5-[5-({3-[(1R,5S/1S,5R)-1-(4-tert-Butylphenyl)-3-azabicyclo[3.1.0]hex-3-yilpropyl}thio)-4-methyl-4H-1,2,4-triazol-3-yl]-2-methylquinoline;
5-[5-({3-[(1S,5R)-1-(4-tert-Butylphenyl)-3-azabicyclo[3.1.0]hex-3-yl]propyl}thio)-4-methyl-4H-1,2,4-triazol-3-yl]-2-methylquinoline, Enantiomer 2;
4-[(1R,5S/1S,5R)-3-(3-{[4-Methyl-5-(2-methylquinolin-5-yl)-4H-1,2,4-triazol-3-yl]thio}propyl)-3-azabicyclo[3.1.0]hex-1-yl]benzonitrile;
4-[(1R,5S/1S,5R)-3-(3-{[4-Methyl-5-(2-methylquinolin-5-yl)-4H-1,2,4-triazol-3-yl]thio}propyl)-3-azabicyclo[3.1.0]hex-1-yl]phenol;
(1R,5S/1S,5R)-3-(3-{[4-methyl-5-(4-methyl-1,3-oxazol-5-yl)-4H-1,2,4-triazol-3-yl]thio}propyl)-1-phenyl-3-azabicyclo[3.1.0]hexane;
(1R,5S/1S,5R)-1-(4-Bromophenyl)-3-(3-{[4-methyl-5-(4-methyl-1,3-oxazol-5-yl)-4H-1,2,4-triazol-3-yl]thio}propyl)-3-azabicyclo[3.1.0]hexane;
(1S,5R)-1-(4-Bromophenyl)-3-(3-{[4-methyl-5-(4-methyl-1,3-oxazol-5-yl)-4H-1,2.4-triazol-3-yl]thio}propyl)-3-azabicyclo[3.1.0]hexane, Enantiomer 1;
(1R,5S/1S,5R)-1-(4-tert-Butylphenyl)-3-(3-{[4-methyl-5-(4-methyl-1,3-oxazol-5-yl)-4H-1,2,4-triazol-3-yl]thio}propyl)-3-azabicyclo[3.1.0]hexane;
(1R,5S/1S,5R)-1-(3,4-Dichlrophenyl)-3-(3-{[4-methyl-5-(4-methyl-1,3-oxazol-5-yl)-4H-1,2,4-triazol-3-yl]thio}propyl)-3-azabicyclo[3.1.0]hexane;
(1S,5R)-1-(3,4-Dichlorophenyl)-3-(3-{[4-methyl-5-(4-methyl-1,3-oxazol-5-yl)-4H-1,2,4-triazol-3-yl]thio}propyl)-3-azabicyclo[3.1.0]hexane, Enantiomer 2;
(1R,5S/1S,5R)-1-(4-methoxyphenyl)-3-(3-{[4-methyl-5-(4-methyl-1,3-oxazol-5-yl)-4H-1,2,4-triazol-3-yl]thio}propyl)-3-azabicyclo[3.1.0]hexane;
(1S,5R)-1-(4-methoxyphenyl)-3-(3-{[4-methyl-5-(4-methyl-1,3-oxazol-5-yl)-4H-1,2,4-triazol-3-yl]thio}propyl)-3-azabicyclo[3.1.0]hexane, Enantiomer 2:
(1R,5S/1S,5R)-1-[4-(5-methyl-3-isoxazolyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane;
(1*S*,5*R*)-3-(3-{[4-Methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane;_(1*S*,5*R*)-3-(3-{[4-Methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane; (1*R*,5*S*/1*S*,5*R*)-1-[2-Fluoro-4-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]-hexane;
(1*R*,5*S*/1*S*,5*R*)-3-(3-{[4-Methyl-5-(4-methyl-1,3-oxazol-5-yl)-4*H*-1,2,4-triazol-3-yl]thio}propyl)-1-[3-(trifluoromethyl)phenyl]-3-azabicyclo[3.1.0]hexane;
(1*R*,5*S*/1*S*,5*R*)-1-[4-Fluoro-3-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1.0]hexane;
1-[5-[(*1S*,*5R*/*1R*,5*S*)-3-(3-{[4-Methyl-5-(4-methyl-1,3-oxazol-5-yl)-4*H*-1,2,4-triazol-3-yl]thio}propyl)-3-azabicyclo[3.1.0]hex-1-yl]-2-(methyloxy)phenyl]ethanone;
1-[5-[(*1S*,*5R*)-3-(3-{[4-Methyl-5-(4-methyl-1,3-oxazol-5-yl)-4*H*-1,2,4-triazol-3-yl]thio}propyl)-3-azabicyclo[3.1.0]hex-1-yl]-2-(methyloxy)phenyl]ethanone, Enantiomer 1;
(*1S*,*5R*/*1R*,*5S*)-1-(4-Chlorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane;
(*1S*,*5R*)-1-(4-Chlorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane, Enantiomer 1:
(*1S*,*5R*/*1R*,*5S*)-1-(4-Fluorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane;
(1*S,*5*R*)-1-(4-Fluorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane, Enantiomer 1;
(*1S*,*5R*/*1R*,*5S*)-1-(3-Chlorophenyl)-5-methyl-3-(3-{[4-methyl-5-(4-methyl-1,3-oxazol-5-yl)-4*H*-1,2,4-triazol-3-yl]thio}propyl)-3-azabicyclo[3.1.0]hexane;
(*1S*,*5R*)-1-(3-Chlorophenyl)-5-methyl-3-(3-{[4-methyl-5-(4-methyl-1,3-oxazol-5-yl)-4*H-*1,2,4-triazol-3-yl]thio}propyl)-3-azabicyclo[3.1.0]hexane, Enantiomer 1;
(*1S*,*5R*/*1R*,*5S*)-1-(3-Fluorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane;
(*1S*,*5R*)-1-(3-Fluorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane, Enantiomer 1:
(*1S*,*5R*/*1R*,*5S*)-3-(3-{[4-methyl-5-(4-methyl-1,3-oxazol-5-yl)-4*H*-1,2,4-triazol-3-yl]thio}propyl)-1-[3-(methyloxy)phenyl]-3-azabicyclo[3.1.0]hexane;
(*1S*,*5R*)-3-(3-([4-methyl-5-(4-methyl-1,3-oxazol-5-yl)-4*H*-1,2,4-triazol-3-yl]thio)propyl)-1-[3-(methyloxy)phenyl]-3-azabicyclo[3.1.0]hexane, Enantiomer 1;
(*1S*,*5R*)-1-(4-Bromophenyl)-3-(3-{[4-methyl-5-(tetrahydro-2*H*-pyran-4-yl)-4*H*-1,2,4-triazol-3-yl]thio}propyl)-3-azabicyclo[3.1.0]hexane;
(*1S*,*5R*)-1-(4-Bromophenyl)-3-[3-({4-methyl-5-[4-(trifluoromethyl)phenyl]-4*H*-1,2,4-triazol-3-yl}thio)propyl]-3-azabicyclo[3.1.0]hexane;
(*1S*,*5R*)-1-(4-Bromophenyl)-3-(3-{[4-methyl-5-(3-pyridinyl)-4*H*-1,2,4-triazol-3-yl]thio}-propyl)-3-azabicyclo[3.1.0]hexane,
(*1S*,*5R*)-1-(4-Bromophenyl)-3-(3-{[5-(3,4-difluorophenyl)-4-methyl-4*H*-1,2,4-triazol-3-yl]thio}propyl)-3-azabicyclo[3.1.0]hexane;
5-[5-({3-[(*1S*,*5R*/*1R*,*5S*)-1-(4-Chlorophenyl)-3-azabicyclo[3.1.0]hex-3-yl]propyl}thio)-4-methyl-4*H*-1,2,4-triazol-3-yl]-2-methylquinoline;
5-[5-({3-[(*1S*,*5R*/*1R*,*5S*)-1-(4-Chlorophenyl)-3-azabicyclo[3.1.0]hex-3-yl]propyl}thio)-4-methyl-4*H*-1,2,4-triazol-3-yl]-2-methylquinoline, Enantiomer 1;
(*1S*,*5R*/*1R*,*5S*)-3-(3-{[4-Methyl-5-(4-methyl-1,3-oxazol-5-yl)-4*H*-1,2,4-triazol-3-yl]thio}propyl)-1-{4-[(trifluoromethyl)oxy]phenyl}-3-azabicyclo[3.1.0]hexane;
(*1S*,*5R*)-3-(3-{[4-Methyl-5-(4-methyl-1,3-oxazol-5-yl)-4*H*-1,2,4-triazol-3-yl]thio}propyl)-1-{4-[(trifluoromethyl)oxy]phenyl}-3-azabicyclo[3.1.0]hexane, Enantiomer 1
(*1S*,*5R*/*1R*,*5S*)-3-(3-{[4-Methyl-5-(4-methyl-1,3-oxazol-5-yl)-4*H*-1,2,4-triazol-3-yl]-thio}propyl)-1-[2-methyl-4-(trifluoromethyl)phenyl]-3-azabicyclo[3.1.0]hexane;
(1*S*,5*R*/1*R*,5*S*)-3-(3-{[4-Methyl-5-(tetrahydro-2*H-*pyran-4-yl)-4*H-*1,2,4-triazol-3-yl]thio}propyl)-1-(4-[(trifluoromethyl)oxy]phenyl}-3-azabicyclo[3.1.0]hexane;
(*1S*,*5R*)-3-(3-{[4-Methyl-5-(tetrahydro-2*H*-pyran-4-yl)-4*H*-1,2,4-triazol-3-yl]thio}propyl)-1-{4-[(trifluoromethyl)oxy]phenyl}-3-azabicyclo[3.1.0]hexane, Enantiomer 2;
(1*R*,5*S*/1*S*,5*R*)-1-(3-Bromophenyl)-3-(3-{[4-methyl-5-(4-methyl-1,3-oxazol-5-yl)-4*H*-1,2,4-triazol-3-yl]thio}propyl)-3-azabicyclo[3.1.0]hexane;
(*1S*,*5R*)-3-(1-Methyl-3-{[4-methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane;
(*1S*,*5R*)-3-(1-Methyl-3-{[4-methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane, Diastereoisomer1:
(*1S*, *5R*)-3-(1-Methyl-3-{[4-methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane, Diastereoisomer 2:
(1*R*,5*S*/1*S*,5*R*)-1-[2-Fluoro-5-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]hexane;
(1*S*,5*R*)-1-[2-Fluoro-5-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]hexane, Enantiomer 2;
1-[4-[(1*R*,5*S*/1*S*,5*R*)-3-(3-{[4-Methyl-5-(4-methyl-1,3-oxazol-5-yl)-4*H*-1,2,4-triazol-3-yl]thio}propyl)-3-azabicyclo[3.1.0]hex-1-yl]-2-(methyloxy)phenyl]ethanone;
1-[4-[(1*R*,5*S*/1*S*,5*R*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]-2-(methyloxy)phenyl]-1-propanone;
(1*R*,5*S*/1*S*,5*R*)-3-(3-{[4-Methyl-5-(4-methyl-1,3-oxazol-5-yl)-4*H*-1,2,4-triazol-3-yl]thio}propyl)-1-[2-(trifluoromethyl)phenyl]-3-azabicyclo[3.1.0]hexane;
(1*S*,5*R*)-1-[2-Fluoro-4-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]hexane;
(1S,5R)-3-(3-{[4-Methyl-5-(2-methyl-3-pyridinyl)-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(4-pyridazinyl)-4H-1,2,4-triazol-3-yl]thio}propyl)-1-[4-(trifluoromethyl)phenyl]-3-azabicyclo[3.1.0]hexane;
(1S,5R)-3-(3-{[5-(1,5-Dimethyl-1H-pyrazol-4-yl)-4-methyl-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(5-pyrimidinyl)-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(3-methyl-2-furanyl)-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(6-methyl-3-pyridinyl)-4H-1,2,4-triazol-3-yl]thio}propyl)-1-[4-(trifluoromethyl)phenyl]-3-azabicyclo[3.1.0]hexane;
(1S,5R)-3-(3-{[5-(2,4-Dimethyl-1,3-thiazol-5-yl)-4-methyl-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(5-methyl-2-pyrazinyl)-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(tetrahydro-2H-pyran-4-yl)-4H-1,2,4-triazol-3-yl]thio}propyl)-1-[4-(trifluoromethyl)phenyl]-3-azabicyclo[3.1.0]hexane;
2-Methyl-6-{4-methyl-5-[(3-{(1S,5R)-1-[4-(trifluoromethyl)phenyl]-3-azabicyclo[3.1.0]hex-3-yl}propyl)thio]-4H-1,2,4-triazol-3-yl}quinoline;
8-Fluoro-2-methyl-5-{4-methyl-5-[(3-{(1S,5R)-1-[4-(trifluoromethyl)phenyl]-3-azabicyclo[3.1.0]hex-3-yl}propyl)thio]-4H-1,2,4-triazol-3-yl}quinoline;
2-Methyl-5-{4-methyl-5-[(3-{(1S,5R)-1-[4-(trifluoromethyl)phenyl]-3-azabicyclo[3.1.0]hex-3-yl}propyl)thio]-4H-1,2,4-triazol-3-yl}quinoline;
(1S,5R)-1-[2-Fluoro-4-(trifluoromethyl)phenyl]-3-(3-{[4-methyl-5-(2-methyl-3-pyridinyl)-4H-1,2,4-triazol-3-yl]thio}propyl)-3-azabicyclo[3.1.0]hexane;
(1S,5R)-1-[2-Fluoro-4-(trifluoromethyl)phenyl]-3-(3-{[4-methyl-5-(4-pyridazinyl)-4H-1,2,4-triazol-3-yl]thio}propyl)-3-azabicyclo[3.1.0]hexane;
(1S,5R)-1-[2-Fluoro-4-(trifluoromethyl)phenyl]-3-(3-{[4-methyl-5-(5-pyrimidinyl)-4H-1,2,4-triazol-3-yl]thio}propyl)-3-azabicyclo[3.1.0]hexane;
(1S,5R)-3-(3-{[5-(2,4-Dimethyl-1,3-thiazol-5-yl)-4-methyl-4H-1,2,4-triazol-3-yl]thio}propyl)-1-[2-fluoro-4-(trifluoromethyl)phenyl]-3-azabicyclo[3.1.0]hexane; (1S,5R)-1-[2-Fluoro-4-(trifluoromethyl)phenyl]-3-(3-{[4-methyl-5-(5-methyl-2-pyrazinyl)-4H-1,2,4-triazol-3-yl]thio}propyl)-3-azabicyclo[3.1.0]hexane;
(1*S*,5*R*)-1-[2-Fluoro-4-(trifluoromethyl)phenyl]-3-[3-({4-methyl-5-[4-(trifluoromethyl)phenyl]-4H-1,2,4-triazol-3-yl}thio)propyl]-3-azabicyclo[3.1.0]hexane;
1-{4-[(1*R*,5*S*/1*S*,5*R*)-3-(3-{[4-Methyl-5-(2-methyl-5-quinolinyl)-4*H*-1,2,4-triazol-3-yl]thio}propyl)-3-azabicyclo[3.1.0]hex-1-yl]phenyl}-2-pyrrolidinone;
5-{5-[(3-{(1*R*,5*S*/1*S*,5*R*)-1-[4-(1,1-Dioxido-2-isothiazolidinyl)phenyl]-3-azabicycle-[3.1.0]hex-3-yl}propyl)thio]-4-methyl-4*H*-1,2,4-triazol-3-yl}-2-methylquinoline;
(1*S*,5*R*)-1-[3-Fluoro-4-(trifluoromethyl)phenyl]-5-methyl-3-(3{[4-methyl-5-(4-methyl-1,3-oxazol-5-yl)-4*H*-1,2,4-triazol-3-yl]thio}propyl)-3-azabicyclo[3.1.0]hexane Enantiomer 1;
1-(2-(Methyloxy)-5-{(1*R*,5*S*/*1S*, *5R*)-3-[3-({4-methyl-5-[4-(trifluoromethyl)pheny)]-4*H*-1,2,4-triazol-3-yl}thio)propyl]-3-azabicyclo[3.1.0]hex-1-yl}phenyl)ethanone;
1-[5-[(1*R*,5*S*/*1S*,*5R*)-3-(3-{[5-(3,4-Difluorophenyl)-4-methyl-4*H*-1,2,4-triazol-3-yl]thio}propyl)-3-azabicyclo[3.1.0]hex-1-yl]-2-(methyloxy)phenyl]ethanone;
1-{2-(Methyloxy)-5-[(1*R*,5*S*/*1S*,*5R*)-3-(3-{[4-methyl-5-(3-pyridinyl)-4*H*-1,2,4-triazol-3-yl]thio}propyl)-3-azabicyclo[3.1.0]hex-1-yl]phenyl}ethanone;
1-[5-[(1*R*,5*S*/*1S*,*5R*)-3-(3-{[4-Methyl-5-(2-methyl-5-quinolinyl)-4*H*-1,2,4-triazol-3-yl]thio}propyl)-3-azabicyclo[3.1.0]hex-1-yl]-2-(methyloxy)phenyl]ethanone;
1-{2-(Methyloxy)-5-[(1*R*,5*S*/*1S*,*5R*)-3-(3-{[4-methyl-5-(tetrahydro-2*H*-pyran-4-yl)-4*H*-1,2,4-triazol-3-yl]thio}propyl)-3-azabicyclo[3.1.0]hex-1-yl]phenyl}ethanone;
1-(2-Hydroxy-5-{(1*R*,5*S*/*1S*,*5R*)-3-[3-({4-methyl-5-[4-(trifluoromethyl)phenyl]-4*H*-1,2,4-triazol-3-yl}thio)propyl]-3-azabicyclo[3.1.0]hex-1-yl}phenyl)ethanone;
1-{5-[(1*R*,5*S*/*1S*,*5R*)-3-(3-{[5-(3,4-Difluorophenyl)-4-methyl-4*H*-1,2,4-triazol-3-yl]thio}propyl)-3-azabicyclo[3.1.0]hex-1-y1]-2-hydroxyphenyl}ethanone;
1-{2-Hydroxy-5-[(1*R*,5*S*/*1S*,*5R*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]phenyl}ethanone;
1-{2-Hydroxy-5-[(1*R*,5*S*/*1S*,*5R*)-3-(3-{[4-methyl-5-(2-methyl-5-quinolinyl)-4*H*-1,2,4-triazol-3-yl]thio}propyl)-3-azabicyclo[3.1.0]hex-1-yl]phenyl}ethanone;
1-[5-[(1*R*,5*S*/1*S*,5*R*)-3-(3-{[4-Methyl-5-(4-methyl-1,3-oxazol-5-yl)-4*H*-1,2,4-triazol-3-yl]thio}propyl)-3-azabicyclo[3.1.0]hex-1-yl]-2-(methyloxy)phenyl]-1-propanone;
1-[5-[(*1S*,*5R*)-3-(3-{[4-Methyl-5-(4-methyl-1,3-oxazol-5-yl)-4*H*-1,2,4-triazol-3-yl]thio}-propyl)-3-azabicyclo[3.1.0]hex-1-yl]-2-(methyloxy)phenyl]-1-propanone Enantiomer 1;
2-Methyl-5-[(1*R*,5*S*/*1S*,*5R*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]-1,3-benzothiazole;
2-Methyl-5-[(*1S*,*5R*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]-1,3-benzothiazole, Enantiomer 1;
2-Methyl-6-[(1*R*,5*S*/*1S*,*5R*)-3-(3-{[4-methyl-5-(4-methyl-1,3-oxazo-1-5-yl)-4*H*-1,2,4-triazol-3-yl]thio}propyl)-3-azabicyclo[3.1.0]hex-1-yl]-1,3-benzothiazole;
1-Methyl-5-[(1*R*,5*S*/*1S*,*5R*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]-1*H*-indazole;
1-Methyl-5-[(*1S*,*5R*)-3-(3-{[4-methyl-5-(4-methyl-1,3-oxazol-5-yl)-4*H*-1,2,4-triazol-3-yl]thio}propyl)-3-azabicyclo[3.1.0]hex-1-yl]-1*H*-indazole, Enantiomer 1;
(1*R*,5*S*/*1S*,*5R*)-3-(3-{[4-Methyl-5-(4-methyl-1,3-oxazol-5-yl)-4*H*-1,2,4-triazol-3-yl]thio}propyl)-1-[6-(trifluoromethyl)-3-pyridinyl]-3-azabicyclo[3.1.0]hexane;
(1*R*,*5S*/*1S*,*5R*)-1-(4-Bromophenyl)-3-(3-{[4-methyl-5-(2-methyl-3-pyridinyl)-4H-1,2,4-triazol-3-yl]thio}propyl)-3-azabicyclo[3.1.0]hexane;
(1*R*,*5S*/*1S*,*5R*)-1-(4-Bromophenyl)-3-(3-{[4-methyl-5-(4-pyridazinyl)-4H-1,2,4-triazol-3-yl]thio}propyl)-3-azabicyclo[3.1.0]hexane;
(1*R*,*5S*/*1S*,*5R*)-1-(4-Bromophenyl)-3-(3-{[5-(5-chloro-1-methyl-1H-pyrazol-4-yl)-4-methyl-4H-1,25,4-triazol-3-yl]thio}propyl)-3-azabicyclo[3.1.0]hexane;
(1*R*,*5S*/*1S*,*5R*)-1-(4-Bromophenyl)-3-(3-{[4-methyl-5-(1-methyl-1H-1,2,3-triazol-4-yl)-4H-1,2,4-triazol-3-yl]thio}propyl)-3-azabicyclo[3.1.0]hexane;
(1*R*,*5S*/*1S*,*5R*)-1-(4-Bromophenyl)-3-(3-{[5-(1,5-dimethyl-1H-pyrazol-4-yl)-4-methyl-4H-1,2,4-triazol-3-yl]thio}propyl)-3-azabicyclo[3.1.0]hexane;
(1*R*,*5S*/*1S*,*5R*)-1-(4-Bromophenyl)-3-(3-{[4-methyl-5-(5-pyrimidiny1)-4H-1,2,4-triazol-3-yl]thio}propyl)-3-azabicyclo[3.1.0]hexane;
(1*R*,5*S*/*1S*,*5R*)-1-(4-Bromophenyl)-3-[3-({4-methyl-5-[1-methyl-3-(trifluoromethyl)-1H-pyrazol-4-yl]-4H-1,2,4-triazol-3-yl}thio)propyl]-3-azabicyclo[3.1.0]hexane;
(1*R*,5*S*/1*S*,*5R*)-1-(4-Bromophenyl)-3-(3-{[4-methyl-5-(3-methyl-2-furanyl)-4H-1,2,4-triazol-3-yl]thio}propyl)-3-azabicyclo[3.1.0]hexane;
(1*R*,5*S*/1*S*,*5R*)-1-(4-Bromophenyl)-3-(3-{[4-methyl-5-(3-methyl-5-isoxazolyl)-4H-1,2,4-triazol-3-yl]thio}propyl)-3-azabicyclo[3.1.0]hexane;
(1*R*,5*S*/*1S*,*5R*)-1-(4-Bromophenyl)-3-(3-{[4-methyl-5-(6-methyl-3-pyridinyl)-4H-1,2,4-triazol-3-yl]thio}propyl)-3-azabicyclo[3.1.0]hexane;
(1*R*,*5S*/*1S*,*5R*)-1-(4-Bromophenyl)-3-(3-{[4-methyl-5-(1-methyl-1H-pyrazol-5-yl)-4H-1,2,4-triazol-3-yl]thio}propyl)-3-azabicyclo[3.1.0]hexane;
(1*R*,5*S*/*1S*,*5R*)-1-(4-Bromophenyl)-3-(3-{[4-methyl-5-(5-methyl-3-pyridinyl)-4H-1,2,4-triazol-3-yl]thio}propyl)-3-azabicyclo[3.1.0]hexane;
(1*R*,5*S*/*1S*,*5R*)-1-(4-Bromophenyl)-3-[3-({4-methyl-5-[2-methyl-5-(trifluoromethyl)-1,3-oxazol-4-yl]-4H-1,2,4-triazol-3-yl}thio)propyl]-3-azabicyclo[3.1.0]hexane;
(1*R*,5*S*/1S,5*R*)-1-(4-Bromophenyl)-3-(3-{[4-methyl-5-(3-methyl-2-pyridinyl)-4H-1,2,4-triazol-3-yl]thio}propyl)-3-azabicyclo[3.1.0]hexane;
(1*R*,5*S*/1*S*,5*R*)-1-(4-Bromophenyl)-3-(3-{[5-(2,4-dimethyl-1,3-thiazol-5-yl)-4-methyl-4H-1,2,4-triazol-3-yl]thio}propyl)-3-azabicyclo[3.1.0]hexane;
(1*R*,5*S*/1*S*,5*R*)-1-(4-Bromophenyl)-3-(3-{[5-(2,5-dimethyl-3-furanyl)-4-methyl-4H-1,2,4-triazol-3-yl]thio}propyl)-3-azabicyclo[3.1.0]hexane;
(1*R*,5*S*/1*S*,*5R*)-1-(4-Bromophenyl)-3-(3-{-[5-(5-chloro-2-thienyl)-4-methyl-4*H*-1,2,4-triazol-3-yl]thio}propyl)-3-azabicyclo[3.1.0]hexane;
(1*R*,5*S*/1,5*R*)-1-(4-Bromophenyl)-3-(3-{[4-ethyl-5-(3-pyridinyl)-4H-1,2,4-triazol-3-yl]thio}propyl)-3-azabicyclo[3.1.0]hexane;
(1*R*,5*S*/1*S,*5*R*)-1-(4-Bromophenyl)-3-[3-({4-methyl-5-[2-methyl-6-(trifluoromethyl)-3-pyridinyl]-4H-1,2,4-triazol-3-yl}thio)propyl]-3-azabicyclo[3.1.0]hexane.
5-[5-({3-[(1*R,*5*S*/*1*S, 5*R*)-1-(4-Bromophenyl)-3-azabicyclo[3.1.0]hex-3-yl]propyl}thio)-4-methyl-4H-1,2,4-triazol-3-yl]-1-methyl-3-(trifluoromethyl)-1H-thieno[2,3-c]pyrazole;
3-(3-{[4-Methyl-5-(4-methyl-1,3-oxazol-5-yl)-4*H*-1,2,4-triazol-3-yl]thio}propyl)-(1*R*,5*R*/*1S*,*5S*)-1-[5-(trifluoromethyl)-2-pyridinyl]-3-azabicyclo[3.1.0]hexane;
3-(3-{[4-Methyl-5-(4-methyl-1,3-oxazol-5-yl)-4*H*-1,2,4-triazol-3-yl]thio}propyl)-(1*R*,5*R*)-1-[5-(trifluoromethyl)-2-pyridinyo-3-azabicyclo[3.1.0]hexane, Enantiomer 2;
3-(3-{[4-methyl-5-(4-methyl-1,3-oxazol-5-yl)-4*H*-1,2,4-triazol-3-yl]thio}propyl)-(1*R*,5R/*1S*,*5S*)-1-[6-(trifluoromethyl)-2-pyridinyl]-3-azabicyclo[3.1.0]hexane;
(1*R*,5*S*/*1S,5R*)-1-[3-Fluoro-4-(1H-pyrrol-1-ylmethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane;
(1S,5R/1R,5S)-3-(3-{[4-Methyl-5-(5-methyl-2-pyrazinyl)-4*H*-1,2,4-triazol-3-yl]thio)propyl)-1-[6-(trifluoromethyl)-3-pyridinyl]-3-azabicyclo[3.1.0]hexane;
(1S,5R/1R,5S)-3-(3-{[4-Methyl-5-(6-methyl-3-pyridinyl)-4*H*-1,2,4-triazol-3-yl]thio}propyl)-1-[6-(trifluoromethyl)-3-pyridinyl]-3-azabicyclo[3.1.0]hexane;
(1S,5R/1R,5S)-3-(3-{[4-Methyl-5-(2-methyl-3-pyridinyl)-4*H*-1,2,4-triazol-3-yl]thio}propyl)-1-[6-(trifluoromethyl)-3-pyridinyl]-3-azabicyclo[3.1.0]hexane;
(1S,5R/1R,5S)-3-{3-[(4-Methyl-5-phenyl-4*H*-1,2,4-triazol-3-yl)thio]propyl)-1-[6-(trifluoromethyl)-3-pyridinyl]-3-azabicyclo[3.1.0]hexane;
(1S,5R/1R,5S)-3-(3-{[5-(2,4-Dimethyl-1,3-thiazol-5-yl)-4-methyl-4H-1,2,4-triazol-3-yl]thio}propyl)-1-[6-(trifluoromethyl)-3-pyridinyl]-3-azabicyclo[3.1.0]hexane;
(1S,5R/1R,5S)-3-[3-({4-Methyl-5-[4-(trifluoromethyl)phenyl]-4*H*-1,2,4-triazol-3-yl}thio)propyl]-1-[6-(trifluoromethyl)-3-pyridinyl]-3-azabicyclo[3.1.0]hexane;
(1S,5R/1R,5S)-2-Methyl-5-[3-(3{[4-methyl-5-(5-methyl-2-pyrazinyl)-4*H*-1,2,4-triazol-3-yl]thio}propyl)-3-azabicyclo[3.1.0]hex-1-yl]-1,3-benzothiazole;
(1S,5R/1R,5S)-2-Methyl-5-[3-(3-{[4-methyl-5-(6-methyl-3-pyridinyl)-4*H*-1,2,4-triazol-3-yl]thio}propyl)-3-azabicyclo[3.1.0]hex-1-yl]-1,3-benzothiazole;
(1S,5R/1R,5S)-2-Methyl-5-(3-{3-[(4-methyl-5-phenyl-4*H*-1,2,4-triazol-3-yl)thio]propyl}-3-azabicyclo[3.1.0]hex-1-yl)-1,3-benzothiazole;
(1S,5R/1R,5S)-5-[3-(3{[5-(2,4-Dimethyl-1,3-thiazol-5-yl)-4-methyl-4*H*-1,2,4-triazol-3-yl]thio}propyl)-3-azabicyclo[3.1.0]hex-1-yl]-2-methyl-1,3-benzothiazole;
(1S,5R/1R,5S)-2-Methyl-5-{3-[3-({4-methyl-5-[4-(trifluoromethyl)phenyl]-4*H*-1,2,4-triazol-3-yl}thio)propyl]-3-azabicyclo[3.1.0]hex-1-yl}-1,3-benzothiazo)e;
(1*R*,5*S*/1*S*,5*R*)-1-[3-Fluoro-5-(trifluoromethyl)phenyl]-3-(3{[4-methyl-5-(4-methyl-1,3-oxazol-5-yl)-4*H*-1,2,4-triazol-3-yl]thio}propyl)-azabicyclo[3.1.0]hexane;
(1*S*,5*R*)-1-[3-Fluoro-5-(trifluoromethyl)phenyl]-3-(3-{[4-methyl-5-(4-methyl-1,3-oxazol-5-yl)-4*H*-1,2,4-triazol-3-yl]thio}propyl)-azabicyclo[3.1.0]hexane, Enantiomer 1;
(1*R*,5*S*/1*S*,5*R*)-1-[2-Fluoro-3-(trifluoromethyl)phenyl]-3-(3-{[4-methyl-5-(4-methyl-1,3-oxazol-5-yl)-4H-1,2,4-triazol-3-yl]thio}propyl)-azabicyclo[3.1.0]hexane;
(1*S*,5*R*)-1-[2-Fluoro-3-(trifluoromethyl)phenyl]-3-(3-{[4-methyl-5-(4-methyl-1,3-oxazol-5-yl)-4*H*-1,2,4-triazol-3-yl]thio}propyl)-azabicyclo[3.1.0]hexane, Enantiomer 2;
(1*R*,5*S*/1*S*,5*R*)-1-[4-(Methyloxy)-5-(trifluoromethyl)phenyl]-3-(3-{[4-methyl-5-(4-methyl-1,3-oxazol-5-yl)-4*H*-1,2,4-triazol-3-yl]thio}propyl)-azabicyclo[3.1.0]hexane;
(1*R*,5*S*/1*S*,5*R*)-1-[4-(4-Chloro-2-fluorophenyl]-3-(3-{[4-methyl-5-(4-methyl-1,3-oxazol-5-yl)-4*H*-1,2,4-triazol-3-yl]thio}propyl)-azabicyclo [3.1.0]hexane;
(1*R*,5*S*/1*S*,5*R*)-1-[3-(2-{[4-Methyl-5-(4-methyl-1,3-oxazol-5-yl)-4H-1,2,4-triazol-3-yl]thio}propyl)-1-{3-[(trifluoromethyl)oxy]phenyl}-3-azabicyclo[3.1.0]hexane:
(1*R*,5*S*/1S,5*R*)-1-(2-Chloro-4-methylphenyl)-3-(2-{[4-methyl-5-(4-methyl-1,3-oxazol-5-yl)-4*H*-1,2,4-triazol-3-yl]thio}propyl)-3-azabicyclo[3.1.0]hexane;
(1*R*,5*S*/1*S*,5*R*)-1-[3-Chloro-4-(methyloxy)phenyl]-3-(2-{[4-methyl-5-(4-methyl-1,3-oxazol-5-yl)-4*H*-1,2,4-triazol-3-yl]thio}propyl)-3-azabicyclo[3.1.0]hexane;
(1*R*,5*S*/1*S*,5*R*)-1-[4-(2,4-Dimethyl-1,3-thiazol-5-yl)phenyl]-3-(3-{[4-methyl-5-(4-methyl-1,3-oxazol-5-yl)-4H-1,2,4-triazol-3-yl]thio}propyl)-3-azabicyclo[3.1.0]hexane;
(1*R*,5*S*/1*S*,5*R*)-3-(3-{[4-methyl-5-(4-methyl-1,3-oxazol-5-yl)-4H-1,2,4-triazol-3-yl]thio}propyl)-1-{4-[6-(trifluoromethyl}-2-pyridinyl]phenyl}-3-azabicyclo[3.1.0]hexane;
(1 *R*,5*S*/1*S*,5*R*)-1-[3-(2,4-dimethyl-1,3-thiazol-5-yl)phenyl]-3-(3-{[4-methyl-5-(4-methyl-1,3-oxazol-5-yl)-4H-1,2,4-triazol-3-yl]thio}propyl)-3-azabicyclo[3.1.0]hexane;
(1*R*,5*S*/*S*,5*R*)-3-(3-{[4-methyl-5-(4-methyl-1,3-oxazol-5-yl)-4H-1,2,4-triazol-3-yl]thio)propyl)-1-[3-(5-methyt-2-thienyl)phenyl]-3-azabicyclo[3.1.0]hexane;
(1*R*,5*S*/1*S*,5*R*)-1-[4-(3,5-dimethyl-4-isoxazolyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane;
(1S,5R)-3-(3-{[5-(2,4-Dimethyl-1,3-oxazol-5-yl)-4-methyl-4*H*-1,2,4-triazol-3-yl]thio}propyl)-1-[2-fluoro-4-(trifluoromethyl)phenyl]-3-azabicydo[3.1.0]hexane;
   and salts thereof, in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, ; and premature ejaculation in a mammal.

Thus, a still further aspect of the invention provides a method of treating a somatoform disorder as defined above and premature ejaculation which comprises administering to a mammal (e.g. human) in need thereof an effective amount of a compound of formula (I) selected from the list of compounds above or a salt thereof.

Also provided is a compound of formula (I) selected from the list of compounds above cited or a salt thereof for use in the treatment of a somatorm disorder as defined above and premature ejaculation.

In a further embodiment of the present invention a new use is provided of a compound selected from the group consisting of:
(1*R*,5*S*/1*S*,5*R*)-3-(3-{[4-Methyl-5-(4-methyl-1,3-oxazol-5-yl)-4*H*-1,2,4-triazol-3-yl]thio}propyl)-1-[3-(trifluoromethyl)phenyl]-3-azabicyclo[3.1.0]hexane;
(1*S*,5*R*)-1-[2-Fluoro-4-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]hexane;
(1*S*,5*R*)-3-(3-{[4-Methyl-5-(tetrahydro-2*H*-pyran-4-yl)-4*H*-1,2,4-triazol-3-yl]thio}propyl)-1-{4-[(trifluoromethyl)oxy]phenyl}-3-azabicyclo[3.1.0]hexane, Enantiomer 1; (1S,5R)-3-(3-{[4-Methyl-5-(2-methyl-3-pyridinyl)-4H-1,2,4-triazol-3-yl]thio}propyl)-1-[4-(trifluoromethyl)phenyl]-3-azabicyclo[3.1.0]hexane;
(1S,5R)-3-(3-{[4-Methyl-5-(4-pyridazinyl)-4H-1,2,4-triazol-3-yl]thio}propyl)-1-[4-(trifluoromethyl)phenyl]-3-azabicyclo[3.1.0]hexane;
(1S,5R)-3-(3-{[5-(2,4-Dimethyl-1,3-thiazol-5-yl)-4-methyl-4H-1,2,4-triazol-3-yl]thio}propyl)-1-[4-(trifluoromethyl)phenyl]-3-azabicyclo[3.1.0]hexane;
(1S,5R)-1-[2-Fluoro-4-(trifluoromethyl)phenyl]-3-(3-{[4-methyl-5-(4-pyridazinyl)-4H-1,2,4-triazol-3-yl]thio}propyl)-3-azabicyclo[3.1.0]hexane;
(1S,5R)-3-(3-{[5-(2,4-Dimethyl-1,3-thiazol-5-yl)-4-methyl-4H-1,2,4-triazol-3-yl]thio}propyl)-1-[2-fluoro-4-(trifluoromethyl)phenyl]-3-azabicyclo[3.1.0]hexane;
(1*S*,5*R*)-1-[3-Fluoro-4-(trifluoromethyl)phenyl]-5-methyl-3-(3-{[4-methyl-5-(4-methyl-1,3-oxazol-5-yl)-4*H*-1,2,4-triazol-3-yl]thio}propyl)-3-azabicyclo[3.1.0]hexane Enantiomer 1;
(1*R*,5*S*/1*S*,5*R*)-1-(4-Bromophenyl)-3-(3-{[4-methyl-5-(6-methyl-3-pyridinyl}-4H-1,2,4-triazol-3-yl]thio}propyl)-3-azabicyclo[3.1.0]hexane;
3-(3-{[4-Methyl-5-(4-methyl-1,3-oxazol-5-yl)-4*H*-1,2,4-triazol-3-yl]thio}propyl)-(1*R*,5*R*)-1-[5-(trifluoromethyl)-2-pyridinyl]-3-azabicyclo[3.1.0]hexane, Enantiomer 2;
(1*S*,5*R*)-1-[3-Fluoro-5-(trifluoromethyl)phenyl]-3-(3-{[4-methyl-5-(4-methyl-1,3-oxazol-5-yl)-4*H*-1,2,4-triazol-3-yl]thio}propyl)-azabicyclo[3.1.0]hexane, Enantiomer 1;
(1S,5R)-3-(3-{[5-(2,4-Dimethyl-1,3-oxazol-5-yl)-4-methyl-4*H*-1,2,4-triazol-3-yl]thio}propyl)-1-[2-fluoro-4-(trifluoromethyl)phenyl]-3-azabicyclo[3.1.0]hexane;
and salts thereof, in the manufacture of a medicament for the treatment of of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and premature ejaculationin a mammal.

Thus, a still further aspect of the invention provides a method of treating of a somatoform disorder such as body dysmorphic disorder or hyperchondriasis, bulimia nervosa, anorexia nervosa, binge eating, paraphilia and nonparaphilic sexual addictions, Sydeham's chorea, torticollis, and premature ejaculation, which comprises administering to a mammal (e.g. human) in need thereof an effective amount of a compound of formula (I) selected from the list of compounds above and salts thereof.

Also provided is a compound of formula (I) selected from the list of compounds above, and salts thereof, for use in the treatment of a somatoform disorder such as body dysmorphic disorder or hyperchondriasis, bulimia nervosa, anorexia nervosa, binge eating, paraphilia and nonparaphilic sexual addictions, Sydeham's chorea, torticollis, autism, a movement disorder including Tourette's syndrome; and premature ejaculation.

In a further aspect, a somatoform disorder is binge eating.

It is intended that reference to particular compounds herein be interpreted to mean that the salts, solvates and prodrugs of those compounds may also be employed.

### Examples

The invention is further illustrated by the following non-limiting examples. Preparations 1 to 5 were carried out in analogy to the synthetic route described in J.Med.Chem. 1981, 24, 481-490.

All temperatures refer to °C. Infrared spectra were measured on a FT-IR instrument. Compounds were analysed by direct infusion of the sample dissolved in acetonitrile into a mass spectra operated in positive electro spray (ES+) ionisation mode. Proton Magnetic Resonance (¹H-NMR) spectra were recorded at 400 MHz, chemical shifts are reported in ppm downfield (d) from Me₄Si, used as internal standard, and are assigned as singlets (s), broad singlets (bs), doublets (d), doublets of doublets (dd), triplets (t), quartets (q) or multiplets (m).

Experimental vibrational circular dichroism (VCD) spectra were measured using a ChirallRTM VCD spectrometer operating in the 2000-800 cm-1 frequency range. Spectra were measured at room temperature (23o C) using a sealed transmission cell with barium fluoride windows and a path length of 100 microns. (Scan times varied from 60 to 120 minutes per isomer.) Sample solutions were typically prepared by dissolving 10 milligrams of each enantiomer in 100 microliters of deutero-chloroform (CDCl₃). For ab initio assignments, VCD and unpolarized IR spectra were calculated using the Gaussian 98 software package.1.

Optical rotations were measured using a (Perkin Elmer Model 241) polarimeter operating at 589 nm (Sodium source). Measurements were made using a 1 decimeter microcell thermostated at 23o C. Concentrations were typically 10 mg/ml (c=0.01). For ab initio OR assignments, the Dalton Quantum Chemistry Program was used.

Column chromathography was carried out over silica gel (Merck AG Darmstaadt, Germany). The following abbreviations are used in the text: NBS = N-bromosuccinimide, Vitride = "Red-Al®", HOBt = 1-hydroxybenzotriazole EtOAc = ethyl acetate, Et₂O = dietyl ether, DMF = N,N'-dimethylformamide, MeOH = methanol, TFA = trifluoroacetic acid, tetrahydrofuran = tetrahydrofuran, IPA = isopropanol, TEA = triethylamine, DCC = 1,3-dicyclohexylcarbodiimide, SCX = strong cation exchanger, Tlc refers to thin layer chromatography on silica plates, and dried refers to a solution dried over anhydrous sodium sulphate, r.t. (RT) refers to room temperature, Rt = retention time, DMSO = dimethyl sulfoxide.

### Preparation 1: Methyl bromo(4-methoxyphenyl)acetate

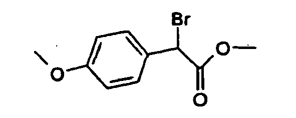

To a mixture of methyl 4-methoxyphenylacetate (20 g, 0.11 mol) and NBS (0.11 mol) in CCl₄ (0.2 l) were added 3 drops of 48% HBr and this mixture was heated to reflux for 8 h. The cooled solution was filtered through a pad of silica gel and the filtrate was evaporated *in vacuo* to give 29 g of the title compound as pale yellow oil, which was used in the subsequent step without further purification.

NMR (¹H, CDCl₃): δ 7.3 (d, 2H), 6.8 (d, 2H), 5.1 (s, 1H), 3.8 (s, 3H), 3.5 (s, 3H).

### Preparation 2: Dimethyl 1-(4-methoxyphenyl)-1,2-cyclopropanedicarboxylate

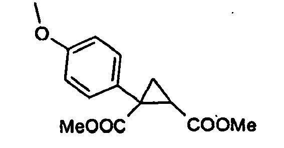

To a stirred slurry of of NaH (4.4 g , 60% in mineral oil) in anhydrous Et₂O (0.3 l) was added methanol (10.3 mL) followed by a solution of bromo ester obtained in Prep. 1 methyl bromo(4-methoxyphenyl)acetate (29 g) in methyl acrylate (19.8 mL) (for examples starting from an ethyl phenylacetate derivative ethanol and ethyl acrylate were used, respectively) and methanol (3 mL) at 0 °C, over a 30 min.. The mixture was stirred at 25 °C for 24 h and then unreacted NaH was decomposed with 3 mL methanol. Water was added (75 mL), the organic phase separated, dried over Na₂SO₄ and filtered. Volatiles were evaporated *in vacuo* to give 31.5 g of the title compound as an oil, which was used in the subsequent step without further purification.

NMR (¹H, CDCl₃): δ 7.3 (d, 2H), 6.8 (d, 2H), 3.77 (s, 3H), 3.73 (s, 3H), 3.64 (s, 3H), 2.18 (dd, 1H), 2.05 (dd, 1H), 1.46 (dd, 1H). MS (m/z): 265.4[MH]⁺.

### Preparation 3: 1-(4-Methoxyphenyl)-1,2-cyclopropanedicarboxylic acid

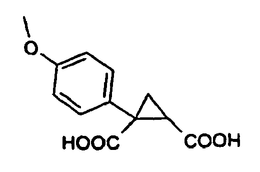

A mixture of diester obtained in Prep. 2 (31.5 g) and KOH (13.5 g) in 1:1 EtOH:H₂O (240 mL) was heated at reflux for 6 h and then concentrated to half the original volume. The aqueous solution was extracted with Et₂O, chilled in ice, and then made acidic with 25 mL of 12N HCl. White crystalline product was collected by filtration and dried under *vacuo* to give 12.8 of the title compound (overall yield from methyl bromo(4-methoxyphenyl)acetate: 50%).

NMR (¹H, DMSO): δ 12.5 (bs,2H), 7.25 (d, 2H), 6.85 (d, 2H), 3.7 (s, 3H), 2.0 (dd, 1H), 1.85 (dd, 1H), 1.38 (dd, 1H). MS (*m*/*z*): 235.0[M-H]⁻.

### Preparation 4: (1R,5S/1S,5R)-1-[4-(Methoxy)phenyl]-3-azabicyclo[3.1.0]hexane-2,4-dione

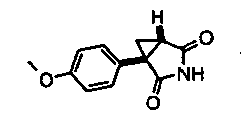

A mixture of 12.8 g of the diacid obtained in Preparation 3 and 6.5 g of urea in 300 mL of m-xylene was heated at reflux for 8 h and then concentrated to dryness *in vacuo.* The crude was purified by column chromatography (AcOEt:cyclohexane=1 (?):10 to 4:6) to give 5.5 g of the title compound (y= 46%).

MS (*m*/*z*): 218.1 [MH]⁺.

### Preparation 5: (1R,5S/1S,5R)-1-[4-(Methoxy)phenyl]-3-azabicyclo[3.1.0]hexane

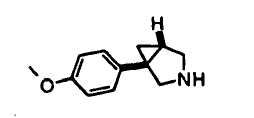

To a stirred slurry of 5.5 g of the imide obtained in Preparation 4 in 170 mL of toluene was slowly added 45 mL of Vitride (3.4 M in toluene) under N₂. This solution was stirred at reflux for 2 h. To the cooled solution was cautiously added aqueous NaOH (10 M, 40 mL) and the organic layer was washed with two portions of water and dried over Na₂SO₄. This solution was filtered, and the filtrate was evaporated in vacuo to give 4.8 g of the title compound (y= 100%).

NMR (¹H, CDCl₃): δ 7.10 (d, 2H), 6.82 (d, 2H), 3.77 (s, 3H), 3.35-2.98 (m, 4H), 2.58 (dd, 1H), 0.87 (dd, 1H), 0.78 (dd, 1H), NH not observed. MS (*m*/*z*): 190.1[MH]⁺.

### Preparation 6: (1R,5S/1S,5R)-1-(4-Bromophenyl)-3-azabicyclo[3.1.0]hexane

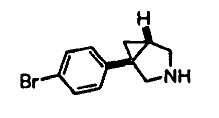

To 20 mL of 1 M BH₃-tetrahydrofuran, stirred at 0°C under N₂, was slowly added a solution of 1.32 g (5 mmol) of (1*R*,5*S*/1*S*,5*R*)-1-(4-bromophenyl)-3-azabicyclo[3.1.0]hexane-2,4-dione, prepared in analogy to Preparation 4, in 20mL of dry tetrahydrofuran. This solution was stirred at room temperature for 15 min and then warmed on a steam bath for 1 h. The solution was then cooled in an ice bath, 2.5 mL of 6 M HCl was added cautiously, and the solvent was removed *in vacuo.* The residual material was combined with 12.5 mL of 5 M NaOH and the mixture was extracted with ether. The ether extract was washed twice with water, dried over Na₂SO₄ and filtered to give 1.19 g of the title compound (y= 100%).

NMR (¹H, CDCl₃): δ 7.35 (d, 2H), 7.02 (d, 2H), 3.25-2.96 (m, 4H), 1.63 (dd, 1H), 1.55 (dd, 1H), 1.30 (dd, 1H), NH not observed. MS (*m*/*z*): 238.1[MH]⁺,1Br.

### Preparation 7: (1R,5S/1S,5R)-4-[3-(Trifluoroacetyl)-3-azabicyclo[3.1.0]hex-1-yl]benzonitrile

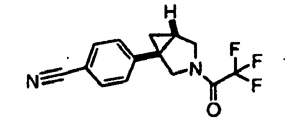

Triflouroacetic anhydride (0.21 mL) was added to a solution of 4-[3-azabicyclo[3.1.0]hex-1-yl]benzonitrile (280 mg, prepared in analogy to the method described in Preparation 5), and triethylamine (0.25 mL) in dichloromethane (15 mL) at 0°C. The reaction mixture was allowed to warm to room temperature over 2h, then washed with saturated NaHCO₃, the organic layer dried and evaporated to give 269 mg of the title compound.

MS (*m*/*z*): 281.2[MH]⁺.

### Preparation 8: (1R,5S/1S,5R)-4-[3-(Trifluoroacetyl)-3-azabicyclo[3.1.0]hex-1-yl]benzaldehyde

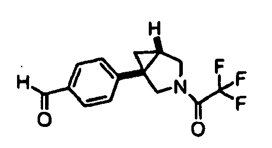

A mixture of 4-[3-(trifluoroacetyl)-azabicyclo[3.1.0]hex-1-yl]benzonitrile (283 mg), Ni-Al alloy (450 mg), formic acid (3.9 mL) and water (1.1 mL) was stirred at 80 °C for 3h. The reaction mixture was cooled to room temperature and filtered. The filtrate was extracted with ethyl acetate and the organic phase washed with NaHCO₃, dried over Na₂SO₄ and evaporated *in vacuo* to give 195 mg of the title compound as yellow oil..

MS (*m*/*z*): 284.2[MH]⁺.

### Preparation 9: (1R,5S/1S,5R)-4-[3-(Trifluoroacetyl)-3-azabicyclo[3.1.0]hex-1-yl]benzaldehyde oxime

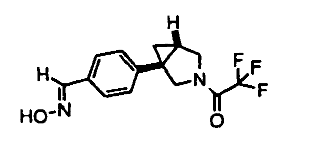

To a solution of 4-[3-(trifluoroacetyl)-3-azabicyclo[3.1.0]hex-1-yl]benzaldehyde (195 mg) in 5 mL of pyridine was added hydroxylamine hydrochloride (57.5 mg) and the mixture was stirred for 3 h at room temperature. The solvent was evaporated, the crude dissolved in ethyl acetate and the organic phase washed with 10% aqueous Na₂CO₃ and brine, dried over Na₂SO₄ and evaporated *in vacuo* to give 225 mg of the title compound as yellow oil..

MS (*m*/*z*): 299.2[MH]⁺.

### Preparation 10: (1R,5S/1S,5R)- 4-[3-(Trifluoroacetyl)-3-azabicyclo[3.1.0]hex-1-yl]-N-hydroxybenzenecarboximidoyl chloride

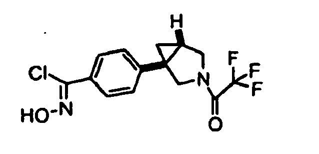

To a solution of 4-[3-(trifluoroacetyl)-(3-azabicyclo[3.1.0]hex-1-y]benzaldehyde oxime (0.69 mmol) in 3.5 mL of dimethylformamide was added portion wise *N*-chlorosuccinimide (97 mg) at 0 °C. After stirring for 1.5 h at 40 °C the solvent was evaporated. The crude product was dissolved in diethyl ether / dichloromethane (4/1) and the organic phase washed with water, dried over Na₂SO₄ and concentrated *in vacuo* to give 243 mg of the title compound as a brown oil.

### Preparation 11: (1R,5S/1S,5R-1-[4-(5-Methyl-3-isoxazolyl)phenyl)-3-(trifluoroacetyl)-3-azabicyclo[3,1.0]hexane

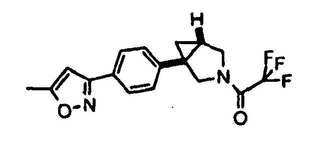

To a solution of 4-[3-(trifluoroacetyl)3-azabicyclo[3.1.0]hex-1-yl)-*N-*hydroxybenzenecarboximidoyl chloride (0.69 mmol) in 6 mL of chloroform triethylamine (0.24 mL) and 2-chloro propene (0.29 mL) were added and the reaction stirred for 18 h at room temperature. The solution was washed with water, dried over Na₂SO₄ and volatiles evaporated *in vacuo.* The crude was purified by column chromatography (AcOEt:cyclohexane=1:10 to 4:6) to give 180 mg of the title compound.

MS (*m*/*z*)*:* 337.2[MH]⁺.

### Preparation 12: (1R,5S/1S,5R)-1-[4-(5-Methyl-3-isoxazolyl)phenyl]-3-azabicyclo[3.1.0]hexane

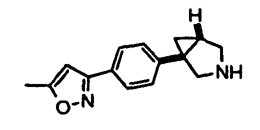

A mixture of 1-[4-(5-methyl-3-isoxazolyl)phenyl]-3-(trifluoroacetyl)-3-azabicyclo[3.1.0]hexane (0.54 mmol) and K₂CO₃ (296 mg)in methanol (5 mL) and water (5 mL) was stirred for 4 h at 50 °C. The solvent was evaporated *in vacuo* and the product treated with dichloromethane / isopropanol 1/1 and filtered. The filtrate was dried over Na₂SO₄ and volatiles evaporated *in vacuo* to give 105 mg of the title compound (y= 81%).

MS (*m*/*z*)*:* 241.2[MH]⁺.

### Preparation 13: 5-{5-[(3-Chloropropyl)thio]-4-methyl-4H-1,2,4-triazol-3-yl}-2-methylquinoline

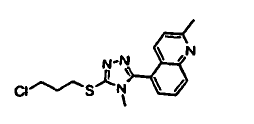

To 4-methyl-5-(2-methyl-5-quinolinyl)-2,4-dihydro-3*H*-1,2,4-triazole-3-thione (3.6 g, prepared in analogy to the method described in WO200240471) in ethanol (60 mL) containing 1-bromo-3-chloropropane (2.0 mL) was carefully added with stirring sodium hydride (0.60 g, 60% in petrolium). The mixture was heated at reflux for 45 min. Volatiles were evaporated *in vacuo* and the residue submitted to column chromatography (EtOAc - acetone gradient). The material thus obtained was precipitated from hot EtOAc (20 mL) by adding petroleum ether (40-60, 50 mL), cooled and collected by filtration to provide the title compound as colourless crystals (2.1 g).

NMR (¹H, CDCl₃): δ 8.18 (d, 1H), 8.12 (d, 1H), 7.76 (t, 1H), 7.55 (d, 1H), 7.30 (d, 1H), 3.75 (t, 2H), 3.50 (t, 2H), 3.40 (s, 3H), 2.76 (s, 3H), 2.37 (m, 2H).

### Preparation 14: 3-[(3-Chloropropyl)thio]-4-methyl-5-(4-methyl-1,3-oxazol-5-yl)-4H-1,2,4-triazole

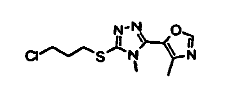

Ethyl-2-chloroacetoacetate (1 wt; 1 eq., 1000 g) was aged with formamide (0.68 vol; ca. 2.8 eq.) and the resulting solution was heated to 120 °C. After 5 hours the mixture was allowed to cool to room temperature and allowed to age under nitrogen over night. The mixture was treated with NaOH (3 M, 6 vol, reaction moderately exothermic) and stirred at room temperature for 4 hours. Ethyl acetate (6 vol) was added and the phases allowed to separae. The organic layer was discarded while the aqueous was acidified with conc. (32%) aqueous HCl to pH 2 (ca. 2.0 vol). A precipitate started to form. The suspension was treated with AcOEt (8 vol) and vigorously stirred until the bulk of the precipitate had dissolved. The aqueous phase was further extracted with AcOEt twice (6 vol each) and the combined organic layers distilled to low volume (again a suspension was observed at low volume). Fresh AcOEt (8 vol) was added and the mixture evaporated to dryness. The collected solid was placed in the oven at 40°C over night under reduced pressure to give 4-methyl-1,3-oxazole-5-carboxylic acid (498 g, 64.5%).
This material (498 g, 1 wt) was dissolved in dry tetrahydrofuran (5 vol), under nitrogen, cooled to 0 °C. DCC (1.62 wt, 1 eq) was added portionwise followed by HOBt (1.07 wt, 1 eq). The mixture was warmed to 25±2 °C and stirred for 30 min. 4-Methyl-3-thiosemicarbazide (0.83 wt, 1 eq) was then added and the mixture further stirred for 2 h at 25±2°C. The mixture was filtered and the cake was washed with fresh tetrahydrofuran (1 vol) and dried on the filter for a few hours. The cake was suspended in 1 M aqueous NaOH (13 vol) and heated to 70°C for 30 min. After this time, the mixture was cooled to 25±2 °C and a solid was removed by filtration. The cake was washed with 1 M aqueous NaOH (10 vol). The combined mother liquors were cooled to 0 °C and acidified to ca. pH 5 with HCl (aqueous, 16%; NOTE: keep temperature while adding HCl below +10 °C). The suspended product was isolated by filtration washing with water (2x3 vol). The cake was dried at 40°C for 18 h in high vacuum to obtain 4-methyl-5-(4-methyl-1,3-oxazol-5-yl)-2,4-dihydro-3*H*-1,2,4-triazole-3-thione (respectively a tautomeric form thereof; 290 g, 37%). NaOEt (21% solution in EtOH, 2.08 vol, 1.1 eq) was added to EtOH (20 vol) under nitrogen atmosphere. 4-Methyl-5-(4-methyl-1,3-oxazol-5-yl)-2,4-dihydro-3*H*-1,2,4-triazole-3-thione (respectively a tautomeric form thereof; 290 g, 1 wt) was added in one portion and the resulting mixture stirred at 25±2°C until a clear solution was obtained. Then 1-bromo-3-chloropropane (0.54 vol, 1.1 eq) was added and the solution stirred at 40 °C for 24 h then cooled to 25 °C. After filtration water (20 vol) was added and the ethanolic phase was removed by vacuum distillation (internal temperature -40 °C). The mixture was extracted with EtOAc (41 vol). The aqueous layer was removed and the organic phase was evaporated to dryness. Dichloromethane (4 vol) was added. The organic solution is purified through a short silica gel column (18 wt of silica), eluting with EtOAc (200 vol) to give the title compound as a solid foam (267.64 g, 66%).

NMR (¹H, CDCl₃): δ 7.90 (s, 1H), 3.70 (s, 5H), 3.40 (t, 2H), 2.52 (s, 3H), 2.30 (m, 2H).

### Preparation 15: 3-[4-(Trifluoromethyl)phenyl]-1H-pyrrole-2,5-dione

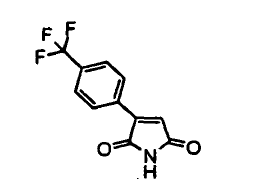

A mixture of hydrochloric acid (37%, 285 mL) and water (190 mL) was added to 4-(trifluoromethyl)aniline (150 g, 116 mL) at room temperature with vigorous stirring and the formed precipitate was allowed to stir for further 30 minutes. Temperature was reduced to 0 °C and sodium nitrite (70.6 g) in 180 mL of water was added dropwise to the stirred suspension. At the end of diazotisation, a clear yellow solution was obtained. Maleimide (180 g) in acetone (1.1 l) was added dropwise at 0 °C and then the pH of the solution was adjusted to 3-3.5 by adding sodium acetate. Copper (II) chloride (18.8 g) was added to the vigorously stirred mixture. After a few minutes a gas started to develop (conspicuous foaming). The reaction mixture was allowed to stir at 0 °C for 1 h and overnight at room temperature.

Acetone was removed *in vacuo,* the residue was filtered and dried overnight *in vacuo* to give the title compound (155 g) as a light brown solid (y = 63%).

MS (m/z): 242.2 [MH]⁺.

### Preparation 16: (1R,5S/1S,5R)-1-[4-Trifluoromethyl)phenyl]-3-azabicyclo[3.1.0]-hexane-2,4-dione

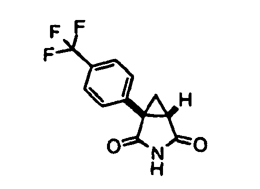

Milled sodium hydroxide (40 g) was added in small portions to a stirred solution of trimethylsulfoxonium iodide (219 g) in DMSO (anhydrous, 2 l). The resulting mixture was allowed to stir at room temperature for 1.5 h.

3-[4-(Trifluoromethyl)phenyl]-1*H*-pyrrole-2,5-dione (Preparation 15, 120 g) dissolved in DMSO (anhydrous, 0.5 l) was then added dropwise and the resulting mixture was allowed to stir at room temperature for 20 minutes. Temperature was then reduced to 0 °C and NH₄Cl (aqueous saturated solution, 2 l) was slowly added, followed by Et₂O (1 l). After separation of the two phases, the aqueous layer was repeatedly extracted with Et₂O (3 x 1 I). Combined organic layers were washed with brine (2 x 1 l) and then dried over Na₂SO₄. Evaporation of the solvent gave a light brown solid which was suspended in 1 l of dichloromethane and 1 l of cyclohexane. The mixture was allowed to stir at room temperature for 45 minutes and then filtered to give the title compound (116 g) as white solid (y = 71%).

MS (m/z): 256.1 [MH]⁺.

### Preparation 17: (1R,5S/1S,5R)-1-[4-(Trifluoromethyl)phenyl]-3-azabicyclo[3.1.0]-hexane

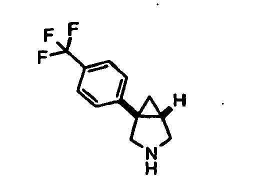

Borane (1 M in tetrahydrofuran, 1.4l) was charged into a 5 l reactor under N₂ and cooled at 0 °C. (1*R*,5*S*/1*S*,5*R*)-1-[4-(Trifluoromethyl)phenyl]-3-azabicyclo[3.1.0]hexane-2,4-dione (Preparation 16, 101 g) dissolved in tetrahydrofuran (anhydrous, 1 l) was then added dropwise with vigorous stirring whereby the temperature was constantly kept below 5 °C and gas evolution was monitored. At the end of the addition the resulting mixture was allowed to stir at 0 °C for 1 h and then at room temperature overnight.

The mixture was then cooled to 0 °C and methanol (200 mL) followed by hydrochloric acid (6 M solution, 0.8 l) were cautiously added monitoring gas evolution. tetrahydrofuran was then removed *in vacuo,* the residue was cooled to 0 °C and sodium hydroxide (5 M solution) was added until pH 9-10 had been reached. The aqueous layer was extracted with Et₂O (3 x 1 l). Removal of solvent *in vacuo* gave the title compound (140 g) as colorless oil.

MS (m/z): 228.1 [MH]⁺.

### Preparation 18: (1S,5R)-1-[4-(Trifluoromethyl)phenyl]-3-azabicyclo[3.1.0]hexane

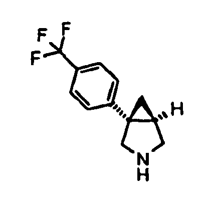

(S) - (+) - Mandelic acid (94 g) was added in portions to a stirred solution of (1*R*,5*S*/1*S*,5*R*)-1-[4-(trifluoromethyl)phenyl]-3-azabicyclo[3.1.0]hexane (Preparation 17, 140 g) in 1.4 l of tetrahydrofuran. The resulting mixture was stirred at room temperature for 2 h until a white precipitate was formed. The mixture was then warmed up to reflux temperature, stirred for 45 minutes and then slowly cooled down to room temperature. The white solid was collected by filtration and dried *in vacuo.* This material was recrystallised 4 times from tetrahydrofuran (10 volumes) to give 32.5 g of a white solid. This material was then suspended in sodium hydroxide (1 M solution, 400 mL) and Et₂O (400 mL) and allowed to stir at room temperature until complete dissolution. After separation of the two phases, the aqueous layer was extracted again with Et₂O (3 x 250 mL). Combined organic layers were washed with sodium hydroxide (1 M solution, 3 x 200 mL) and then dried over Na₂SO₄. Evaporation of solvent *in vacuo* gave the title compound (19 g) as white solid (y = 37%).

The absolute configuration of the optical isomers was assigned using comparative VCD (vibrational circular dichroism) and OR (optical rotation) analyses.

The configuration of the title compound was assigned by comparing its experimental VCD spectrum and observed specific rotation to the data observed for (1*S*,5*R*)-1-(3,4-dichlorophenyl)-3-azabicyclo[3.1.0]hexane (see Preparation 48) as the reference sample. The assignment of the absolute configuration of the title compound was confirmed by a single crystal X-ray structure obtained from a crystal of (1*S*,5*R*)-1-[4-(trifluoromethyl)phenyl]-3-azabicyclo[3.1.0]hexane, (S)-(+)-mandelic acid salt. Both, analysis based on the known configuration of the (S)(+)- mandelic acid and on the basis of anomalous dispersion effects confirmed the assignment of the title compound as being (1S,5R)-1-[4-(trifluoromethyl)phenyl]-3-azabicyclo[3.1.0]hexane.

NMR (¹H, CDCl₃): δ 7.51 (d, 2H), 7.25 (d, 2H), 3.20 (d, 1H), 3.0-3.1 (m, 3H), 1.69 (m, 1H), 0.8-1.0 (m, 2H), NH not observed. MS (*m*/*z*): 228.1 [MH]⁺.

### Analytical chromatography

Column: chiralcel OD 10 um, 250 x 4.6 mm
Mobile phase: A: n-Hexane; B: Isopropanol +0.1% Isopropyl amine
Gradient: isocratic 2% B
Flow rate: 1 mL/min
UV wavelengh range: 200-400 nm
Analysis time 25 min
ret. time (min) % a/a
16.5 0.4 (1*R*,5*S*)-1-[4-(trifluoromethyl)phenyl]-3-azabicyclo[3.1.0]hexane
21.7 99.6 title compound
Specific Optical Rotation: [□]_{D} = - 10° (CDCl₃, T = 20 °C, c ≅ 0.004 g/0.8 mL).

### Preparation 19: 3-{(1S,5R)-1-[4-(Trifluoromethyl)phenyl]-3-azabicyclo[3.1.0]hex-3-yl}-1-butanol

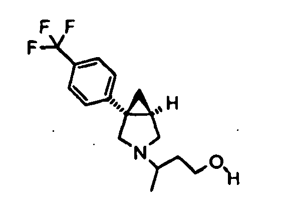

To a suspension of (1*S*, 5*R*)-1-[4-(trifluoromethyl)phenyl]-3-azabicyclo[3.1.0]hexane (Preparation 18, 100 mg) in tetrahydrofuran (1.1 mL), 4-hydroxy-2-butanone (0.66 mmol), acetic acid (0.66 mmol) and NaBH(OAc)₃ (0.88 mmol) were added. The mixture was stirred at room temperature for 2 h. After addition of NaOH (1M), the solvent was eliminated under *vacuo,* the residue was dissolved in ethyl acetate and the organic layer was washed with H₂O and dried over Na₂SO₄. This solution was concentrated *in vacuo* to give 130 mg of the title compound which was used without further purification.
MS (*m*/*z*): 300 [MH]⁺.

### Preparation 20: (1S,5R)-3-(3-Chloro-1-methylpropyl)-1-[4-(trifluoromethyl)phenyl]-3-azabicyclo[3.1.0]hexane

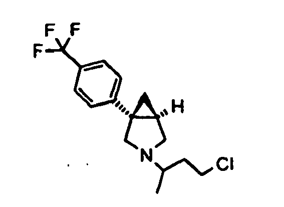

To a solution of 3-{(1*S*,5*R*)-1-[4-(trifluoromethyl)phenyl]-3-azabicyclo[3.1.0]hex-3-yl}-1-butanol (Preparation 19, 130 mg) in chloroform (4 mL), thionyl chloride (0.87 mmol) was added and the mixture was stirred at room temperature for 6 h. After addition of NaOH (1 M), dichloromethane was added and the organic layer was washed with Brine and dried over Na₂SO₄. The solution was concentrated *in vacuo* and the crude product purified by flash chromatography (ethyl acetate: cyclohexane= 5:95) to give 106 mg of the title compound.

MS (*m*/*z*)*:* 318 [MH]⁺.

### Preparation 21: 1-{5-[(1S,5R/1R,5S)-3-Azabicyclo[3.1.0]hex-1-yl)-2-(methyloxy)phenyl}ethanone

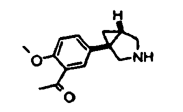

The title compound was prepared in 32 mg yield from 1-[4-(methyloxy)phenyl]-3-(trifluoroacetyl)-3-azabicyclo[3.1.0]hexane (94 mg) as described for preparation 34.

MS (*m*/*z*): 232 [MH]+. HPLC: condition 1, Rt= 3.393 min.

### Preparation 22: (1S,5R/1R,5S)-1-(4-Chlorophenyl)-3-azabicyclo[3.1.0]hexane

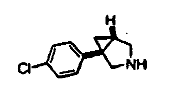

The title compound was prepared in 230 mg yield from commercially available methyl 4-chlorophenylacetate (1 g, 5.5 mmol) following the methods described in preparations 1, 2, 3, 4, 6.

MS (*m*/*z*): 194 [MH]+,.

### Preparation 23: (1S,5R/1R,5S)-1-(4-Fluorophenyl)-3-azabicyclo[3.1.0]hexane

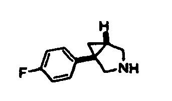

The title compound was prepared in 160 mg yield from commercially available methyl 4-fluorophenylacetate (1 g, 6 mmol) following the methods described in preparations 1, 2, 3, 4, 6.

MS (*m*/*z*): 178 [MH]+.

### Preparation 24: (1S,5R/1R,5S)-1-(3-Chlorophenyl)-3-azabicyclo[3.1.0]hexane

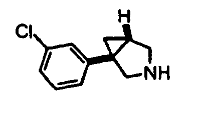

The title compound was prepared in 1.25g yield from commercially available methyl 3-chlorophenylacetate (5 g, 27 mmol) following the methods described in preparations 1, 2, 3, 4, 5.

MS (*m*/*z*): 194 [MH]+. HPLC: condition 1, Rt= 3.469 min.

### Preparation 25: (1S,5R/1R,5S)-1-(3-Fluorophenyl)-3-azabicyclo[3.1.0]hexane

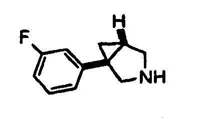

The title compound was prepared in 1.97 g yield from commercially available methyl 3-fluorophenylacetate (5 g, 29.7 mmol) following the methods described in preparations 1, 2, 3, 4, 5.

MS (*m*/*z*): 178 [MH]+.

### Preparation 26: (1S,5R/1R,5S)-1-[3-(Methyloxy)phenyl]-3-azabicyclo[3,1.0]hexane

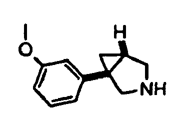

The title compound was prepared in 1.2 g yield from commercially available methyl 3-methoxyphenylacetate (5 g, 27.7 mmol) following the methods described in preparations 1, 2, 3, 4, 5.

MS (*m*/*z*): 190 [MH]+. HPLC: condition 1, Rt= 3.219 min.

### Preparation 27: (1S,5R/1R,5S)-1-[2-Methyl-4-(trifluoromethyl)phenyl]-3-azabicyclo[3.1.0]hexane

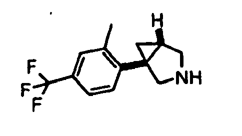

The title compound was prepared in 71 mg yield from commercially available 2-methyl-4-(trifluoromethyl)aniline (1 g, 5.7 mmol) following the methods described in preparations 15, 16, 17.

MS (*m*/*z*): 242 [MH]+.

### Preparation 28: Methyl bromo{4-[(trifluoromethyl)oxy]phenyl}acetate

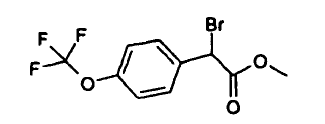

To a solution of 4-trifluoromethoxyphenylacetic acid (5 g, 23 mmol) in carbon tetrachloride oxalyl chloride (25 mmol) and two drops of DMF were added at 0°C. After stirring the solution at room temperature for 1 h, NBS (25 mmol) and few drops of 48% HBr were added and the mixture was heated to reflux for 4 h. The solution was allowed to cool, MeOH (5 mL) was added and the mixture was stirred at room temperature for 1 h. After filtration through a pad of silica gel, the filtrate was evaporated *in vacuo* to give 7.2 g of the title compound as yellow foam, which was used in the subsequent step without further purification.

MS (*m*/*z*): 314 [MH]+.

### Preparation 29: (1S,5R/1R,5S)-1-{4-[(Trifluoromethyl)oxy]phenyl}-3-azabicyclo[3.1.0]hexane

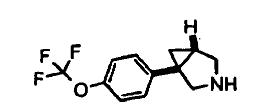

The title compound was prepared in 1.2 g yield from methyl 3-triflouromethoxyphenylacetate (Preparation M, 23 mmol) following the methods described in preparations 2, 3, 4, 5.

MS (*m*/*z*): 244 [MH]+. HPLC: condition 1, Rt= 3.942 min.

### Preparation 30: (1S,5R/1R,5S)-1-[3-(Trifluoromethyl)phenyl]-3-azabicyclo[3.1.0]hexane

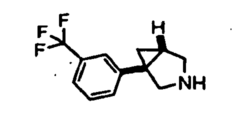

The title compound was prepared in 1.5 g yield from commercially available 3-trifluoromethylphenylacetic acid (5 g, 24.5 mmol), following the methods described in preparations 28, 2, 3, 4, 5.

MS (*m*/*z*): 228 [MH]+. HPLC: condition 1, Rt= 3.665 min.

### Preparation 31: (1R,5S/1S,5R)-1-(3-Bromophenyl)-3-azabicyclo[3.1.0]hexane

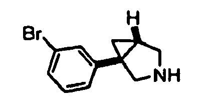

The title compound was prepared in 1.6 g yield from commercially available 3-bromophenylacetic acid (5 g, 23.2 mmol), following the methods described in preparations 28, 2, 3, 4, 6.

MS (*m*/*z*): 239 [MH]+. HPLC: condition 1, Rt= 3.528 min.

### Preparation 32: (1S,5R)-1-(4-Bromophenyl)-3-azabicyclo[3.1.0]hexane

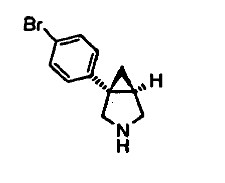

(S)(+)-Acetyl mandelic acid (3.22 g) was added in portions to a stirred solution of (1*R*,5*S*/1*S*,5*R*)-1-[4-bromophenyl]-3-azabicyclo[3.1.0]hexane (Preparation 6, 3.96 g) in 80 mL of IPA. The resulting mixture was stirred at room temperature for 2 h until a white precipitate was formed. The mixture was then warmed up to reflux temperature, stirred for 45 minutes and then slowly allowed to cool to room temperature. The white solid was collected by filtration and dried *in vacuo.* This material was recrystallised 4 times from IPA (10 volumes) to give 2.3 g of a white solid.

This material was then suspended in sodium hydroxide (1M aqueous solution, 400 mL) and Et₂O (400 mL) and allowed to stir at room temperature until complete dissolution. After separation of the two phases, the aqueous layer was extracted again with Et₂O (3 x 250 mL). Combined organic layers were washed with sodium hydroxide (1 M solution, 3 x 200 mL) and then dried over Na₂SO₄. Evaporation of solvent *in vacuo* gave the title compound (1.24 g) as white solid.

The absolute configuration of the optical isomers was assigned as described for Preparation 18.

The assignment of the absolute configuration of the title compound was confirmed by a single crystal X-ray structure obtained from a crystal of (1*S*,5*R*)-1-(4-bromophenyl)-3-azabicyclo[3.1.0]hexane, (S)-(+)-O-acetyl mandelic acid salt. Both, analysis based on the known configuration of the (S)(+)-acetyl mandelic acid and on the basis of anomalous dispersion effects confirmed the assignment of the title compound as being (1S,5R)-1-(4-bromophenyl)-3-azabicyclo[3.1.0]hexane.

NMR (¹H, CDCl₃): δ 7.43 (d, 2H), 7.09 (d, 2H), 3.25 (d, 1H), 3.15 (m, 2H), 3.06 (d, 1H), 1.71 (m, 1H), 0.95 (dd, 1H), 0.89 (t, 1H), NH not observed.

MS (*m*/*z*): 239 [MH]⁺.

### Analytical chromatography

| | |
|---|---|
| Column: | chiralcel OD 5 µm, 250 x 4.6 mm |
| Mobile phase: | A: n-Hexane; B: Isopropanol +0.1% Isopropyl amine |
| Gradient: | isocratic 2% B |
| Flow rate: | 1 mUmin |
| UV wavelengh range: | 200-400 nm |
| Analysis time | 25 min |
| Ret. time | 22.3 min, purity > 99 % a/a |
| Specific Optical Rotation: | [□]_{D} = - 86° (CDCl₃, T = 20 °C, c = 0.0053 g/0.8 mL). |

### Preparation 33: (1R,5S/1S,5R)-1-[2-(Trifluoromethyl)phenyl]-3-azabicyclo[3.1.0]hexane

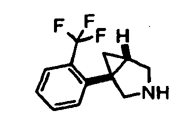

The title compound was prepared in 53 mg yield from commercially available methyl [2-(trifluoromethyl)phenyl]acetate (944 mg) following the methods described in preparations 1,2,3,4and5.

MS (*m*/*z*): 228 [MH]⁺.

Preparation 34: 1-[4-[(1*R*,5*S*/1*S*,5*R*)-3-Azabicyclo[3.1.0]hex-1-yl]-2-(methyloxy)phenyl]ethanone

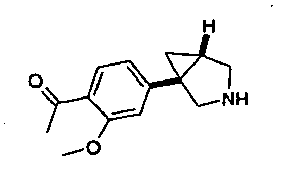

To a mixture of AlCl₃ (2 eq) in 1,2-dichloroethane (anhydrous, 9 mL) at 0 °C was added acetyl chloride (1.05 eq). The reaction mixture was stirred at 0 °C for 15 min and a solution of 1-[3-(methyloxy)phenyl]-3-(trifluoroacetyl)-3-azabicyclo[3.1.0]hexane (1.1 g, obtained in analogy to the method described in preparation 7 from 1-[3-(methyloxy)phenyl]-3-azabicyclo[3.1.0]hexane) in 1,2-dichloroethane (anhydrous, 9 mL) was added. The reaction mixture was stirred at RT for 1.5 h. HCl (1 M, 4 mL) was added followed by water (20 mL) and the mixture was extracted with dichloromethane. The organic layer was washed with saturated aqueous NaHCO₃ and dried over Na₂SO₄. The solution was filtered and the filtrate was concentrated *in vacuo.* The crude product was purified by flash chromatography (cyclohexanes:EtOAc 6:4) to give 593 mg as a colourless liquid of the protected amine. 143 mg of the protected amine was dissolved in MeOH: H₂O (3 mL:3 mL) and K₂CO₃ (4 eq) was added stirring the mixture at 50 °C for 2.5 h. The reaction mixture was extracted with dichloromethane and the organic layer was washed with saturated aqueous NaHCO₃ and dried over Na₂SO₄. The solution was filtered and the filtrate was concentrated *in vacuo* to give the title compound as a white solid (88 mg).

MS (*m*/*z*): 232 [MH]⁺.

### HPLC: conditions 1

### Analytical

| | |
|---|---|
| Column: | Supelcosil ABZ+Plus 33 x 4.6 mm, 3 □m |
| Mobile phase: | A: H2O+0.1% HCOOH, B: CH3CN |
| Gradient: | 0% (B) for 1 min, from 0% (B) to 95% (B) in 5 min, 95% (B) for 2 min |
| Flow rate: | 1 mUmin |
| UV wavelength: | 285 nm, band width 130 nm |
| Mass range: | 100-1000 amu |
| Ionization: | ES+ |
| Rₜ 2.971 min | |

### Preparation 35: -[4-[(1R,5S/1S,5R)-3-Azabicyclo[3.1.0]hex-1-yl)-2. (methyloxy)phenyl]-1-propanone

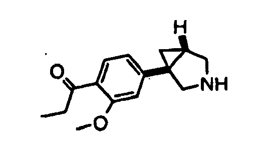

The title compound was prepared using propionyl chloride in place of acetyl chloride, in 106 mg yield from 147 mg of protected amine obtained in 705 mg from 1-[3-(methyloxy)phenyl]-3-(trifluoroacetyl)-3-azabicyclo[3.1.0]hexane (1.07 g) as described for preparation 34.

MS (*m*/*z*): 246 [MH]⁺.

### HPLC: conditions 1

Rₜ 3.249 min

### Preparation 36: 1(1R,5S/1S,5R)-[2-Fluoro-5-(trifluoromethyl)phenyl]-3-azabicyclo[3.1.0]hexane

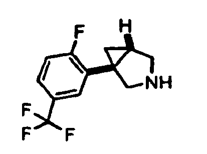

The title compound was prepared in 112 mg yield from 2-fluoro-5-(trifluoromethyl)anline (1.09 g) following the procedures reported for preparations 37 and 6.

NMR (¹H, CDCl₃): δ 7.45 (m, 2H), 7.1 (m, 1H), 3.2 (m, 2H), 3.05 (m, 2H), 1.7 (m, 1H), 0.95 (m, 1H), 0.9 (m, 1H), NH not observed. MS (*m*/*z*): 246[MH]⁺.

### Preparation 37:1 (1R,5S/1S,5R)-[2-Fluoro-4-trifluoromethyl)phenyl]-3 azabicyclo[3.1.0]hexane-2,4-dione

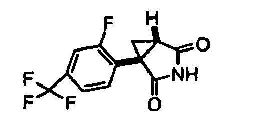

To a slurry of maleimide (1.7 eq), anhydrous CuCl₂ (1.2 eq) and *tert*-butyl nitrite (1.5 eq) in CH₃CN (35 mL) at 0°C a solution of 2-fluoro-4-(trifluoromethyl)aniline (16.3 g) in CH₃CN (6.5 mL) was added dropwise. The reaction mixture was stirred at room temperature for 1 h and HCl (10%, aqueous, 196 mL) was added. The mixture was extracted with EtOAc, the organic layer was washed with saturated aqueous NaCl and dried over Na₂SO₄. The solution was filtered and the filtrate was concentrated *in vacuo.* By NMR analysis the crude mixture resulted a 1:4 mixture of the arylated maleimide hydrogen chloride adduct (component A) and unreacted maleimide (component B).

A DMSO (140 mL) solution of this crude product was added dropwise to a preformed solution of trimethylsulfoxonium iodide (2 eq with respect to component A plus 2 eq with respect to component B) in anhydrous DMSO (412 mL) to which NaH (3 eq with respect to component A plus 2 eq with respect to component B) had been added portionwise. The reaction mixture was stirred for 30 min and AcOH (2 eq) was added followed by water. The reaction mixture was extracted with Et₂O and then with EtOAc, the combined organic layers were washed with saturated aqueous NaCl and dried over Na₂SO₄. The solution was filtered and the filtrate was concentrated *in vacuo.* The crude product obtained was triturated with water and then with cyclohexanes to give the title compound as light brown solid (5.98 g).

NMR (¹H, CDCl₃): δ 7.55-7.3 (m, 3H), 2.8-2.7 (m, 1H), 2.1 (m, 1H), 2.0 (m, 1H) , NH not observed. MS (*m*/*z*): 274[MH]⁺.

### Preparation 38 (1R,5S/1S,5R)-1-[2-Fluoro-4-(trifluoromethyl)phenyl]-3-azabicyclo[3.1.0]hexane

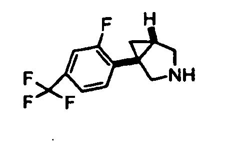

To a solution of (1*R*,5*S*/1*S*,5*R*)-1-[2-fluoro-4-(trifluoromethyl)phenyl]-3-azabicyclo-[3.1.0]hexane-2,4-dione (2.6 g) in anhydrous tetrahydrofuran (56 mL), BH₃ in tetrahydrofuran (1 M, 4 eq) was added at 0 °C. The reaction mixture was stirred at 65°C for 24 h, cooled to RT and MeOH was added until gas evolution ceased. Solvent was removed *in vacuo,* MeOH was added (200 mL) p-tolueneulfonic acid (3 eq) was added and the reaction mixture was stirred at 65°C for 6 h, the reaction mixture was cooled to room temperature and a saturated solution of K₂CO₃ (1.7 eq) was added. The mixture was extracted with dichloromethane, the organic layer was washed with saturated aqueous NaCl and dried over Na₂SO₄. The solution was filtered and the filtrate was concentrated *in vacuo* to give the title compound as colourless oil (2.1 g).

NMR (¹H, CDCl₃): δ 7.2-7.4 (m, 3H), 3.2 (m, 2H), 3.1 (m, 2H), 1.8 (m, 1H), 0.8 (m, 2H), NH not observed. MS (m/z): 246[MH]⁺.

### Preparation 39: (1S,5R)-1-[2-Fluoro-4-(trifluoromethyl)phenyl]-3-azabicyclo[3.1.0]hexane

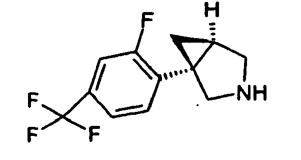

(1*R*)-(-)-10-Camphorsulfonic acid (4.19 g) was added in portions to a stirred solution of (1*R*,5*S*/1*S*,5*R*)-1-[2-fluoro-4-(trifluoromethyl)phenyl]-3-azabicyclo[3.1.0]hexane (4.4 g) in CH₃CN (44 mL). The resulting mixture was stirred at room temperature for 20 min until a white precipitate formed. The mixture was then warmed up to reflux temperature, stirred for 45 minutes and then slowly allowed to cool to room temperature. The white solid was collected by filtration and dried *in vacuo.* This material was recrystallised 2 times from CH₃CN (25 mL per g solid) to give 1.57 g of a white solid.

This material was then suspended in sodium hydroxide (1M solution, 1.1 eq) and dichloromethane (100 mL) and allowed to stir at room temperature until complete dissolution. After separation of the two phases, the aqueous layer was extracted again with dichloromethane. The combined organic layers were washed with sodium hydroxide and then dried over Na₂SO₄. Evaporation of solvent *in vacuo* gave the title compound (874 mg) as colorless liquid.

### Analytical chromatography

| | |
|---|---|
| Column: | chiralcel OD 10 □m, 250 x 4.6 mm |
| Mobile phase: | A: n-Hexane; B: Isopropanol +0.1 % Isopropyl amine |
| Gradient: | isocratic 2% B |
| Flow rate: | 0.8 mL/min |
| UV wavelengh range: | 200-400 nm |
| Analysis | |
| ret. time (min) | % a/a |
| 17.18 | >99.5 (1*S*,5*R*)-1-[2-fluoro-4-(trifluoromethyl)phenyl]-3-azabicyclo[3.1.0]hexane |

### Preparation 40: (1S,5R)-3-(3-Chloropropyl)-1-[4-(trifluoromethyl)phenyl]-3-azabicyclo[3.1.0]hexane

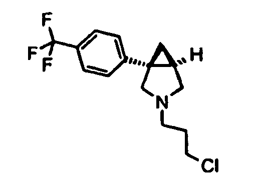

To a solution of (1*S*,5*R*)-1-[4-(trifluoromethyl)phenyl]-3-azabicyclo[3.1.0]hexane (1.00 g) in dry tetrahydrofuran (5 mL), diisopropylethylamine (2.4 mL) and 1-bromo-3-chloropropane (3.7 mL) were added and the resulting mixture was heated at reflux for 3 hours. After cooling at room temperature it was diluted with ethyl acetate (30 mL) washed twice with a saturated solution of NH₄Cl in water (20 mL) and once with a saturated solution of NaHCO₃ in water (20 mL), dried over anhydrous Na₂SO₄ and concentrated under reduced pressure. The crude product was purified by silica gel chromatography eluting with cyclohexane/EtOAc 7:3 to give the title compound as a colourless oil (1.26 g).

NMR (¹H, CDCl₃): δ 7.50 (d, 2H) 7.19 (d, 2H), 3.59 (t, 2H), 3.33 (d, 1H), 3.09 (d, 1H), 2.58 (m, 2H), 2.66 (dd, 1H), 2.46 (dd, 1H), 1.92 (m, 2H), 1.74 (m, 1H), 1,67 (t, 1H), 0.81 (dd, 1H). MS (*m*/*z*): 304 [MH]⁺.

### Preparation 41: (1S,5R)-3-(3-Chloropropyl)-1-[2-fluoro-4-(trifluoromethyl)pheny)]-3-azabicyclo[3.1.0]hexane

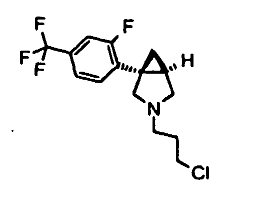

To a solution of (1*S*,5*R*)-1-[2-fluoro-4-(trifluoromethyl)phenyl]-3-azabicyclo[3.1.0]hexane (300 mg) in dry tetrahydrofuran (3 mL), diisopropylethylamine (0.65 mL) and 1-bromo-3-chloropropane (1.01 mL) were added and the resulting mixture was refluxed for 3 hours. After cooling at room temperature it was diluted with ethyl acetate (15 mL) washed twice with a saturated solution of NH₄Cl in water (10 mL) and once with a saturated solution of NaHCO₃ in water (10 mL), dried over anhydrous Na₂SO₄ and concentrated under reduced pressure. The crude product was purified by chromatography (silica gel) eluting with cyclohexane/EtOAc 6:4 to give the title compound as yellow oil (345 mg).

NMR (¹H, CDCl₃): δ 7.24 (d, 2H), 7.16 (t, 1H), 3.51 (t, 2H), 3.18 (dd, 1H), 3.03 (d, 1H), 2.54 (t, 2H), 2.48 (dd, 1H), 2.37 (d, 1H), 1.83 (m, 2H), 1.69 (m, 1H), 1.34 (t, 1H), 0.70 (dd, 1H). MS (*m*/*z*): 322 [MH]⁺.

### Preparation 42: (1R,5S/1S,5R)-1-[3-Fluoro-4-(trifluoromethyl)phenyl]-3-azabicyclo[3.1.0]hexane

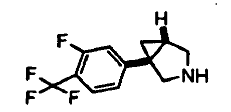

The title compound was prepared in 338 mg yield from 3-fluoro-4-(trifluoromethyl)aniline (2 g) following the procedures reported for Preparations 37 and 6.

NMR (¹H, CDCl₃): δ 7.5 (m, 1H), 6.9 (m, 2H), 3.3-3.0 (m, 4H), 1.7 (m, 1H), 0.95 (m, 2H), NH not observed. MS (*m*/*z*): 246 [MH]⁺.

### Preparation 43: (1R,5S/1S,5R-1-[4-(Methyloxy)phenyl]-3-(trifluoroacetyl)-3-azabicyclo[3.1.0]hexane

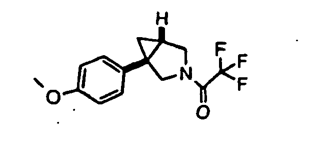

The title compound was prepared in 1.80 g yield (95%) as a colorless oil from (1*R*,5*S*/1*S*,5*R*)-1-[4-(methoxy)phenyl]-3-azabicyclo[3.1.0]hexane (1.25 g) in analogy to the method described in Preparation 7.

MS (*m*/*z*): 286 [MH]⁺.

### Preparation 44: 1-{2-(Methyloxy)-5-[(1R,5S/1S,5R)-3-(trifluoroacetyl)-3-azabicyclo[3.1.0]hex-1-yl]phenyl}ethanone and 1-{2-hydroxy-5-[(1R,5S/1S,5R)-3-(trifluoroacetyl)-3-azabicyclo[3.1.0]hex-1-yl]phenyl}ethanone

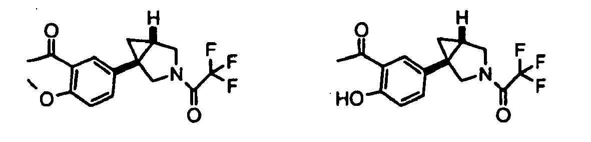

To a suspension of AlCl₃ (12.6 mmol) in dry 1,2-dichloroethane (16 mL) at 0°C acetyl chloride (6.6 mmol) was added and the mixture was stirred at this temperature for 15 min. A solution of (1*R*,5*S*/1*S*,5*R*)-1-[4-(methyloxy)phenyl]-3-(trifluoroacetyl)-3-azabicyclo[3.1.0]hexane (1.81 g, 6.3 mmol) in 1,2-dichloroethane (16 mL) was then added. The reaction mixture was allowed to stir at 0 °C for 15 min and overnight at room temperature. 1 M aqueous HCl was then added and the mixture was extracted with dichloromethane. The organic phase was washed with 5% NaHCO₃ and water, dried over Na₂SO₄ and concentrated *in vacuo.* The two products were separated by flash chromatography (cyclohexane/ethyl acetate from 95/5 to 80/20) to give 965 mg of 1-{2-(methyloxy)-5-[(1*R*,5*S*/1*S*,5*R*)-3-(trifluoroacetyl)-3-azabicyclo[3.1.0]hex-1-yl]phenyl}ethanone (48%) and 266 mg of 1-{2-hydroxy-5-[(1*R*,5*S*/1*S*,5*R*)-3-(trifluoroacetyl)-3-azabicyclo[3.1.0]hex-1-yl]phenyl}ethanone (18%) as yellow oils.

MS (*m*/*z*): 328 [MH]⁺. 1-{2-(methyloxy)-5-[(1R,5S/1S,5R)-3-(trifluoroacetyl)-3-azabicyclo[3.1.0]hex-1-yl]phenyl}ethanone; 312 [M-H]⁻, 1-{2-hydroxy-5-[(1*R*,5*S*/1*S*,5*R*)-3-(trifluoroacetyl)-3-azabicyclo[3.1.0]hex-1-yl]phenyl}ethanone.

### Preparation 45: 1-[5-[(1R,5S/1S,5R)-3-Azabicyclo[3.1.0]hex-1-yl]-2-(methyloxy)phenyl]ethanone

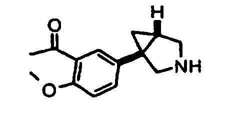

The title compound was prepared in 624 mg yield (91%) as a colorless oil from 1-{2-(methyloxy)-5-[(1*R*,5*S*/1*S*,5*R*)-3-(trifluoroacetyl)-3-azabicyclo[3.1.0]hex-1-yl]phenyl}ethanone (965 mg) in analogy to the method described in Preparation 12.

MS (*m*/*z*): 232 [MH]⁺.

### Preparation 46:1-{5-[(1R,5S/1S,5R)-3-Azabicyclo[3.1.0]hex-1-yl]-2-hydroxyphenyl}ethanone

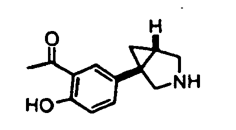

The title compound was prepared in 151 mg yield (82%) as a colorless oil from 1-{2-hydroxy-5-[(1R,5S/1S,5R)-3-(trifluoroacetyl)-3-azabicyclo[3.1.0]hex-1-yl]phenyl}ethanone (266 mg) in analogy to the method described in Preparation 12.

MS (m/z): 216 [M-H]⁻.

### Preparation 47: (1S,5R/1R,5S)-1-(4-Bromophenyl)-3-(3-chloropropyl)-3-azabicyclo[3.1.0]hexane

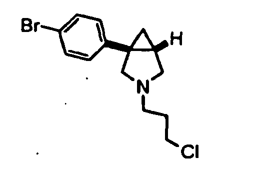

To a solution of racemic (1S,5R/1*R*,5*S*)-1-(4-bromophenyl)-3-azabicyclo[3.1.0]hexane (0.12 g) in dry tetrahydrofuran (2 mL), diisopropylethylamine (0.22 mL) and 1-bromo-3-chloropropane (0.062 mL) were added and the resulting mixture was heated at reflux for 3 hours. After cooling to room temperature the solvent was removed *in vacuo* and the resulting crude oil was taken up in dichloromethane (10 mL). This solution was then washed twice with a saturated solution of NH₄Cl in water (5 mL), dried over anhydrous Na₂SO₄ and concentrated under reduced pressure. The crude product was purified passing the sample through a 2 g silica cartridge (Varian) with a gradient elution from cyclohexane to cyclohexane/EtOAc 7:3, to give the title compound as a colourless oil (0.10 g).

NMR (¹H, DMSO): δ 7.45 (d, 2H), 7.10 (d, 2H), 3.65 (t, 2H), 3.30 (d, 1H), 3.00 (d, 1H), 2.55 (t, 2H), 2.45 (m, 1H), 2.40 (dd, 1H), 1.85 (m, 2H), 1.80 (m, 1H), 1.30 (t, 1H), 0.70 (m, 1H). MS (*m*/*z*): 314, 316, 318 [MH]⁺.

### Preparation 48: (1R,5S/1S,5R)- 1-(3,4-Dichlorophenyl)-3-azabicyclo[3.1.0]hexane

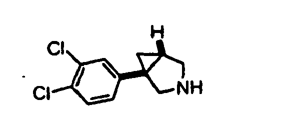

The crude title compound was prepared in 0.36 g yield from commercially available methyl 3,4-dichlorophenylacetate (1 g, 4.57 mmol) following the methods described in preparations 1, 2, 3, 4, 6.

The title compound was separated to give the separated enantiomers by preparative chromatography using a chiral column chiralcel AD 10 um, 250 x 21 mm, eluent A: n-hexane; B: isopropanol + 0.1% isopropyl amine, gradient isocratic 2% B, flow rate 7 mL/min, detection UV at 200-400 nm. Retention times given were obtained using an analytical HPLC using a chiral column chiralcel AD 5 um, 250 x 4.6 mm, eluent A: n-hexane; B: isopropanol +0.1% Isopropyl amine, gradient isocratic 2% B, flow rate 1.2 mL/min, detection UV at 200-400 nm.

Enantiomer 1, (1*R*,5*S*)-1-(3,4-dichlorophenyl)-3-azabicyclo[3.1.0]hexane, was recovered in 20 mg yield as white solid from the racemate (60 mg). Rt. = 41 min.

Enantiomer 2, (1*S*,5*R*)1-(3,4-dichlorophenyl)-3-azabicyclo[3.1.0]hexane, was recovered in 28 mg yield as white solid from the racemate (60 mg). Rt. = 43.4 min.

The absolute configuration of (1*S*,5*R*)-1-(3,4-dichlorophenyl)-3-azabicyclo[3.1.0]hexane was assigned using ab *initio* VCD and *ab initio* OR analyses.

Specific Optical Rotation of (1*S*,5*R*)-1-(3,4-dichlorophenyl)-3-azabicyclo[3.1.0]hexane: [□]_{D} = - 67.9° (CDCl₃, T = 20 °C, c ≅ 0.01g/mL).

NMR (¹H, CDCl₃): δ 7.35 (d, 1H), 7.27 (s, 1H), 7.02 (dd, 1H), 3.25 (d, 1H), 3.13 (bm, 2H), 3.06 (d, 1H), 1.71 (m, 1H), 0.93 (m, 2H), NH not observed. MS (m/z): 228 [MH]⁺,

### Preparation 49: 1-(Phenylmethyl)-3-(4,4,5,5-tetramethyl-1,3,2-dioxaborolan-2-yl)-2,5-dihydro-1H-pyrrole

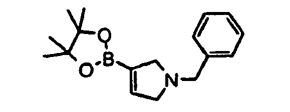

Diisopinocampheylborane was prepared following the procedure reported in *J*. *Org. Chem.* 1984, *49*, 945-947. 2-[(1Z)-3-Chloro-1-(chloromethyl)-1-propen-1-yl]-4,4,5,5-tetramethyl-1,3,2-dioxaborolane (previously described in Tetrahedron Lett. 1993, 34, 4827-4828) was prepared following the general procedure reported in Tetrahedron Lett. 1989, 30, 2929, using 1,4-dichloro-2-butyne. The material thus obtained was further converted following the procedure reported in Synlett 2002, 5, 829-831. This latter procedure was modified in that isolation of the the title product was achieved (rather than by distillation) by extraction of a solution of the crude reaction products in acetonitrile with cyclohexane, to provide the title compound (containing ~ 10% in moles of benzylamine) after evaporation of the volatiles from the cyclohexane phase.

### Preparation 50: 2-[1-(Phenylmethyl)-2,5-dihydro-1H-pyrrol-3-yl]-5-(trifluoromethyl)pyridine

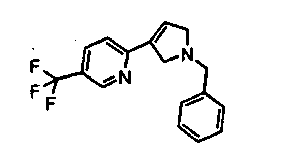

To a solution of 2-bromo-5-(trifluoromethyl)pyridine (4.42 mmol) in dry tetrahydrofuran (45 mL) 1-(phenylmethyl)-3-(4,4,5,5-tetramethyl-1,3,2-dioxaborolan-2-yl)-2,5-dihydro-1*H-*pyrrole (3.4 mmol), tetrakis(triphenylphosphine)palladium(0) (0.196 mmol) and cesium fluoride (13,2 mmol) were added at room temperature. The resulting mixture was stirred at 80 °C for 1.5 hours. After cooling solvent was evaporated under reduced pressure and the residue was partitioned between dichloromethane (25 mL) and sodium hydroxyde (15 mL, 1M). The organic phase was evaporated under reduced pressure. The crude product was purified by silica gel column chromatography (AcOEt:cyclohexane=1:10 to 4:6) to give 0.33 g of the title compound (y= 24%).

NMR (¹H, CDCl₃): δ 9.8 (s,1H), 7.85 (dd, 1H), 7.5 - 7.2 (m, 6H), 6.7 (s, 1H), 3.95 (m, 2H), 3.9 (s, 2H), 3.75 (m, 2H). MS (*m*/*z*): 305 [MH]⁺.

### Preparation 51: 2-[1-(Phenylmethyl)-2,5-dihydro-1H-pyrrol-3-yl]-6-(trifluoromethyl)pyridine

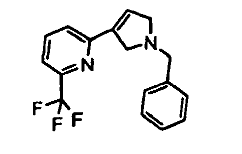

2-[1-(Phenylmethyl)-2,5-dihydro-1*H*-pyrrol-3-yl]-6-(trifluoromethyl)pyridine was prepared in analogy to the method described in Preparation 50 in 0.56 g (y= 42%) as an oil.

NMR (¹H, CDCl₃): δ 7.7 (t, 1H), 7.85 (dd, 1H), 7.4-7.1 (m, 6H), 6.5 (s, 1H), 3.90 (sb, 2H), 3.8 (s, 2H), 3.6 (m, 2H). MS (*m*/*z*): 305 [MH]⁺.

### Preparation 52: 3-(Phenylmethyl)-1-[5-(trifluoromethyl)-2-pyridinyl]-3-azabicyclo[3.1.0]hexane

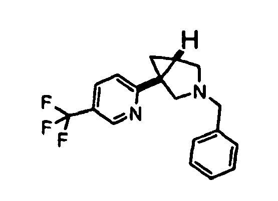

To a slurry of sodium hydride (83 mg) and trimethylsulfoxonium iodide (0.46 g) DMSO (anhydrous, 3 mL) was added dropwise (gas evolution). The resulting mixture was allowed to stir at room, temperature for 0.5 h. A solution of 2-[1-(phenylmethyl)-2,5-dihydro-1*H*-pyrrol-3-yl]-5-(trifluoromethyl)pyridine (330 mg) in DMSO (anhydrous, 6 mL) was added at room temperature. After 1 h a saturated solution of ammonium chloride (4 mL) was added and the mixture extracted with dichloromethane (2 x 10 mL). Volatiles from the organic phase were evaporated under reduced pressure, the residue charged onto an SCX column and eluted with MeOH followed by MeOH/NH₃ 0.25 M. The methanole/ammonia fractions were concentrated under reduced pressure to give 0.31 g of the title compound (y= 89%).

NMR (¹H, CDCl₃): δ 8.78 (s,1H), 8.03 (dd, 1H), 7.32 (m, 5H), 7.25 (m, 1H), 3.66 (dd, 2H), 3.25 (d, 1H), 2.96 (d, 1H), 2.80 (d, 1H), 2.46 (sb, 1H), 2.05 (q, 1H), 1.58 (m, 1H), 1.22 (m, 1H). MS (*m*/*z*): 317 [MH]⁺.

### Preparation 53: 3-(Phenylmethyl)-1-[6-(trifluoromethyl)-2-pyridinyl]-3-azabicyclo[3.1.0]hexane

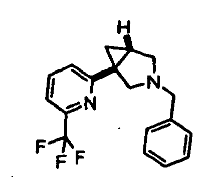

3-(Phenylmethyl)-1-[6-(trifluoromethyl)-2-pyridinyl]-3-azabicyclo[3.1.0]hexane was prepared in analogy to the method described in Preparation 52 (0.46 g, 79%) as an oil.

NMR (¹H, CDCl₃): δ 7.7 (t, 1H), 7.4 (d, 1H), 7.35 (m, 5H), 7.2 (d, 1H), 3.7 (s, 2H), 3.4 (d, 1H), 3.1 (d, 1H), 2.85 (d, 1H), 2.55 (m, 1H), 2.1 (m, 1H), 1.7 (m, 1H), 1.3 (m, 1H). MS (*m*/*z*): 317 [MH]⁺.

### Preparation 54: 1-[5-(Trifluoromethyl)-2-pyridinyl]-3-azabicyclo[3.1.0]hexane

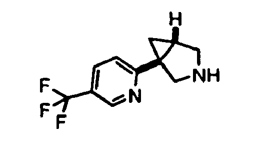

3-(Phenylmethy)-1-[5-(trifluoromethyl)-2-pyridinyl]-3-azabicyclo[3.1.0]hexane was dissolved in ethanol (15 mL), hydrochloridic acid (3M, 0.76 mL) was added followed, in inert atmosphere, by Pd/C 10% w/w (120 mg). After 20 h under a hydrogen atmosphere (1 atm) the mixture was filtered. Solvent was removed under reduced pressure. A saturated solution of sodium bicarbonate was added (10 mL) and the mixture extracted with diethyl ether (2 x 10 mL) to provide the title compound (0.14 g, 81%) after evaporation of volatiles.

NMR (¹H, DMSO-d⁶): δ 8.7 (s, 1H), 7.8 (d, 1H), 7.15 (d, 1H), 3.4 - 3.2 (dd, 2H), 3.1 (m, 2H), 2.05 (m, 1H), 1.4 (m, 1H), 1.05 (t, 1H).

### Preparation 55: 2-Fluoro-4-[1-(phenylmethyl)-2,5-dihydro-1H-pyrrol-3-yl]benzonitrile

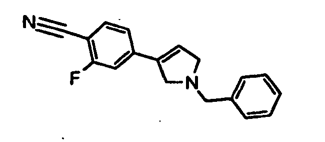

The title compound was prepared in analogy to the method described in Preparation 50 in 0.44 g (y= 31%) as an oil.

NMR (¹H, CDCl₃): δ 7.55 (t,1H), 7.4 - 7.2 (m, 5H), 7.2 (d, 1H), 7.1 (d, 1H), 6.4 (bs, 1H), 3.9 (s, 2H), 3.8 (m, 2H), 3.75 (m, 2H). MS (*m*/*z*): 279 [MH]⁺.

### Preparation 56: 2-Fluoro-4-[3-(phenylmethyl)-3-azabicyclo[3.1.0]hex-1-yl]benzonitrile

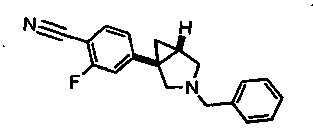

The title compound was prepared in analogy to the method described in Preparation 52 in 0.39 g (y= 84%) as an oil.

NMR (¹H, CDCl₃): δ 7.41 (t,1H), 7.25 - 7.15 (m, 5H), 6.85 - 6.8 (dd, 2H), 3.64 - 3.56 (dd, 2H), 3.19 (dd, 1H), 3.01 (dd, 1H), 2.53 (dd, 1H), 2.47 (dd, 1H), 1.73 (q, 1H), 1.67 (m, 1H), 0.81 (m, 1H). MS (*m*/*z*): 293 [MH]⁺.

### Preparation 57: 1-[3-Fluoro-4-(1H-pyrrol-1-ylmethyl)phenyl]-3-azabicyclo[3.1.0]hexane

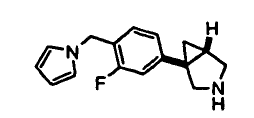

To a solution of {[4-(3-azabicyclo[3.1.0]hex-1-yl)-2-fluorophenyl]methyl}amine dihydrochloride in methanol/tetrahydrofuran (anhydrous, 1/1, 5 mL), which was prepared in analogy to the method described in Preparation 54 starting from 1.1 mmol of 2-fluoro-4-[3-(phenylmethyl)-3-azabicyclo[3.1.0]hex-1-yl]benzonitrile and used without further purification, a solution of 2,5-bis(methyloxy)tetrahydrofuran (2.53 mmol), H₂SO₄ (4.4 mmol) in methanol/tetrahydrofuran (anhydrous, 1/1, 5 mL) was added dropwise over 5 min at room temperature. After standing over night at room temperature a saturated solution of NaHCO₃ was slowly added, extraction with 2x 15 mL of dichloromethane followed by preparative HPLC purification provided 14 mg of titled compound as an oil (y = 5%).

NMR (¹H, COCl₃): δ 6.88 - 6.82 (m, 3H), 6.67 (t, 2H), 6.14 (t, 2H), 5.04 (s, 2H), 3.21 (d, 1H), 3.1 (d, 1H), 3.09 (d, 1H), 3.01 (d, 1H), 1.67 (m, 1H), 0.88 (m, 2H). MS *(mlz):* 257 [MH]⁺.

### Preparation 58: (1R,5S/1S,5R)-3-(3-Chloropropyl)-1-[6-(trifluoromethyl)-3-pyridinyl]-3-azabicyclo[3.1.0]hexane

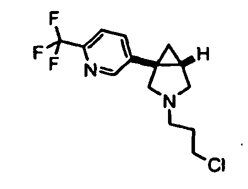

The title compound was prepared in 522 mg yield (84%) as a colorless oil from (1R,5S/1S,5R)-1-[6-(trifluoromethyl)-3-pyridinyl]-3-azabicyclo[3.1.0]hexane (584 mg) in analogy to the method described in Preparation 40.

NMR (¹H, CDCl₃): δ 8.47 (s, 1H), 7.55 (m, 2H), 3.59 (t, 2H), 3.33 (d, 1H), 3.09 (d, 1H), 2.6 (m, 3H), 2.52 (dd, 1H), 1.92 (m, 2H), 1.78 (m, 1H), 0.85 (m, 1H), 0.81 (dd, 1H). MS (*m*/*z*): 305 [MH]⁺.

### Preparation 59: 5-[(1R,5S/1S,5R)-3-(3-Chloropropyl)-3-azabicyclo[3.1.0]hex-1-yl]-2-methyl-1,3-benzothiazole

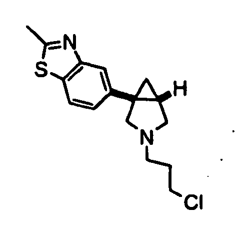

The title compound was prepared in 480 mg yield (84%) as a colorless oil from 5-[(1R,5S/1S,5R5)-3-azabicyclo[3.1.0]hex-1-yl]-2-methyl-1,3-benzothiazole (374 mg) in analogy to the method described in Preparation 40.

NMR (¹H, CDCl₃): δ 7.70 (m, 2H), 7.11 (d, 1H), 3.59 (t, 2H), 3.38 (d, 1H), 3.09 (d, 1H), 2.8 (s, 3H), 2.66 (m, 3H), 2.53 (dd, 1H), 1.95 (m, 2H), 1.74 (m, 1H), 1,44 (t, 1H), 0.83 (dd, 1H). MS (*m*/*z*): 307 [MH]⁺.

### Preparation 60: 1(1R,5S/1S,5R)-[3-Fluoro-5-(trifluoromethyl)phenyl]-3-azabicyclo[3.1.0]hexane-2,4-dione

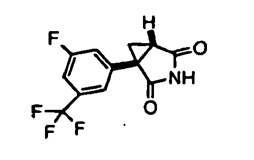

To a slurry of maleimide (1.8 eq), anhydrous CuCl₂ (1.2 eq) and *tert*-butyl nitrite (1.5 eq) in CH₃CN (5 mL) at 0 °C a solution of 3-fluoro-5-(trifluoromethyl)aniline (2.2 g) in CH₃CN (4 mL) was added dropwise. The reaction mixture was stirred at room temperature for 2 h and HCl (aqueous 6 M, 30 mL) was added. The mixture was extracted with EtOAc. the organic layer dried over Na₂SO₄. The solution was filtered and the filtrate was concentrated *in vacuo*. The filtered was triturated with water and dried *in vacuo.*

A DMSO (10 mL) solution of this crude product was added dropwise to a preformed solution of trimethylsulfoxonium iodide (2 eq) in anhydrous DMSO (20 mL) to which NaH (15 eq) had been added portionwise. The reaction mixture was stirred for 30 min and water was added followed by a satured solution of NH₄Cl (until pH 6.5). The reaction mixture was extracted with Et₂O, the combined organic layers were washed with saturated aqueous NaCl and dried over Na₂SO₄. The solution was filtered and the filtrate was concentrated *in vacuo.* The crude product obtained was triturated with cyclohexane to give the title compound as light green solid (1.02 g).

NMR (¹H, CDCl₃): δ 7.4-7.20 (m, 3H), 2.85-2.75 (m, 1H), 2.0 (m, 1H), 1.85 (m, 1H), NH not observed. MS (*m*/*z*): 274[MH]⁺.

### Preparation 61: (1R,5S/1S,5R)-1-[3-Fluoro-5-(trifluoromethyl)phenyl]-3-azabicyclo[3.1.0]hexane

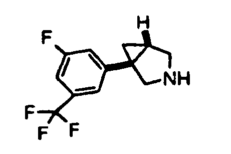

The title compound was prepared in 650 mg yield from (1*R*,5*S*/1*S*,5*R*)-1-[3-fluoro-5-(trifluoromethyl)phenyl]-3-azabicyclo[3.1.0]hexane-2,4-dione following the procedure reported for Preparation 38.

NMR (¹H, CDCl₃): δ 7.05-7.40 (m, 3H), 3.1-3.3 (m, 4H), 1.7 (m, 1H), 0.9 (m, 2H), NH not observed. MS (*m*/*z*): 246[MH]⁺.

### Preparation 62: (1R,5S/1S,5R)-1-[2-Fluoro-3-(trifluoromethyl)phenyl]-3-azabicyclo[3.1.0]hexane

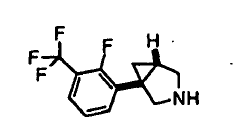

The title compound was prepared in 947 mg yield from 2-fluoro-3-(trifluoromethyl) aniline (3 g) following the procedures reported for Preparations 60 and 38.

NMR (¹H, CDCl₃): δ 7.2 (m, 2H), 6.9 (m, 1H), 3.0-2.7 (m, 4H), 1.6 (m, 1H), 0.7 (m, 2H); MS (*m*/*z*): 246[MH]⁺.

### Preparation 63: (1R,5S/1S,5R)-1-[4-(Methyloxy)-5-(trifluoromethyl)phenyl]-3-azabicyclo[3.1.0]hexane

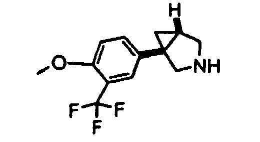

The title compound was prepared in 430 mg yield from 4-(methyloxy)-5-(trifluoromethyl) aniline (2.2 g) following the procedures reported for Preparations 60 and 38.

NMR (¹H, CDCl₃): δ 7.4-7.3 (m, 2H), 6.9 (m, 1H), 3.9 (s, 3H), 3.2-3.0 (m, 4H), 1.9 (s, 1H), 1.65 (m, 1H), 0.8 (m, 2H). MS (*m*/*z*): 258[MH]⁺.

### Preparation 64: (1R,5S/1S,5R)-1-(4-Chloro-2-fluorophenyl)-3-azabicyclo[3.1.0]hexane

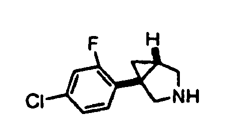

The title compound was prepared in 360 mg yield from 4-chloro-2-fluoro aniline (1.87 g) following the procedures reported for Preparations 60 and 38.

NMR (¹H, CDCl₃): δ 7.2-7.0 (m, 3H), 3.2-3.0 (m, 4H), 2.0 (s, 1H), 1.75 (m, 1H), 0.8 (m, 2H). MS (*m*/*z*): 212[MH]⁺.

### Preparation 65: (1R,5S/1S,5R)-1-{3-[(Trifluomethyl)oxy]phenyl}-3-azabicyclo[3.1.0]hexane

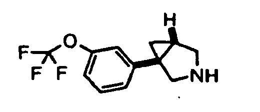

The title compound was prepared in 600 mg yield from 3-trifuoromethyloxy aniline (2.65 g) following the procedures reported for Preparations 60 and 38.

NMR (¹H, CDCl₃): δ 7.3-7 (m, 4H), 3.3-3.0 (m, 4H), 1.8 (s, 1H), 1.75 (m, 1H), 0.95 (m, 2H); MS (*m*/*z*): 212[MH]⁺.

### Preparation 66: (1R,5S/1S,5R)-1-(2-Fluoro-4-methylphenyl)-3-azabicyclo[3.1.0]hexane

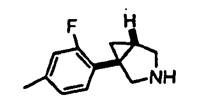

The title compound was prepared in 148 mg yield from 2-fluoro-4-methyl aniline (2.18 g) following the procedures reported for Preparations 60 and 38.

NMR (¹H, CDCl₃): δ 7.2 (m, 1H), 6.85 (m, 2H), 3.2-2.9 (m, 4H), 2.25 (s, 3H), 1.75 (s, 1H), 1.65 (m, 1H), 0.9 (m, 2H); MS (*m*/*z*): 192 [MH]⁺.

### Preparation 67: (1R,5S/1S,5R)-1-[3-Chloro-4-(methyloxy)phenyl]-3-azabicyclo[3.1.0]hexane

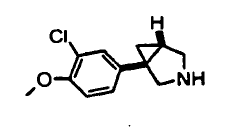

The title compound was prepared in 60 mg yield from 2-chloro-4-methyl aniline (2.36 g) following the procedures reported for Preparations 60 and 38.

NMR (¹H, CDCl₃): δ 7.15-7 (m, 2H), 6:85 (m, 1H), 3.85 (s, 3H), 3.2-2.9 (m, 4H), 1.8-1.6 (m, 2H), 0.75 (m, 2H); MS (*m*/*z*): 224[MH]⁺.

### Preparation 68: (1R,5S/1S,5R)-1,1-Dimethylethyl1-(4-bromophenyl)-3-azabicyclo[3.1.0]hexane-3-carboxylate

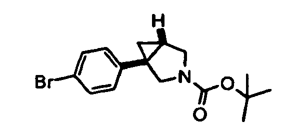

To a stirred solution of (1*R*,5*S*/1*S*,5*R*)-1-(4-bromophenyl)-3-azabicyclo[3.1.0]hexane (Preparation 6, 1.3 g) in dichloromethane (20 mL) at room temperature, triethylamine (0.99 mL) and bis(1,1-dimethylethyl) dicarbonate were added. Stirring was continued over 6 h, then the reaction mixture was concentrated under vacuum and the crude product treated with diethyl ether and water. The organic phase was washed with saturated ammonium chloride solution, dried over sodium sulphate and the solvent evaporated under vacuum to give a crude product that was purified by chromatography over silica gel (cyclohexane/ETOAC 9/1) affording the title compound (1.68 g, 91%).

MS (*m*/*z*): 282.1 [MH -C₄H₈]⁺, 1 Br.

### Preparation 69: (1R,5S/1S,5R)-1,1-Dimethylethyl1-[4-(4,4,5,5-tetramethyl-1,3,2-dioxaborolan-2-yl)phenyl]-3-azabicyclo[3.1.0]hexane-3-carboxylate

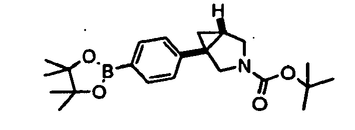

To a stirred solution of (1R,5S/1S,5R)-1,1-dimethylethyl 1-(4-bromophenyl)-3-azabicyclo[3.1.0]hexane-3-carboxylate (2 g) in DMF (30 mL), at RT, bis(pinacolate)di boron (2.25 g), potassium acetate (1.75 g) and PdCl₂(dppf) (0.15 g) were subsequently added. The reaction mixture was heated at 85 °C for 1.5 h, poured into water and extracted twice with diethylether, and the organic phase was washed with brine and dried over sodium sulphate. The solvent was evaporated under vacuum and the crude product purified by chromatography over silica gel (cyclohexane/ETOAC 9/1) affording the title compound as a white solid (2.1 g. 92%).

MS (*m*/*z*): 330.3 [MH - C₄H₈]⁺, 1 Br.

### Preparation 70: (1R,5S/1S,5R)-1,1-Dimethylethyl 1-(3-bromophenyl)-3-azabicyclo[3.1.0]hexane-3-carboxylate

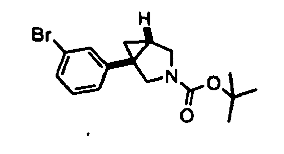

The title compound was prepared in 94% yield as a white solid in analogy to the method described for Preparation 68 starting from (1*R*,5*S*/1*S*,5*R*)-1-(3-bromophenyl)-3-azabicyclo[3.1.0]hexane (7.4 g).

MS (*m*/*z*): 282.1 [MH -C₄H₈]⁺, 1Br.

### Preparation 71: (1R,5S/1S,5R)-1,1-Dimethylethyl 1-[3-(4,4,5,5-tetramethyl-1,3,2-dioxaborolan-2-yl)phenyl]-3-azabicyclo[3.1.0]hexane-3-carboxylate

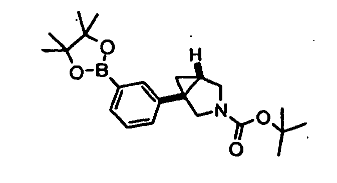

The title compound was prepared in 84% yield as a white solid in analogy to the method described for Preparation 69 starting from (1*R*,5*S*/1*S*,5*R*)-1,1-dimethylethyl 1-(3-bromophenyl)-3-azabicyclo[3.1.0]hexane-3-carboxylate (2.5 g).

MS (*m*/*z*): 330.3 [MH - C₄H₈]⁺, 1Br.

### Preparation 72: (1R,5S/1S,5R)-1-[4-(2,4-Dimethyl-1,3-thiazol-5-yl)phenyl]-3-azabicyclo[3.1.0]hexane

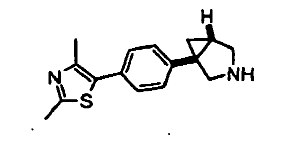

To a stirred solution of (1*R*,5*S*/1*S*,5*R*)-1,1-dimethylethyl 1-[4-(4,4,5,5-tetramethyl-1,3,2-dioxaborolan-2-yl)phenyl]-3-azabicyclo[3.1.0]hexane-3-carboxylate (0.3 g) in tetrahydrofuran (12 mL), at RT and under a nitrogen atmosphere, 5-bromo-2,4-dimethyl-1,3-thiazole (0.22 g), cesium fluoride (0.47 g) and tetrakis-(triphenylphosphin)-palladium(0) (0.06 g) were subsequently added. The reaction mixture was heated at 80 °C for 4 h and the solvent evaporated under vacuum. The crude product was treated with diethyl ether and saturated aqueous ammonium chloride solution, the organic phase was washed with brine, dried over sodium sulphate and concentrated under vacuum. The crude product was purified by chromatography over silica gel (cyclohexane/ETOAC 8/1). The purified product was then dissolved in CH₂Cl₂ (10 mL) and trifluoroacetic acid was added (4 mL). After 2 h the reaction mixture was treated with solid sodium carbonate and the solvent evaporated. The residue was treated with water and extracted with CH₂Cl₂, the organic phase washed with brine, dried over sodium sulphate and evaporated to give the title compound (0.1g, 34%).

MS (*m*/*z*): 271.2 [MH]⁺.

### Preparation 73: (1R,5S/1S,5R)-1-{4-[6-(Trifluoromethyl)-2-pyridinyl]phenyl}-3-azabicyclo[3.1.0]hexane

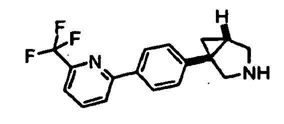

The title compound was prepared in analogy to the method described for Preparation 72 (using 2-bromo-6-(trifluoromethyl)pyridine) in 60% yield.

MS (*m*/*z*): 305.3 [MH]⁺.

### Preparation 74: (1R,5S/1S,5R)-1-[4-(3,5-Dimethyl-4-isoxazolyl)phenyl]-3-azabicyclo[3.1.0]hexane

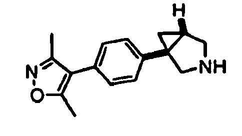

To a stirred solution of (1*R*,5*S*/1*S*,5*R*)-1,1-dimethylethyl 1-(4-bromophenyl)-3-azabicyclo[3.1.0]hexane-3-carboxylate (0.37 g) in toluene (5 mL) and ethyl alcohol (2 mL), at RT and under a nitrogen atmosphere, (3,5-dimethyl-4-isoxazolyl)boronic acid (0.25 g), tetrakis-(triphenylphosphin)-palladium(0) (0.03 g) and a saturated solution of potassium carbonate (2 mL) were subsequently added. The reaction mixture was heated at 88 °C for 2 h, and the solvents evaporated under vacuum. The crude product was treated with diethyl ether and water, the organic phase washed with brine, dried over sodium sulphate, concentrated under vacuum and extracted twice with ether. The solvent was evaporated and the crude product purified by chromatography over silica gel (cyclohexane/ETOAC 8/1). The recovered product was then dissolved in CH₂Cl₂ (10 mL) and trifluoroacetic acid was added (4 mL). After 3 h the reaction mixture was treated with solid sodium carbonate and the solvent evaporated. The residue was treated with water and extracted with CH₂Cl₂, the organic phase washed with brine, dried over sodium sulphate and evaporated to give the title compound (0.12 g, 45%).

MS (*m*/*z*): 255.2[MH]⁺.

### Preparation 75: (1R,5S/1S,5R)-1-[3-(2,4-Dimethyl-1,3-thiazol-5-yl)phenyl]-3-azabicyclo[3.1.0]hexane

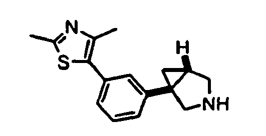

The title compound was prepared in analogy to the method described for Preparation 72, using (1*R*,5*S*/1*S*,5*R*)-1,1-dimethyfethyl 1-[3-(4,4,5,5-tetramethyl-1,3,2-dioxaborolan-2-yl)phenyl]-3-azabicyclo[3.1.0]hexane-3-carboxylate intermediate 4), in 50% yield.

MS (*m*/*z*): 271.3 [MH]⁺.

### Preparation 76: (1R,5S/1S,5R)-1-[3-(5-Methyl-2-thienyl)phenyl]-3-azabicyclo[3.1.0]hexane

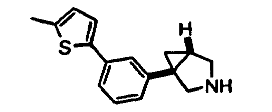

The title compound was prepared in analogy to the method described for Preparation 72, using (1*R*,5*S*/1*S*,5*R*)-1,1-dimethylethyl 1-[3-(4,4,5,5-tetramethyl-1,3,2-dioxaborolan-2-yl)phenyl]-3-azabicyclo[3.1.0]hexane-3-carboxylate intermediate 4), in 55% yield.

MS (*mlz*): 256.2[MH]⁺.

### Preparation 77: 5-(2,4-Dimethyl-1,3-oxazol-5-yl)-4-methyl-2,4-dihydro-3H-1,2,4-triazole-3-thione

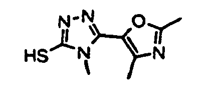

2,4-Dimethyl-1,3-oxazole-5-carboxylic acid (0.8 g), *N*-methylhydrazinecarboxamide (0.6 g), 1-(3-Dimethylaminopropyl)-3-ethyl carbodiimide hydrochloride (1.09 g), HOBt (0.038 g) and triethylamine (0.86 ml) were dissolved, under nitrogen, in dry DMF (15 ml) at room temperature. The mixture was stirred overnight, then DMF was removed under vacuum. NaOH (0.75M, 10 ml) was added and mixture was heated at 80 °C for 3 h. The reaction mixture was cooled to 0 °C and acidified to ca. pH 5 with HCl (aqueous, 37%). The suspended product was isolated by filtration, washing with water (2x3 ml). The cake was dried at room temperature overnight under vacuum to give the title compound in a 3:2 mixture with 2,4-dimethyl-1,3-oxazole-5-carboxylic acid as a solid foam (0.68 g, 57% yield).

NMR (¹H, CDCl₃): δ 3.80 (s, 3H), 2.60 (s, 3H), 2.40 (s, 3H), NH/SH not observed.

### Preparation 78: 3-[(3-Chloropropyl)thio]-5-(2,4-dimethyl-1,3-oxazol-5-yl)-4-methyl-4H-1,2,4-triazole

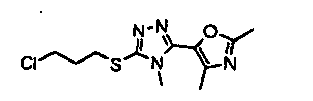

The product mixture from Preparation 77 was suspended in EtOH (10 ml). NaOEt (21% solution in EtOH, 1.14 ml) was added followed by 1-bromo-3-chloropropane (0.41 ml), the solution stirred at 90 °C for 45 min, then cooled to 25 °C. Acetic acid (0.1 eq.) was added than solvent was removed under vacuum. The solid was purified by silica gel column chromatography, eluting with cyclohexane/EtOAc to give the title compound as a solid foam (0.44 g, 54% yield).

NMR (¹H, CDCl₃): δ 3.70 (t+s, 5H), 3.35 (t, 2H), 2.50 (s, 3H), 2.4 (s, 3H), 2.30 (m, 2H). MS (*mlz*): 287 [MH]⁺.

### Example 1: 5-[5-({3-[(1R,5S/1S,5R)-1-(4-Methoxyphenyl)-3-azabicyclo[3.1.0]hex-3-yl]propyl}thio)-4-methyl-4H-1,2,4-triazol-3-yl]-2-methylquinoline hydrochloride

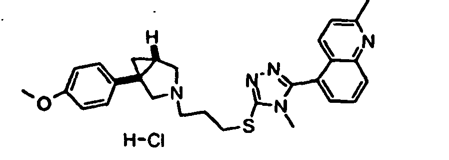

A mixture of (1*R*,5*S*/1*S*,5*R*)-1-[4-(methoxy)phenyl]-3-azabicyclo[3.1.0]hexane (Preparation 5, 42 mg), 5-{5-[(3-chloropropyl)thio]-4-methyl-4*H*-1.2,4-triazol-3-yl}-2-methylquinoline (0.26 mmol), Na₂CO₃ (0.44 mmol) and Nal (0.22 mmol) in DMF (anhydrous, 0.4 mL) was heated at 60 °C for 24 h. After elimination of the solvent under *vacuo*, the residue was dissolved in ethyl acetate and the organic layer was washed with saturated aqueous NaHCO₃ and dried over Na₂SO₄. This solution was filtered and the filtrate was concentrated *in vacuo.* The crude was purified by flash chromatography (dichloromethane to 10% MeOH in dichloromethane) to give 65 mg of the free base of the title compound. To a solution of this material in dichloromethane (0.2 mL) was added 0.14 mmol of HCl (1 M in Et₂O), the solvent evaporated under *vacuo* and the material thus obtained triturated with Et₂O to give 69 mg of the title compound as a white slightly hygroscopic solid (59% yield).

[The procedure may in analogy be adapted to other combinations of 1-substituted 3-azabicyclo[3.1.0]hexanes and 3-substituted 5-[(3-chloropropyl)thio]-4-methyl-4*H*-1,2,4-triazols. An equivalent molar amount of K₂CO₃ may be used to replace Na₂CO₃.]

NMR (¹H, DMSO): δ 10.57 (bs,1H), 8.28 (bs, 1H), 8.2 (d, 1H), 7.94 (t, 1H), 7.82 (d, 1H), 7.56 (d, 1H), 7.25 (d, 2H), 6.91 (d, 2H), 4.01 (dd, 1H), 3.7 (m, 1H), 3.74 (s, 3H), 3.6-3.2 (m, 6H), 3.42 (s, 3H), 2.75 (s, 3H), 2.24 (quint, 2H), 2.08 (quint, 1H), 1.62/1.05 (t/t, 2H). MS (*m*/*z*): 486.3[MH]⁺.

Example 1 was separated to give the separated enantiomers by semipreparative Supercritical Fluid Chromatography (Gilson) using a chiral column Chiralpak AD-H, 25 x 2.1 cm, eluent CO₂ containing 20% (ethanol + 0.1 % isopropanol), flow rate 25 mL/min, P 194 bar, T 35 °C, detection UV at 220 nm, loop 1 mL. Retention times given were obtained using an analytical Supercritical Fluid Chromatography (Gilson) using a chiral column Chiralpak AD-H, 25 x 0.46 cm, eluent CO₂ containing 20% (ethanol + 0.1% isopropanol), flow rate 2.5 mL/min, P 194 bar, T 35 °C, detection UV at 220 nm.

Enantiomer 1 was recovered in 15 mg yield as white solid (y=27%) from the racemate (60 mg). Rt. = 39.2 min.

Enantiomer 2 was recovered in 17 mg yield as white solid (y=30%) from the racemate (60 mg). Rt. = 43.4 min.

Enantiomer 1 showed fpKi (D3) > 1 log-unit higher than Enantiomer 2.

### Example 2: 5-[5-({3-[(1R,5S/1S,5R)-1-(4-Bromophenyl)-3-azabicyclo[3.1.0]hex-3-yl]propyl}thio)-4-methyl-4H-1,2,4-triazol-3-yl]-2-methylquinoline hydrochloride

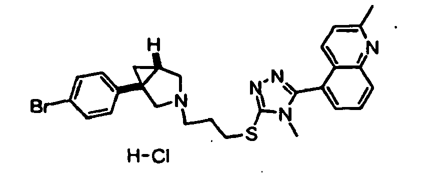

The title compound was prepared in analogy to the method described in Example 1 in 39 mg yield as a white slightly hygroscopic solid (y=40%) from (1*R*,5*S*/1*S*,5*R*)-1-(4-bromophenyl)-3-azabicyclo[3.1.0]hexane (40 mg).

NMR (¹H, DMSO): δ 10.28 (bs,1H), 8.16 (dd, 2H), 7.89 (dd, 1H), 7.76 (d, 1H), 7.55 (d, 2H), 7.49 (d, 1H), 7.28 (d, 2H), 4.06 (bm, 1H), 3.77 (bm, 1H), 3.6 (bm, 2H), 3.44 (s, 3H), 3.5-3.2 (bm, 4H), 2.71 (s, 3H), 2.23 (m, 3H), 1.58/1.14 (t/m, 2H). MS (*m*/*z*): 534.1[MH]⁺, 1 Br.

Example 2 was separated to give the separated enantiomers by semipreparative Supercritical Fluid Chromatography (Gilson) using a chiral column Chiralcel OJ-H, 25 x 2.1 cm, eluent CO₂ containing 12% (ethanol + 0.1% isopropylamine), flow rate 2.5 mL/min, P 196 bar, T 36 °C, detection UV at 220 nm, loop 1 mL. Retention times given were obtained using an analytical Supercritical Fluid Chromatography (Gilson) using a chiral column Chiralcel OJ-H, 25 x 0.46 cm, eluent CO₂ containing 10% (ethanol + 0.1% isopropylamine), flow rate 2.5 mL/min, P 196 bar, T 35 °C, detection UV at 220 nm. Enantiomer 1 was recovered in 7 mg yield as white solid, hydrochloride salt from the racemate (39 mg). Rt. = 56.8 min. Purity >99% a/a by UV.

Enantiomer 2 was recovered in 7 mg yield as white solid, hydrochloride salt from the racemate (39 mg). Rt. = 62.5 min. Purity >99% a/a by UV.

The absolute configuration of Enantiomer 1 was assigned using comparative VCD and comparative OR analyses of the corresponding free base to be 5-[5-({3-[(1*R*,5*S*)-1-(4-bromophenyl)-3-azabicyclo[3.1.0]hex-3-yl]propyl}thio)-4-methyl-4*H*-1,2,4-triazol-3-yl]-2-methylquinoline. (1*S*,5*R*)-1-(3,4-dichlorophenyl)-3-azabicyclo[3.1.0]hexane (see Preparation 48) was used used as the reference.

The absolute configuration of Enantiomer 2 was assigned as described for Enantiomer 1 to be 5-[5-({3-[(1*S*,5*R*)-1-(4-bromophenyl)-3-azabicycto[3.1.0]hex-3-yl]propyl}thio)-4-methyl-4H-1,2,4-triazol-3-yl]-2-methylquinoline.
Enantiomer 1: Specific Optical Rotation of the corresponding free base: [□]_{D} = +47 ° (CHCl₃, T = 20 °C, c = 0.066 g/mL).
Enantiomer 2: Specific Optical Rotation of the corresponding free base: [□]_{D} = -42 ° (CHCl₃, T = 20 °C, c = 0.065 g/mL).
Enantiomer 2 showed fpKi (D3) > 1 log-unit higher than Enantiomer 1.

### Example 3: 2-Methyl-5-[4-methyl-5-({3-[(1R,5S/1S,5R)-1-phenyl-3-azabicyclo[3.1.0]hex-3-yl]propyl}thio)-4H-1,2,4-triazol-3-yl]quinoline hydrochloride

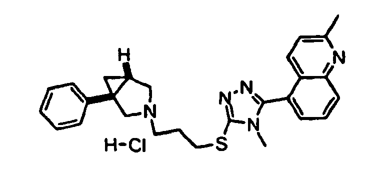

The title compound was prepared in analogy to the method described in Example 1 in 74 mg yield as a white slightly hygroscopic solid (y=59%) from (1*R*,5*S*/1*S*,5*R*)-1-phenyl-3-azabicyclo[3.1.0]hexane (40 mg).

NMR (¹H, DMSO): δ 10.4 (bs,1H), 8.3 (bs, 1H), 8.2 (d, 1H), 7.9 (t, 1H), 7.8 (d, 1H), 7.6 (bd, 1H), 7.4-7.3 (m, 5H), 4.0-3.5 (m/m, 2H), 3.7-3.45 (m/m, 2H), 3.5-3.3 (m, 7H), 2.73 (s, 3H), 2.3 (m, 3H), 1.60,1.1 (t,t 2H). MS (*m*/*z*): 456.3[MH]⁺.

Example 3 was separated to give the separated enantiomers by semi-preparative HPLC using a chiral column Chiralcel OD 10 µm, 250 x 20 mm, eluent A: n-hexane; B: isopropanol, gradient isocratic 35% B, flow rate 7 mL/min, detection UV at 200-400 nm, CD 230 nm. Retention times given were obtained using an analytical HPLC using a chiral column Chiralcel OD 5 µm, 250 x 4.6 mm, eluent A: n-hexane; B: isopropanol, gradient isocratic 25% B, flow rate 1 mL/min, detection UV at 200-400 nm.

Enantiomer 1 was recovered in 15 mg yield as white solid (y=27%) from the racemate (60 mg). Rt. = 39.2 min.

Enantiomer 2 was recovered in 17 mg yield as white solid (y=30%) from the racemate (60 mg). Rt. = 43.4 min.

Enantiomer 2 showed fpKi (D3) > 1 log-unit higher than Enantiomer 1.

### Example 4: 5-[5-({3-[(1R,5S/1S,5R)-1-(3,4-Dichlorophenyl)-3-azabicyclo[3.1.0]hex-3-yl]propyl}thio)-4-methyl-4H-1,2,4-triazol-3-yl]-2-methylquinoline hydrochloride

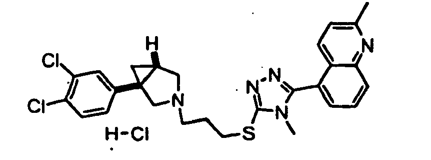

The title compound was prepared in analogy to the method described in Example 1 in 65 mg yield as a white slightly hygroscopic solid (y=52%) from (1*R*,5*S*/1*S*,5*R*)-1-(3,4-dichlorophenyl)-3-azabicyclo[3.1.0]hexane (50 mg).

NMR (¹H, DMSO): δ 10.6 (s,1H), 8.32 (bs, 1H), 8.21 (d, 1H), 7.96 (d, 1H), 7.84 (d, 1H), 7.66 (d, 1H), 7.61 (d, 1H), 7.6 (d, 1H), 7.31 (dd, 1H), 4.06 (m, 2H), 3.74 (m, 2H), 3.7-3.2 (m, 4H), 3.36 (s, 3H), 2.76 (s, 3H), 2.25 (m, 4H), 1.69 (m, 1H), 1.2 (m, 1H). MS (*mlz*): 524.3[MH]⁺, 2Cl.

Example 4 was separated to give the separated enantiomers by semipreparative Supercritical Fluid Chromatography (Gilson) as described in Example 1.

Enantiomer 1 was recovered in 19 mg yield as white solid (y=36%) from the racemate (56 mg). Rt. = 26.9 min.

The absolute configuration of Enantiomer 1 was assigned using comparative VCD and comparative OR analyses of the corresponding free base to be 5-[5-({3-[(1*S*,5*R*)-1-(3,4-dichlorophenyl)-3-azabicydo[3.1.0]hex-3-yl] propyl}thio)-4-methyl-4*H*-1,2,4-triazol-3-yl]-2-methylquinoline. (1*S*,5*R*)-1-(3,4-dichlorophenyl)-3-azabicyclo[3.1.0]hexane (see Preparation 48) was used used as the reference.

The absolute configuration of Enantiomer 2 was assigned as described for Enantiomer 1 to be 5-[5-({3-[(1*R*,5*S*)-1-(3,4-dichlorophenyl)-3-azabicyclo[3.1.0]hex-3-yl]propyl}thio)-4-methyl-4*H*-1,2,4-triazol-3-yl]-2-methylquinoline.
Enantiomer 1: Specific Optical Rotation of the corresponding free base: [□]_{D} = -38.4 ° (CDCl₃, T = 20 °C, c = 0.010 g/mL).
Enantiomer 2 was recovered in 14 mg yield as white solid (y=26%) from the racemate (56 mg). Rt. = 31.4 min.
Enantiomer 2: Specific Optical Rotation of the corresponding free base: [□]_{D} = +34.4 ° (CDCl₃, T = 20 °C, c = 0.010 g/mL).

Enantiomer 1 showed fpKi (D3) > 0.6 log-unit higher than Enantiomer 2.

### Example 5: 5-[5-({3-[(1R,5S/1S,5R)-1-(4-tert-Butylphenyl)-3-azabicyao[3.1.0]hex-3-yl]propyl}thio)-4-methyl-4H-1,2,4-triazol-3-yl]-2-methylquinoline hydrochloride

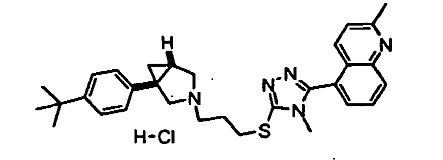

The title compound was prepared in analogy to the method described in Example 1 in 38 mg yield as a white slightly hygroscopic solid (y=51%) from (1*R*,5*S*/1*S*,5*R*)-1-(4-*tert-*butylphenyl)-3-azabicyclo[3.1.0]hexane (29 mg).

NMR (¹H, DMSO): δ 10.16 (bs,1H), 8.15 (dd, 2H), 7.89 (t, 1H), 7.76 (d, 1H), 7.49 (d, 1H), 7.36 (d, 2H), 7.23 (d, 2H), 4.05 (dd, 1H), 3.77 (dd, 1H), 3.58 (m, 2H), 3.44 (s, 3H), 2.7 (bm, 4H), 2.34 (s, 3H), 2.23 (t, 2H). 2.15 (t, 1H), 1.51 (t, 1H), 1.27 (s, 9H), 1.14 (m, 1H). MS (*mlz*): 512.4 [MH]⁺.

### Example 5 was separated to give the separated enantiomers by semipreparative Supercritical Fluid Chromatography (Gilson) as described in Example 1 but applying a pressure of 200 bar instead of 194 bar.

Enantiomer 1 was recovered in 6.5 mg yield as white solid (y=30%) from the racemate (23 mg). Rt. = 7.0 min.
Enantiomer 2 was recovered in 5 mg yield as white solid (y=23%) from the racemate (23 mg). Rt. = 7.8 min.
Enantiomer 2 showed fpKi (D3) > 0.9 log-unit higher than Enantiomer 1.

### Example 6: 4-[(1R,5S/1S,5R)-3-(3-{[4-Methyl-5-(2-methylquinolin-5-yl)-4H-1,2,4-triazol-3-yl]thio}propyl)-3-azabicycto[3.1.0]hex-1-yl]benzonitrile hydrochloride

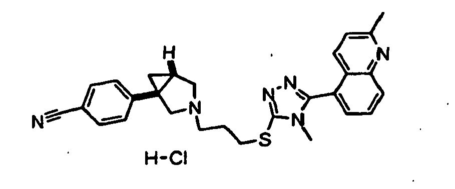

The title compound was prepared in analogy to the method described in Example 1 in 19 mg yield as a white slightly hygroscopic solid (y=27%) from (1*R*,5*S*/1*S*,5*R*)-1-(4-cyanophenyl)-3-azabicyclo[3.1.0]hexane (25 mg).

NMR (¹H, DMSO): δ 10.45 (bs,1H), 8.26 (bd, 1H), 8.17 (d, 1H), 7.93 (t, 1H), 7.8 (d/d, 3H), 7.5 (d, 1H), 7.46 (d, 2H), 4.09 (d, 1H), 3.76 (d, 1H), 3.67 (t, 1H), 3.6-3.2 (bm, 5H), 3.43 (s, 3H), 2.73 (s, 3H), 2.34 (m, 1H), 2.25 (quint., 2H),1.71/1.22 (dt, 2H).

### Example 7:4-[(1R,5S/1S,5R)-3-(3-{[4-Methyl-5-(2-methylquinolin-5-yl)-4H-1,2,4-triazol-3-yl]thio}propyl)-3-azabicyclo[3.1.0]hex-1-yl]phenol hydrochloride

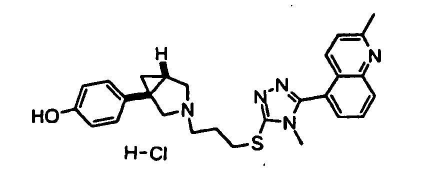

The title compound was prepared in analogy to the method described in Example 1 in 10 mg yield as a white slightly hygroscopic solid (y=11%) from (1*R*,5*S*/1*S*,5*R*)-1-(4-hydroxyphenyl)-3-azabicyclo[3.1.0]hexane (38 mg).

NMR (¹H, DMSO): δ 10.17 (bs,1H), 9.4 (s,1H), 8.15 (bd, 2H), 7.89 (d, 1H), 7.75 (d, 1H), 7.48 (d, 1H), 7.12(d, 2H), 6.73 (d, 2H), 3.98 (dd, 1H), 3.74 (m, 1H), 3.5 (bm, 2H), 3.44 (s, 3H), 3.5-3.2 (bm, 4H), 2.7 (s, 3H), 2.22 (bquint. 2H), 2.03 (m, 1H),1.46/1.03 (dm, 2H). MS (*m*/*z*): 486.2[MS]⁺.

### Example 8: (1R,5S/1S,5R)-3-(3-{[4-methyl-5-(4-methyl-1,3-oxazol-5-yl)-4H-1,2,4-triazol-3-yl]thio}propyt)-1-phenyl-3-azabicyclo[3.1.0]hexane hydrochloride

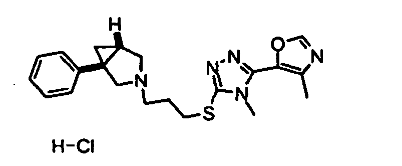

The title compound was prepared in analogy to the method described in Example 1 in 75 mg yield as a white slightly hygroscopic solid (y=70%) from (1*R*,5*S*/1*S*,5*R*)-1-phenyl-3-azabicyclo[3.1.0]hexane (40 mg).

NMR (¹H, DMSO): δ 10.46 (bs,1H), 8.58 (s, 1H), 7.4-7.2 (m, 5H), 4.04 (dd, 1H), 3.73 (m, 1H), 3.7 (s, 3H), 3.7-3.4 (m, 2H), 3.4-3.2 (m+t, 4H), 2.39 (s, 3H), 2.17 (m, 3H), 1.64,1.1 (2t, 2H).

### Example 9: (1R,5S/1S,5R)-1-(4-Bromophenyl)-3-(3{[4-methyl-5-(4-methyl-1,3-oxazol-5-yl)-4H-1,2,4-triazol-3-yl]thio}propyl)-3-azabicyclo[3.1.0]hexane hydrochloride

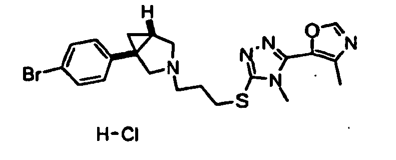

The title compound was prepared in analogy to the method described in Example 1 in 24 mg yield as a white slightly hygroscopic solid (y=28%) from (1*R*,5*S*/1*S*,5*R*)-1-(4-bromophenyl)-3-azabicyclo[3.1.0]hexane (40 mg).

NMR (¹H, DMSO): δ 10.29 (bs,1H), 8.58 (s, 1H), 7.55 (dd, 2H), 7.27 (dd, 2H), 4.03 (dd, 1H), 3.73 (dd, 1H), 3.7 (s, 3H), 3.55 (m, 2H), 3.5-3.2/3.28 (m+t, 4H), 2.39 (s, 3H), 2.19 (m, 3H), 1.59/1.12 (2t, 2H). MS (*m*/*z*): 474.1 [MH]⁺.

Example 9 was separated to give the separated enantiomers by semipreparative Supercritical Fluid Chromatography (Gilson) using a chiral column Chiralcel AS-H, 25 x 2.1 cm, eluent CO₂ containing 11 % (ethanol + 0.1 % isopropylamine), flow rate 22 mL/min, P 192 bar, T 36 °C, detection UV at 220 nm, loop 2 mL. Retention times given were obtained using an analytical Supercritical Fluid Chromatography (Gilson) using a chiral column Chiralpak AS-H, 25 x 0.46 cm, eluent CO₂ containing 10% (ethanol + 0.1% isopropyilamine), flow rate 2.5 mL/min, P 199 bar, T 35 °C, detection UV at 220 nm.

Enantiomer 1 was recovered in 59 mg yield as white solid, hydrochloride salt from the racemate (138 mg). Rt. = 22.2 min. Purity >99% a/a by UV.

Enantiomer 2 was recovered in 50 mg yield as white solid, hydrochloride salt from the racemate (138 mg). Rt. = 30.8 min. Purity >99% a/a by UV.

The absolute configuration of Enantiomer 1 was assigned using comparative VCD and comparative OR analyses of the corresponding free base to be (1*S*,5*R*)-1-(4-bromophenyl)-3-(3-{[4-methyl-5-(4-methyl-1,3-oxazol-5-yl)-4*H*-1,2,4-triazol-3-yl]thio}propyl)-3-azabicyclo[3.1.0]hexane. (1*R*,5*S*)-1-(4-Bromophenyl)-3-azabicyclo[3.1.0]hexane (compare Preparation 32) was used used as the reference.

The absolute configuration of Enantiomer 2 was assigned as described for Enantiomer 1 to be (1*R*,5*S*)-1-(4-bromophenyl)-3-(3-{[4-methyl-5-(4-methyl-1,3-oxazol-5-yl)-4*H*-1,2,4-triazol-3-yl]thio}propyl)-3-azabicyclo[3.1.0]hexane.
Enantiomer 1: Specific Optical Rotation of the corresponding free base: [□]_{D} = -51 ° (CHCl₃, T = 20 °C, c = 0.00913 g/mL).
Enantiomer 2: Specific Optical Rotation of the corresponding free base: [□]_{D} = +27 ° (CHCl₃, T = 20 °C, c = 0.0113 g/mL).
Enantiomer 1 showed fpKi (D3) > 1 log-unit higher than Enantiomer 2.

### Example 10: (1R,5S/1S,5R)-1-(4-tert-Butylphenyl)-3-(3-{[4-methyl-5-(4-methyl-1,3-oxazol-5-yl)-4H-1,2,4-triazol-3-yl]thio}propyl)-3-azabicydo[3.1.0]hexane hydrochloride

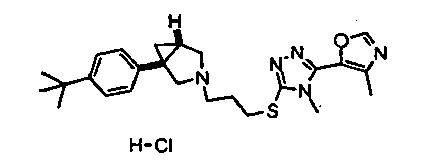

The title compound was prepared in analogy to the method described in Example 1 in 52 mg yield as a white slightly hygroscopic solid (y=57%) from (1*R*,5*S*/1*S*,5*R*)-1-(4-*tert-*butylphenyl)-3-azabicyclo[3.1.0]hexane (40 mg).

NMR (¹H, CD3OD): δ 8.4 (s, 1H), 7.42 (d, 2H), 7.28 (d, 2H), 4.11 (d, 1H), 3.88 (d, 1H), 3.8 (s, 3H), 3.65 (m, 2H), 3.43 (t, 2H), 3.39 (t, 2H), 2.47 (s, 3H), 2.29 (m, 2H), 2.21 (m, 1H), 1.44 (m, 1H), 1.33 (s, 9H), 1.3 (m, 1H). MS (*m*/*z*): 452.3[MH]⁺.

### Example 11: (1R,5S/1S,5R)-1-(3,4-Dichlorophenyl)-3-(3-{[4-methyl-5-(4-methyl-1,3-oxazol-5-yl)-4H-1,2,4-triazol-3-yl]thio}propyl)-3-azabicyclo[3.1.0]hexane hydrochloride

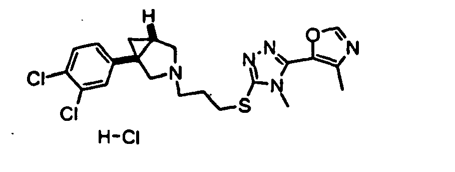

The title compound was prepared in analogy to the method described in Example 1 in 35 mg yield as a white slightly hygroscopic solid (y=32%) from (1*R*,5*S*/1*S*,5*R*)-1-(3,4-dichlorophenyl)-3-azabicyclo[3.1.0]hexane (50 mg).

NMR (¹H, DMSO): δ 10.11 (vbs, 1H), 8.58 (s, 1H), 7.6 (d+d, 2H), 6.29 (dd, 1H), 4.04/3.74 (2dd, 2H), 3.7 (s, 3H), 3.6-3.2 (m, 4H), 3.28 (t, 2H), 2.39 (s, 3H), 2.26 (quint, 1H), 2.15 (quint., 2H), 1.53/1.2 (2t, 2H). MS (*m*/*z*): 464.1[MH]⁺, 2Cl.

Example 11 was separated to give the separated enantiomers by semipreparative Supercritical Fluid Chromatography (Gilson) using a chiral column Chiralpak AS-H, 25 x 2.1 cm, eluent CO₂ containing 8% (ethanol + 0.1% isopropylamine), flow rate 22 mL/min, P 194 bar, T 36 °C, detection UV at 220 nm, loop 1 mL. Retention times given were obtained using an analytical Supercritical Fluid Chromatography (Gilson) using a chiral column Chiralpak AS-H, 25 x 0.46 cm, eluent CO₂ containing 8% (ethanol + 0.1% isopropylamine), flow rate 2.5 mL/min, P 190 bar, T 35 °C, detection UV at 220 nm.

Enantiomer 1 was recovered in 12.5 mg yield as white solid, hydrochloride salt from the racemate (29 mg). Rt. = 38.0 min. Purity 98.6% a/a by UV.

Enantiomer 2 was recovered in 12.5 mg yield as white solid, hydrochloride salt from the racemate (29 mg). Rt. = 40.8 min. Purity 98.6% a/a by UV.
Enantiomer 1 showed fpKi (D3) > 0.5 log-units higher than Enantiomer 2.

### Example 12: (1R,5S/1S,5R)-1-(4-methoxyphenyl)-3-(3{[4-methyl-5-(4-methyl-1,3-oxazol-5-yl)-4H-1,2,4-triazo-3-yl]thio}propyl)-3-azabicyclo[3.1.0]hexane hydrochloride

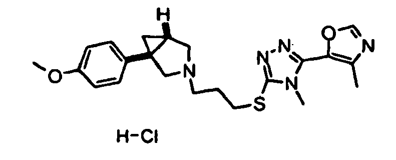

The title compound was prepared in analogy to the method described in Example 1 in 38 mg yield as a white slightly hygroscopic solid (y=39%) from (1*R*,5*S*/1*S*,5*R*)-1-(4-methoxyphenyl)-3-azabicyclo[3.1.0]hexane (40 mg).

NMR (¹H, DMSO): δ 10.18 (bs, 1H), 8.58 (s, 1H), 7.24 (d, 2H), 6.91 (d, 2H), 3.97 (dd, 1H), 3.74 (s, 3H), 3.7 (s, 3H), 3.7 (m, 1H), 3.6-3.2 (m, 4H), 3.27 (t, 2H), 2.39 (s, 3H), 2.15 (quint, 2H), 2.07 (quint., 1H), 1.49/1.05 (2t, 2H). MS (*mlz*)*:* 426.2[MH]⁺.

Example 12 was separated to give the separated enantiomers by semipreparative Supercritical Fluid Chromatography (Gilson) using a chiral column Chiralpak AS-H, 25 x 2.1 cm, eluent CO₂ containing 9% (ethanol + 0.1% isopropylamine), flow rate 22 mL/min, P 192 bar, T 36 °C, detection UV at 220 nm, loop 1 mL. Retention times given were obtained using an analytical Supercritical Fluid Chromatography (Gilson) using a chiral column Chiralpak AS-H, 25 x 0.46 cm, eluent CO₂ containing 8% (ethanol + 0.1% isopropylamine), flow rate 2.5 mL/min, P 190 bar, T 35 °C, detection UV at 220 nm.
Enantiomer 1 was recovered in 5 mg yield as white solid, hydrochloride salt from the racemate (30 mg). Rt. = 28.7 min. Purity >99% a/a by UV.
Enantiomer 2 was recovered in 12.5 mg yield as white solid, hydrochloride salt from the racemate (30 mg). Rt. = 36.4 min. Purity >99% a/a by UV.
Enantiomer 1 showed fpKi (D3) > 1 log-unit higher than Enantiomer 2.

### Example 13: (1R,5S/1S,5R)-1-[4-(5-methyl-3-isoxazolyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane hydrochloride

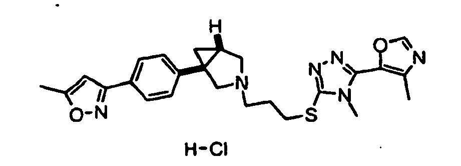

The title compound was prepared in analogy to the method described in Example 1 in 30 mg yield as a white slightly hygroscopic solid (y=25%) from (1*R*,5*S*/1*S*,5*R*)-1-[4-(5-methyl-3-isoxazolyl)phenyl]-3-azabicyclo[3.1.0]hexane (55 mg).

NMR (¹H, CD₃OD): δ 8.37 (s, 1H), 7.8 (d, 2H), 7.43 (d, 2H), 6.55 (s, 1H), 4.16/3.88 (2d, 2H), 3.78 (s, 3H), 3.7 (m, 2H), 3.48-3.4 (2t, 4H), 2.48 (s, 3H), 2.45 (s, 3H), 2.29 (m, 3H), 1.51/1.37 (2t, 2H). MS (*m*/*z*): 477.2[MH]⁺.

### Example 14: (1S,5R)-3-(3{[4-Methyl-5-(4-methyl-1,3-oxazol-5-yl)-4H-1,2,4-triazol-3-yl]thio}propyl)-1-[4-(trifluoromethyl)phenyl]-3-azabicyclo[3.1.0]hexane

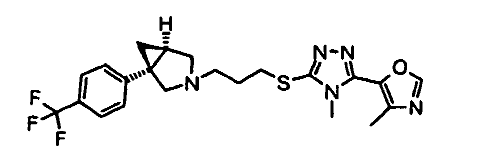

A mixture of (1*S*,5*R*)-1-[4-(trifluoromethyl)phenyl]-3-azabicyclo[3.1.0]hexane (Preparation 18, 10.4 g), 3-[(3-chloropropyl)thio]-4-methyl-5-(4-methyl-1,3-oxazol-5-yl)-4*H*-1,2,4-triazole (Preparation 14, 15.0 g), K₂CO₃ (7.5 g) and Nal (8.23 g) in DMF (anhydrous, 100 mL) were heated at 60 °C for 15 h. The mixture was then allowed to cool to room temperature, diluted with Et₂O (250 mL) and water (200 mL). After separation of the two phases, the aqueous layer was extracted again with Et₂O (2 x 200 mL). The combined organic layers were washed with water (2 x 150 mL) and then dried over Na₂SO₄. After evaporation of the solvent *in vacuo,* the crude product was purified by flash chromatography (dichloromethane to 10% MeOH in dichloromethane) to give 16.5 g of a yellow solid. The material thus obtained was triturated with Et₂O to provide the title compound (13 g) as white solid (y = 61%).

Assignment of the configuration of the title compound is based on two lines of evidence: The fact that it was prepared from (1*S*,5*R*)-1-[4-(trifluoromethyl)phenyl]-3-azabicyclo[3.1.0]hexane (of known configuration, see Preparation 14) and by comparison with the spectroscopic data obtained for (1*S*,5*R*)-1-[4-(trifluoromethyl)-phenyl]-3-azabicyclo[3.1.0]hexane: Bands in the VCD spectrum of the title compound are coincident with the corresponding bands in the spectrum of (1*S*,5*R*)-1-[4-(trifluoromethyl)phenyl]-3-azabicyclo[3.1.0]hexane, additionally the sign of the specific rotation is the same for both compounds.

NMR (¹H, CDCl₃): δ 7.89 (m, 1H), 7.49 (d, 2H), 7.18 (d, 2H), 3.67 (s, 3H), 3.31 (m, 2H), 3.30 (d, 1H), 3.09 (d, 1H), 2.61 (m, 2H), 2.56 (d, 1H), 2.5 (s, 3H), 2.45 (d, 1H), 1.97 (m, 2H), 1.73 (m, 1H), 1.47 (t, 1H), 0.8 (dd, 1H). MS (*m*/*z*): 464 [MH]⁺.

### Example 15: (1S,5R)-3-(3-{[4-Methyl-5-(4-methyl-1,3-oxazol-5-yl)-4H-1,2,4-triazol-3-yl]thio}propyl)-1-[4-(trifluoromethyl)phenyl]-3-azabicyclo[3.1.0]hexane hydrochloride

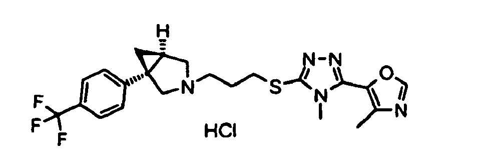

Hydrochloric acid (1M solution in Et₂O; 19.4 mL) was added dropwise under N₂ to a solution of (1*S*,5*R*)-3-(3-{[4-methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane (Example 14, 9 g) in Et₂O (anhydrous, 135 mL). The resulting suspension was allowed to stir at room temperature for 2 h. The solid was then filtered, washed with Et₂O and dried *in vacuo* overnight to provide the title compound (8.9 g) as off white solid (y = 92%).

NMR (¹H, DMSO): δ 10.16 (bs, 1H), 8.58 (s, 1H), 7.72 (d, 2H), 7.51 (d, 2H), 4.1 (dd, 1H), 3.78 (dd, 1H), 3.70 (s, 3H), 3.66 (m, 2H), 3.29 (t, 2H), 2.5 (bm, 2H), 2.39 (s, 3H), 2.33 (quint, 2H), 2.19 (m, 1H), 1.62/1.23 (t/t. 2H). MS (*m*/*z*): 464 [MH]⁺.

**Example 16: (1*R*,5*S*/1*S*,5*R*)-1-[2-Fluoro-4-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]-hexane hydrochloride**

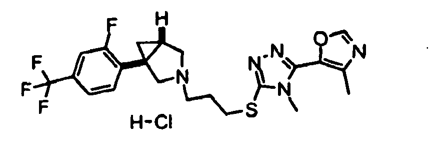

A mixture of (1*R*,5*S*/1*S*,5*R*)-1-[2-Fluoro-4-(trifluoromethyl)phenyl]-3-azabicyclo-[3.1.0]hexane (Preparation 38, 700 mg, 2.8 mmol), 3-[(3-Chloropropyl)thio]-4-methyl-5-(4-methyl-1,3-oxazol-5-yl)-4*H*-1,2,4-triazole (Preparation 14, 3.4 mmol), Na₂CO₃ (3.4 mmol) and Nal (3.4 mmol) in DMF (anhydrous, 6 mL) was heated at 60 °C for 24 h. After elimination of the solvent under *vacuo,* the residue was dissolved in ethyl acetate and the organic layer was washed with saturated aqueous NaHCO₃ and dried over Na₂SO₄. This solution was filtered and the filtrate was concentrated *in vacuo*. The crude was purified by flash chromatography (dichloromethane to 10% MeOH in dichloromethane) to give 503 mg of the free base of the title compound.

NMR (¹H, CDCl₃): δ 7.89 (s, 1H), 7.32-7.2 (m, 3H), 3.70 (s, 3H), 3.30 (t, 2H), 3.26 (dd, 1H), 3.10 (dd, 1H), 2.60 (t, 2H), 2.52 (dd, 1H), 2.51 (s, 3H), 2.43 (dd, 1H), 1.94 (m, 2H), 1.74 (m, 1H), 1.40 (t, 1H), 0.76 (dd, 1H). MS (*m*/*z*): 482.2[MH]⁺.

The title compound was obtained as a white solid following the method described for Example 15.

NMR (¹H, DMSO): δ 10.28 (bs,1H), 8.58 (s, 1H), 7.73 (d, 1H), 7.6 (m, 2H), 4/ 3.57 (d/m, 2H), 3.79 (d, 1H), 3.69 (s, 3H), 3.5-3.2 (vbm, 1H), 3.27 (t, 2H), 2.5 (m, 2H), 2.4 (m, 1H), 2.38 (s, 3H), 2.14 (quint., 2H), 1.62/1.16 (2t, 2H).

### Example 16 was separated to give the separated enantiomers by semi-preparative HPLC using a chiral column Chiralpak AD 10 µm, 250 x 21 mm, eluent A: n-hexarie; B: isopropanol + 0.1% isopropyl amine, gradient isocratic 9% B, flow rate 7 mUmin, detection UV at 200-400 nm. Retention times given were obtained using an analytical HPLC using a chiral column Chiralpak AD-H 5 µm, 250 x 4.6 mm, eluent A: n-hexane; B: isopropanol, gradient isocratic 15% B, flow rate 0.8 mL/min, detection UV at 200-400 nm.

Enantiomer 1 was recovered as white solid, Rt. = 15.4 min.
Enantiomer 2 was recovered as white solid, Rt. = 16.3 min.
Enantiomer 2 showed fpKi (D3) > 1 log-unit higher than Enantiomer 1.

### Example 17: (1R,5S/1S,5R)-3-(3{[4-Methyl-5-(4-methyl-1,3-oxazol-5-yl)-4H-1,2,4-triazol-3-yl]thio}propyl)-1-[3-(trifluoromethyl)phenyl]-3-azabicyclo[3.1.0]hexane hydrochloride

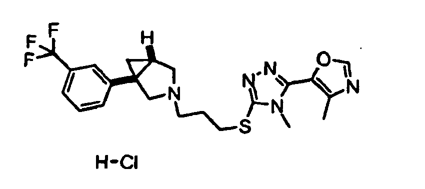

(1*R*,5*S*/1*S*,5*R*)-1-[3-(Trifluoromethyl)phenyl]-3-azabicyclo[3.1.0]hexane was prepared in analogy to the method described in Preparations 15, 16 and 17. From this material the title compound was obtained as a white slightly hygroscopic solid following the method described for Examples 14 and 15.

NMR (¹H, DMSO): δ 10.5 (bs,1H), 8.58 (s, 1H), 7.7-7.5 (m, 4H), 4.09 (m, 1H), 3.8-3.2 (m, 8H), 3.29 (t, 2H), 2.39 (s, 3H), 2.3 (m, 1H), 2.18 (m, 2H), 1.68 (t, 1H), 1.21 (t, 1H). MS (*m*/*z*): 464 [MH]⁺.

Example 17 was separated to give the separated enantiomers by semipreparative Supercritical Fluid Chromatography (Gilson) using a chiral column Chiralpak AD-H. 25 x 0.46 cm, eluent CO₂ containing 10% (ethanol +0.1% isopropanol), flow rate 2.5 mL/min, P 180 bar, T 35 °C, detection UV at 220 nm, loop 1 mL. Retention times given were obtained using an analytical Supercritical Fluid Chromatography (Gilson) using a chiral column Chiralpak AD-H, 25 x 0.46 cm, eluent CO₂ containing 10% (ethanol + 0.1% isopropanol), flow rate 22 mL/min, P 190 bar, T 36 °C, detection UV at 220 nm.
Enantiomer 1 was recovered as white solid, Rt. = 17.6 min.
Enantiomer 2 was recovered as white solid, Rt. = 18.4 min.
Enantiomer 1 showed fpKi (D3) > 1 log-unit higher than Enantiomer 2.

### Example 18: (1R,5S/1S,5R)-1-[4-Fluoro-3-(trifluoromethyl)phenyl]-3-(3-{[4-methyl-5-(4-methyl-1,3-oxazol-5-yl)-4H-1,2,4-triazol-3-yl]thio}propyl)-3-azabicyclo[3.1.0]-hexane hydrochloride

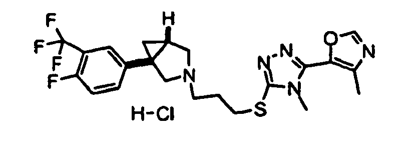

(1*R*,5*S*/1*S*,5*R*)-1-[4-Fluoro-3-(trifluoromethyl)phenyl]-3-azabicyclo[3.1.0]hexane was prepared in analogy to the method described in Preparations 15, 16 and 17. From this material the title compound was obtained as a white slightly hygroscopic solid following the method described for Examples 14 and 15.

NMR (¹H, DMSO): δ 10.2 (bs, 1H), 8.58 (s, 1H), 7.75 (dm, 1H), 7.72 (m, 1H), 7.53 (t, 1H), 4.06 (dd, 1H), 3.74 (dd, 1H), 3.7 (s, 3H), 3.6 (m, 2H), 3.4 (m, 2H), 3.28 (t, 2H), 2.39 (s, 3H), 2.26 (m, 1H), 2.18 (m, 2H), 1.54 (t, 1H), 1.22 (dd, 1H). MS (*m*/*z*): 481 [MH]⁺.

### Example 19: 1-[5-[(1S,5R/1R,5S)-3-(3-{[4-Methyl-5-(4-methyl-1,3-oxazol-5-yl)-4H-1,2,4-triazol-3-yl]thio}propyl)-3-azabicyclo[3.1.0]hex-1-yl]-2-(methyloxy)phenyl]ethanone hydrochloride

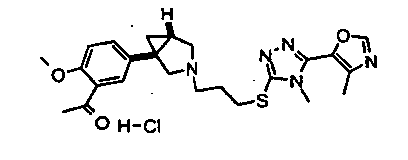

The title compound was prepared in analogy to the method described in Example 1 in 25 mg yield as a white slightly hygroscopic solid from (1*R*,5*S*/1*S*,5*R*)-1-[5-(3-azabicyclo[3.1.0]hex-1-yl)-2-(methyloxy)phenyl]ethanone (32 mg).

NMR (¹H, DMSO): δ 10.31 (bs,1H), 8.58 (s, 1H), 7.52 (d, 1H), 7.49 (dd, 1H), 7.16 (d, 1H), 3.98 (dd, 1H), 3.89 (s, 3H), 3.7 (m, 4H), 3.6-3.2 (bm, 4H), 3.27 (t, 2H), 2.5 (m, 3H), 2.39 (s, 3H), 2.15 (quint, 2H), 2.09 (quint, 1H), 1.54-1.08 (2t, 2H). MS (*m*/*z*): 468[MH]⁺.

Example 19 was separated to give the separated enantiomers by semipreparative Supercritical Fluid Chromatography (Gilson) using a chiral column Chiralpak AS-H, 25 x 2.1 cm, eluent CO₂ containing 15% (ethanol + 0.1% isopropylamine), flow rate 22 mL/min, P 196 bar, T 36 °C, detection UV at 220 nm, loop 1 mL. Retention times given were obtained using an analytical Supercritical Fluid Chromatography (Gilson) using a chiral column Chiralpak AS-H, 25 x 0.46 cm, eluent CO₂ containing 15% (ethanol + 0.1% isopropylamine), flow rate 2.5 mL/min, P 190 bar, T 35 °C, detection UV at 220 nm.
Enantiomer 1 was recovered in 14 mg yield as white solid, hydrochloride salt from the racemate (40 mg). Rt. = 12.5 min. Purity >99% a/a by UV.
Enantiomer 2 was recovered in 16 mg yield as white solid, hydrochloride salt from the racemate (40 mg). Rt. = 16.8 min. Purity >99% a/a by UV.
Enantiomer 1 showed fpKi (D3) > 1 log-unit higher than Enantiomer 2.

### Example 20: (1S,5R/1R,5S)-1-(4-Chlorophenyl)-3-(3-{[4-methyl-5-(4-methyl-1,3-oxazol-5-yl)-4H-1,2,4-triazol-3-yl]thio}propyl)-3-azabicyclo[3.1.0]hexane hydrochloride

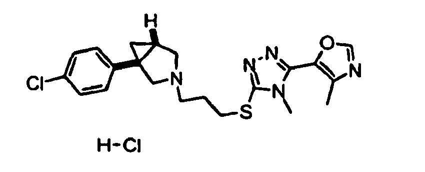

The title compound was prepared in analogy to the method described in Example 1 in 99 mg yield as a white slightly hygroscopic solid from (*1R,5S*/*1S,5R*)-1-(4-chlorophenyl)-3-azabicyclo[3.1.0]hexane (58 mg). NMR (¹H, DMSO): δ 9.93(bs,1H), 8.58 (s, 1H), 7.42 (d, 2H), 7.33 (d, 2H), 4.04 (dd, 1H), 3.75 (dd, 1H), 3.7 (s, 3H), 3.5 (m, 2H), 3.3 (bm, 4H), 2.39 (s, 3H), 2.2 (m, 1H), 2.15 (m, 2H), 1.47-1.14 (2t, 2H). MS (*m*/*z*): 431[MH]⁺.

Example 20 was separated to give the separated enantiomers by semipreparative Supercritical Fluid Chromatography (Gilson) using a chiral column Chiralpak AS-H, 25 x 2.1 cm, eluent CO₂ containing 15% (ethanol + 0.1% isopropylamine), flow rate 22 mL/min, P 192 bar, T 36°C, detection UV at 220 nm, loop 1 mL. Retention times given were obtained using an analytical Supercritical Fluid Chromatography (Gilson) using a chiral column Chiralpak AS-H, 25 x 0.46 cm, eluent CO₂ containing 15% (ethanol + 0.1% isopropylamine), flow rate 2.5 mL/min, P 190 bar, T 35°C, detection UV at 220 nm.
Enantiomer 1 was recovered in 17 mg yield as white solid, hydrochloride salt from the racemate (40 mg). Rt. = 7.8 min. Purity >99% a/a by UV.
Enantiomer 2 was recovered in 17 mg yield as white solid, hydrochloride salt from the racemate (40 mg). Rt. = 9.7 min. Purity >99% a/a by UV. ,

The absolute configuration of Enantiomer 1 was assigned using comparative VCD and comparative OR analyses of the corresponding free base to be (1*S*,5*R*)-1-(4-chlorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane. 5-[5-({3-[(1*R*,5*S*)-1-(4-Bromophenyl)-3-azabicyclo[3.1.0]hex-3-yl]propyl}thio)-4-methyl-4*H*-1,2,4-triazol-3-yl]-2-methylquinoline (compare Example 2) was used used as the reference.

The absolute configuration of Enantiomer 2 was assigned as described for Enantiomer 1 to be (1*R*,5*S*)-1-(4-chlorophenyl)-3-(3-{[4-methyl-5-(4-methyl-1,3-oxazol-5-yl)-4*H*-1,2,4-triazol-3-yl]thio}propyl)-3-azabicyclo[3.1.0]hexane.
Enantiomer 1: Specific Optical Rotation of the corresponding free base: [□]_{D} = -25° (CHCl₃, T = 20°C, c = 0.0066 g/mL).
Enantiomer 2: Specific Optical Rotation of the corresponding free base: [□]_{D} = +29° (CHCl₃, T = 20°C, c = 0.0068 g/mL).
Enantiomer 1 showed fpKi (D3) > 1 log-unit higher than Enantiomer 2.

### Example 21: (1S,5R/1R,5S)-1-(4-Fluorophenyl)-3-(3-{[4-methyl-5-(4-methyl-1,3-oxazol-5-yl)-4H-1,2,4-triazol-3-yl]thio}propyl)-3-azabicyclo[3.1.0]hexane hydrochloride

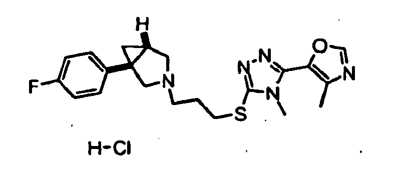

The title compound was prepared in analogy to the method described in Example 1 in 78 mg yield as a white slightly hygroscopic solid from (1*R*,5*S*/1*S*,5*R*)-1-(4-florophenyl)-3-azabicyclo[3.1.0]hexane (49 mg).

NMR (¹H, DMSO): δ 10.06 (bs, 1H), 8.58 (s, 1H), 7.36 (dd, 2H), 7.19 (t, 2H), 4.02 (dd, 1H), 3.74 (dd, 1H), 3.7 (s, 3H), 3.55 (m, 2H), 3.5-3.2 (bm, 4H), 2.39 (s, 3H), 2.15 (m, 3H), 1.49-1.1 (2t, 2H). MS (*m*/*z*): 414[MH]⁺.

Example 21 was separated to give the separated enantiomers by semipreparative Supercritical Fluid Chromatography (Gilson) using a chiral column Chiralpak AS-H, 25 x 2.1 cm, eluent CO₂ containing 7% (ethanol + 0.1% isopropylamine), flow rate 22 mUmin, P 196 bar, T 36°C, detection UV at 220 nm, loop 1 mL. Retention times given were obtained using an analytical Supercritical Fluid Chromatography (Gilson) using a chiral column Chiralpak AS-H, 25 x 0.46 cm, eluent CO₂ containing 6% (ethanol + 0.1% isopropylamine), flow rate 2.5 mL/min, P 190 bar, T 35°C, detection UV at 220 nm.
Enantiomer 1 was recovered in 14 mg yield as white solid, hydrochloride salt from the racemate (40 mg). Rt. = 26.2 min. Purity >99% a/a by UV.
Enantiomer 2 was recovered in 16 mg yield as white solid, hydrochloride salt from the racemate (40 mg). Rt. = 32.4 min. Purity >99% a/a by UV.
Enantiomer 1 showed fpKi (D3) > 1 log-unit higher than Enantiomer 2.

### Example 22: (1S,5R/1R,5S)-1-(3-Chlorophenyl)-5-methyl-3-(3-{[4-methyl-5-(4-methyl-1,3-oxazol-5-yl)-4H-1,2,4-triazol-3-yl]thio}propyl)-3-azabicyclo[3.1.0]hexane hydrochloride

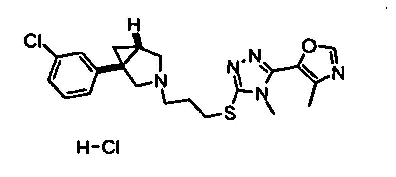

The title compound was prepared in analogy to the method described in Example 1 in 184 mg yield as a white slightly hygroscopic solid from (1*R*,5*S*/1*S*,5*R*)-1-(3-chlorophenyl)-3-azabicyclo[3.1.0]hexane (116 mg).

NMR (¹H, DMSO): δ 9.88 (bs, 1H), 8.58 (s, 1H), 7.43 (d, 1H), 7.4-7.2 (m, 3H), 4.06 (dd, 1H), 3.75 (dd, 1H), 3.7 (s, 3H), 3.62-3.54 (t/m, 2H), 3.5-3.3 (bm, 4H), 2.39 (s, 3H), 2.25 (m, 1H), 2.15 (m, 2H), 1.46-1.19 (2m, 2H). MS (*m*/*z*): 431[MH]⁺.

Example 22 was separated to give the separated enantiomers by semipreparative Supercritical Fluid Chromatography (Gilson) using a chiral column Chiralpak AD-H, 25 x 0.46 cm, eluent CO₂ containing 15% (ethanol + 0.1% isopropylamine), flow rate 22 mL/min, P 192 bar, T 36°C, detection UV at 220 nm, loop 1 mL. Retention times given were obtained using an analytical Supercritical Fluid Chromatography (Berger) using a chiral column Chiralpak AD-H, 25 x 0.46 cm, eluent CO₂ containing 15% (ethanol + 0.1% isopropylamine), flow rate 2.5 mL/min. P 180 bar, T 35°C, detection UV at 220 nm.
Enantiomer 1 was recovered in 18 mg yield as white solid, hydrochloride salt from the racemate (40 mg). Rt. = 29.6 min. Purity 100% a/a by UV.
Enantiomer 2 was recovered in 16 mg yield as white solid, hydrochloride salt from the racemate (40 mg). Rt. = 32.0 min. Purity 100% a/a by UV.
Enantiomer 1 showed fpKi (D3) > 1 log-unit higher than Enantiomer 2.

### Example 23: (1S,5R/1R,5S)-1-(3-Fluorophenyl)-3-(3-{[4-methyl-5-(4-methyl-1,3-oxazol-5-yl)-4H-1,2,4-triazol-3-yl]thio}propyl)-3-azabicyclo[3.1.0]hexane hydrochloride

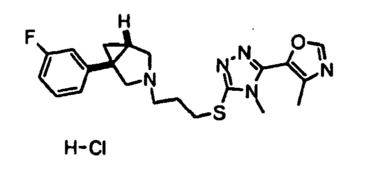

The title compound was prepared in analogy to the method described in Example 1 in 150 mg yield as a white slightly hygroscopic solid from (1*R*,5*S*/1*S*,5*R*)-1-(3-fluorophenyl)-3-azabicyclo[3.1.0]hexane (116 mg).

NMR (¹H, DMSO): δ 10.21 (bs, 1H), 8.58 (d, 1H), 7.4 (m, 1H), 7.2-7.0 (m, 3H), 4.03 (dd, 1H), 3.75 (dd, 1H), 3.7 (s, 3H), 3.61 (t/m, 1H), 3.52 (m, 1H), 3.3 (m, 2H), 3.28 (t, 2H), 2.38 (s, 3H), 2.25 (m, 1H), 2.16 (m, 2H), 1.57-1.17 (t/m, 2H). MS (*m*/*z*): 414 [MH]⁺.

Example 23 was separated to give the separated enantiomers by semipreparative Supercritical Fluid Chromatography (Gilson) using a chiral column Chiralpak AS-H, 25 x 2.1 cm, eluent CO₂ containing 7% (ethanol + 0.1% isopropylamine), flow rate 22 mL/min, P 192 bar, T 36°C, detection UV at 220 nm, loop 1 mL. Retention times given were obtained using an analytical Supercritical Fluid Chromatography (Gilson) using a chiral column Chiralpak AS-H, 25 x 0.46 cm, eluent CO₂ containing 6% (ethanol + 0.1% isopropylamine), flow rate 2.5 mL/min, P 190 bar, T 35°C, detection UV at 220 nm.
Enantiomer 1 was recovered in 12 mg yield as white solid, hydrochloride salt from the racemate (40 mg). Rt. = 24.6 min. Purity >99% a/a by UV.
Enantiomer 2 was recovered in 14.5 mg yield as white solid, hydrochloride salt from the racemate (40 mg). Rt. = 26.0 min. Purity >99% a/a by UV.
Enantiomer 1 showed fpKi (D3) > 1 log-unit higher than Enantiomer 2.

### Example 24: (1S,5R/1R,5S)-3-(3-{[4-methyl-5-(4-methyl-1,3-oxazol-5-yl)-4H-1,2,4-triazol-3-yl]thio}propyl)-1-[3-(methyloxy)phenyl]-3-azabicyclo[3.1.0]hexane hydrochloride

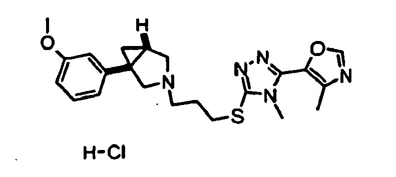

The title compound was prepared in analogy to the method described in Example 1 in 140 mg yield as a white slightly hygroscopic solid from (1*R*,5*S*/1*S*,5*R*)-1-(3-methoxyphenyl)-3-azabicyclo[3.1.0]hexane (116 mg).

NMR (¹H, DMSO): δ 10.16 (bs, 1H), 8.58 (d, 1H), 7.26 (dd, 1H), 6.85 (m, 3H), 4.03 (dd, 1H), 3.77 (s, 3H), 3.72 (dd, 1H), 3.7 (s, 3H), 3.6-3.3 (bm, 4H), 3.28 (t/m, 2H), 2.39 (s, 3H), 2.18 (m, 3H), 1.53-1.1 (t/m, 2H). MS (*m*/*z*): 426 [MH]⁺.

Example 24 was separated to give the separated enantiomers by semipreparative Supercritical Fluid Chromatography (Gilson) using a chiral column Chiralcel OJ-H, 25 x 2.1 cm, eluent CO₂ containing 13% (2-propanol + 0.1% isopropylamine), flow rate 22 mL/min, P 200 bar, T 36°C, detection UV at 220 nm. Retention times given were obtained using an analytical Supercritical Fluid Chromatography (Berger) using a chiral column Chiralcel OJ-H, 25 x 0.46 cm, eluent CO₂ containing 13% (2-propanol + 0.1% isopropylamine), flow rate 2.5 mL/min, P 180 bar, T 35°C, detection UV at 220 nm.
Enantiomer 1 was recovered in 13.5 mg yield as white solid, hydrochloride salt from the racemate (40 mg). Rt. = 24.2 min. Purity >99% a/a by UV.
Enantiomer 2 was recovered in 13.5 mg yield as white solid, hydrochloride salt from the racemate (40 mg). Rt. = 26.8 min. Purity >99% a/a by UV.
Enantiomer 1 showed fpKi (D3) > 1 log-unit higher than Enantiomer 2.

### Example 25: (1S,5R)-1-(4-Bromophenyl)-3-(3-{[4-methyl-5-(tetrahydro-2H-pyran-4-yl)-4H-1,2,4-triazol-3-yl]thio}propyl)-3-azabicyclo[3.1.0]hexane hydrochloride

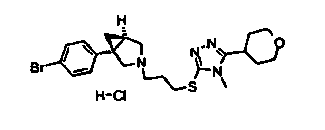

The title compound was prepared in analogy to the method described in Example 1 in 33 mg yield as a white slightly hygroscopic solid from (1*S*,5*R*)-1-(4-bromophenyl)-3-azabicyclo[3.1.0]hexane (Preparation 32, 30 mg).

NMR (¹H, CD3OD): δ 7.54 (d, 2H), 7.28 (d, 2H), 4.08 (m, 3H), 3.8-3.6 (m, 2H), 3.72 (s, 3H), 3.65 (m, 3H), 3.47 (t, 2H), 3.3 (m, 3H), 2.25 (m, 3H), 1.93 (m, 4H), 1.48-1.34 (2m, 2H). MS (*m*/*z*): 478[MH]⁺.

### Example 26: (1S,5R)-1-(4-Bromophenyl)-3-[3-({4-methyl-5-[4-(trifluoromethyl)phenyl]-4H-1,2,4-triazol-3-yl}thio)propyl]-3-azabicyclo[3.1.0]hexane hydrochloride

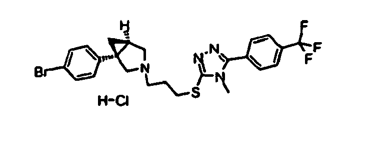

The title compound was prepared in analogy to the method described in Example 1 in 36 mg yield as a white slightly hygroscopic solid from (1*S*,5*R*)-1-(4-bromophenyl)-3-azabicyclo[3.1.0]hexane (Preparation 32, 30 mg).

NMR (¹H, CD30D): δ 7.96 (m, 4H), 7.54 (d, 2H), 7.29 (t, 2H), 4.14 (d, 1H), 3.90 (m, 1H), 3.75 (s, 3H), 3.66 (m, 2H), 3.50 (m, 2H), 3.43 (t, 2H), 2.30 (m, 3H), 1.50 (m, 1H), 1.34 (t, 1H). MS (*m*/*z*): 578[MH]⁺.

### Example 27: (1S,5R)-1-(4-Bromophenyl)-3-(3-{[4-methyl-5-(3-pyridinyl)-4H-1,2,4-triazol-3-yl]thio}propyl)-3-azabicyclo[3.1.0]hexane hydrochloride

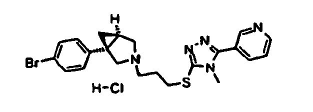

The title compound was prepared in analogy to the method described in Example 1 in 49 mg yield as a white slightly hygroscopic solid from (1*S*,5*R*)-1-(4-bromophenyl)-3-azabicyclo[3.1.0]hexane (Preparation 32, 30 mg).

NMR (¹H, CD30D): δ 8.97 (m, 1H), 8.82 (m, 1H), 8.31 (m, 1H), 7.75 (m, 1H), 7.53 (d, 2H), 7.29 (t, 2H), 4.15 (d, 1H), 3.90 (d, 1H), 3.75 (s, 3H), 3.67 (m, 2H), 3.50 (m, 2H), 3.42 (t, 2H), 2.29 (m, 3H), 1.51 (m, 1H), 1.34 (t, 1H). MS (*m*/*z*): 471[MH]⁺.

### Example 28: (1S,5R)-1-(4-Bromophenyl)-3-(3-{[5-(3,4-difluorophenyl)-4-methyl-4H-1,2,4-triazol-3-yl]thio}propyl)-3-azabicyclo[3.1.0]hexane hydrochloride

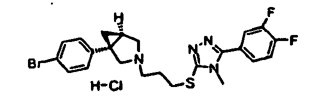

The title compound was prepared in analogy to the method described in Example 1 in 26 mg yield as a white slightly hygroscopic solid from (1*S*,5*R*)-1-(4-bromophenyl)-3-azabicyclo[3.1.0]hexane (Preparation 32, 30 mg).

NMR (¹H, CD3OD): δ 7.72 (m, 1H), 7.55 (m, 4H), 7.28 (d, 2H), 4.13 (d, 1H), 3.89 (d, 1H), 3.7 (s, 3H), 3.64 (m, 2H), 3.43 (t, 2H), 3.38 (m, 2H), 2.29 (m, 3H), 1.48 (m, 1H), 1.34 (t, 1H). MS (*m*/*z*): 506[MH]⁺.

### Example 29: 6-[5-({3-[(1S,5R/1R,5S)-1-(4-Chlorophenyl)-3-azabicyclo[3.1.0]hex-3-yl]propyl}thio)-4-methyl-4H-1,2,4-triazol-3-yl]-2-methylquinoline hydrochloride

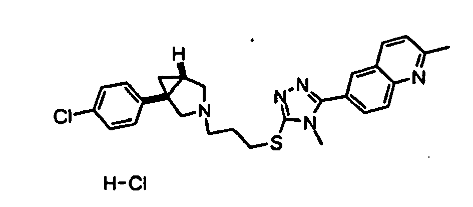

The title compound was prepared in analogy to the method described in Example 1 in 110 mg yield as a white slightly hygroscopic solid from (1*R*,5*S*/1*S*,5*R*)-1-(4-chlorophenyl)-3-azabicyclo[3.1.0]hexane (87 mg).

NMR (¹H, CD30D): δ 8.95 (d, 1H), 8.39 (d, 1H), 8.28 (t, 1H), 8.13 (d, 1H), 7.96 (d, 1H), 7.37 (m, 4H), 4.17 (d, 1H), 3.93 (d, 1H), 3.71 (m, 2H), 3.62 (s, 3H), 3.5 (2m, 4H), 3.04 (s, 3H), 2.37 (m, 2H), 2.27 (m, 1H), 1.55 (m, 1H), 1.31 (m, 1H). MS (*m*/*z*): 490 [MH]⁺.

Example 29 was separated to give the separated enantiomers by semipreparative Supercritical Fluid Chromatography (Gilson) using a chiral column Chiralpak AD-H, 25 x 0.46 cm, eluent CO₂ containing 25% (ethanol + 0.1% isopropylamine), flow rate 22 mL/min, P 199 bar, T 36°C, detection UV at 220 nm. Retention times given were obtained using an analytical Supercritical Fluid Chromatography (Berger) using a chiral column Chiralpak AD-H, 25 x 0.46 cm, eluent CO₂ containing 25% (ethanol + 0.1% isopropylamine), flow rate 2.5 mL/min, P 180 bar, T 35°C, detection UV at 220 nm. Enantiomer 1 was recovered in 13.5 mg yield as white solid, hydrochloride salt from the racemate (40 mg). Rt. = 24.3 min. Purity 87.6% a/a by UV.
Enantiomer 2 was recovered in 5 mg yield as white solid, hydrochloride salt from the racemate (40 mg). Rt. = 26.5 min. Purity 100% a/a by UV.
Enantiomer 1 showed fpKi (D3) > 1 log-unit higher than Enantiomer 2.

### Example 30: (1S,5R/1R,5S)-3-(3-{[4-Methyl-5-(4-methyl-1,3-oxazol-5-yl)-4H-1,2,4-triazol-3-yl]thio}propyl)-1-{4-[(trifluoromethyl)oxy]phenyl}-3-azabicyclo[3.1.0]hexane hydrochloride

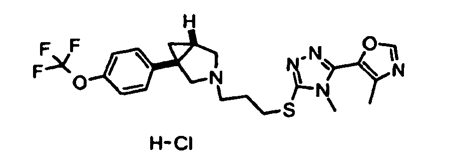

The title compound was prepared in analogy to the method described in Example 1 in 246 mg yield as a white slightly hygroscopic solid from (1*R*,5*S*/1*S*,5*R*)-1-{4-[(trifluoromethyl)oxy]phenyl}-3-azabicyclo[3.1.0]hexane (205 mg).

NMR (¹H, DMSO): δ 10.33 (bs,1H), 8.58 (s, 1H), 7.43 (d, 2H), 7.36 (d, 2H), 4.04 (dd, 1H), 3.73 (dd, 1H), 3.7 (s, 3H), 3.6-3.2 (bm, 6H), 2.39 (s, 3H), 2.2 (m, 3H), 1.61-1.16 (2t, 2H). MS (*m*/*z*): 480[MH]⁺.

Example 30 was separated to give the separated enantiomers by semi-preparative HPLC using a chiral column Chirapak AS-H, 25 x 2 cm, eluent A: n-hexane; B: isopropanol, gradient isocratic 15% B v/v, flow rate 7 mUmin, detection UV at 220 nm. Retention times given were obtained using chiral column Chiracel OD, 25 x 0.46 cm, eluent A: n-hexane; B: isopropanol, gradient isocratic 10% B v/v, flow rate 1 mUmin, detection UV at 220 nm.
Enantiomer 1 was recovered in 15 mg yield as white solid, hydrochloride salt from the racemate (40 mg). Rt. = 28.3 min. Purity >99% a/a by UV.
Enantiomer 2 was recovered in 16 mg yield as white solid, hydrochloride salt from the racemate (40 mg). Rt. = 50.6 min. Purity >99% a/a by UV.
Enantiomer 1 showed fpKi (D3) > 1 log-unit higher than Enantiomer 2.

### Example 31: (1S,5R/1R,5S)-3-(3-{[4-Methyl-5-(4-methyl-1,3-oxazol-5-yl)-4H-1,2,4-triazol-3-yl]thio}propyl)-1-[2-methyl-4-(trifluoromethyl)phenyl]-3-azabicyclo[3.1.0]hexane hydrochloride

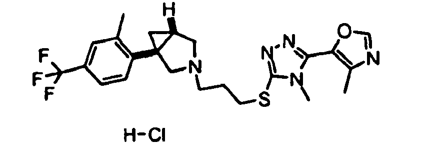

The title compound was prepared in analogy to the method described in Example 1 in 46 mg yield as a white slightly hygroscopic solid from (1*R*,5*S*/1*S*,5*R*)-1-[2-methyl-4-(trifluoromethyl)phenyl]-3-azabicyclo[3.1.0]hexane (71.5 mg).

NMR (¹H, DMSO): δ 10.25 (bs, 1H), 8.58 (s, 1H), 7.6 (m, 3H), 3.97-3.7 (dd/m, 2H), 3.79/3.4 (dd/m, 2H), 3.69 (s, 3H), 3.27 (t, 2H), 2.5 (m, 2H), 2.48 (s, 3H), 2.38 (s, 3H), 2.2 (m, 1H), 2.13 (quint., 2H), 1.61-1.01 (2t, 2H). MS (*m*/*z*): 478[MH]⁺.

### Example 32: not used

### Example 33: (1S,5R/1R,5S)-3-(3-{[4-Methyl-5-(tetrahydro-2H-pyran-4-yl)-4H-1,2,4-triazol-3-yl]thio}propyl)-1-{4-[(trifluoromethyl)oxy]phenyl}-3-azabicyclo[3.1.0]hexane hydrochloride

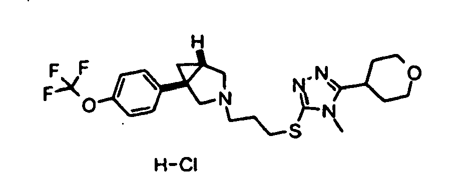

The title compound was prepared in analogy to the method described in Example 1 in 72 mg yield as a white slightly hygroscopic solid from (1*R*,5*S*/1*S*,5*R*)-1-{4-[(trifluoromethyl)oxy]phenyl}-3-azabicyclo[3.1.0]hexane (100 mg).

NMR (¹H, DMSO): δ 10.45 (bs, 1H), 7.44 (d, 2H), 7.36 (d, 2H), 4.04 (bm, 1H), 3.94 (dm, 2H), 3.73 (bm, 1H), 3.55 (s, 3H), 3.6-3.3 (bm, 6H), 3.22 (t, 2H), 3.13 (m, 1H), 2.23 (m, 1H), 2.21 (m, 2H), 1.9-1.7 (m, 4H), 1.63-1.16 (2t, 2H). MS (*m*/*z*): 483 [MH]⁺.

Example 33 was separated to give the separated enantiomers by semipreparative Supercritical Fluid Chromatography (Gilson) using a chiral column Chiralpak AS-H, 25 x 2.1 cm, eluent CO₂ containing 8% (2-propanol + 0.1% isopropylamine), flow rate 22 mL/min, P 200 bar, T 36°C, detection UV at 220 nm. Retention times given were obtained using an analytical Supercritical Fluid Chromatography (Berger) using a chiral column Chiralpak AS-H, 25 x 0.46 cm, eluent CO₂ containing 8% (2-propanol + 0.1% isopropylamine), flow rate 2.5 mL/min, P 180 bar, T 35°C, detection UV at 220 nm.
Enantiomer 1 was recovered in 15 mg yield as white solid, hydrochloride salt from the racemate (65 mg). Rt. = 23.2 min. Purity 100% a/a by UV.
Enantiomer 2 was recovered in 12 mg yield as white solid, hydrochloride salt from the racemate (65 mg). Rt. = 24.6 min. Purity 100% a/a by UV.
Enantiomer 1 showed fpKi (D3) > 1 log-unit higher than Enantiomer 2.

### Example 34: (1R,5S/1S,5R)-1-(3-Bromophenyl)-3-(3-{[4-methyl-5-(4-methyl-1,3-oxazol-5-yl)-4H-1,2,4-triazol-3-yl]thio}propyl)-3-azabicyclo[3.1.0]hexane hydrochloride

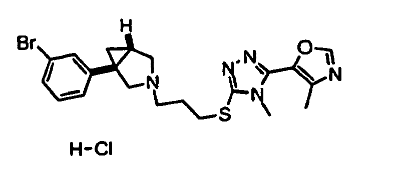

The title compound was prepared in 23 mg yield as a white slightly hygroscopic solid from (1*R*,5*S*/1*S*,5*R*)-1-(3-bromophenyl)-3-azabicyclo[3.1.0]hexane (140 mg) in analogy to the method described in Example 1 and purifying the free base of the title compound by preparative HPLC using a column -X Terra MS C18 5µm, 100 x19 mm, eluent A: H₂O+0.1% TFA; B: CH₃CN+0.1% TFA, gradient 10% (B) for 1 min, from 10% (B) to 35% (B) in 12 min, flow rate 17 mL/min, detection UV at 200-400 nm. Retention times given were obtained using column X Terra MS C18 5µm, 50 x 4.6 mm, eluent A: H₂O+0.1% TFA; B: CH₃CN+0.1% TFA, gradient isocratic 25% B v/v, flow rate 1 mL/min, detection UV at 200-400 nm. Rt. = 6.26 min. Purity 96.4% a/a by UV.

NMR (1H, DMSO): δ 9.9 (bs, 1H), 8.58 (s, 1H), 7.57 (s, 1H), 7.47 (m, 1H), 7.3 (m, 2H), 4.04 (m, 1H), 3.75 (dd, 1H), 3.7-3.2 (m, 6H), 3.7 (s, 3H), 2.39 (s, 3H), 2.23 (m, 1H), 2.15 (m, 2H), 1.47 (t, 1H), 1.2 (t, 1H). MS (*m*/*z*): 512[MH]⁺.

### Example 35: (1S,5R)-3-(1-Methyl-3-{[4-methyl-5-(4-methyl-1,3-oxazol-5-yl)-4H-1,2,4-triazol-3-yl]thio}propyl)-1-[4-(trifluoromethyl)phenyl]-3-azabicyclo[3.1.0]hexane hydrochloride

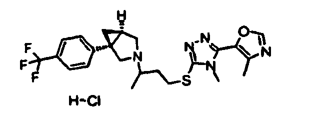

A mixture of (1*S*,5*R*)-3-(3-chloro-1-methylpropyl)-1-[4-(trifluoromethyl)phenyl]-3-azabicyclo[3.1.0]hexane hexane (Preparation 20, 105 mg), 4-methyl-5-(4-methyl-1,3-oxazol-5-yl)-2,4-dihydro-3H-1,2,4-triazole-3-thione (0.43 mmol), TEA (0.46 mmol) and Nal (0.43 mmol) in DMF (anhydrous, 1.6 mL) was heated at 60°C for 12 h. After elimination of the solvent under vacuo, the residue was dissolved in ethyl acetate and the organic layer was washed with H₂O and dried over Na₂SO₄. This solution was concentrated *in vacuo,* treated with cyclohexane and filtered to give 125 mg of the free base of the title compound. To a solution of this material in dichloromethane (0.2 mL) was added 0.34 mmol of HCl (1M in Et₂O), the solvent evaporated under *vacuo* and the material thus obtained triturated with Et₂O to give 105 mg of the title compound as a white slightly hygroscopic solid.

MS(*m*/*z*): 478[MH]⁺.

Example 35 was separated to give the separated diastereoisomers by semi-preparative HPLC using a chiral column Chirapak AD, 25 x 2 cm, eluent A: n-hexane; B: ethanol+ 0.1% isopropylamine, gradient isocratic 15% B v/v, flow rate 7 mL/min, UV wavelength range 220-400 nm. Retention times given were obtained using a chiral column Chiralpak AD-H, 25 x 0.46 cm, eluent A: n-hexane; B: ethanol+ 0.1% isopropylamine, gradient isocratic 17% B v/v, flow rate 1 mL/min, UV wavelength range 200-400 nm.

Diastereoisomer 1 was recovered in 30 mg yield as a white solid, hydrochloride salt from the diastereomeric mixture (105mg). Rt. = 17.9 min. Purity 99.4% a/a by UV NMR (¹H, DMSO): δ 10.33 (bs, 1H), 8.58 (s, 1H), 7.71 (d, 2H), 7.53 (d, 2H), 4.07 (dd, 1H), 3.78 (dd, 1H), 3.7 (s, 3H), 3.7 (m, 1H), 3.56 (bs, 2H), 3.4 (m, 1H), 3.18 (m, 1H), 2.4 (s, 3H), 2.4-2.3 (m, 1H), 2.26-2.09 (m, 2H), 1.72 (m, 1H), 1.42 (d, 3H), 1.2 (m, 1H). MS (*m*/*z*): 478[MH]⁺.

Diastereoisomer 2 was recovered in 46 mg yield as white solid, hydrochloride salt from the diastereomeric mixture (105mg). Rt. = 21.2 min. Purity >99% a/a by UV

NMR (¹H, DMSO): δ 10.26 (bs, 1H), 8.58 (s, 1H), 7.7 (d, 2H), 7.51 (d, 2H), 4.14 (dd, 1H), 3.8-3.6 (m, 3H), 3.7 (s, 3H), 3.53 (bs, 1H), 3.4 (m, 1H), 3.18 (m, 1H), 2.38 (s, 3H), 2.4-2.25 (m, 2H), 2.1 (m, 1H), 1.69 (m, 1H), 1.39 (d, 3H), 1.2 (m, 1H). MS (*m*/*z*): 478[MH]⁺.

### Example 36: (1R,5S/1S,5R)-1-[2-Fluoro-5-(trifluoromethyl)phenyl]-3-(3-{[4-methyl-5-(4-methyl-1,3-oxazol-5-yl)-4H-1,2,4-triazol-3-yl]thio}propyl)-3-azablcyclo[3.1.0]hexane hydrochloride

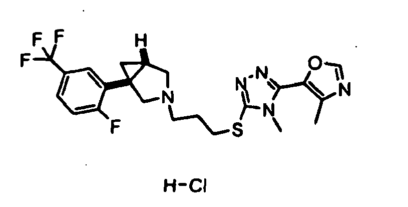

The title compound was prepared in analogy to the method described in Example 1 in 144 mg yield as a white slightly hygroscopic solid from 1(1*R*,5*S*/1*S*,5*R*) -[2-fluoro-5-(trifluoromethyl)phenyl]-3-azabicyclo[3.1.0]hexane (109 mg).

NMR (¹H, CD₃OD): δ 8.41 (s, 1H), 7.8 (m, 1H), 7.74 (m, 1H), 7.39 (t, 1H), 4.13 (d, 1H), 3.95 (d, 1H), 3.81 (s, 3H), 3.73 (bd, 1H), 3.54 (d, 1H), 3.48 (m, 2H), 3.41 (m, 2H), 2.48 (s, 3H), 2.39 (m, 1H), 2.28 (q, 2H), 1.58 (m, 1H), 1.35 (m, 1H). MS (*m*/*z*): 482[MH]⁺.

Example 36 was separated to give the separated enantiomers by semi-preparative HPLC using a chiral column Chirapak AS-H, 25 x 2 cm, eluent A: n-hexane; B: isopropanol + 0.1% isopropylamine, gradient isocratic 10% B v/v, flow rate 7 mL/min, detection UV at 220 nm. Retention times given were obtained using an analytical Supercritical Fluid Chromatography (Berger) using a chiral column Chiralpak AD-H, 25 x 0.46 cm, eluent CO₂ containing 7% (ethanol + 0.1% isopropylamine), flow rate 2.5 mL/min, P 180 bar, T 35°C, detection UV at 220 nm.
Enantiomer 1 was recovered in 48 mg yield as a white solid, hydrochloride salt from the racemate (138 mg). Rt. = 21.2 min. Purity 100% a/a by UV.
Enantiomer 2 was recovered in 46 mg yield as white solid, hydrochloride salt from the racemate (138 mg). Rt. = 22.7 min. Purity 99% a/a by UV.
Enantiomer 2 showed fpKi (D3) > 1 log-unit higher than Enantiomer 1.

### Example 37: 1-[4-[(1R,5S/1S,5R)-3-(3-{[4-Methyl-5-(4-methyl-1,3-oxazol-5-yl)-4H-1,2,4-triazol-3-yl]thio}propyl)-3-azabicyclo[3.1.0]hex-1-yl]-2-(methyloxy)phenyl]ethanone hydrochloride

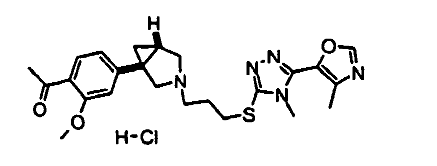

The title compound was prepared in analogy to the method described in Example 1 in 70 mg yield as a white slightly hygroscopic solid from 1-[4-[(1*S*,5*R* /1*R*,5*S*)-3-azabicyclo[3.1.0]hex-1-yl]-2-(methyloxy)phenyl]ethanone (87 mg).

NMR (¹H, CDCl₃) of the corresponding free base: δ 8.0 (s, 1H), 7.7 (d, 1H), 6.7-6.8 (m, 2H), 3.9 (s, 3H), 3.7 (s, 3H), 3.35 (m, 4H), 3.1 (d, 1H), 2.6 (m, 3H), 2.55 (s, 3H), 2.5 (s, 3H), 2.45 (m, 1H), 2.0 (m, 2H), 1.75 (m, 1H), 0.8 (m, 1H). MS (*m*/*z*): 468[MH]⁺.

### Example 38: 1-[4-[(1R,5S/1S,5R)-3-(3-{[4-methyl-5-(4-methyl-1,3-oxazol-5-yl)-4H-1,2,4-triazol-3-yl]thio}propyl)-3-azabicyclo[3.1.0]hex-1-yl]-2-(methyloxy)phenyl]-1-propanone hydrochloride

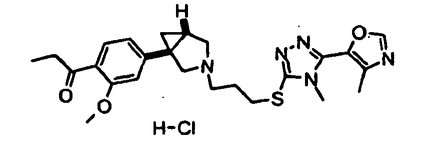

The title compound was prepared in analogy to the method described in Example 1 in 75 mg yield as a white slightly hygroscopic solid from 1-[(1*S*,5*R* /1*R*,5*S*)-3-azabicyclo[3.1.0]hex-1-yl)-2-(methyloxy)phenyl]-1-propanone (106 mg).

Free base NMR (¹H, CDCl₃): δ 7.9 (s, 1H), 6.65 (d, 1H), 6.7 (m, 2H), 3.9 (s, 3H), 3.7 (s, 3H), 3.35 (m, 3H), 3.1 (d, 1H), 2.9 (m, 2H), 2.6 (m, 3H), 2.5 (s, 3H), 2.45 (m, 1H), 2.0 (m, 2H), 1.8 (m, 1H), 1.1 (m, 3H), 0.8 (m, 1H). MS (*m*/*z*): 482[MH]⁺.

### Example 39: (1R,5S/1S,5R)-3-(3-{[4-Methyl-5-(4-methyl-1,3-oxazol-5-yl)-4H-1,2,4-triazol-3-yl]thio}propyl)-1-[2-(trifluoromethyl)phenyl]-3-azabicyclo[3.1.0]hexane hydrochloride

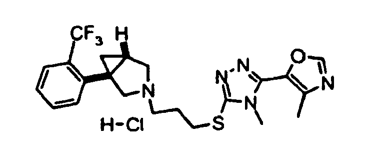

The title compound was prepared in analogy to the method described in Example 1 in 7 mg yield as a white slightly hygroscopic solid from (1*R*,5*S*/1*S*,5*R*)-1-[2-(trifluoromethyl)phenyl]-3-azabicyclo[3.1.0]hexane (53 mg).

NMR (¹H, DMSO): δ 10.48 (bs, 1H), 8.55 (s, 1H), 7.9-7.6 (m, 4H), 3.9-3.1 (bm, 8H), 3.68 (s, 3H), 2.36 (s, 3H), 2.13 (m, 2H), 1.66 (m, 1H), 1.2 (m, 1H), 1.1 (m, 1H). MS (*m*/*z*): 464[MH]⁺.

### Example 40: (1S,5R)-1-[2-Fluoro-4-(trifluoromethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane hydrochloride

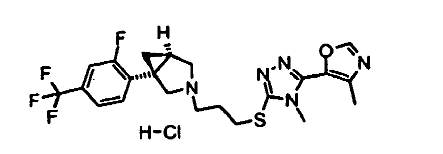

The free base of the title compound was prepared in analogy to the method described in Example 1 from (1*S*,5*R*)-1-[2-fluoro-4-(trifluoromethyl)phenyl]-3-azabicyclo[3.1.0]hexane. A mixture of (1*S*,5*R*)-1-[2-fluoro-4-(trifluoromethyl)phenyl]-3-azabicyclo[3.1.0]hexahe (Preparation 39, 727mg, 2.97mmol), 3-[(3-Chloropropyl)thio]-4-methyl-5-(4-methyl-1,3-oxazol-5-yl)-4*H*-1,2,4-triazole (Preparation 14, 3.6mmol.), K₂CO₃ (3.6mmol.) and Nal (2.97mmol) in DMF anhydrous was heated at 60°C for 24 h. After elimination of the solvent under *vacuo,* the residue was dissolved in ethyl acetate and the organic layer was washed with saturated aqueous NaHCO₃ and dried over Na₂SO₄. This solution was filtered and the filtrate was concentrated *in vacuo.* The crude was purified by flash chromatography (dichloromethane to 10% MeOH in dichloromethane) to give 940 mg of the free base of the title compound.

This free base (886 mg) was converted to the hydrochloride salt (847 mg) according to the method described in Example 1. The title compound was obtained as a white solid.

Analytical Chiral HPLC confirmed the product to be identical to Enantiomer 2 of Example 16.

NMR and MS data corresponded to those reported for Example 16.

The absolute configuration of the title compound was confirmed using comparative VCD and comparative OR analyses of the corresponding free base to be (1*S*,5*R*)-1-[2-fluoro-4-(trifluoromethyl)phenyl]-3-(3-{[4-methyl-5-(4-methyl-1,3-oxazol-5-yl)-4*H*-1,2,4-triazol-3-yl]thio}propyl)-3-azabicyclo[3.1.0]hexane. (1*S*,5*R*)-3-(3-{[4-Methyl-5-(4-methyl-1,3-oxazol-5-yl)-4*H*-1,2,4-triazol-3-yl]thio}propyl)-1-[4-(trifluoromethyl)phenyl]-3-azabicyclo[3.1.0]hexane (see Example 14) was used used as the reference.

Specific Optical Rotation of the corresponding free base: [α]_{D} = -42° (CDCl₃, T = 25 °C, c ≅ 0.005 g/0.8 mL).

### Examples 41-52:

To a solution of the respective 3-thio-5-aryl-1,2,4-triazole (prepared in analogy to the method described in Preparation 13, 0.131 mmol) in dry acetonitrile (2 mL) 2-*tert-*butylimino-2-diethylamino-1,3-dimethyl-perhydro-1,3,2-diaza-phosphorine on polystyrene (90 mg, 2.2 mmol/g) was added and the resulting mixture was shaken for 30 minutes at room temperature. (1*S*,5*R*)-3-(3-Chloropropyl)-1-[4-(trifluoromethyl)phenyl]-3-azabicyclo[3.1.0]hexane (40 mg) was added and the resulting mixture was shaken at 70 °C for three hours. After cooling the resin was removed by filtration, washed with methanol (2 mL), and then the solvent was removed under reduced pressure. Purifications were carried out using mass directed HPLC using a Waters XTerra Prep MS C18 10µm, 30x150 mm column using the following conditions:

| | **Time** | **Flow** | **% A** | **% B** |
|---|---|---|---|---|
| **Prerun** | 0 | 40 ml/min | 99 | 1 |
| | 1 | 40 ml/min | 99 | 1 |
| **Run** | 0 | 40 ml/min | 99 | 1 |
| | 10 | 40 ml/min | 75 | 25 |
| | 14.5 | 40 ml/min | 10 | 90 |
| | 15 | 40 ml/min | 0 | 100 |
| **Postrun** | 0 | 40 ml/min | 0 | 100 |
| | 0.2 | 45 ml/min | 0 | 100 |
| | 1.5 | 45 ml/min | 0 | 100 |
| | 2 | 40 ml/min | 0 | 100 |

| | | | | |
|---|---|---|---|---|
| A= H2O + 0.1 % formic acid B = ACN + 0.1 % formic acid | | | | |

Then solvent was removed under reduced pressure to give the respective compounds as formate salts. The residues were taken up with methanol (1 mL) and loaded on SCX SPE cartridges (1g), washed with methanol (3 mL) and eluted with a 2 M ammonia solution in methanol (3 mL), then the solvent was removed under reduced pressure. The residues were taken up with dichloromethane (1 mL) and a 1.0 M HCl solution in diethylether was added (0.131 mmol), then the solvent was removed under reduced pressure to give product compounds summarised in TABLE 1 as hydrochloride salts.

Analytical Chromatographic conditions:

| | |
|---|---|
| Column: | X Terra MS C18 5 mm, 50 x 4.6 mm |
| Mobile phase: | A: NH4HCO3 sol. 10 mM, pH10; B: CH3CN |
| Gradient: | 10% (B) for 1 min, from 10% (B) to 95% (B) in 12 min, 95% (B) for 3 min |
| Flow rate: | 1 mL/min |
| UV wavelengh range: | 210-350 nm |
| Mass range: | 100-900 amu |
| Ionization: | ES+ |

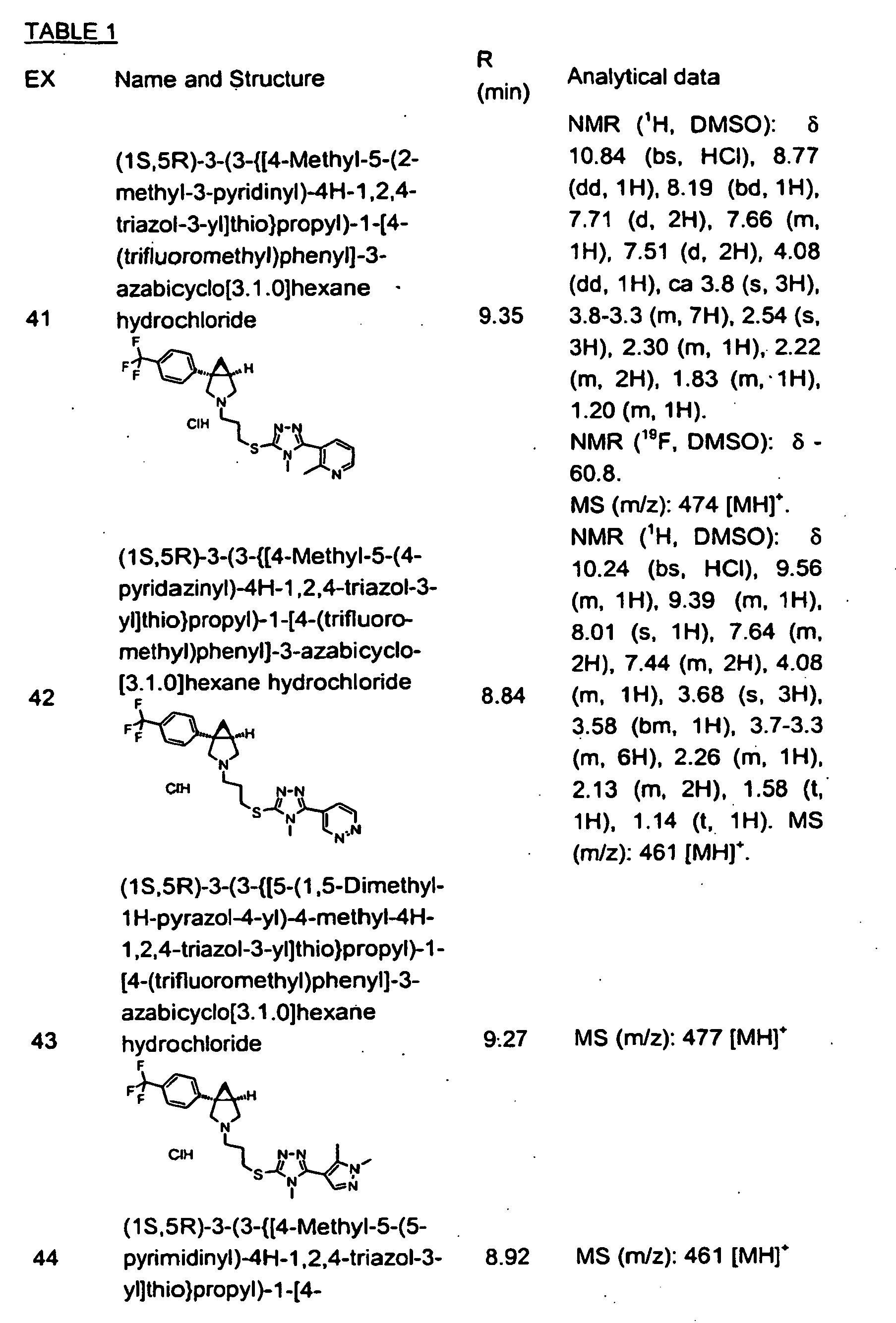

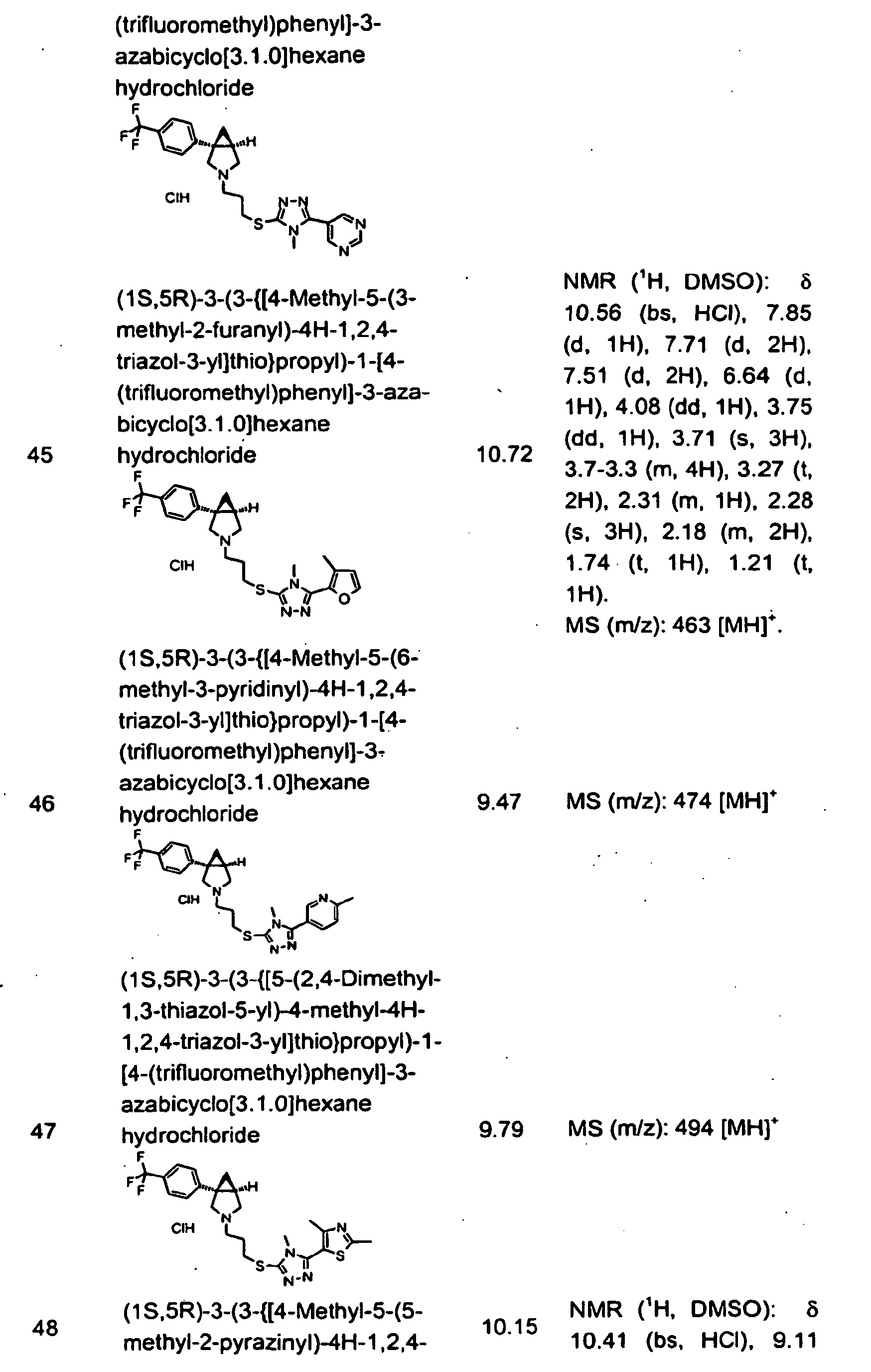

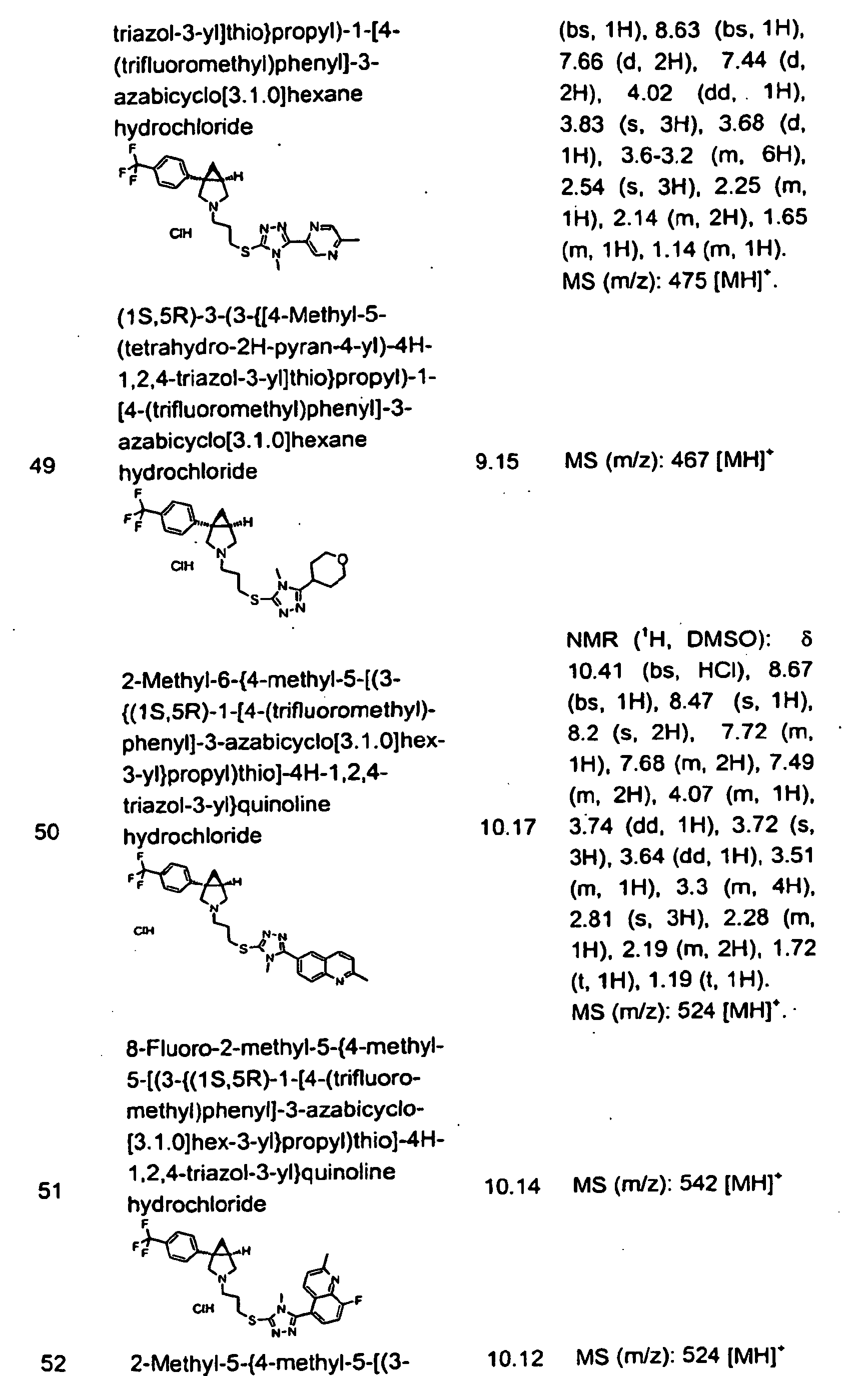

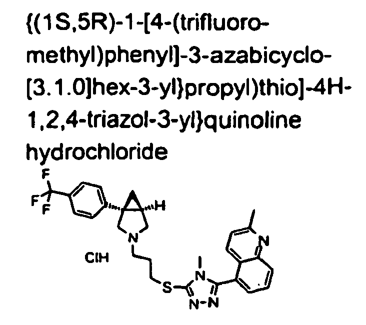

### Examples 53-58:

To a solution of the respective 3-thio-5-aryl-1,2,4-triazole (0.124 mmol) in dry acetonitrile (2 mL) 2-*tert*-butylimino-2-diethylamino-1,3-dimethyl-perhydro-1,3,2-diaza-phosphorine on polystyrene (85 mg, 2.2 mmol/g) was added and the resulting mixture was shaken for 30 minutes at room temperature, then (1*S*,5*R*)-3-(3-chloropropyl)-1-[2-fluoro-4-(trifluoromethyl)phenyl]-3-azabicyclo[3.1.0]hexane (40 mg) was added and the resulting mixture was shaken at 50°C overnight. After cooling the resin was removed by filtration, washed with methanol (2mL) and then the solvent was removed under reduced pressure. Purifications were carried out using mass directed HPLC:

### Preparative chromatographic conditions (prep. HPLC of 6 out of 6 compounds)

| | |
|---|---|
| Column: | X Terra MS C18 5 mm, 100 x 19 mm |
| Mobile phase: | A: NH4HCO3 sol. 10 mM, pH10; B: CH3CN |
| Gradient: | 30% (B) for 1 min, from 30% (B) to 95% (B) in 9 min, 95% (B) for 3 min |
| Flow rate: | 1 mL/min |
| UV wavelenght range: | 210-350 nm |
| Mass range: | 100-900 amu |
| Ionization: | ES+ |

Then solvent was removed under reduced pressure to give compounds as free bases. The residues were taken up with dichloromethane (2 mL) and a 1.0 M HCl solution in diethylether was added (0.124 mmol) then solvent was removed under reduced pressure to give to give product compounds summarised in TABLE 2 as hydrochloride salts.

### Analytical chromatographic conditions

| | |
|---|---|
| Column: | X Terra MS C18 5 mm, 50 x 4.6 mm |
| Mobile phase: | A: NH4HCO3 sol. 10 mM, pH10; B: CH3CN |
| Gradient: | 30% (B) for 1 min, from 30% (B) to 95% (B) in 9 min, 95% (B) for 3 min |
| Flow rate: | 1 mL/min |
| UV wavelenght | range: 210-350 nm |
| Mass range: | 100-900 amu |
| Ionization: | ES+ |

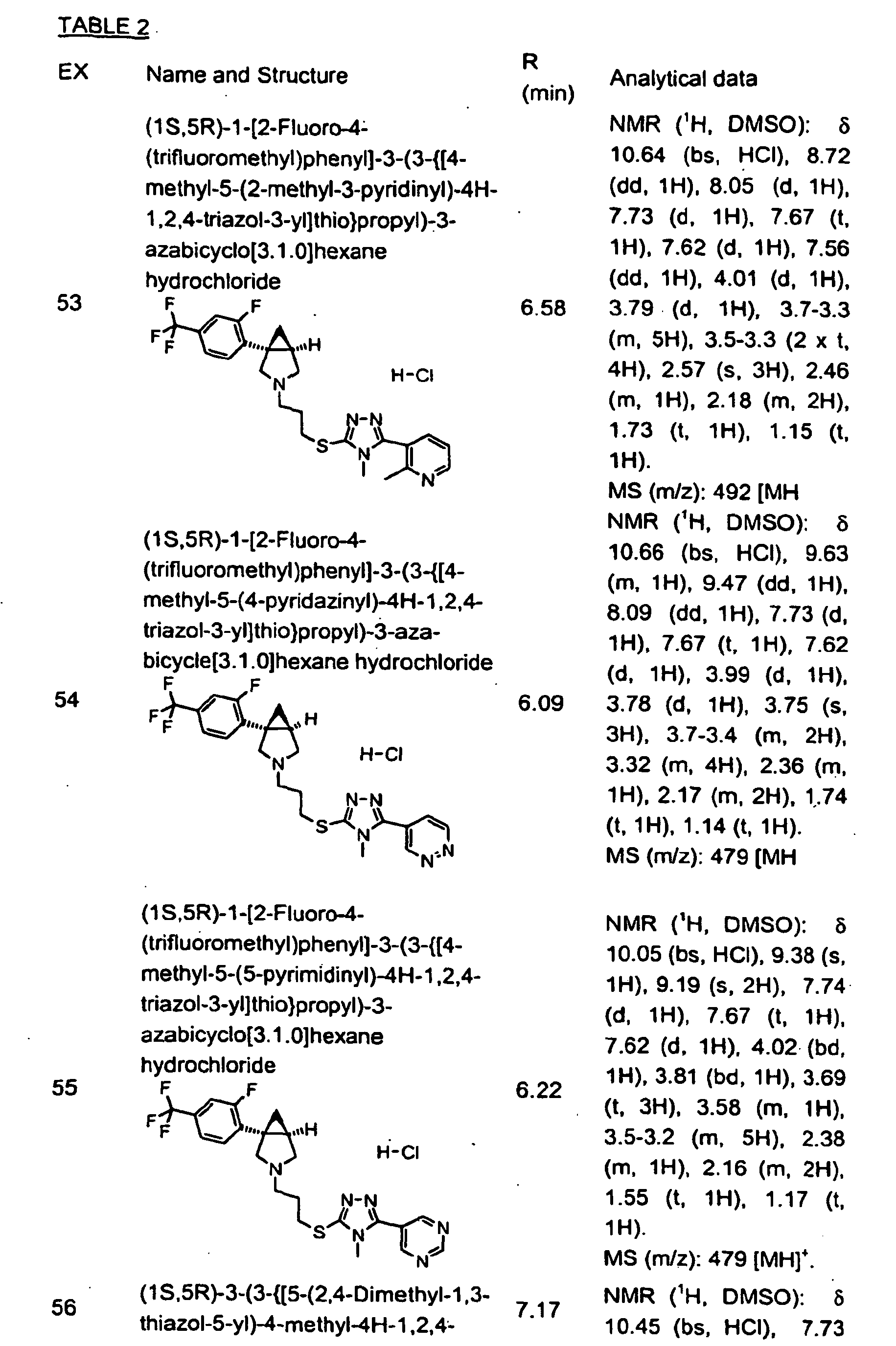

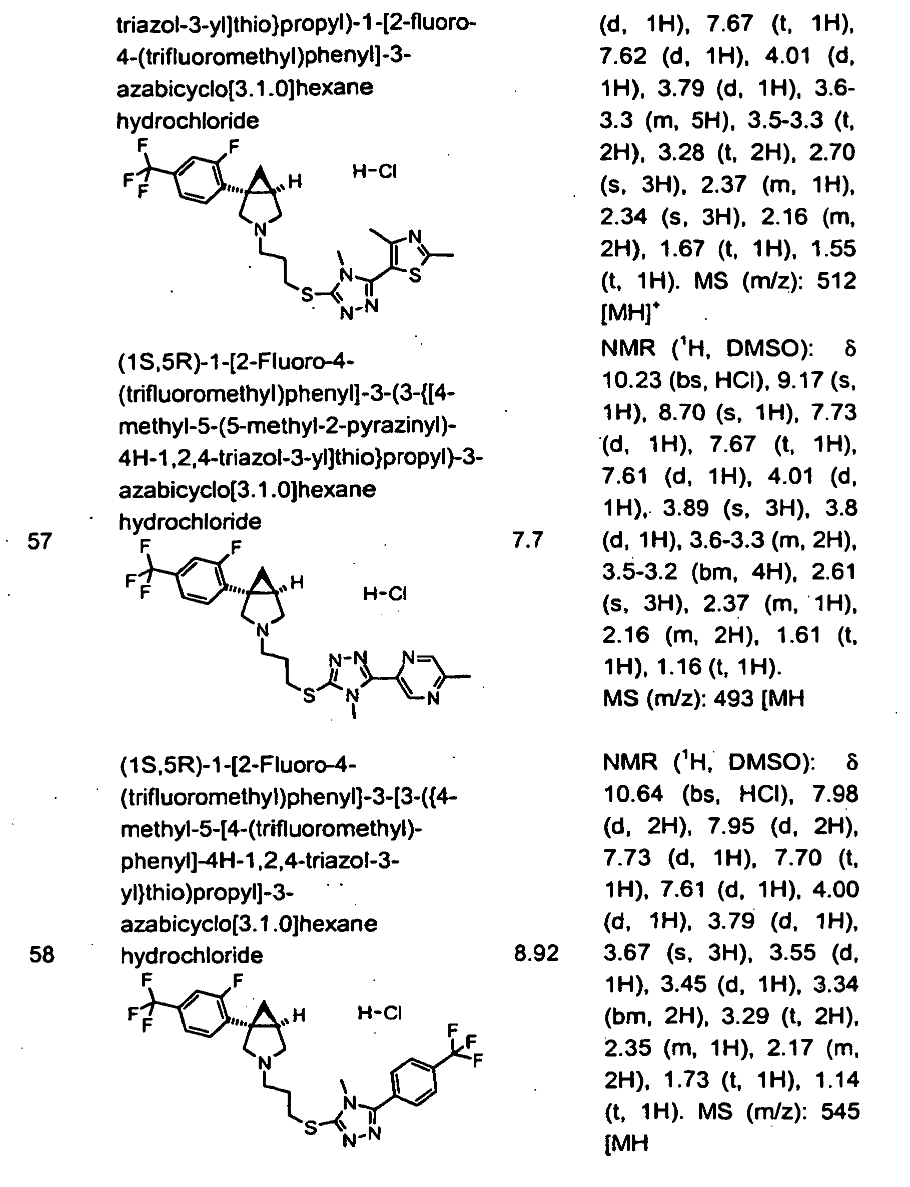

### Example 59: 1-{4-[(1R,5S/1S,5R)-3-(3-{[4-Methyl-5-(2-methyl-5-quinolinyl)-4H-1,2,4-triazol-3-yl]thio}propyl)-3-azabicyclo[3.1.0]hex-1-yl]phenyl}-2-pyrrolidinone hydrochloride

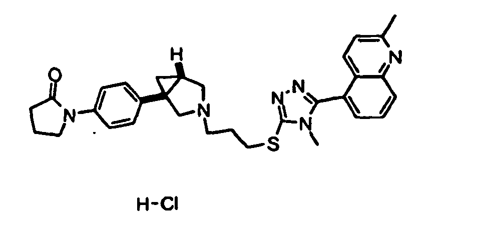

A Schlenk tube was charged with 5-[5-({3-[(1*R*,5*S*/1*S*,5*R*)-1-(4-bromophenyl)-3-azabicyclo[3.1.0]hex-3-yl]propyl}thio)-4-methyl-4*H*-1,2,4-triazol-3-yl]-2-methylquinoline (cf. Example 2; 0.15 g), 2-pyrrolidinone (32 mg), tris(dibenzylideneacetone)-dipalladium(0) (6 mg), 4,5-bis(diphenylphosphino)-9,9-dimethylxanthene (10 mg), cesium carbonate (130 mg) and 1,4-dioxane (2 mL). The Schlenk tube was sealed with a teflon screwcap and the reaction mixture was stirred at 100°C for 12 h. The reaction mixture was allowed to cool to room temperature, diluted with dichloromethane (10 mL), filtered and concentrated *in vacuo.* The crude product was purified by flash chromatography (dichloromethane to 10% MeOH in dichloromethane) to give 60 mg of the free base of the title compound. To a solution of this material in dichloromethane (0.4 mL) was added HCl (0.11 mL, 1M in Et₂O), the solvent evaporated *in vacuo* and the material thus obtained triturated with Et₂O to give 64 mg of the title compound as a white solid.

NMR (¹H, DMSO): δ 10.48 (bs,1H), 8.24 (bd, 1H), 8.18 (d, 1H), 7.93 (t, 1H), 7.81 (d, 1H), 7.62 (d, 2H), 7.54 (d, 1H), 7.31 (d, 2H), 4.04 (dd, 1H), 3.82 (t, 2H), 3.76 (dd, 1H), 3.70/3.10 (bm, 8H), 3.45 (s, 3H), 2.74 (s, 3H), 2.25 (m, 2H), 2.16 (m, 1H), 2.07 (m, 2H), 1.63/1.10 (t/t, 2H). MS (*m*/*z*): 539 [MH]⁺.

### Example 60: 5-{5-[(3-{(1R,5S/1S,5R)-1-[4-(1,1-Dioxido-2-isothiazolidinyl)phenyl]-3-azabicyclo[3.1.0]hex-3-yl}propyl)thio]-4-methyl-4H-1,2,4-triazol-3-yl}-2-methylquinoline hydrochloride

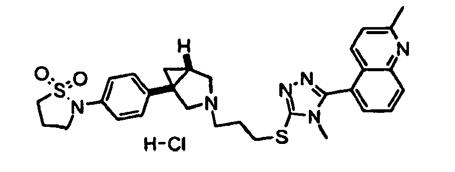

A Schlenk tube was charged with 5-[5-({3-[(1*R*,5*S*/1*S*,5*R*)-1-(4-bromophenyl)-3-azabicyclo[3.1.0]hex-3-yl]propyl}thio)-4-methyl-4*H*-1,2,4-triazol-3-yl]-2-methylquinoline (cf. Example 2; 0.15 g), isothiazolidine 1,1-dioxide (46 mg), tris(dibenzylideneacetone)-dipalladium(0) (6 mg), 4,5-bis(diphenylphosphino)-9,9-dimethylxanthene (10 mg), cesium carbonate (130 mg) and 1,4-dioxane (2 mL). The Schlenk tube was sealed with a teflon screwcap and the reaction mixture was stirred at 100°C for 12 h. The reaction mixture was allowed to cool to room temperature, diluted with dichloromethane (10 mL), filtered and concentrated *in vacuo.* The crude product was purified by flash chromatography (dichloromethane to 10% MeOH in dichloromethane) to give 50 mg of the free base of the title compound. To a solution of this material in dichloromethane (0.3 mL) was added HCl (0.087 mL, 1 M in Et₂O), the solvent evaporated *in vacuo* and the material thus obtained triturated with Et₂O to give 52 mg of the title compound as a white solid.

NMR (¹H, DMSO): δ 10.57 (bs, 1H), 8.27 (bd, 1H), 8.19 (d, 1H), 7.94 (t, 1H), 7.82 (d, 1H), 7.55 (d, 1H), 7.32 (d, 2H), 7.18 (d, 2H), 4.03 (dd, 1H), 3.72 (m, 3H), 3.60/3.20 (bm, 8H), 3.45 (s, 3H), 2.75 (s, 3H), 2.41 (m, 2H), 2.25 (m, 2H), 2.14 (m, 1H), 1.66/1.10 (t/m, 2H). MS (*m*/*z*): 575 [MH]⁺.

### Example 61: (1R,5S/1S,5R)-1-[3-Fluoro-4-(trifluoromethyl)phenyl]-5-methyl-3-(3-{[4-methyl-5-(4-methyl-1,3-oxazol-5-yl)-4H-1,2,4-triazol-3-yl]thio}propyl)-3-azabicyclo[3.1.0]hexane hydrochloride

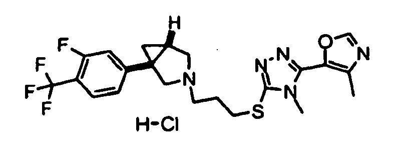

The title compound was prepared in analogy to the method described in Example 1 in 247 mg yield as a white slightly hygroscopic solid from (1*R*,5*S*/1*S*,5*R*)-1-[3-fluoro-4-(trifluoromethyl)phenyl]-3-azabicyclo[3.1.0]hexane (338 mg).

NMR (¹H, CD3OD): δ 8.4 (s, 1H), 7.55 (t, 1H), 7.37 (d, 1H), 7.32 (d, 1H), 4.2 (d, 1H), 3.91 (d, 1H), 3.81 (s, 3H), 3.76 (d, 1H), 3.67 (d, 1H), 3.51 (t, 2H), 3.43 (t, 2H), 2.47 (s, 3H), 2.41 (m, 1H), 2.31 (m, 2H), 1.61 (t, 1H), 1.45 (t, 1H). MS (*m*/*z*): 496 [MH]⁺.

Example 61 was separated to give the separated enantiomers by semipreparative Supercritical Fluid Chromatography (Gilson) using a chiral column Chiralpak AD-H, 25 x 2.1 cm, eluent CO₂ containing 12% (Ethanol + 0.1% isopropylamine), flow rate 22 mL/min, P 194 bar, T 36°C, detection UV at 220 nm. Retention times given were obtained using an analytical Supercritical Fluid Chromatography (Berger) using a chiral column Chiralpak AD-H, 25 x 0.46 cm, eluent CO₂ containing 10% (ethanol + 0.1% isopropylamine), flow rate 2.5 mL/min, P 180 bar, T 35°C, detection UV at 220 nm.
Enantiomer 1 was recovered in 42 mg yield as white solid, hydrochloride salt from the racemate (100 mg). Rt. = 27.1 min. Purity 100% a/a by UV.
Enantiomer 2 was recovered in 34 mg yield as white solid, hydrochloride salt from the racemate (100 mg). Rt. = 31.0 min. Purity 100% a/a by UV.
Enantiomer 1 showed fpKi (D3) > 2 log-unit higher than Enantiomer 2.

### Example 62: 1-(2-(Methyloxy)-5-{(1R,5S/1S,5R)-3-[3-({4-methyl-5-[4-(trifluoromethyl)phenyl]-4H-1,2,4-triazol-3-yl}thio)propyl]-3-azabicyclo[3.1.0]hex-1-yl}phenyl)ethanone hydrochloride

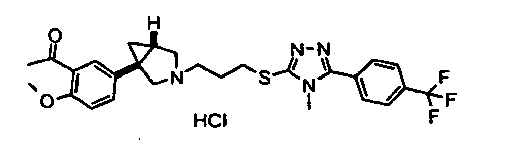

The title compound was prepared in analogy to the method described in Example 1 in 51 mg yield as a white solid (y=60%) from 1-[5-[(1*R*,5*S*/*1S*,*5R*)-3-azabicyclo[3.1.0]hex-1-yl]-2-(methyloxy)phenyl]ethanone (35 mg) and 3-[(3-chloropropyl)thio]-4-methyl-5-[4-(trifluoromethyl)phenyl]-4*H*-1,2,4-triazole (60 mg, prepared in analogy to the method described in Preparation 13).

NMR (¹H, CDCl₃, free base): δ 7.80-7.70 (m, 4H), 7.50 (s, 1H), 7.27-7.20 (m, 1H), 6.85 (d, 1H), 3.86 (s, 3H), 3.62 (s, 3H), 3.40-3.24 (m, 3H), 3.15 (d, 1H), 2.58 (s, 3H), 2.65-2.55 (m, 2H), 2.54-2.45 (m, 2H), 2.10-1.90 (quint, 2H), 1.65-1.57(m, 1H), 1.35 (m, 1H), 0.75 (m, 1H). MS *(m*/*z):* 531 [MH]⁺.

### Example 63: 1-[5-[(1R,5S/1S,5R)-3-(3-{[5-(3,4-Difluorophenyl)-4-methyl-4H-1,2,4-triazol-3-yl]thio}propyl)-3-azabicyclo[3.1.0]hex-1-yl]-2-(methyloxy)phenyl]ethanone hydrochloride

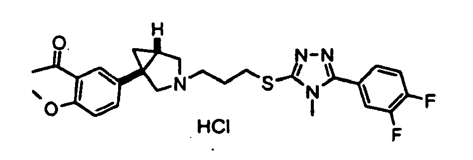

The title compound was prepared in analogy to the method described in Example 1 in 40 mg yield as a white solid (y=50%) from 1-[5-[(1*R*,5*S*/*1S*,*5R*)-3-azabicyclo[3.1.0]hex-1-yl]-2-(methyloxy)phenyl]ethanone (35 mg) and 3-[(3-chloropropyl)thio]-5-(3,4-difluorophenyl)-4-methyl-4*H*-1,2,4-triazole (54 mg, prepared in analogy to the method described in Preparation 13).

NMR (¹H, CDCl₃, free base): δ 7.56-7.19 (m, 5H), 6.84 (d, 1H), 3.86 (s, 3H), 3.62 (s, 3H), 3.38-3.24 (m, 3H), 3.10 (d, 1H), 2.58 (s, 3H), 2.65-2.42 (m, 4H), 2.10-1.90 (quint, 2H), 1.65-1.57(m, 1H), 1.35 (m, 1H), 0.75 (m, 1H). MS *(m*/*z):* 499 [MH]⁺.

### Example 64: 1-{2-(Methyloxy)-5-[(1R,5S/1S,5R)-3-(3-{[4-methyl-5-(3-pyridinyl)-4H-1,2,4-triazol-3-yl]thio}propyl)-3-azabicyclo[3.1.0]hex-1-yl]phenyl}ethanone hydrochloride

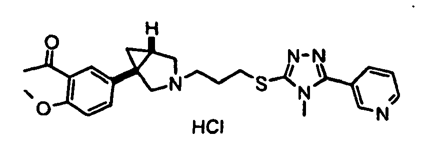

The title compound was prepared in analogy to the method described in Example 1 in 32 mg yield as a yellow solid (y=42%) from 1-[5-[(1*R*,5*S*/*1S*,*5R*)-3-azabicyclo[3.1.0]hex-1-yl]-2-(methyloxy)phenyl]ethanone (35 mg) and 3-{5-[(3-chloropropyl)thio]-4-methyl-4*H*-1,2,4-triazol-3-yl}pyridine (48 mg, prepared in analogy to the method described in Preparation 13).

NMR (¹H, CDCl₃, free base): δ 8.87 (s, 1H), 8.70 (d, 1H), 8.0 (d, 1H), 7.48 (s, 1H), 7.43 (m, 1H), 7.23 (m, 1H), 6.84 (d, 1H), 3.86 (s, 3H), 3.62 (s, 3H), 3.40-3.25 (m, 3H), 3.10 (d, 1H), 2.58 (s, 3H), 2.67-2.42 (m, 4H), 2.10-1.90 (quint, 2H), 1.65-1.57(m, 1H), 1.35 (m, 1H), 0.75 (m, 1H). MS *(m*/*z):* 464 [MH]⁺.

### Example 65: 1-[5-[(1R,5S/1S,5R)-3-(3-{[4-Methyl-5-(2-methyl-5-quinolinyl)-4H-1,2,4-triazol-3-yl]thio}propyl)-3-azabicyclo[3.1.0]hex-1-yl]-2-(methyloxy)phenyl]ethanone hydrochloride

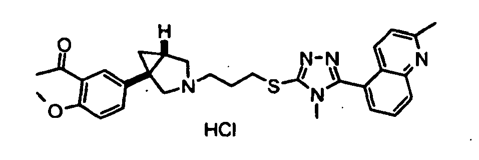

The title compound was prepared in analogy to the method described in Example 1 in 50 mg yield as a yellow solid (y=60%) from 1-[5-[(1*R*,5*S*/*1S*,*5R*)-3-azabicyclo[3.1.0]hex-1-yl]-2-(methyloxy)phenyl]ethanone (35 mg) and 5-{5-[(3-chloropropyl)thio]-4-methyl-4*H*-1,2,4-triazol-3-yl}-2-methylquinoline (60 mg).

NMR (¹H, DMSO): δ 10.38 (bs,1H), 8.2 (m, 2H), 7.91 (t, 1H), 7.78 (d, 1H), 7.5 (m, 3H), 7.17 (d, 1H), 4.02 (d, 1H), 3.89 (s, 3H), 3.74 (dd, 1H), 3.6-3.2 (m, 6H), 3.45 (s, 3H), 2.72 (s, 3H), 2.5 (s, 3H), 2.23 (quint, 2H), 2.11 (quint, 1H), 1.57 (t, 1H), 1.1 (t, 1H). MS *(m*/*z):* 528 [MH]⁺.

### Example 66: 1-{2-(Methyloxy)-5-[(1R,5S/1S,5R)-3-(3-{[4-methyl-5-(tetrahydro-2H-pyran-4-yl)-4H-1,2,4-triazol-3-yl]thiol}propyl)-3-azabicyclo[3.1.0]hex-1-yl]phenyl}ethanone hydrochloride

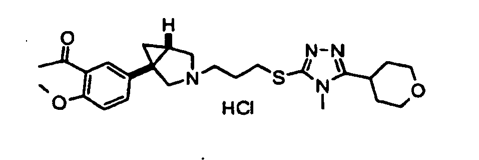

The title compound was prepared in analogy to the method described in Example 1 in 24 mg yield as a white solid (y=32%) from 1-[5-[(1*R*,5*S*/*1S*,*5R*)-3-azabicyclo[3.1.0]hex-1-yl]-2-(methyloxy)phenyl]ethanone (35 mg) and 3-[(3-chloropropyl)thio]-4-methyl-5-(tetrahydro-2*H*-pyran-4-yl)-4*H*-1,2,4-triazole (50 mg, prepared in analogy to the method described in Preparation 13).

NMR (¹H, CDCl₃, free base): δ 7.49 (s, 1H), 7.24 (m, 1H), 6.85 (d, 1H), 4.14-4.05 (m, 2H), 3.86 (s, 3H), 3.62 (s, 3H), 3.57-3.40 (m, 2H), 3.29-3.15 (m, 3H), 3.05 (d, 1H), 2.82-2.95 (m, 1H), 2.63-2.40 (m, 4H), 2.58 (s, 3H), 2.15-1.77 (m, 6H), 1.62 (m, 1H), 1.32 (m, 1H), 0.70 (m, 1H). MS *(m*/*z):* 471 [MH]⁺.

### Example 67: 1-(2-Hydroxy-5-{(1R,5S/1S,5R)-3-[3-({4-methyl-5-[4-(trifluoromethyl)phenyl]-4H-1,2,4-triazol-3-yl}thio)propyl]-3-azabicyclo[3.1.0]hex-1-yl}phenyl)ethanone hydrochloride

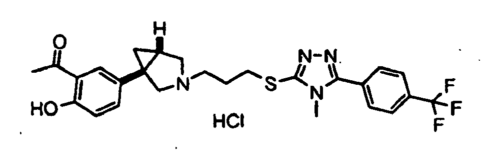

The title compound was prepared in analogy to the method described in Example 1 in 35 mg yield as a white solid (y=33%) from 1-{5-[(1*R*,5*S*/*1S*,*5R*)-3-azabicyclo[3.1.0]hex-1-yl]-2-hydroxyphenyl}ethanone (43 mg) and 3-[(3-chloropropyl)thio]-4-methyl-5-[4-(trifluoromethyl)phenyl]-4*H*-1,2,4-triazole (80 mg, prepared in analogy to the method described in Preparation 13).

NMR (¹H, CDCl₃, free base): δ 12.2 (s, 1H), 7.80-7.70 (m, 4H), 7.50 (s, 1H), 7.30 (m, 1H), 6.88 (d, 1H), 3.86 (s, 3H), 3.40-3.25 (m, 3H), 3.10 (d, 1H), 2.58 (s, 3H), 2.65-2.35 (m, 4H), 2.0 (quint, 2H), 1.58 (m, 1H), 1.35 (m, 1H), 0.70 (m, 1H). MS *(m*/*z):* 517 [MH]⁺.

### Example 68: 1-{5-[(1R,5S/1S,5R)-3-(3-{[5-(3,4-Difluorophenyl)-4-methyl-4H-1,2,4-triazol-3-yl]thio}propyl)-3-azabicyclo[3.1.0]hex-1-yl]-2-hydroxyphenyl}ethanone hydrochloride

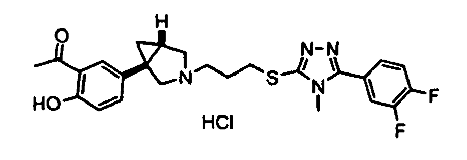

The title compound was prepared in analogy to the method described in Example 1 in 27 mg yield as a white solid (y=29%) from 1-{5-[(1*R*,5*S*/*1S*,*5R*)-3-azabicyclo[3.1.0]hex-1-yl]-2-hydroxyphenyl}ethanone (40 mg) and 3-[(3-chloropropyl)thio]-5-(3,4-difluorophenyl)-4-methyl-4*H*-1,2,4-triazole (67 mg, prepared in analogy to the method described in Preparation 13).

NMR (¹H, DMSO): δ 11.82 (s,1H), 10.26 (bs, 1H), 7.85 (m, 1H), 7.76 (d, 1H), 7.67 (m, 1H), 7.61 (m, 1H), 7.51 (dd, 1H), 6.97 (d, 1H), 4.02 (dd, 1H), 3.74 (dd, 1H), 3.64 (s, 3H), 3.55 (m, 2H), 3.3 (m, 4H), 2.67 (s, 3H), 2.15 (m, 3H), 1.52 (t, 1H), 1.13 (t, 1H). MS *(mlz)*: 485 [MH]⁺.

### Example 69: 1-{2-Hydroxy-5-[(1R,5S/1S,5R)-3-(3-{[4-methyl-5-(4-methyl-1,3-oxazol-5-yl)-4H-1,2,4-triazol-3-yl]thio}propyl)-3-azabicyclo[3.1.0]hex-1-yl]phenyl}ethanone hydrochloride

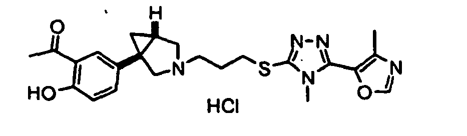

The title compound was prepared in analogy to the method described in Example 1 in 36 mg yield as a white solid (y=43%) from 1-{5-[(1*R*,5*S*/*1S*,*5R*)-3-azabicyclo[3.1.0]hex-1-yl]-2-hydroxyphenyl}ethanone (38 mg) and 3-[(3-chloropropyl)thio]-4-methyl-5-(4-methyl-1,3-oxazol-5-yl)-4*H*-1,2,4-triazole (57 mg).

NMR (¹H, CDCl₃, free base): δ 12.2 (s, 1H), 7.88 (s, 1H), 7.50 (s, 1H), 7.24 (m, 1H), 6.58 (d, 1H), 3.68 (s, 3H), 3.32 (m, 3H), 3.10 (d, 1H), 2.58-2.47 (m, 4H), 2.58 (s, 3H), 2.47 (s, 3H), 2.0 (m, 2H), 1.62 (m, 1H), 1.35 (m, 1H), 0.68 (m, 1H). MS *(m*/*z):* 454 [MH]⁺.

### Example 70: 1-{2-Hydroxy-5-[(1R,5S/1S,5R)-3-(3-{[4-methyl-5-(2-methyl-5-quinolinyl)-4H-1,2,4-triazol-3-yl]thio}propyl)-3-azabicyclo[3.1.0]hex-1-yl]phenyl}ethanone hydrochloride

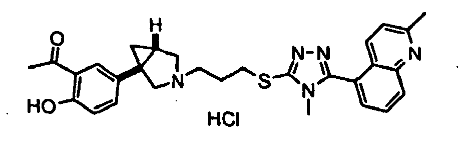

The title compound was prepared in analogy to the method described in Example 1 in 24 mg yield as a yellow solid (y=32%) from 1-{5-[(1*R*,5*S*/*1S*,*5R*)-3-azabicyclo[3.1.0]hex-1-yl]-2-hydroxyphenyl}ethanone (30 mg) and 5-{5-[(3-Chloropropyl)thio]-4-methyl-4*H*-1,2,4-triazol-3-yl}-2-methylquinoline (55 mg).

NMR (¹H, CDCl₃, free base): δ 12.1 (s, 1H), 8.10 (dd, 2H), 7.67 (t, 1H), 7.50 (m, 2H), 7.23 (m, 2H), 6.85 (d, 1H), 3.45-3.23 (m, 3H), 3.40 (s, 3H), 3.08 (d, 1H), 2.67 (s, 3H), 2.65-2.41 (m, 4H), 2.55 (s, 3H), 2.02 (m, 2H), 1.58 (m, 1H), 1.32 (m, 1H), 0.64 (m, 1H). MS (m/z): 514 [MH]⁺.

### Example 71: 1-[5-[(1R,5S/1S,5R)-3-(3-{[4-Methyl-5-(4-methyl-1,3-oxazol-5-yl)-4H-1,2,4-triazol-3-yl]thio}propyl)-3-azabicyclo[3.1.0]hex-1-yl]-2-(methyloxy)phenyl]-1-propanone hydrochloride

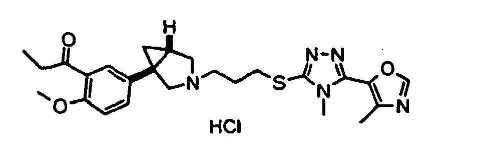

The title compound was prepared in analog y to the method described in Example 1 in 51 mg yield as a white solid (y=47%) from 1-[5-[(1*R*,5*S*)-3-azabicyclo[3.1.0]hex-1-yl]-2-(methyloxy)phenyl]-1-propanone (52 mg, prepared in analogy to the method described in Preparations 43-45) and 3-[(3-chloropropyl)thio]-4-methyl-5-(4-methyl-1,3-oxazol-5-yl)-4*H-*1,2,4-triazole (69 mg).

NMR (¹H, DMSO): δ 10.24 (bs,1H), 8.52 (m, 1H), 7.42 (d, 1H), 7.40 (dd, 1H), 7.08 (d, 1H), 3.93 (dd, 1H), 3.81 (s, 3H), 3.67 (dd, 1 H), 3.64 (s, 3H), 3.48 (m, 2H), 3.28 (m, 2H), 3.22 (t, 2H), 2.86 (q, 2H), 2.33 (s, 3H), 2.1 (m, 2H), 2.03 (m, 1H), 1.48 (t, 1H), 1.0 (t, 1H), 1.04 (t, 3H). MS *(m*/*z)*: 482 [MH]⁺.

The title compound was separated to give the separated enantiomers by semi-preparative HPLC using a chiral column chiralpak AS-H 5 µm, 250 x 21 mm, eluent A: n-hexane; B: ethanol + 0.1% isopropylamine, gradient isocratic 40% B, flow rate 7 mL/min, detection UV at 200-400 nm. Retention times given were obtained using an analytical HPLC using a chiral column chiralpak AS-H 5 µm, 250 x 4.6 mm, eluent A: n-hexane; B: ethanol + 0.1% isopropylamine, gradient isocratic 40% B, flow rate 0.8 mL/min, detection UV at 200-400 nm.
Enantiomer 1 was recovered in 10 mg yield as white solid (y=30%) from the racemate (66 mg). Rt. = 17.2 min.
Enantiomer 2 was recovered in 10 mg yield as white solid (y=30%) from the racemate (66 mg). Rt. = 19.1 min.
Enantiomer 1 showed fpKi (D3) > 1 log-unit higher than Enantiomer 2.

### Example 72: 2-Methyl-5-[(1R,5S/1S,5R)-3-(3-{[4-methyl-5-(4-methyl-1,3-oxazol-5-yl)-4H-1,2,4-triazol-3-yl]thio}propyl)-3-azabicyclo[3.1.0]hex-1-yl]-1,3-benzothiazole hydrochloride

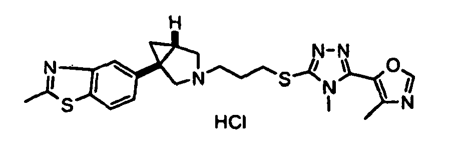

5-[(1*R*,5*S*)-3-Azabicyclo[3.1.0]hex-1-yl]-2-methyl-1,3-benzothiazole was prepared from 2-methyl-1,3-benzothiazol-5-amine dihydrochloride in analogy to the method described in Preparations 15, 16 and 17. From this material the title compound was obtained as a yellow solid following the method described for Examples 14 and 15.

NMR (¹H, DMSO): δ 10.54 (bs,1H), 8.58 (m, 1H), 8.0 (d, 1H), 7.9 (d, 1H), 7.34 (dd, 1H), 4.0 (dd, 1H), 3.75 (dd, 1H), 3.70 (s, 3H), 3.65 (m, 1H), 3.57 (m, 1H), 3.35 (m, 2H), 3.30 (t, 2H), 2.8 (s, 3H), 2.39 (s, 3H), 2.27 (m, 1H), 2.19 (m, 2H), 1.7 (t, 1H), 1.19 (t, 1H). MS *(m*/*z)*: 467 [MH]⁺.

The title compound was separated to give the separated enantiomers by semi-preparative HPLC using a chiral column chiralpak AS-H 5 µm, 250 x 21 mm, eluent A: n-hexane; B: ethanol + 0.1% isopropylamine, gradient isocratic 13% B, flow rate 7 mL/min, detection UV at 200-400 nm. Retention times given were obtained using an analytical HPLC using a chiral column chiralpak AS-H 5 µm, 250 x 4.6 mm, eluent A: n-hexane; B: ethanol + 0.1% isopropylamine, gradient isocratic 13% B, flow rate 1 mUmin, detection UV at 200-400 nm.
Enantiomer 1 was recovered in 17 mg yield as white solid (y=62%) from the racemate (55 mg). Rt. = 17.1 min.
Enantiomer 2 was recovered in 18 mg yield as white solid (y=65%) from the racemate (55 mg). Rt. = 19.3 min.
Enantiomer 1 showed fpKi (D3) > 1 log-unit higher than Enantiomer 2.

### Example 73: 2-Methyl-6-[(1R,5S/1S,5R)-3-(3-{[4-methyl-5-(4-methyl-1,3-oxazol-5-yl)-4H-1,2,4-triazol-3-yl]thio}propyl)-3-azabicyclo[3.1.0]hex-1-yl]-1,3-benzothiazole hydrochloride

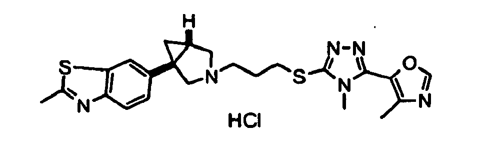

6-[(1*R*,5*S*/*1S*,*5R*)-3-Azabicyclo[3.1.0]hex-1-yl]-2-methyl-1,3-benzothiazole was prepared from 2-methyl-1,3-benzothiazol-6-amine in analogy to the method described in Preparations 15, 16 and 5. From this material the title compound was obtained as a yellow solid following the method described for Examples 14 and 15.

NMR (¹H, CD₃OD): δ 8.39 (s,1H), 7.98 (d, 1H), 7.89 (d, 1H), 7.5 (dd, 1H), 4.19 (d, 1H), 3.92 (d, 1H), 3.8 (s, 3H), 3.72 (d, 2H), 3.52 (t, 2H), 3.42 (t, 2H), 2.85 (s, 3H), 2.47 (s, 3H), 2.31 (m, 3H), 1.54 (t, 1H), 1.41 (t, 1H). MS *(m*/*z):* 467 [MH]⁺.

### Example 74: 1-Methyl-5-[(1R,5S/1S,5R)-3-(3-{[4-methyl-5-(4-methyl-1,3-oxazol-5-yl)-4H-1,2,4-triazol-3-yl]thio}propyl)-3-azabicyclo[3.1.0]hex-1-yl]-1H-indazole hydrochloride

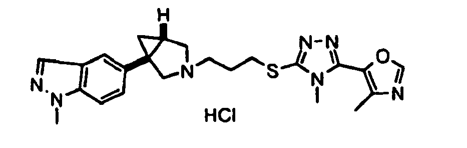

5-[(1*R*,5*S*/*1S*,*5R*)-3-Azabicyclo[3.1.0]hex-1-yl]-1-methyl-1*H*-indazole was prepared from 1-methyl-1*H*-indazol-5-amine in analogy to the method described in Preparations 15, 16 and 5. From this material the title compound was obtained as a yellow solid following the method described for Examples 14 and 15.

NMR (¹H, DMSO): δ 10.4 (bs, 1H), 8.58 (m, 1H), 8.01 (s, 1H), 7.70 (d, 1H), 7.63 (d, 1H), 7.39 (dd, 1H), 4.05 (m, 1H), 4.04 (s, 3H), 3.75 (d, 1H), 3.70 (s, 3H), 3.59 (m, 2H), 3.39 (t, 2H), 3.26 (t, 2H), 2.39 (s, 3H), 2.18 (m, 3H), 1.61 (t, 1H), 1.14 (t, 1H). MS *(m*/*z):* 450 [MH]⁺.

The title compound was separated to give the separated enantiomers by semi-preparative SFC (Gilson) using a chiral column chiralpak AS-H, 250 x 21 mm, modifier: ethanol + 0.1% isopropylamine 12%, flow rate 22 mL/min, P 200 bar, T 36 °C, detection UV at 220 nm. Retention times given were obtained using an analytical SFC (Berger) using a chiral column chiralpak AS-H 5 µm, 250 x 46 mm, modifier: ethanol + 0.1% isopropylamine 12%, flow rate 2.5 mL/min, P 180 bar, T 35 °C, detection UV at 220 nm.
Enantiomer 1 was recovered in 25 mg yield as white solid (y=62%) from the racemate (80 mg). Rt. = 19.5 min.
Enantiomer 2 was recovered in 28 mg yield as white solid (y=70%) from the racemate (80 mg). Rt. = 22.8 min.
Enantiomer 1 showed fpKi (D3) > 1 log-unit higher than Enantiomer 2.

### Example 75: (1R,5S/1S,5R)-3-(3-{[4-Methyl-5-(4-methyl-1,3-oxazol-5-yl)-4H-1,2,4-triazol-3-yl]thio}propyl)-1-[6-(trifluoromethyl)-3-pyridinyl]-3-azabicyclo[3.1.0]hexane hydrochloride

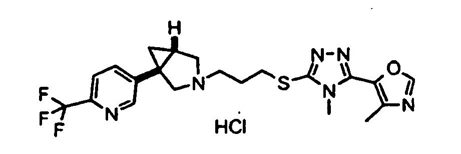

(1*R*,5*S*/*1S*,*5R*)-1-[6-(Trifluoromethyl)-3-pyridinyl]-3-azabicyclo[3.1.0]hexane was prepared from 6-(trifluoromethyl)-3-pyridinamine in analogy to the method described in Preparations 37 and 5. From this material the title compound was obtained as a yellow solid following the method described for Examples 14 and 15.

NMR (¹H, DMSO): δ 10.46 (bs,1H), 8.73 (bs, 1H), 8.58 (m, 1H), 8.0 (dd, 1H), 7.90 (d, 1H), 4.12 (m, 1H), 3.78 (d, 1H), 3.70 (s, 3H), 3.7 (m, 1H), 3.54 (m, 1H), 3.39 (t, 2H), 3.29 (s, 2H), 2.39 (m, 3H), 2.47 (m, 1H), 2.18 (m, 2H), 1.71 (m, 1H), 1.33 (m, 1H). NMR (¹⁹F, DMSO): δ -66.2 (s). MS *(mlz)*: 465 [MH]⁺.

### Examples 76-94:

To a solution of the respective 3-thio-5-aryl-1,2,4-triazole (prepared in analogy to the method described in Preparation 13, 0.063 mmol) in dry acetonitrile (2 mL) 2-*tert-*butylimino-2-diethylamino-1,3-dimethyl-perhydro-1,3,2-diaza-phosphorine on polystyrene (43 mg, 2.2 mmol/g) was added and the resulting mixture was shaken for 1 h at room temperature, then (1*R*,5*S*/*1S*,*5R*)-1-(4-bromophenyl)-3-(3-chloropropyl)-3-azabicycle-[3.1.0]hexane (20 mg) was added and the resulting mixture was shaken at 70 °C for 3.5 hours. After cooling the resin was removed by filtration, washed with dichloromethane (2 mL) and methanol (2 mL) and the collected liquid phase was evaporated under reduced pressure. Two isomers were formed by *S*- and *N*- alkylation, the major isomer being the desired S-alkylated. Those isomers were separated using mass directed HPLC using a Waters XTerra Prep MS C18 10 µm, 30x150 mm column using the following conditions:

| | **Time** | **Flow** | **%A** | **% B** |
|---|---|---|---|---|
| **Prerun** | 0 | 40 ml/min | 99 | 1 |
| | 1 | 40 ml/min | 99 | 1 |
| **Run** | 0 | 40 ml/min | 99 | 1 |
| | 10 | 40 ml/min | 75 | 25 |
| | 14.5 | 40 ml/min | 10 | 90 |
| | 15 | 40 ml/min | 0 | 100 |
| **Postrun** | 0 | 40 ml/min | 0 | 100 |
| | 0.2 | 45 ml/min | 0 | 100 |
| | 1.5 | 45 ml/min | 0 | 100 |
| | 2 | 40 ml/min | 0 | 100 |

| | | | | |
|---|---|---|---|---|
| A= H2O + 0.1% formic acid B = acetonitrile + 0.1% formic acid | | | | |

Then solvent was removed under reduced pressure to give title compounds was formate salts.

In the case of Examples 93 and 94 the isomers were separated by silica gel flash chromatography. The S-alkylated isomers were dissolved in dry diethyl ether and cooled at 0 °C. 1.2 eq of HCl (as 1.0 M solution in diethyl ether) were slowly added. The resulting precipitate was decanted, washed with pentane and filtered, yielding the products as the hydrochloride salts.

### Analytical conditions:

### Examples 76-90:

| | |
|---|---|
| Column | X-Terra MS C18 5 um, 50 x 4.6 mm |
| Mobile Phase | A: H20 + 0.1 % TFA; B: CH3CN + 0.1 % TFA |
| Gradient | 10% (B) for 1 min; from 10% (B) to 90% (B) in 12 min; 90% (B) for 3 min |
| Flow rate | 1 mL/min |
| UV wavelength | 200-400 nm |
| range | |
| Mass range | 100-900 amu |
| lonisation | ES+ |

### Example 91:

| | |
|---|---|
| Column | X-Terra MS C18 5 um, 50 x 4.6 mm |
| Mobile Phase | A: H20 + 0.2 % HCOOH; B: CH3CN + 0.2 % HCOOH |
| Gradient | 10% (B) for 1 min; from 10% (B) to 95% (B) in 12 min; 95% (B) |
| | for 3 min |
| Flow rate | 1 mL/min |
| UV wavelength | 210-400 nm |
| range | |
| Mass range | 100-900 amu |
| lonisation | ES+ |

### Example 93:

| | |
|---|---|
| Analytical Column | ZORBAX SB C18, 50 mm, 4.6 mm i.d.; 1.8 um |
| Mobile phase | Amm.Acet., 5mM/ Acetonitrile+0.1%Formic Acid |
| Gradient | 97/3 > 36/64 v/v in 3.5 min >0/100 v/v in 3.5 min |
| Flow rate | 2 mL/min |
| Detection | DAD, 210-350 nm |
| MS | ES+ |
| Retention time | 2.42 min |
| [M+H]+ | 484/486 (1 Br pattern) |
| Assay | 98.17 % ala (by DAD) |

### Example 94:

| | |
|---|---|
| Analytical Column | ZORBAX SB C18, 50 mm, 4.6 mm i.d.; 1.8 um |
| Mobile phase | Amm.Acet., 5mM/ Acetonitrite+0.1%Formic Acid |
| Gradient | 97/3 > 36/64 v/v in 3.5 min >0/100 v/v in 3.5 min |
| Flow rate | 2 mL/min |
| Detection | DAD, 210-350 nm |
| MS | ES+ |
| Retention time | 3.02 min |
| [M+H]+ | 552/554 (1 Br pattern) |
| Assay | 99.51 % ala (by DAD) |

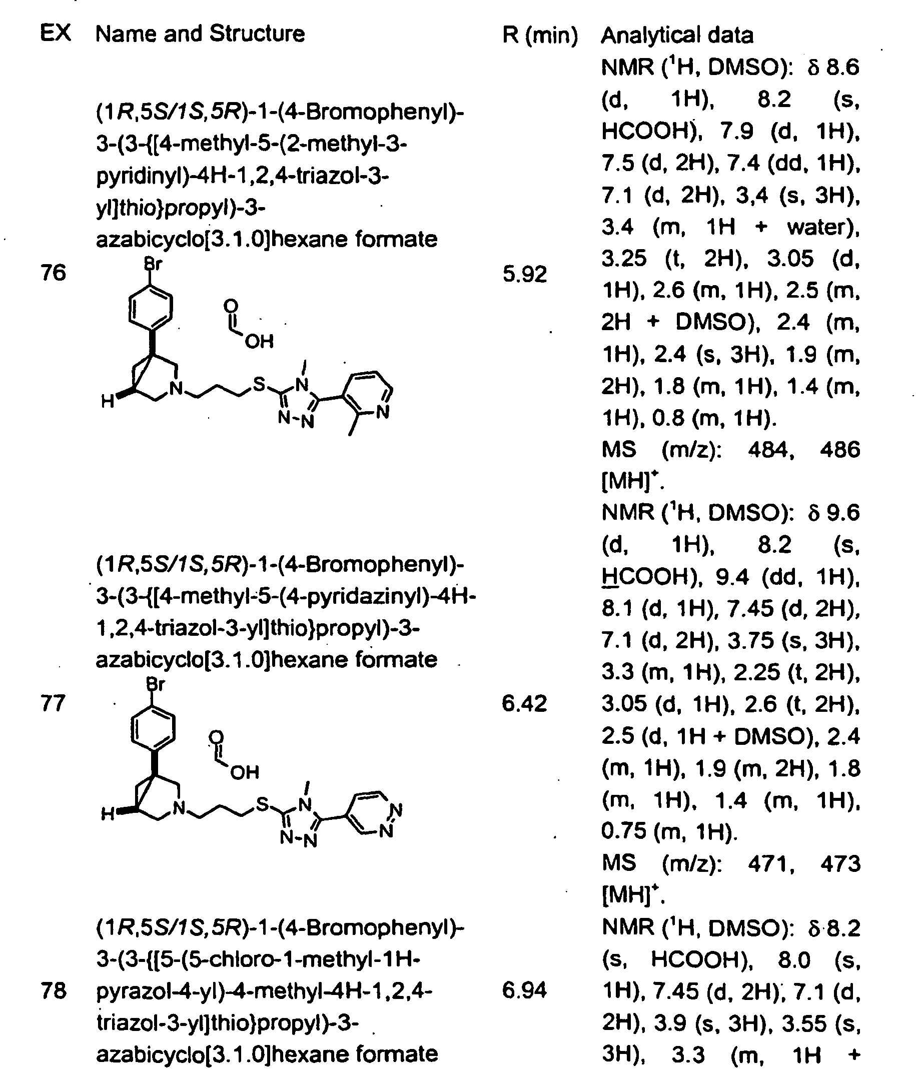

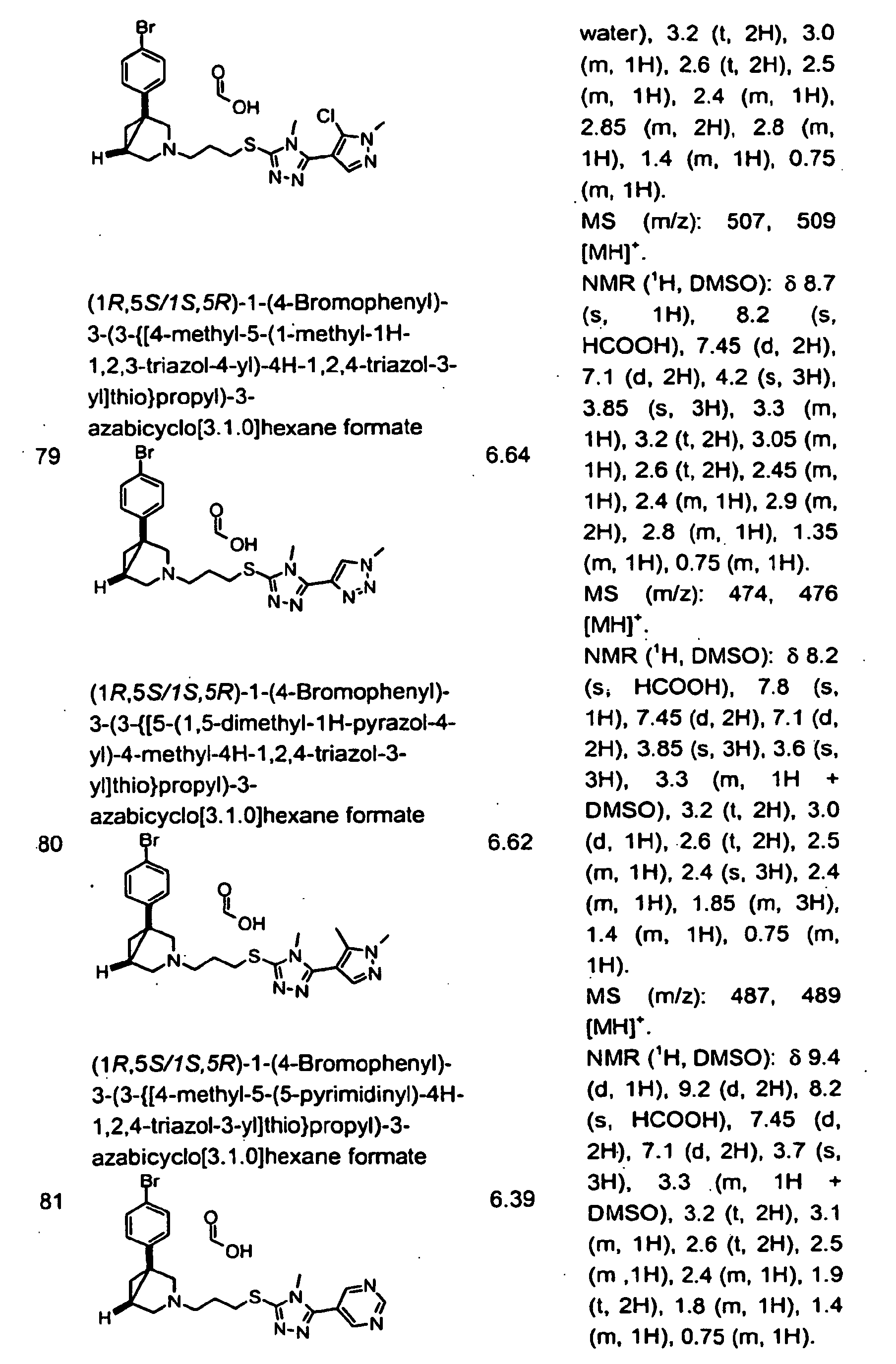

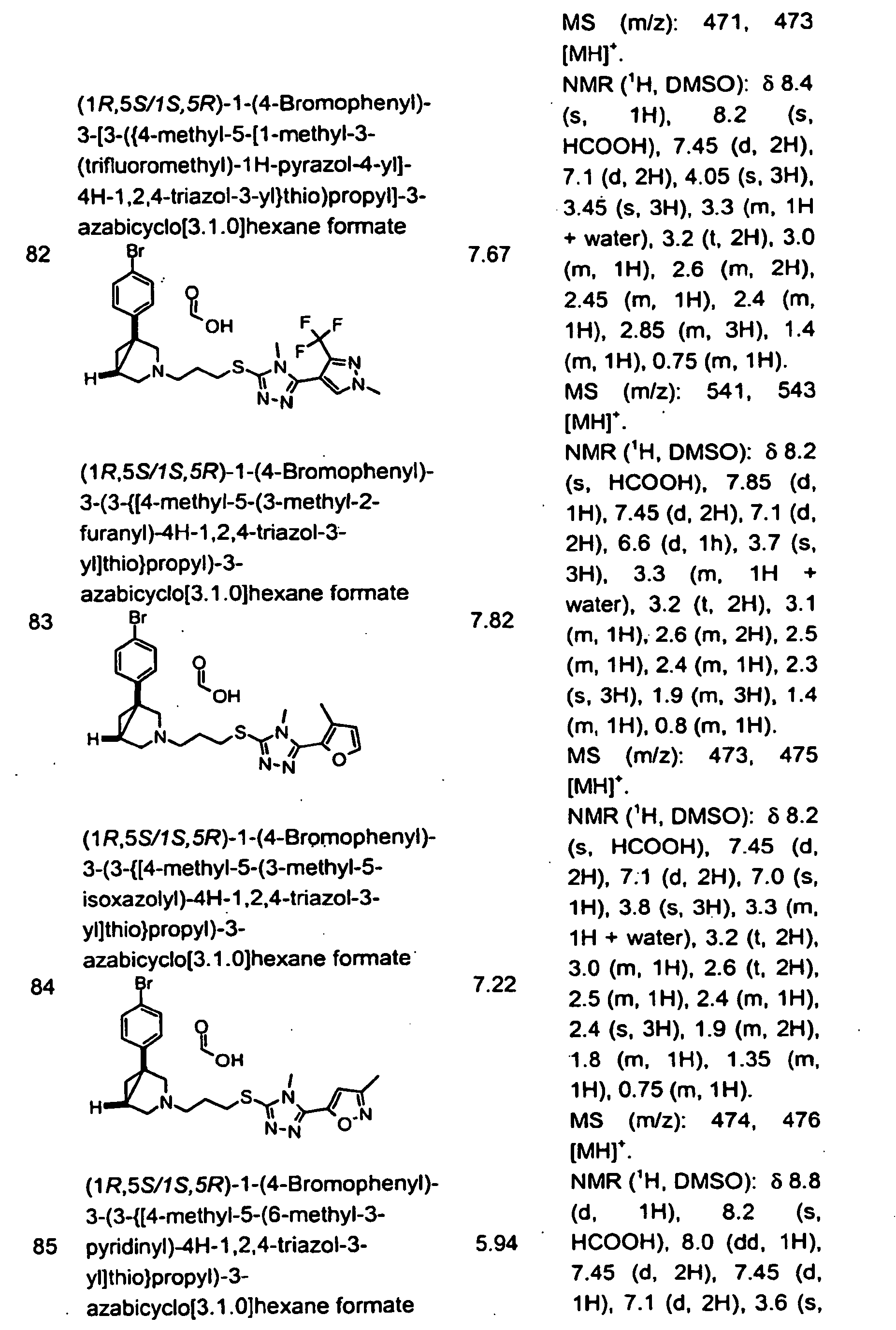

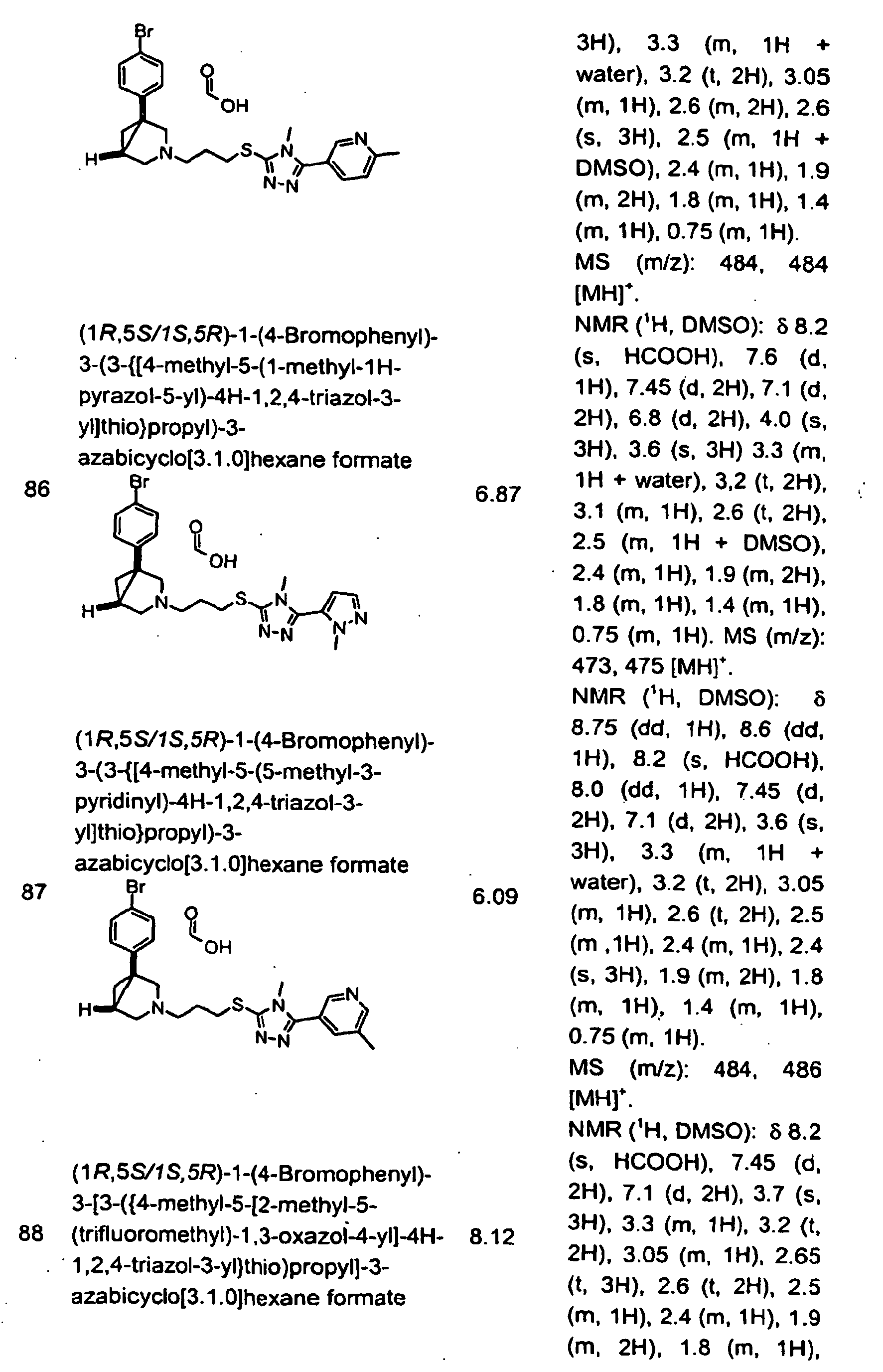

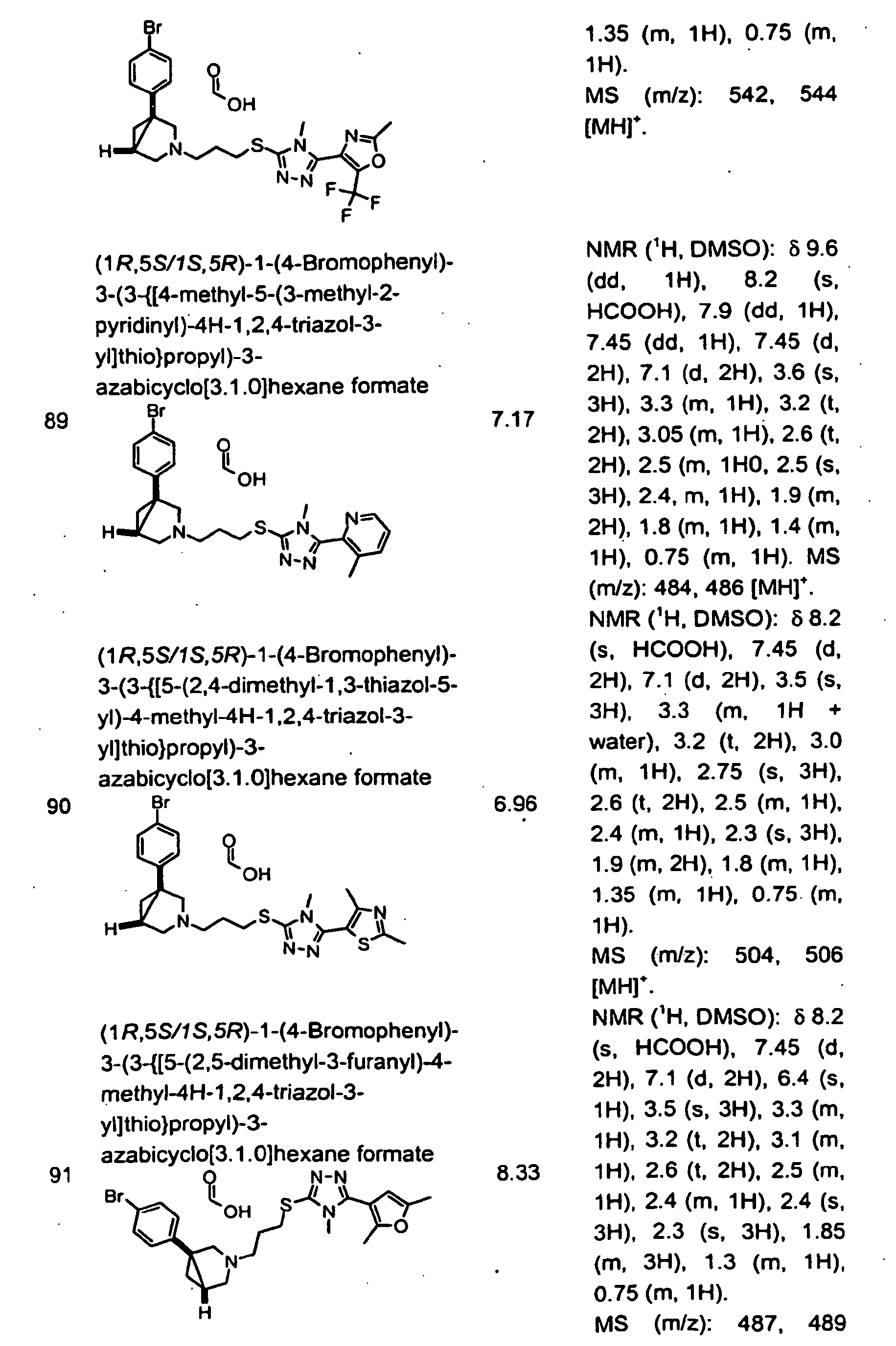

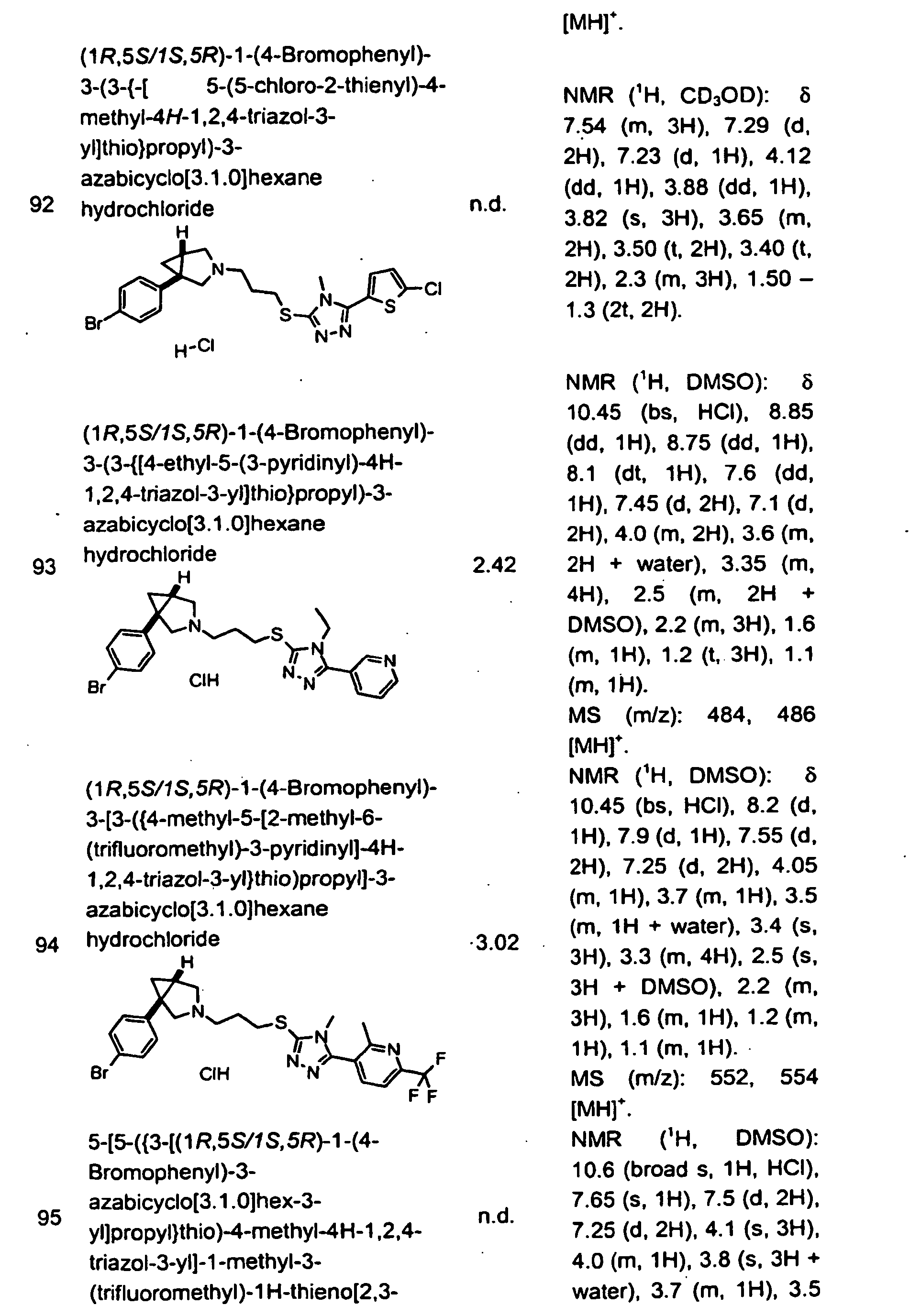

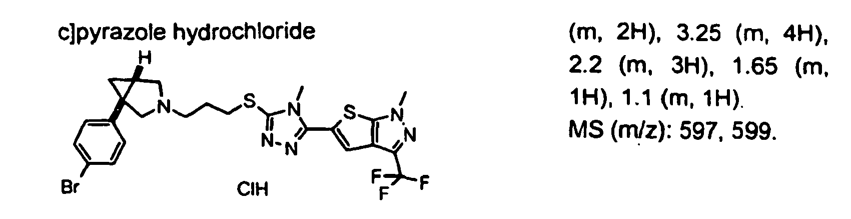

### Example 96: 3-(3-{[4-Methyl-5-(4-methyl-1,3-oxazol-5-yl)-4H-1,2,4-triazol-3yl]thio}propyl)-(1R,5R/1S,5S)-1-[5-(trifluoromethyl)-2-pyridinyl]-3-azabicyclo[3.1.0]hexane hydrochloride

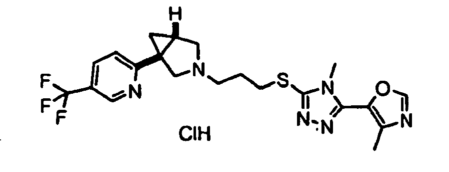

The title compound was prepared in analogy to the method described in Example 1 as a white slightly hygroscopic solid (70 mg, 45%), from 1-[5-(trifluoromethyl)-2-pyridinyl]-3-azabicyclo[3.1.0]hexane.

NMR (corresponding free base, ¹H, CD₃OD): δ 8.71 (s, 1H), 7.93 (s, 1H), 7.78 (dd, 1H), 7.15 (d, 1H), 3.72 (s, 3H), 3.41 (d, 1H), 3.36 (t, 2H), 3.13 (d, 1H), 2.8 (d, 1H), 2.68 (m, 2H), 2.54 (s, 3H), 2.48 (dd, 1H), 2.04 (m, 3H), 1.6 (m, 1H), 1.26 (dd, 1H). MS *(m*/*z):* 465 [MH]⁺.

Example 96 was separated to give the separated enantiomers by semi-preparative HPLC using a chiral column Chirapak AD-H 5 µm, 250 x 4.6 mm, eluent A: n-hexane; B: Ethanol + 0.1% isopropylamine, gradient isocratic 30% B v/v, flow rate 6 mL/min, detection UV at 270 nm. Retention times given were obtained using a chiral column Chirapak AD-H 5 µm, 250 x 4.6 mm, eluent A: n-hexane; B: Ethanol, gradient isocratic 30% B v/v, flow rate 0.8 mUmin, detection UV at 200 - 400 nm.
Enantiomer 1 was recovered in 18 mg yield as a white solid, hydrochloride salt from the racemate (70 mg). Rt. = 19.09 min. Purity 100% a/a by UV.
Enantiomer 2 was recovered in 18 mg yield as white solid, hydrochloride salt from the racemate (70 mg). Rt. = 21.6 min. Purity 99% a/a by UV.
Enantiomer 2 showed fpKi (D3) > 1 log-unit higher than Enantiomer 1.

### Example 97: 3-(3-{[4-methyl-5-(4-methyl-1,3-oxazol-5-yl)-4H-1,2,4-triazol-3-yl]thio}propyl)-(1R,5R/1S,5S)-1-[6-(trifluoromethyl)-2-pyridinyl]-3-azabicyclo[3.1.0]hexane dihydrochloride

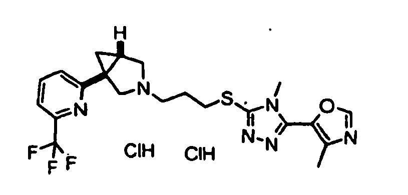

3-(Phenylmethyl)-1-[5-(trifluoromethyl)-2-pyridinyl]-3-azabicyclo[3.1.0]hexane (0.19 mmol) was dissolved in 1,2-dichloroethane (1 mL) and 1-chloroethyl chloridocarbonate was added (0.21 mmol). After two microwave cycles (5 min at 120 °C and 10 min at 140 °C) the solvent was removed at reduced pressure. Methanol (2 mL) was added and the solution was submitted to an additional microwave cycle (10 min, 120 °C). The solvent was removed at reduced pressure to give 47 mg of intermediate which was used without further purification and treated in analogy to the method described in Example 1 to give the title compound (5 mg, 5%) as a white slightly hygroscopic solid.

NMR (¹H, CD₃OD): δ 8.38 (s, 1H), 7.99 (dd, 1H), 7.67 (dd, 1H), 7.48 (dd, 1H), 4.18 (d, 1H), 4.06 (d, 1H), 3.88 (d, 1H), 3.79 (s, 3H), 3.63 (dd, 1 H), 3.50 (m, 2H), 3.41 (t, 2H), 2.49 (m, 1 H), 2.44 (s, 3H), 2.31 (m, 2H), 1.69 (m, 1H), 1.65 (dd, 1H). MS (m/z): 465 [MH]⁺.

### Example 98: (1R,5S/1S,5R)-1-[3-Fluoro-4-(1H-pyrrol-1-ylmethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane hydrochloride

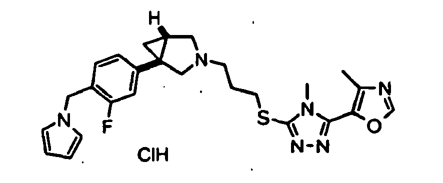

The title compound was prepared in analogy to the method described in Example 1 as a white slightly hygroscopic solid (5.4 mg, yield = 19%), from 1-[3-fluoro-4-(1*H*-pyrrol-1-ylmethyl)phenyl]-3-azabicyclo[3.1.0]hexane (Preparation 57).

NMR (as formate salt) (1H, CDCl3): δ 7,9 (s, 1H), 6.8 (m, 4H), 6.65 (s, 2H), 6.15 (s, 2H), 5.05 (s, 2H), 3.66 (s, 3H), 3.4 (d, 1H), 3.25 (t, 2H), 3.2 (d, 1H), 2,75 (t, 2H), 2.6 (d, 1H), 2.55 (m, 1H), 2.5 (s, 3H), 2.0 (m, 2H), 1.7 (m, 1H), 1.45 (t, 1H), 0.8 (m, 1H); acidic proton not observed. MS (hydrochloride salt) *(m*/*z):* 475 [MH]⁺.

### Examples 99-104:

### Examples 99-104 were prepared as white slightly hygroscopic solids from (1R,5S/1S,5R)-3-(3-chloropropyl)-1-[6-(trifluoromethyl)-3-pyridinyl]-3-azabicyclo[3.1.0]hexane (40 mg) in analogy to the method described for Examples 53-58.

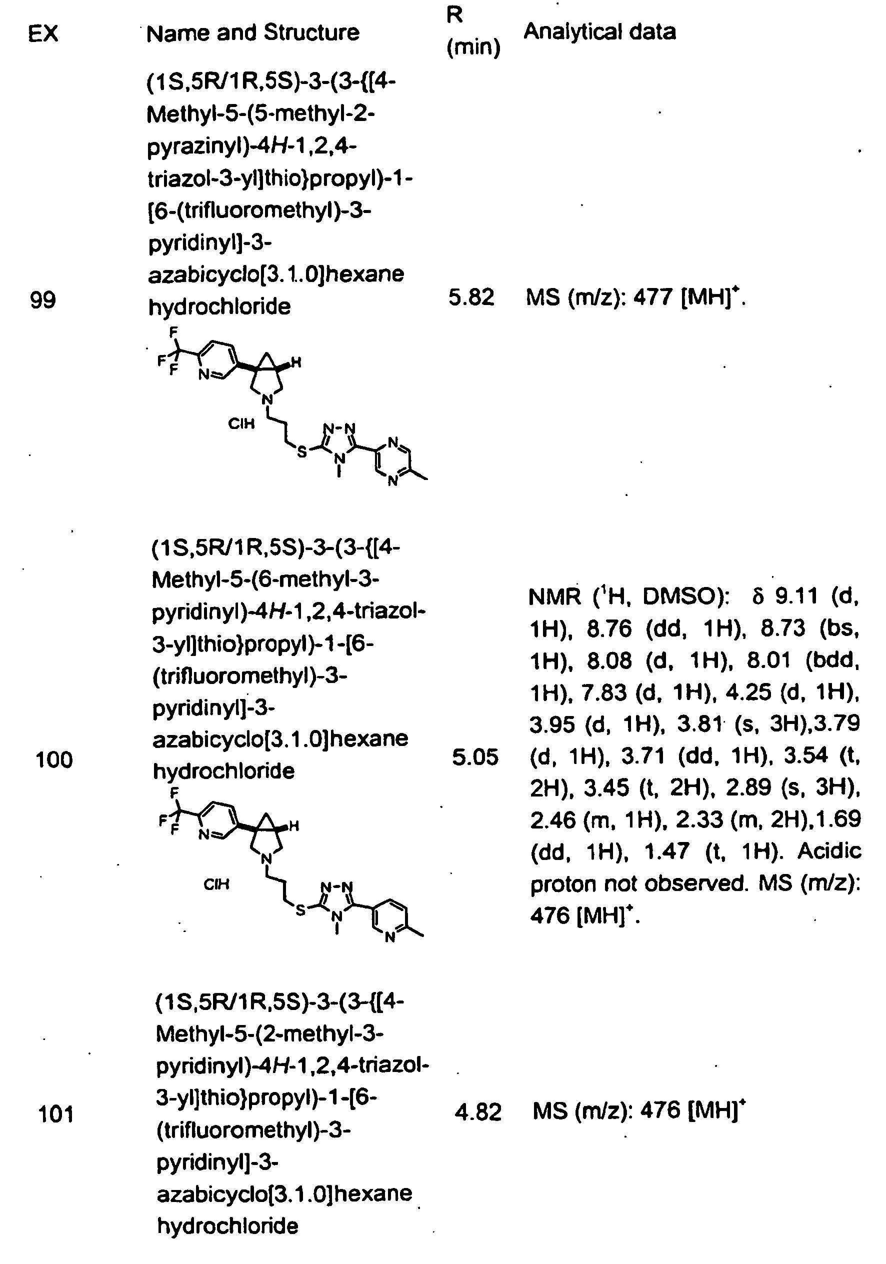

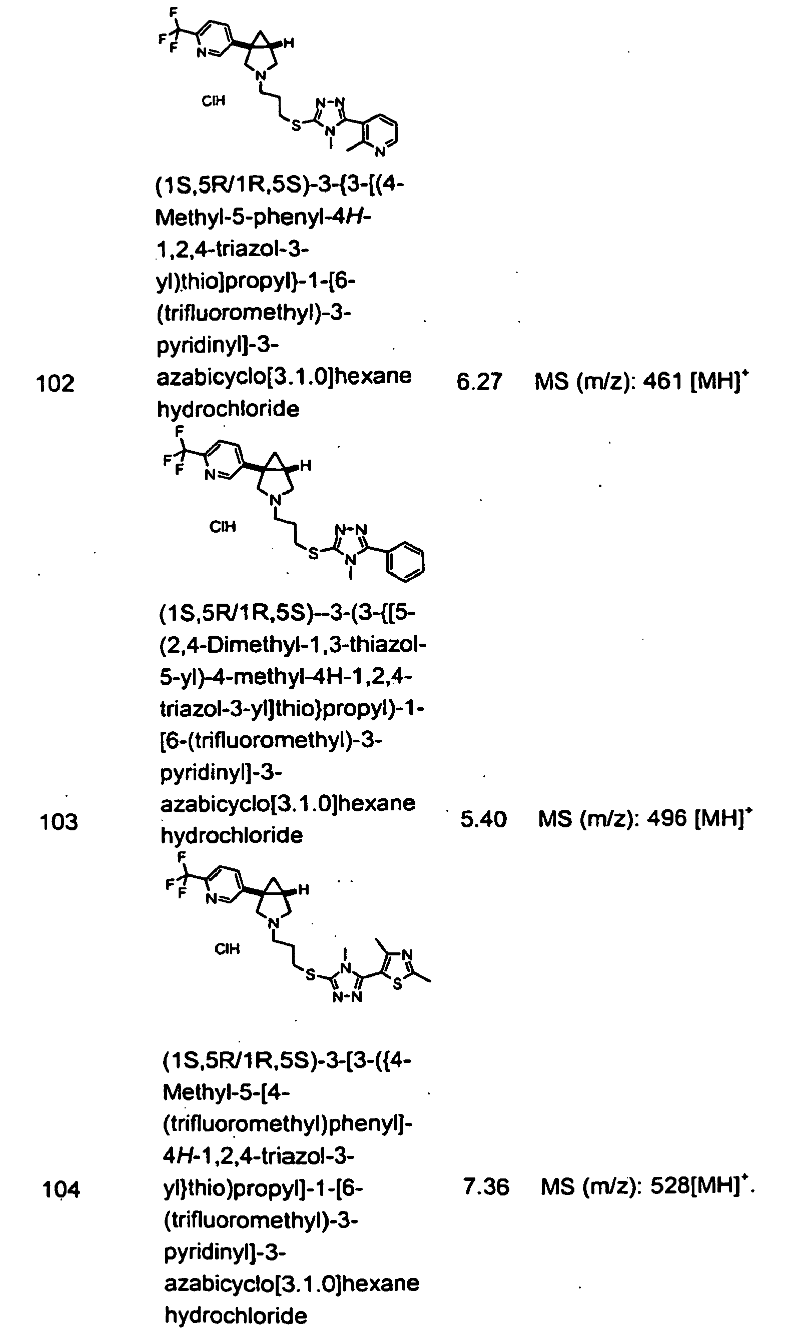

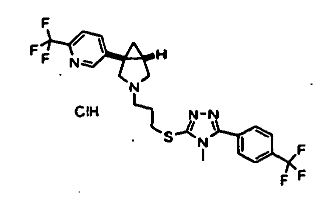

### Examples 105-109:

Examples 105-109 were prepared as white slightly hygroscopic solids from 5-[(1R,5S/1S,5R)-3-(3-chloropropyl)-3-azabicyclo[3.1.0]hex-1-yl]-2-methyl-1,3-benzothiazole (40 mg) in analogy to the method described in Example 53-58.

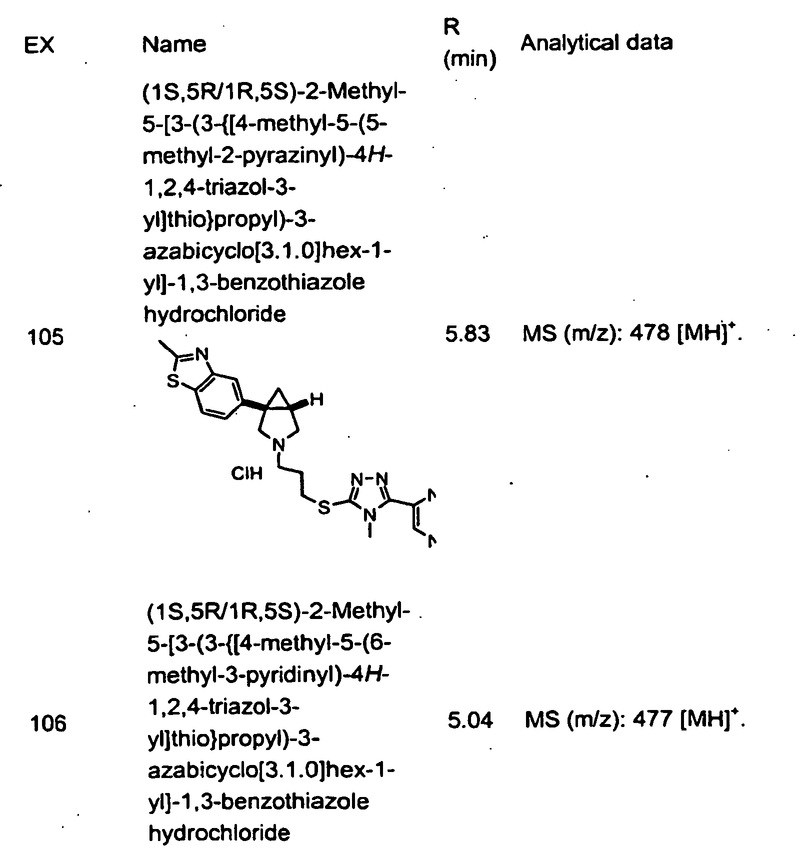

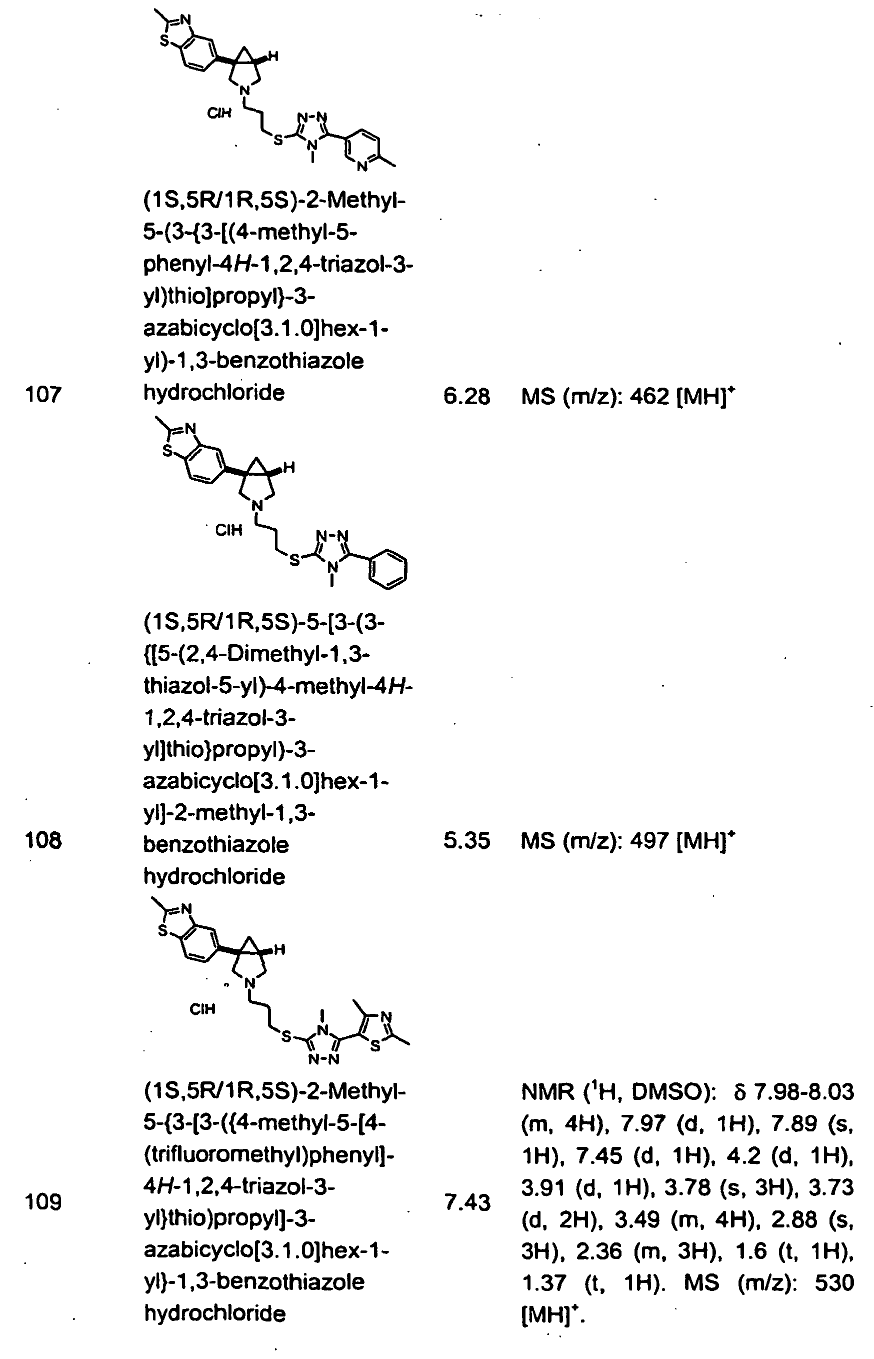

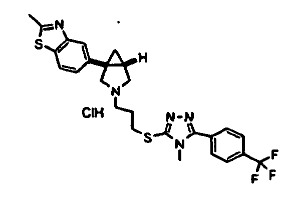

### Example 110: (1R,5S/1S,5R)-1-[3-Fluoro-5-(trifluoromethyl)phenyl]-3-(3-{[4-methyl-5-(4-methyl-1,3-oxazol-5-yl)-4H-1,2,4-triazol-3-yl]thio}propyl)-azabicyclo[3.1.0]hexane hydrochloride

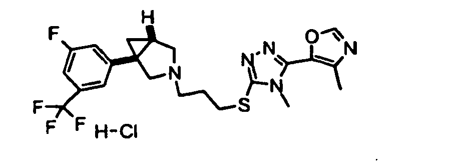

The title compound was prepared in analogy to the method described in Example 1 in 383 mg yield as a white slightly hygroscopic solid (y=46%) from (1*R*,5*S*/*1S*,*5R*)-1-[3-fluoro-5-(trifluoromethyl)phenyl]-3-azabicyclo[3.1.0]hexane (400 mg).

NMR (¹H,CD3OD): δ 8.46 (s, 1H), 7.54 (bs, 1H), 7.47 (bd, 1H), 7.41 (bd, 1H), 4.19 (d, 1H), 3.09 (d, 1H), 3.87 (s, 3H), 3.71 (m, 2H), 3.51 (t, 2H), 3.46 (t, 2H), 2.49 (s, 3H), 2.33 (m, 3H), 1.67 (m, 1H), 1.39 (m, 1H). MS *(m*/*z):* 482 [MH]⁺.

(1*R*,5*S*/*1S*,*5R*)-1-[3-Fluoro-5-(trifluoromethyl)phenyl]-3-(3-{[4-methyl-5-(4-methyl-1,3-oxazol-5-yl)-4*H*-1,2,4-triazol-3-yl]thio}propyl)-azabicyclo[3.1.0]hexane hydrochloride was separated to give the separated enantiomers by semipreparative Supercritical Fluid Chromatography (Gilson) using a chiral column Chiralcel AD-H, 25 x 2.1 cm, eluent CO₂ containing 9% (ethanol + 0.1% isopropylamine), flow rate 22 mL/min, P 192 bar, T 36 °C, detection UV at 220 nm, loop 2 mL. Retention times given were obtained using an analytical Supercritical Fluid Chromatography (Gilson) using a chiral column Chiralpak AD-H, 25 x 0.46 cm, eluent CO₂ containing 10% (ethanol + 0.1% isopropyilamine), flow rate 2.5 mL/min, P 180 bar, T 35 °C, detection UV at 220 nm.
Enantiomer 1 was recovered in 19.4 mg yield as white solid, hydrochloride salt from the racemate (100 mg). Rt. = 12.6 min. Purity >99% a/a by UV.
Enantiomer 2 was recovered in 18.3 mg yield as white solid, hydrochloride salt from the racemate (100 mg). Rt. = 14.7 min. Purity >99% a/a by UV.
Enantiomer 1 showed fpKi (D3) > 1 log-unit higher than Enantiomer 2.

### Example 110: (1R,5S/1S,5R)-1-[3-Fluoro-5-(trifluoromethyl)phenyl]-3-(3-{[4-methyl-5-(4-methyl-1,3-oxazol-5-yl)-4H-1,2,4-triazol-3-yl]thio}propyl)-azabicyclo[3.1.0]hexane hydrochloride

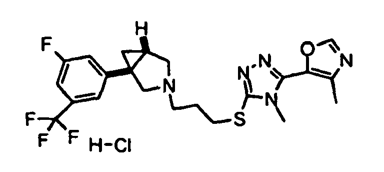

The title compound was prepared in analogy to the method described in Example 1 in 383 mg yield as a white slightly hygroscopic solid (y=46%) from (1*R*,5*S*/*1S*,*5R*)-1-[3-fluoro-5-(trifluoromethyl)phenyl]-3-azabicyclo[3.1.0]hexane (400 mg).

NMR (¹H,CD3OD): δ 8.46 (s, 1H), 7.54 (bs, 1H), 7.47 (bd, 1H), 7.41 (bd, 1H), 4.19 (d, 1H), 3.09 (d, 1H), 3.87 (s, 3H), 3.71 (m, 2H), 3.51 (t, 2H), 3.46 (t, 2H), 2.49 (s, 3H), 2.33 (m, 3H), 1.67 (m, 1H), 1.39 (m, 1H). MS *(m*/*z):* 481 [MH]⁺.

(1*R*,5*S*/*1S*,*5R*)-1-[3-Fluoro-5-(trifluoromethyl)phenyl]-3-(3-{[4-methyl-5-(4-methyl-1,3-oxazol-5-yl)-4*H*-1,2,4-triazol-3-yl]thio}propyl)-azabicyclo[3.1.0]hexane hydrochloride was separated to give the separated enantiomers by semipreparative Supercritical Fluid Chromatography (Gilson) using a chiral column Chiralcel AD-H, 25 x 2.1 cm, eluent CO2 containing 9% (ethanol + 0.1 % isopropylamine), flow rate 22 mL/min, P 192 bar, T 36 °C, detection UV at 220 nm, loop 2 mL. Retention times given were obtained using an analytical Supercritical Fluid Chromatography (Gilson) using a chiral column Chiralpak AD-H, 25 x 0.46 cm, eluent CO2 containing 10% (ethanol + 0.1% isopropyilamine), flow rate 2.5 mL/min, P 180 bar, T 35 °C, detection UV at 220 nm.
Enantiomer 1 was recovered in 19.4 mg yield as white solid, hydrochloride salt from the racemate (100 mg). Rt. = 12.6 min. Purity >99% a/a by UV.
Enantiomer 2 was recovered in 18.3 mg yield as white solid, hydrochloride salt from the racemate (100 mg). Rt. = 14.7 min. Purity >99% a/a by UV.
Enantiomer 1 showed fpKi (D3) > 1 log-unit higher than Enantiomer 2.

### Example 111: (1R,5S/1S,5R)-1-[2-Fluoro-3-(trifluoromethyl)phenyl]-3-(3-{[4-methyl-5-(4-methyl-1,3-oxazol-5-yl)-4H-1,2,4-triazol-3-yl]thio}propyl)-azabicyclo[3.1.0]hexane hydrochloride

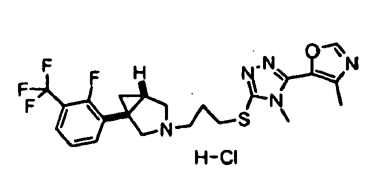

The title compound was prepared in analogy to the method described in Example 1 in 349 mg yield as a white slightly hygroscopic solid (y=45%) from (1*R*,5*S*/1*S*,5*R*)-1-[2-fluoro-3-(trifluoromethyl)phenyl]-3-azabicyclo[3.1.0]hexane (400 mg).

NMR (¹H,CD3OD): δ 8.46 (s, 1H), 7.75-7.65 (m, 2H), 7.38 (t, 1H), 4.1 (d, 1H), 3.93 (d, 1H), 3.78 (s, 3H), 3.71 (d, 1H), 3.54 (d, 1H), 3.48 (t, 2H), 3.38 (t, 2H), 2.45 (s, 3H), 2.36 (m, 1H), 2.25 (m, 2H), 1.54 (m, 1H), 1.34 (m, 1H). MS (*m*/*z*): 481 [MH]⁺.

(1*R*,5*S*/1*S*,5*R*)-1-[2-Fluoro-3-(trifluoromethyl)phenyl]-3-(3-{[4-methyl-5-(4-methyl-1,3-oxazol-5-yl)-4*H-*1,2,4-triazol-3-yl]thio}propyl)-azabicyclo[3.1.0]hexane hydrochloride was separated to give the separated enantiomers by semipreparative Supercritical Fluid Chromatography (Gilson) using a chiral column Chiralpak AD-H, 250 x 4.6 mm, eluent n-Hexane/ Ethanol 88/12 (isocratic), flow rate 1 mL/min, P 200-400 bar, T 36 °C, detection UV at 200-400 nm, loop 2 mL. Retention times given were obtained using an analytical Supercritical Fluid Chromatography (Gilson) using a chiral column Chiralpak AD-H, 250 x 4.6 mm, eluent n-Hexane/ Ethanol 88/12 (isocratic), flow rate 1 mUmin, P 200-400 bar, T 36 °C, detection UV at 200-400 nm.
Enantiomer 1 was recovered in 37 mg yield as white solid, hydrochloride salt from the racemate (98 mg). Rt. = 20.4 min. Purity 98.5% a/a by UV.
Enantiomer 2 was recovered in 35 mg yield as white solid, hydrochloride salt from the racemate (98 mg). Rt. = 23.0 min. Purity 99.5% a/a by UV.
Enantiomer 2 showed fpKi (D3) > 1 log-unit higher than Enantiomer 1.

### Example 112: (1R,5S/1S,5R)-1-[4-(Methyloxy)-5-(trifluoromethyl)phenyl]-3-(3-{[4-methyl-5-(4-methyl-1,3-oxazol-5-yl)-4H-1,2,4-triazol-3-yl]thio}propyl)-azabicyclo[3.1.0]hexane hydrochloride

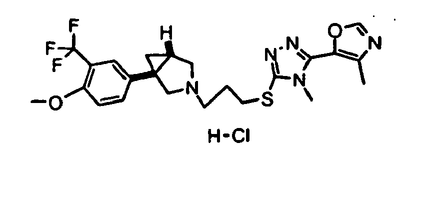

The title compound was prepared in analogy to the method described in Example 1 in 658 mg yield as a white slightly hygroscopic solid (y=76%) from (1*R*,5*S*/1*S*,5*R*)-1-[4-(methyloxy)-5-(trifluoromethyl)phenyl]-3-azabicyclo[3.1.0]hexane (430 mg).

NMR (¹H,CD3OD): δ 8.37 (s, 1H), 7.57 (m, 2H), 7.17 (d, 1H) 3.9 (m, 4H), 3.77 (s, 3H), 3.74 (m, 1H), 3.65-3.30 (m, 6H), 2.44 (s, 3H), 2.21 (m, 2H), 2.13 (m, 1H), 1.43 (t, 1H), 1.24 (m, 1H). MS (*m*/*z*): 494 [MH]⁺.

(1*R*,5*S*/1*S*,5*R*)-1-[4-(Methyloxy)-5-(trifluoromethyl)phenyl]-3-(3-{[4-methyl-5-(4-methyl-1,3-oxazol-5-yl)-4*H-*1,2,4-triazol-3-yl]thio}propyl)-azabicyclo[3.1.0]hexane hydrochloride was separated to give the separated enantiomers by semipreparative Supercritical Fluid Chromatography (Gilson) using a chiral column Chiralpak AD-H, 250 x 4.6 mm, eluent n-Hexane/ Ethanol + 0.1% isopropylamine 70/30 (isocratic), flow rate 6 mUmin, detection UV at 270 nm, loop 2 mL. Retention times given were obtained using an analytical Supercritical Fluid Chromatography (Gilson) using a chiral column Chiralpak AD-H, 250 × 4.6 mm, eluent n-Hexane/ Ethanol 70/30 (isocratic), flow rate 0.8 mL/min, detection UV at 200-400 nm.
Enantiomer 1 was recovered in 18.3 mg yield as white solid, hydrochloride salt from the racemate (100 mg). Rt. = 15.5 min. Purity > 99% a/a by UV.
Enantiomer 2 was recovered in 22.2 mg yield as white solid, hydrochloride salt from the racemate (100 mg). Rt. = 17.5 min. Purity > 99% a/a by UV.
Enantiomer 2 showed fpKi (D3) > 2 log-units higher than Enantiomer 1.

### Example 113: (1R,5S/1S,5R)-1-[4-(4-Chloro-2-fluorophenyl)-3-(3-{[4-methyl-5-(4-methyl-1,3-oxazol-5-yl)-4H-1,2,4-triazol-3-yl]thio}propyl)-azabicyclo [3.1.0]hexane hydrochloride

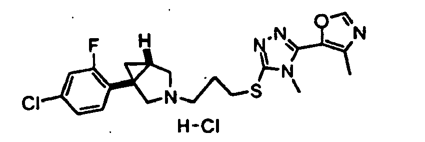

The title compound was prepared in analogy to the method described in Example 1 in 112 mg yield as a white slightly hygroscopic solid from (1*R*,5*S*/1*S*,5*R*)-1-[4-chloro-2-fluorophenyl]-3-azabicyclo[3.1.0]hexane (130 mg).

NMR (¹H, CD3OD): δ 8.27 (s, 1H), 7.3 (t, 1H), 7.1 (m, 2H), 3.95 (d, 1H), 3.8 (d, 1H), 3.67 (s, 3H), 3.56 (dd, 1H), 3.4-3.2 (m, 5H), 2.34 (s, 3H), 2.15 (m, 3H), 1.4 (t, 1H), 1.18 (t, 1H). MS (*m*/*z*): 448 [MS]⁺.

### Example 114: (1R,5S/1S,5R)-1-[3-(2-{[4-Methyl-5-(4-methyl-1,3-oxazol-5-yl)-4H-1,2,4-triazol-3-yl]thio}propyl)-1-{3-[(trifluoromethyl)oxy]phenyl}-3-azabicyclo[3.1.0]hexane hydrochloride

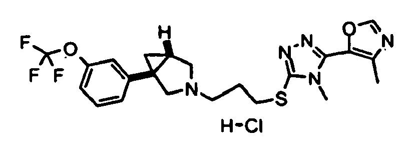

The title compound was prepared in analogy to the method described in Example 1 in 160 mg yield as a white slightly hygroscopic solid from 1-{3-[(trifluoromethyl)oxy]phenyl}-3-azabicyclo[3.1.0]hexane (150 mg).

NMR (¹H,CD3OD): δ 8.41 (s, 1H), 7.49 (t, 1H), 7.3 (s, 1H), 7.37 (d, 1H), 7.24 (m,1H), 4.17 (d, 1H), 3.9 (d, 1H), 3.8 (s, 3H), 3.69 (d, 2H), 3.51 (t, 2H), 3.42 (t, 2H), 2.47 (s, 3H), 2.3 (m, 3H), 1.57 (dd, 1H), 1.36 (t, 1H). MS (*m*/*z*): 480 [MS]⁺.

### Example 115: (1R,5S/1S,5R)-1-(2-fluoro-4-methylphenyl)-3-(3{[4-methyl-5-(4-methyl-1,3-oxazol-5-yl)-4H-1,2,4-triazol-3-yl]thio}propyl)-3-azabicyclo[3.1.0]hexane hydrochloride

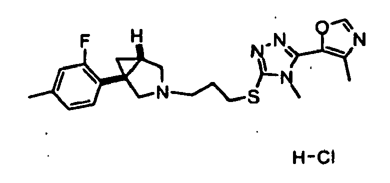

The title compound was prepared in analogy to the method described in Example 1 in 60 mg yield as a white slightly hygroscopic solid from 1-(2-fluoro-4-methylphenyl)-3-azabicyclo[3.1.0]hexane (148mg).

NMR (¹H,CD3OD): δ 8.39 (s, 1H), 7.26 (t, 1H), 7.01 (m, 2H), 3.93 (m, 1H), 3.77 (m, 4H), 3.61 (m, 1H), 3.41-3.38 (m, 5H), 2.47 (s, 3H), 2.36 (s, 3H), 2.23 (m, 2H), 2.19 (m, 1H), 1.45 (t, 1H), 1.21 (t, 1H). MS (*m*/*z*): 428 [MS]⁺.

### Example 116: (1R,5S/1S,5R)-1-[3-Chloro-4-(methyloxy)phenyl]-3-(2-{[4-methyl-5-(4-methyl-1,3-oxazol-5-yl)-4H-1,2,4-triazol-3-yl]thio}propyl)-3-azabicyclo[3.1.0]hexane hydrochloride

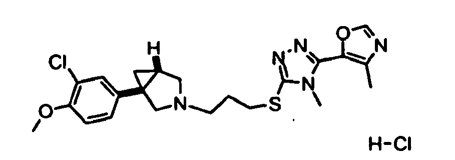

The title compound was prepared in analogy to the method described in Example 1 in 56 mg yield as a white slightly hygroscopic solid from 1-[3-chloro-4-(methyloxy)phenyl]-3-azabicyclo[3.1.0]hexane (60mg).

NMR (¹H,CD3OD): δ 8.4 (s, 1H), 7.35 (m, 1H), 7.1 (m, 2H), 4.01 (m, 1H), 3.89 (m, 4H), 3.8 (s, 3H), 3.6-3.3 (m, 6H), 2.47 (s, 3H), 2.23 (m, 2H), 2.19 (m, 1H), 1.4 (m, 1H), 1.2 (m, 1H). MS (*m*/*z*): 460 [MH]⁺.

### Example 117: (1R,5S/1S,5R)-1-[4-(2,4-Dimethyl-1,3,4-thiazol-5-yl)phenyl]-3-(3-{[4-methyl-5-(4-methyl-1,3-oxazol-5-yl)-4H-1,2,4-triazol-3-yl]thio}propyl)-3-azabicyclo[3.1.0]hexane hydrochloride

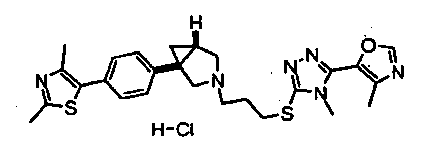

A mixture of (1*R*,5*S*/1*S*,5*R*)-1-[4-(2,4-dimethyl-1,3-thiazol-5-yl)phenyl]-3-azabicyclo[3.1.0]hexane (70 mg), 3-[(3-chloropropyl)thio]-4-methyl-5-(4-methyl-1,3-oxazol-5-yl)-4*H*-1,2,4-triazole (85 mg), potassium carbonate (43 mg), Na₂CO₃ and sodium iodide (45 mg) in anhydrous DMF (0.6 mL) was heated at 60 °C for 24 h. After elimination of the solvent *in vacuo* the residue was dissolved in ethyl acetate and the organic phase was washed with saturated aqueous sodium bicarbonate, dried over sodium sulphate and concentrated *in vacuo.* The crude was purified by flash chromatography (dichloromethane to 10% MeOH in dichloromethane) to give 65 mg of the free base of the title compound. To a solution of this material in dichloromethane (1 mL) was added HCl (1 M in Et₂O, 0.13 mL), the solvent evaporated under *vacuo* and the material thus obtained triturated with Et₂O to give 69 mg of the title compound as a white solid (50% yield).

NMR (¹H, DMSO): δ 10.39 (bs, 1H), 8.56 (s, 1H), 7.39 (d, 2H), 7.35 (d, 2H), 4.02 (m, 1H), 3.72 (m, 1H), 3.68 (s, 3H), 3.60 (t, 1H), 3.51 (bm, 1H), 3.27 (m, 4H), 2.60 (s, 3H), 2.37 (s, 3H), 2.35 (s, 3H), 2.19 (m, 1H), 2.16 (m, 2H), 1.62 (m, 1H), 1.15 (m, 1H); MS (*m*/*z*): 507.2 [MH]⁺.

### Example 118: (1R,5S/1S,5R)-3-(3-{[4-Methyl-5-(4-methyl-1,3-oxazol-5-yl)-4H-1,2,4-triazol-3-yl]thio}propyl)-1-{4-[6-(trifluoromethyl)-2-pyridinyl]phenyl}-3-azabicyclo[3.1.0]hexane hydrochloride

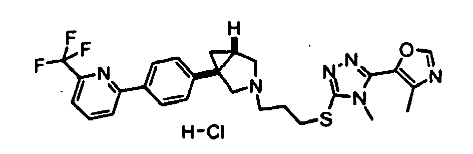

The title compound was prepared in analogy to the method described in Example 117 (using (1*R*,5*S*/1*S*,5*R*)-1-{4-[6-(trifluoromethyl)-2-pyridinyl]phenyl}-3-azabicyclo[3.1.0]-hexane) in 55% yield as a white solid.

NMR (¹H, CDCl₃): δ 10.44 (bs, 1H), 8.56 (s, 1H), 8.29 (d, 1H), 8.17 (t, 1H), 8.09 (d, 2H), 7.84 (d, 1H), 7.43 (d, 2H), 4.08 (m, 1H), 3.75 (m, 1H), 3.68 (s, 3H), 3.64 (t, 1H), 3.53 (bm, 1H), 3.28 (m, 4H), 2.37 (s, 3H), 2.27 (m, 1H), 2.17 (m, 2H), 1.68 (m, 1H), 1.17 (m, 1H); MS (*m*/*z*): 541.2 [MH]⁺.

### Example 119: (1R,5S/1S,5R)-1-[3-(2,4-Dimethyl-1,3-thiazol-5-yl)phenyl]-3-(3-{[4-methyl-5-(4-methyl-1,3-oxazol-5-yl)-4H-1,2,4-triazol-3-yl]thio}propyl)-3-azabicyclo[3.1.0]hexane hydrochloride

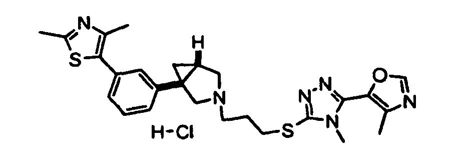

The title compound was prepared in analogy to the method described in Examples 117 (using (1*R*,5*S*/1*S*,5*R*)-1-[3-(2,4-dimethyl-1,3-thiazol-5-yl)phenyl]-3-azabicyclo[3.1.0]-hexane) in 53% yield as a white solid.

NMR (¹H, CDCl₃): δ 10.53 (b, 1H), 8.58 (s, 1H), 7.43 (d, 1H), 7.28-7.38 (m, 3H), 4.07 (dd, 1H), 3.73 (dd, 1H), 3.70 (s, 3H), 3.61 (t, 1H), 3.53 (m, 1H), 3.34 (m, 2H), 3.29 (t, 2H), 2.64 (s, 3H), 2.39 (s, 3H), 2.73 (s, 3H), 2.23 (m, 1H), 2.20 (m, 2H), 1.68 (t, 1H), 1.16 (t, 1H); MS (*m*/*z*): 507.1 [MH]⁺.

### Example 120: (1R,5S/1S,5R)-3-(3-{(4-Methyl-5-(4-methyl-1,3-oxazol-5-yl)-4H-1,2,4triazol-3-yl]thio}propyl)-1-[3-(5-methyl-2-thienyl)phenyl]-3-azabicyclo[3.1.0]hexane hydrochloride

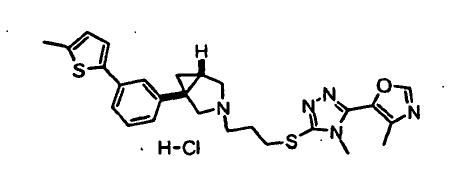

The title compound was prepared in analogy to the method described in Example 117 (using (1*R*,5*S*/1*S*,5*R*)-1-[3-(5-methyl-2-thienyl)phenyl]-3-azabicyclo[3.1.0]hexane) in 51% yield as a white solid.

NMR (¹H, CDCl₃): δ 10.44 (b, 1H), 8.58 (s, 1H), 7.49 (d, 1H), 7.45 (dt, 1H), 7.37 (t, 2H), 7.18 (dt, 1H), 6.84 (t, 1H), 4.08 (dd, 1H), 3.76 (dd, 1H), 3.70 (s, 3H), 3.62 (t, 1H), 3.54 (tm, 1H), 3.28 (t, 4H), 2.48 (s, 3H), 2.39 (s, 3H), 2.24 (m, 1H), 2.19 (t, 2H), 1.65 (t, 1H), 1.16 (t, 1H); MS (*m*/*z*): 472.0 [MH]⁺.

### Example 121: (1R,5S/1S,5R)-1-[4-(3,5.Dimethyl-4-isoxazolyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane

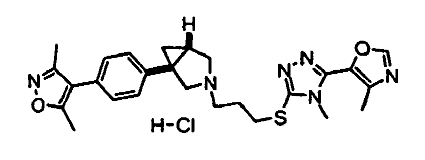

The title compound was prepared in analogy to the method described in Example 117 (using (1*R*,5*S*/1*S*,5*R*)-1-[4-(3,5-dimethyl-4-isoxazolyl)pheny]-3-azabicyclo[3.1.0]hexane), in 55% yield as a white solid.

MS (*m*/*z*): 491.2 [MH]⁺.

### Example 122: (1S,5R)-3-(3-{[5-(2,4-Dimethyl-1,3-oxazol-5-yl)-4-methy-4H-1,2,4-triazol-3-yl]thio}propyl)-1-[2-fluoro-4-(trifluoromethyl)phenyl]-3-azabicyclo[3.1.0]-hexane hydrochloride

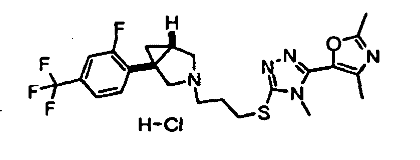

A mixture of (1*S,*5*R*)-1-[4-(trifluoromethyl)phenyl]-3-azabicyclo[3.1.0]hexane (Preparation 18, 60 mg), 3-[(3-chloropropyl)thio]-5-(2,4-dimethyl-1,3-oxazol-5-yl)-4-methyl-4*H-*1,2,4-triazole (Preparation 78, 78 mg), 2-*tert-*butylimino-2-diethylamino-1,3-dimethyl-perhydro-1,3,2-diaza-phosphorine on polystyrene (2.2 mmol/g, 140 mg) and a catalytic amount of Nal in acetonitrile dry (3 ml) was heated at 70 °C for 4 h, then overnight at 55 °C. The resin was removed by filtration and washed with acetonitrile (2 x 3 ml). The solvent was removed under vacuum, the remaining solid dissolved in DMF dry (0.5 ml), 3-[(3-chloropropyl)thio]-5-(2,4-dimethyl-1,3-oxazol-5-yl)-4-methyl-4*H-*1,2,4-triazole (Preparation 78, 60 mg) was added followed by potassium carbonate (118 mg). The resulting suspension was heated at 60 °C overnight. At room temperature a saturated solution of sodium bicarbonate was added (4 ml) and the suspension was extracted with DCM (2 x 6 ml). The resulting solution was charged onto a SCX column and eluted with MeOH followed by MeOH/NH₃ 0.25 M. The resulting material was purified by preparative HPLC and then converted to the hydrochloride salt following the method described for Example 15 to give the title compound as a white slightly hygroscopic solid (37 mg, 27% yield). NMR (¹H, DMSO): δ 10.38 (b,1H), 7.71 (d, 1H), 7.64 (t, 1H), 7.59 (d, 1H), 3.98 (bd, 1H), 3.76 (bd, 1H), 3.65 (s, 3H), 3.54 (b, 1H), 3.44 (bt, 1H), 3.31 (b, 2H), 3.24 (t, 2H), 2.47 (s, 3H), 2.35 (m, 1H), 2.29 (s, 3H), 2.11 (m, 2H), 1.63 (t, 1H), 1.13 (t, 1H). MS *(mlz)*: 496 [MH]⁺.

### Example 123: (1S,5R)-3-(3-{[5-(2,4-Dimethyl-1,3-oxazol-5-yl)-4-methyl-4H-1,2,4-triazol-3-yl]thio}propyl)-1-[2-fluoro-4-(trifluoromethyl)phenyl]-3-azabicyclo[3.1.0]-hexane hydrochloride

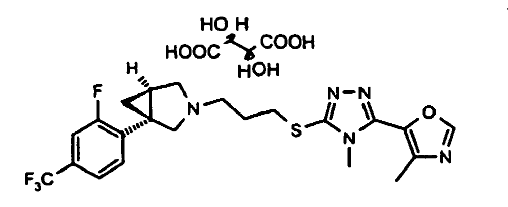

### HPLC Methods

| HPLC Assay (short run): | |
|---|---|
| Column type | Phenomenex LUNA |
| Column length [cm] | 5 |
| Internal diameter [cm] | 0.2 |
| Particle size [um] | 3.0 |
| Mobile phase | A: 0.05%v/v TFA in water / B: 0.05%v/v TFA in acetonitrile |
| Step 1: Time-Reserv.A-Reserv.B | Time 0min 100%A |
| Step 2: Time-Reserv.A-Reserv.B | Time 8min 5%A |
| Step 3: Time-Reserv.A-Reserv.B | Time 8.01min 100%A |
| Flow rate [mL/min] | 1 |
| Column temperature [^C] | 40 |
| Autosampler temperature [^C] | AMB |
| Detector type | UV |
| Wavelength [nm] | 220 |
| Injection volume [uL] | 1 |
| Run Time | 8 min. |

| HPLC chiral 1 | |
|---|---|
| Column type | Chiracel OD-H |
| Column length [cm] | 25 |
| Internal diameter [cm] | 4.6 |
| Particle size [um] | 5 |
| Mobile phase | Heptane/IPA 85/15% v/v |
| Flow rate [mL/min] | 1 |
| Column temperature [^C] | 30 |
| Autosampler temperature [^C] | AMB |
| Detector type | UV |
| Wavelength [nm] | 220 |
| Injection volume [uL] | 10 |
| Dilution Factor 5 | |

| HPLC Assay (long run): | |
|---|---|
| Column type | LUNA 3u phenyl - hexyl |
| Column length [cm] | 15 |
| Internal diameter [cm] | 0.46 |
| Particle size [um] | 3.0 |
| Mobile phase | A: 0.05%v/v TFA in water / B: 0.05%v/v TFA in acetonitrile |
| Step 1: Time-Reserv.A-Reserv.B | Time 0 min 95%A - 5%B |
| Step 2: Time-Reserv.A-Reserv.B | Time 30 min 5%A - 95%B |
| Step 3: Time-Reserv.A-Reserv.B | Time 30.01 min 95%A - 5%B |
| Flow rate [mL/min] | 1 |
| Column temperature [^C] | 40 |
| Autosampler temperature [^C] | AMB |
| Detector type | UV |
| Wavelength [nm] | 220 |
| Injection volume [uL] | 10 |
| Run Time | 30 min. |

| HPLC chiral 2 | |
|---|---|
| Column type | CHIRALPAK AD |
| Column length [cm] | 25 |
| Internal diameter [cm] | 4.6 |
| Particle size [um] | 10 |
| Mobile phase | Heptane/IPA 85/15% v/v |
| Flow rate [mL/min] | 0.8 |
| Column temperature [^C] | 25 |
| Autosampler temperature [^C] | AMB |
| Detector type | UV |
| Wavelength [nm] | 270 |
| Injection volume [uL] | 10 |
| Dilution Factor 10 | |

### Preparation 123(1): 3-[(3-Chloropropyl)thio]-4-methyl-5-(4-methyl-1,3-oxazol-5-yl)-4H-1,2,4-triazole

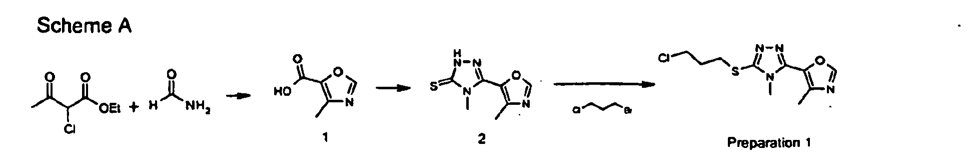

### Preparation 123(1A): 4-methyl-1,3-oxazole-5-carboxylic acid

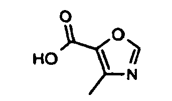

Ethyl-2-chloroacetoacetate (28.6 g, 24.0 mL) was dissolved in DMF (28.6 mL) and formamide (19.5 mL) was added. The resulting solution was heated up to 120 °C (internal temperature) under nitrogen for 21 h. The mixture was allowed to cool down to 20°C, diluted with *ter*-butyl methyl ether (172 mL) and washed with water (115 mL). The aqueous phase was extracted again with 115 mL of *tert-*butyl methyl ether and the combined organic layers were washed twice with water (86 mL) and treated with NaOH 3 N (86 mL). The resulting mixture was stirred at 20°C for 3 hours. The organic layer was discarded while the aqueous was acidified with 20 mL of concentrated HCl (37% sol.) till pH 2 over 10 minutes. A precipitate started to crush out of solution. The suspension was stirred at 20°C for 2 h, filtered and the cake washed with 14.3 mL of cold water (10°C ca.). The collected solid was dried under high vacuum at 40°C for 16 hours. The title compound was obtained in a theoretical yield of 35.3 % (7.81 g).

NMR (1H, DMSO-d6, δ ppm): 13.5 (bs, 1H), 8.47 (s, 1H), 2.38 (s, 3H)

MS (*m*/*z*): 128[MH]⁺

### Preparation 123(1B): 4-methyl-5-(4-methyl-1,3-oxazol-5-yl)-2,4-dihvdro-3H-1,2,4-triazole-3-thione

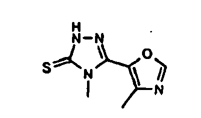

4-Methyl-1,3-oxazole-5-carboxylic acid (prepared according to the method of Preparation 1A, 12.9 g) was dissolved in DMF (60 mL) and treated with 4-methyl-3-thiosemicarbazide (11.61 g). Then disopropylethylamine (DIPEA) (31.0mL) was added at 20°C. Under ice bath cooling, T3P 50% w/w in ethyl acetate (90 mL) was added drop wise, maintaining the temperature below 15°C over 20 minutes. The resulting mixture was then stirred at 20°C for 6 hours.

The mixture was diluted with NaOH 4 M (120.0 mL). The resulting bi-phasic mixture was allowed separating and the upper organic layer discarded. The aqueous layer (pH = 8) was adjusted to pH = 11 with additional NaOH 4 M (60 mL) and then heated to 70°C (internal temperature) for 30 minutes. After cooling down over night, HCl 37% was slowly added until pH=5 was reached.

The suspension was stirred for 8 hours, then the solid was filtered and washed with water (60 mL), and it was dried in a vacuum oven at 40°C overnight. The title compound was obtained in a 53% theoretical yield (10.48 g).

NMR (1H, DMSO-d6, δ ppm): 14.11 (bs, 1H), 8.60 (s, 1H), 3.61 (s, 3H), 2.33 (s, 3H)

MS (*m*/*z*): 197[MH]⁺

### 3-[(3-chloropropyl)thio]-4-methyl-5-(4-methyl-1,3-oxazol-5-yl)-4H-1,2,4-triazole

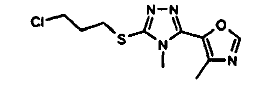

4-methyl-5-(4-methyl-1,3-oxazol-5-yl)-2,4-dihydro-3*H-*1,2,4-triazole-3-thione (prepared according to the method of Preparation 2A, 380 g) was added to a mixture of methanol (1140 mL) and acetone (2660 mL), followed by K₂CO₃ (380 g) and 1-bromo-3-chloropropane (251 mL). The suspension was stirred at 20±2°C. for 4h. The volume of solvent was reduced then ethyl acetate (4800 mL) was added and the organic layer was washed with water twice (2400 mL each). The organic layer was distilled to about 3300mL, diluted with ethyl acetate (3800 mL) and distilled again to the same level as before. Some precipitate was already observed when cooling the mixture that was stirred for 30 minutes. Heptane (3800 mL) was added slowly over a period of 30 minutes upon which more product crashed out as a fine, heavy solid, The suspension was stirred for four additional hours at 20±2°C. The solid was collected by filtration and washed with 1140mL of a ethyl acetate/ heptane (1:2) mixture. The solid was dried in the oven at 40°C under reduced pressure overnight to give 3-[(3-Chloropropyl)thio]-4-methyl-5-(4-methyl-1,3-oxazol-5-yl)-4*H-*1,2,4-triazole in a 59.3 theoretical yield (314 g).

NMR (1H, DMSO-d6, δ ppm): 8.55 (s, 1H), 3.76 (t, 2H), 3.68 (s, 3H), 3.26 (t, 2H), 2.37 (s, 3H), 2.14 (m, 2H)

MS (*m*/*z*): 273[MH]⁺

### Preparation 123(2): 3-[2-fluoro-4-(trifluoromethyl)phenyl]-1H-pyrrole-2,5-dione

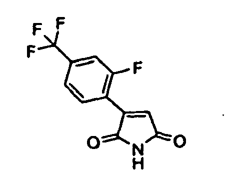

Maleimide (48.6 g) was suspended in acetonitrile (300 mL,) under N₂ and tert-butyl nitrite (38 mL) followed by copper (II) chloride (45 g) were added. The resulting suspension was cooled down to 0°C and neat 4-amino-2-fluorotrifluorobenzene (50g, 35.2 mL) was added drop wise in 45min ca. The internal temperature was kept below 10°C during the aniline addition and gas developing was observed. The reaction mixture was allowed to stir at 0°C for 1 h and then overnight at 20°C. Then 10% HCl (300 mL,) was added. The biphasic mixture obtained was extracted with AcOEt (300 mL). The organic layer was washed with water (300mL, 6vol) and then with 10% NaCl (300 mL). After solvent evaporation to dryness the residue was dissolved in IPA (200 mL) and re-distilled down to dryness. Then IPA (100 mL, 2 vol) and 2,6-Lutidine (17.5 mL) were added and the suspension refluxed for 20 min to obtain a clear dark solution. After cooling down to 20°C the suspension was stirred overnight and then the solid filtered by washing upon the filter with water (200 mL). After drying at 50°C under vacuum the product was obtained as beige solid in a 30.6% theoretical yield (22.13 g).

1H NMR (DMSO-d6) ppm: 11.29 (br.s., 1H); 8.21 (t, 1H); 7.90 (d, 1H); 7.75 (d, 1H); 7.15 (s, 1H)

### Preparation 123(3): 1(1R,5S/1S,5R)-[2-Fluoro-4-(trifluoromethyl)phenyl]-3aza-bicyclo[3.1.0]hexane-2,4-dione

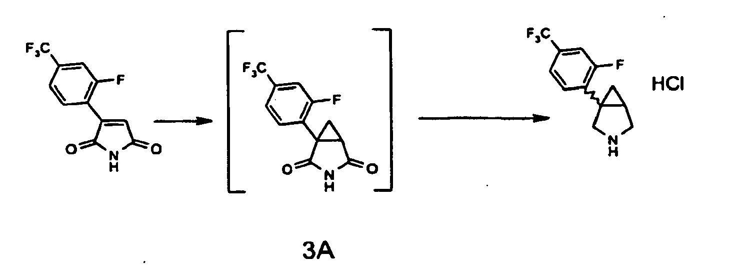

### Preparation 123(3A): 1(1R,5S/1S,5R)-[2-Ftuoro-4-(trifluoromethyl)phenyl]-3 azabicyclo[3.1.0]hexane-2,4-dione

Potassium hydroxide (258.1 g) was added to a stirred suspension of trimethylsulfoxonium iodide (1013 g) in dimethylsulfoxide (4470 mL) under N₂. The resulting mixture was allowed to stir at room temperature for 1 hr (or until a clear solution is observed). 3-[2-fluoro-4-(trifluoromethyl)phenyl]-1H*-*pyrrole-2,5-dione (prepared according to the method of Preparation 2, 596.0 g) dissolved in dimethylsulfoxide (1490 mL) was then added drop wise in 30 minutes keeping the internal temperature below 25°C and the resulting mixture was allowed to stir at room temperature for 2 h.

The mixture was then diluted with tert-butyl methyl ether (6000 mL) and HCl 2N (4800 mL) was slowly added at room temperature. After separation of the two phases, the aqueous layer was extracted again with tert-butyl methyl ether (3000 mL) and the collected organic layers washed twice with water (3000 mL) and then with NaCl 10% (3000 mL).

The organic layer was concentrated to 1800 mL then 4800 mL of tetrahydrofuran were added and the solution concentrated again to 1800 mL. The resulting tetrahydrofuran solution of 1(1*R*,5*S*/1*S*,5*R*)-[2-Fluoro-4-(trifluoromethyl)phenyl]-3-azabicyclo[3.1.0]hexane-2,4-dione was used as such in the following step.

### (1S,5R)-1-[2-Fluoro-4-(trifluoromethyl)phenyl]-3-azabicyclo[3.1.0]hexane salt of hydrochloric acid

NaBH₄ (351 g) was charged under N₂ followed by tetrahydrofuran (3600 mL) then the solution of 1-[2-Fluoro-4-(trifluoromethyl)phenyl]-3-azabicyclo[3.1.0]hexane in tetrahydrofuran prepared in the previous step was added dropwise in 1 h and the resulting suspension allowed to stir at room temperature for 1 hr.

BF₃-THF complex (1440 mL) was then added dropwise in 1 h and 30 min keeping the internal temperature around 25°C and the resulting suspension was stirred at 25°C for 24 hrs.

The mixture was cooled down to 0°C and methanol (2400 mL) was cautiously added in 2.5 h monitoring gas evolution. The suspension was then heated to reflux for 30 min and distilled down to 2400 mL at atmospheric pressure. The resulting suspension was diluted with tert-butyl methyl ether (6000 mL) and HCl 2 N (3600 mL) and the mixture was then stirred at room temperature for 1 hr. The aqueous phase was discharged and the organic phase was washed twice with NaOH 2 N (2400 mL) and then with NaCl 10 % solution (3000 mL).

The organic phase was distilled down to 1800 mL then diluted with 3000 mL of tert-butyl methyl ether and again distilled down to 1800 mL.

3000 mL of tert-butyl methyl ether were added followed by 780 mL of HCl 5-6 N in isopropanol and the precipitation was immediately observed.

The suspension was aged overnight and then the solid filtered off washing with tert-butyl methyl ether (1200 mL). After drying at 40°C for 16 h, 1-[2-fluoro-4-(trifluoromethyl)phenyl]-3-azabicyclo[3.1.0]hexane hydrochloride salt (369.1 g) was obtained as a white solid in 57 mol % theoretical yield.

NMR (1H, DMSO-d6, δ ppm): 9.64 (bs, 2H); 7.70 (dd, 1H); 7.64 (t, 1H); 7.58 (dd, 1H); 3.62 (dd, 1H); 3.50 (dd, 1H); 3.42 (d, 1H); 3.35 (d, 1H); 2.24 (m, 1H); 1.41 (t, 1H); 1.15 (m, 1H)

### Preparation 123(4): (1S,5R)-1-[2-fluoro-4-(trifluoromethyl)phenyl]-3-azabicyclo-[3.1.0]hexane salt of [(1R,4S)-7,7-dimethyl-2-oxobicyclo[2.2.1]hept-1-yl]-methanesulfonic acid

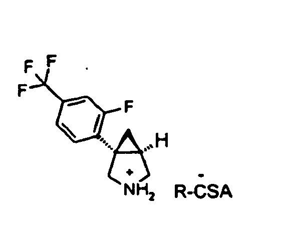

1-[2-fluoro-4-(trifluoromethyl)phenyl]-3-azabicyclo[3.1.0]hexane hydrochloride salt obtained from Preparation 3 (369.0 g) was suspended in tert-butyl methyl ether (2950 ml) and treated with NaOH 1 N (1850 ml). The mixture was stirred for 15 minutes to achieve complete dissolution and then allowed to separate. The organic layer was washed twice with water (1850 ml) and then with 1850 ml of NaCl 10 % w/w solution. The organic layer was concentrated down to 1110 ml, diluted with more tert-butyl methyl ether (1850 ml) and distilled down to 1110 ml.

The solution was diluted with acetonitrile (1850 ml) and distilled down again to 1110 ml. The resulting solution was diluted to 2960 ml and (-)-(R)-Camphorsulfonic acid was added (171.63 g). The exact amount of (-)-(R)-Camphorsulfonic acid was determined introducing a correction based on the assay w/w of the starting material.

Complete dissolution was observed followed after 30 minutes by precipitation. The slurry was aged for 22 hours at 20°C under N₂; then filtered and the cake washed with additional acetonitrile (740 ml). The collected solid was placed in the oven at 40°C under reduced pressure for 18 h. 223.5 g of the title compound were obtained in a 35.8 % mol theoretical yield

1H NMR (DMSO-d6) ppm: 9.12 (br.s.; 2H); 7.72 (dd, 1H); 7.63 (t, 1H); 7.60 (m, 1H); 3.67 (dd, 1H); 3.56 (dd, 1H); 3.47 (d, 1H); 3.42 (d, 1H); 2.90 (d, 1H); 2.67 (m, 1H); 2.41 (d, 1H); 2.26 (m, 2H); 1.95 (t, 1H); 1.87 (m, 1H); 1.79 (d, 1H); 1.30 (m, 3H); 1.19 (m, 1H); 1.05 (s, 3H); 0.76 (s, 3H)

HPLC assay (short run): > 99% a/a

HPLC chiral 1: enantiomeric excess (e.e.) > 80 %

### (1S,5R)-1-[2-Fluoro-4-(trifluoromethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane tartrate

(1*S*,5*R*)-1-[2-fluoro-4-(trifluoromethyl)phenyl]-3-azabicyclo[3.1.0]hexane salt of[(1*R*,4*S*)-7,7-dimethyl-2-oxobicyclo[2.2.1]hept-1-yl]methanesulfonic acid (310 g), prepared in an analogous way as described before in Preparation 123(4), was suspended in tert-butylmethyl ether (3.1 L) and treated with NaOH 1N (1.55 L). After phase separation the organic layer was washed twice with water (1.55 L each) and then evaporated down to about 620 mL. Fresh tert-butylmethylether (620 mL) was added and the solution evaporated down again to 620 mL. After addition of DMF (0.93 L), the solution was evaporated down to about 0.93L. K₂CO₃ 325 mesh (143 g), KI (171 g) and 3-[(3-chloropropyl)thio]-4-methy-5-(4-methyl-1,3-oxazol-5-yl)-4*H-*1,2,4-triazole (283 g) prepared in analogy with Preparation 123(1) were added at room temperature. The obtained suspension was then warmed at 62-63 °C for 5h and then cooled down to 20°C. After dilution with ethyl acetate (1.55 L), water (1.55 L) was added and phases allowed separating. The organic layer was washed twice with water (775 mL each), diluted with further ethyl acetate (0.31 L), concentrated to 620 mL, diluted with additional ethyl acetate (620 mL) and evaporated down again to dryness. A portion of the so obtained yellow waxy solid (315g over a total of 330 g) was dissolved in acetone (2.30 L) and L-Tartaric Acid (93.3 g) was added at 20 °C. After 20 min water (74 mL) was added to dissolve completely the acid. Precipitation of a white solid immediately occurred. The mixture was stirred for 3h at 20°C, then filtered and the cake washed with acetone/water 2/1 mixture (0.9 L). After drying under vacuum at 40°C for 20h, the title compound was obtained as an off-white solid (347 g) and 97.8% a/a typical purity by HPLC (short run).

NMR (1H, DMSO-d6): 8.55 (s, 1H), 7.61 (d, 1H), 7.53 (m, 2H), 4.27 (s, 2H), 3.67 (s, 3H), 3.33 (d, 1H), 3.19 (t, 2H), 3.13 (d, 1H), 2.64 (t, 2H), 2.58 (dd, 1H), 2.50 (m, 1H), 2.37 (s, 3H), 1.94 (m, 1H), 1.86 (m, 2H), 1.35 (t, 1H), 0.82 (dd, 1H). MS (*m*/*z*): 482[MH]⁺.

### Example 124: Effect of acute administration of (1S,5R)-1-[2-Fluoro-4-(trifluoromethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexane hydrochloride on sexual desire and consummatory behaviour in male rats

Background: In order to evaluate the effect on natural rewards and to potentially differentiate this approach compared with clinically active gold standards, the effect of the title compound (0.03, 0.3 and 3 mg/kg i.p.; -1 hour, prepared in an analogous way with Example 40) on sexual desire and consummatory performance was evaluated in the rat after acute administration. The effect of the title compound was assessed according to a Latin squared experimental design in male (about 300g upon arrival) and female (250g upon arrival) Wistar rats.

Method - Male rats, sexually experienced were treated with the title compound and submitted 1-hr later to a sexual incentive motivation test (10 minutes) in which male rats were singly exposed to receptive female and active male incentives (Agmo A., Journal of Comparative Psychology. 117(1), Mar 2003, 3-14). The following parameters were scored: (1) time spent in the incentive zones (close to the incentive cage); (2) number of visits to the zones, and (3) preference score (time spent in the sexual incentive zone / (time in the sexual incentive zone + time in the social incentive zone)). Immediately after the incentive motivation test each subject was transferred to an observation cage in the presence of a receptive female and its copulatory behaviour was then recorded until the end of the 1^{st} post-ejaculatory interval. In the observation cage, the following behavioural parameters were recorded: (1) mount latency (time from the introduction of the female until the first mount with pelvic thrusting); (2) intromission latency (time from the introduction of the female until the first mount with vaginal penetration); (3) ejaculation latency (time from the 1^{st} intromission until ejaculation); (4) post-ejaculatory interval (time from ejaculation until the following intromission); (5) number of mounts, and (6) number of intromission.

Results - Data are expressed as mean ± s.e.m.

**Table 1- Effect of the title compound (0, 0.03, 0.3 and 3 mg/kg i.p. - 1 hour) on sexual incentive motivation of male rats**

| Behaviour | Vehicle (n=9) | Example 40 0.03 mg/kg (n=9) | Example 40 0.3 mg/kg (n=9) | Example 40 3 mg/kg (n=9) |
|---|---|---|---|---|
| Time sexual incentive zone (s) | 350.07 ± 21.95 ++ | 367.57 ± 23.53 ++ | 335.12 ± 32.47 ++ | 355.91 ± 13.70 ++ |
| Time social incentive zone (s) | 82.32 ± 9.16 | 73.69 ± 12.48 | 86.84 ± 13.38 | 76.83 ± 10.05 |
| n.of visits to sex. Incentive zone | 26.11 ± 1.09 ++ | 23.44 ± 1.95 ++ | 23.89 ± 2.06 ++ | 21.78 ± 1.72++ |
| n.of visits social incentive zone | 18.11 ± 1.17 | 15.33 ± 1.60 | 17.44 ± 2.49 | 14.00 ± 1.85 |

| | | | | |
|---|---|---|---|---|
| ++p<0.01; receptive female vs. active male | | | | |

**Table 2- Effect of the title compound (0, 0.03, 0.3 and 3 mg/kg; i.p.. -1 hour) on male consummatory behaviour**

| Behaviour | Vehicle (n=9) | Example 40 0.03 mg/kg (n=9) | Example 40 0.3 mg/kg (n=9) | Example 40 3 mg/kg (n=9) |
|---|---|---|---|---|
| n. of mounts | 2.8 ± 1.2 | 3.4 ± 0.7 | 2.3 ± 0.8 | 4.8 ± 1.2 ** |
| n. of intromissions | 15.6 ± 2.3 | 15.8 ± 1.3 | 15.6 ± 1.5 | 16.8 ± 2.1 |
| Mount latency (s) | 5.6 ± 1.0 | 4.6 ± 0.8 | 5.3 ± 0.8 | 6.4 ± 1.5 |
| Intromission latency(s) | 8.9 ± 2.2 | 6.3 ± 0.8 * | 7.0 ± 1.1 | 8.8 ± 2.0 |
| Ejaculation latency (s) | 214.1 ± 5.8 | 202.2 ± 19.6 | 228.3 ± 53.3 | 272.8 ± 41.6 ** |
| Post ejaculatory interval (s) | 371.6 ± 35.2 | 403.0 ± 37.4 | 455.3 ± 73.4 * | 485.1 ± 67.3** |

| | | | | |
|---|---|---|---|---|
| *p<0.05; **p<0.01; treatment vs. vehicle | | | | |

Conclusions - The title compound did not affect sexual desire of male rats when exposed to sexual and social stimuli. Interestingly a specific effect of the title compound was observed on ejaculation patterns as reflected by a significant increase in the number of mounts, ejaculation latency and post-ejaculatory interval.

### Example 125: Effect of (1S,5R)-1-[2-Fluoro-4-(trifluoromethyl)phenyl]-3-(3-{[4-methyl-5-(4-methyl-1,3-oxazol-5-yl)-4H-1,2,4-triazol-3-yl]thio}propyl)-3-azabicyclo-[3.1.0]hexane tartrate on stress-induced food intake

### Animal model for binge eating

Dieting is the strongest predictor of overeating in response of stress. In this specific case, binge eating or binge/purge behaviours are recognized as key features of the human diseases (bulimia nervosa or anorexia, respectively). Two critical factors seem to modulate the production of binge eating: a history of food restriction and access of high palatable (HP) food (dense in fat and sugar) and the interaction with environmental stress. Based on these premises, preclinical models can be developed to explore the potential therapeutic effect of test compounds on binge eating. One model which may be used is described by Hagan et al (Hagan, M.M. et al., Physiol and Beh 77 (2002): 45-54).

Slight modifications such as those below may be applied to their model, for example:
- Instrument to deliver FS: Passive Avoidance (Gemini Avoidance System, Couboum Instrument , San Diego) may be used instead of the 4 closed runways with metal bar floors (Coulbourn Instruments Habitest System, Allentown, PA).
- Refeeding: 4 days instead of 6 days
- HP food: Baiocchi® (Barilla) instead of Oreo® biscuits
- For the submission of the animals to the pharmacological treatment, see: Placidi et al., Int J Eat Disord 2004 36 (3) 328-41

Based on the model by Hagan et al, and incorporating the above changes, a model for binge eating may be set up as follows. Female Sprague Dawley rats (7 weeks old) can be single housed at 21 ± 1C and under a 12 h/12h light/dark cycle (dark phase: 18:00 - 06:00) for all the period of the experiment. Before beginning, rats can be habituated to these conditions for 7 days. The rats can then be divided into 2 groups (16 animals/group). One group may take food ad lib (non restricted group: NR) for the 24 days of the experiment, while the other (Restricted Group: R) may receive a restricted diet (66 % of the quantity food intake that the other control group was eating) during 4 days, and eat ad lib for the following 4 days. This cycle can be repeated 3 times. Following the last day of refeeding, each group can be subdivided in two. Eight animals of group NR and 8 animals of group R can receive a stress stimulus (4 Foot Shocks (FS) of 0.6 mA separated by 15 sec intervals) while the other 2 subgroups can remain in the same cage where FS is delivered for the same period of time, but without receiving the FS (no shocked: NS). The 4 groups are S/NR, NS/NR, S/R and NS/R. Immediately following time in the shock apparatus, rats can be submitted to a pharmacological treatment (administration of the test compound or of the vehicle) and then be returned to the animal colony. A pre-measured amount of pellets or HP food (Baiocchi®) can be put in each cage, and food intake measured after 4 h. Food intake can be expressed in kcal.

Other relevant pre-clinical models have been described by, for example, Hudson A. L., Man J., Willems R., Nutt D. J. and Ashton D., at the 28th Annual Meeting Canadian College of Neuropsychopharmacology, July 2-5, 2005, St John's, Newfoundland, Canada.

Rationale - The effects of the title compound, prepared in an analogous way with Example 123, were tested in the animal model discussed above. The aim of the present study was to assess the effect of this compound in animals that received 3 cycles of diet (restricted:R) and stress (shocked) conditions similar to binge eating in humans.

Methods - Female Sprague Dawley rats (7-week old) were single housed at 21 ± 1°C and under a 12 h/12h light/dark cycle (dark phase: 18:00 - 06:00) for all the entire experimental period. Rats received a restricted diet (66 % of the food intake vs. control group) during 4 days, and ate ad libitum for the following 4 days. This cycle was repeated 3 times. Additionally, two other groups were eating ad libitum (not restricted: NR) and received the shock (NR/S). Following the last day of refeeding, the animals received a stress stimulus (4 Foot Shocks (FS) of 0.6 mA separated by 15 sec interval) and received vehicle or the title compound (0.03, 0.3 or 3 mg/kg i.p.) immediately after the shock. These were the S/R (shocked/Restricted) rats. The S/NR rats received the shock and vehicle or the title compound (prepared in an analogous way with Example 123; 3 mg/kg i.p.) in the same way as the S/R groups. Immediately following time spent in the shock apparatus, rats were returned to the animal colony. A pre-measured amount of Chow (pellets) or HP food (Baiocchi®) was put in each cage, and food intake was measured after 4 hrs.

### Results -

| Pharmacological Treatment after FS | n | HP Intake after 4 h (Kcal) | Chow Intake after 4 h (Kcal) | Total Food Intake after 4 h (Kcal) |
|---|---|---|---|---|
| S/R + vehicle i.p. | 18 | 19.57 ± 2.51 | 0.80 + 0.39 | 20.37 + 2.53 |
| S/R + 0.03 mg/kg i.p. | 19 | 17.11 ± 2.49 | 1.95 + 0.58 | 19.06 + 2.44 |
| S/R + 0.3 mg/kg i.p. | 19 | 14.44 ± 2.15 | 2.09 ± 0.74 | 16.54 + 1.94 |
| S/R + 3 mg/kg i.p. | 20 | 8.89 ± 1.90 | 2.62 ± 0.79 | 11.51 + 1.99 |
| | | | | |
| S/NR + vehicle i.p. | 8 | 8.30 ± 1.78 | 1.56 ± 1.03 | 9.85 + 1.40 |
| S/NR + 3 mg/kg i.p. | 8 | 8.73 ± 2.75 | 1.92 ± 0.66 | 10.65 ± 2.75 |

| | | | | |
|---|---|---|---|---|
| Data analysis has been performed using a statistical program (Statistica, STASOFT) | | | | |

Analysis of the first 4 groups (S/R): there was a significant effect of the title compound on total Food Intake (1-way ANOVA (F(1, 36)= 7.75; p = 0.008); the highest dose of 3 mg/kg significantly decreased total food intake.

There was a significant effect of the title compound on HP (ANOVA (F(1, 36)= 11.6,p = 0.002); the highest dose of 3 mg/kg significantly decreased HP intake (Dunnett's: p < 0.01). There was a significant effect of the title compound on Chow (F(1, 36)= 4.29, p = 0.04); the highest dose of 3 mg/kg significantly decreased chow Intake (Dunnett's, p < 0.05).

The compound did not show any inhibitory effect in non-stressed animals, in respect to controls.

Conclusion - The title compound produced a significant inhibitory effect on stress-induced overeating. No significant effects were observed in non-stressed animals.

## Claims

1. Use of a compound of formula (I) or a pharmaceutically acceptable salt or solvate salt thereof: 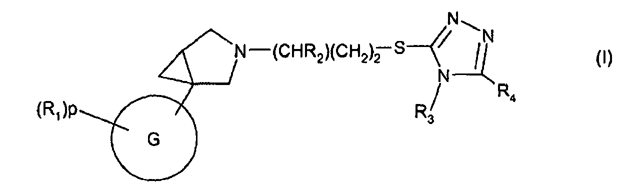 wherein:
G is selected from a group consisting of: phenyl, pyridyl, benzothiazolyl, indazolyl;
p is an integer ranging from 0 to 5;
R₁ is independently selected from a group consisting of: halogen, hydroxyl, cyano, C₁₋ ₄alkyl, haloC₁₋₄alkyl, C₁₋₄alkoxy, haloC₁₋₄alkoxy, C₁₋₄alkanoyl; or corresponds to a group R₅;
R₂ is hydrogen or C₁₋₄alkyl;
R₃ is C₁₋₄alkyl;
R₄ is hydrogen, or a phenyl group, a heterocyclyl group, a 5- or 6-membered heteroaromatic group, or a 8- to 11-membered bicyclic group, any of which group is optionally substituted by 1, 2, 3 or 4 substituents selected from the group consisting of: halogen, cyano, C₁₋₄alkyl, haloC₁₋₄alkyl, C₁₋₄alkoxy, C₁₋₄alkanoyl;
R₅ is a moiety selected from the group consisting of: isoxazolyl, -CH₂-N-pyrrolyl, 1,1- dioxido-2-isothiazolidinyl, thienyl, thiazolyl, pyridyl, 2-pyrrolidinonyl, and such a group is optionally substituted by one or two substituents selected from: halogen, cyano, C₁₋₄alkyl, haloC₁₋₄alkyl, C₁₋₄alkoxy, C₁₋₄alkanoyl; and when R₁ is chlorine and p is 1, such R₁ is not present in the ortho position with respect to the linking bond to the rest of the molecule; and when R₁ corresponds to R₅, p is 1;
in the preparation of a medicament for the treatment of a somatoform disorder such as body dysmorphic disorder or hyperchondriasis, bulimia nervosa, anorexia nervosa, binge eating, paraphilia and nonparaphilic sexual addictions, Sydenham's chorea torticollis ; and in the preparation of a medicament for the treatment of premature ejaculation.

2. Use of a compound of formula (I) according to claim 1 in the preparation of a medicament for the treatment of binge eating.

3. Use of a compound of formula (I) according to claim 1 in the preparation of a medicament for the treatment of premature ejaculation.

4. Use, according to claim 1, in which the compound of formula (I) is (1S,5R)-1-[2-fluoro-4-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1.0]hexane or a pharmaceutically acceptable salt or solvate thereof.

5. Use, according to claim 2, in which the compound of formula (I) is (1S,5R)-1-[2-fluoro-4-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1.0]hexane or a pharmaceutically acceptable salt or solvate thereof.

6. Use, according to claim 3, in which the compound of formula (I) is (1S,5R)-1-[2-fluoro-4-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1.0]hexane or a pharmaceutically acceptable salt or solvate thereof.

7. A compound of formula (I) as defined In claim 1 or a pharmaceutically acceptable salt or solvate thereof for use in the treatment of a somatoform disorder such as body dysmorphic disorder or hyperchondriasis, bulimia nervosa, anorexia nervosa, binge eating, paraphilia and nonparaphilic sexual addictions, Sydenham's chorea, torticollis; and premature ejaculation.

8. A compound of formula (I) as defined in claim 1 or a pharmaceutically acceptable salt or solvate thereof for use in the treatment of binge eating.

9. A compound of formula (I) as defined in claim 1 or a pharmaceutically acceptable salt or solvate thereof for use in the treatment of premature ejaculation.

10. (1S,5R)-1-[2-Fluoro-4-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1.0]hexane or a pharmaceutically acceptable salt or solvate thereof for use in the treatment of a somatoform disorder such as body dysmorphic disorder or hyperchondriasis, bulimia nervosa, anorexia nervosa, binge eating, paraphilia and nonparaphilic sexual addictions, Sydenham's chorea, torticollis; and premature ejaculation.

11. (1S,5R)-1-[2-Fluoro-4-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1.0]hexane or a pharmaceutically acceptable salt or solvate thereof for use in the treatment of binge eating.

12. (1S,5R)-1-[2-Fluoro-4-(trifluoromethyl)phenyl]-3-(3-{[4-methyl-5-(4-methyl-1,3-oxazol-5-yl)-4*H-*1,2,4-triazol-3-yl]thio}propyl)-3-azabicyclo[3.1,0]hexane or a pharmaceutically acceptable salt or solvate thereof for use in the treatment of premature ejaculation.

## Patentansprüche

1. Verwendung einer Verbindung der Formel (I) oder eines pharmazeutisch verträglichen Salzes oder Solvat des Salzes davon: 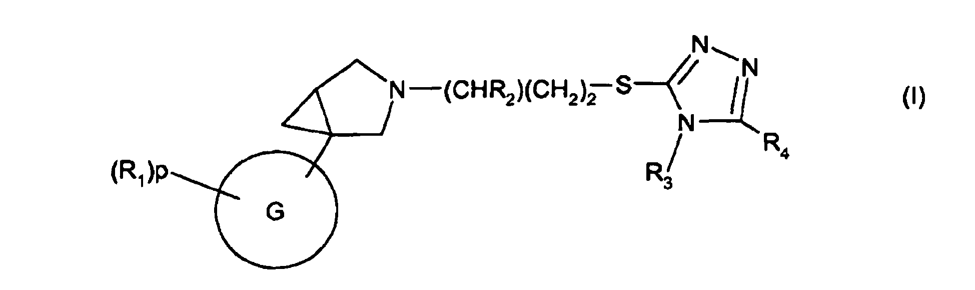 wobei:
G ausgewählt ist aus einer Gruppe bestehend aus Phenyl, Pyridyl, Benzothiazolyl, Indazolyl;
p eine ganze Zahl im Bereich von 0 bis 5 ist;
R₁ unabhängig ausgewählt ist aus einer Gruppe bestehend aus Halogen, Hydroxyl, Cyano, C₁-C₄-Alkyl, Halogen-C₁₋₄-alkyl, C₁₋₄-Alkoxy, Halogen-C₁₋₄-alkoxy, C₁₋₄-Alkanoyl oder einem Rest R₅ entspricht;
R₂ Wasserstoff oder C₁₋₄-Alkyl ist;
R₃ C₁₋₄-Alkyl ist;
R₄ Wasserstoff oder ein Phenylrest, ein Heterocyclylrest, ein 5- oder 6-gliedriger heteroaromatischer Rest oder ein 8- bis 11-gliedriger bicyclischer Rest ist, wobei die Reste gegebenenfalls jeweils mit 1, 2, 3 oder 4 Substituenten, ausgewählt aus der Gruppe bestehend aus Halogen, Cyano, C₁₋₄-Alkyl, Halogen-C₁₋₄-alkyl, C₁₋₄- Alkoxy, C₁₋₄-Alkanoyl, substituiert sind;
R₅ eine Einheit, ausgewählt aus der Gruppe bestehend aus Isoxazolyl, -CH₂-N- Pyrrolyl, 1,1-Dioxido-2-isothiazolidinyl, Thienyl, Thiazolyl, Pyridyl, 2-Pyrrolidinonyl, ist und ein solcher Rest gegebenenfalls mit einem oder zwei Substituenten, ausgewählt aus Halogen, Cyano, C₁₋₄-Alkyl, Halogen-C₁₋₄-alkyl, C₁₋₄-Alkoxy, C₁₋₄-Alkanoyl, substituiert ist; und wenn R₁ Chlor ist und p 1 ist, ein solcher Rest R₁ nicht in ortho-Position, bezogen auf die verbindende Bindung zum Rest des Molküls, vorhanden ist; und wenn R₁ R₅ entspricht, p 1 ist;
zur Herstellung eines Medikaments zur Behandlung einer somatoformen Störung, wie einer körperdysmorphen Störung oder Hyperchondriasis, Bulimie, Anorexie, Binge Eating, Paraphilie und nicht paraphilischen sexuellen Abhängigkeiten, Sydenham-Chorea, Torticollis, und zur Herstellung eines Medikaments zur Behandlung von vorzeitiger Ejakulation.

2. Verwendung einer Verbindung der Formel (I) gemäß Anspruch 1 zur Herstellung eines Medikaments zur Behandlung von Binge Eating.

3. Verwendung einer Verbindung der Formel (I) gemäß Anspruch 1 zur Herstellung eines Medikaments zur Behandlung von vorzeitiger Ejakulation.

4. Verwendung gemäß Anspruch 1, wobei die Verbindung der Formel (I) (1S,5R)-1-[2-Fluor-4-(trifluormethyl)phenyl]-3-(3-{[4-methyl-5-(4-methyl-1,3-oxazol-5-yl)-4H*-*1,2,4-triazol-3-yl]thio}propyl)-3-azabicyclo[3.1.0]hexan oder ein pharmazeutisch verträgliches Salz oder Solvat davon ist.

5. Verwendung gemäß Anspruch 2, wobei die Verbindung der Formel (I) (1S,5R)-1-[2-Fluor-4-(trifluormethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexan oder ein pharmazeutisch verträgliches Salz oder Solvat davon ist.

6. Verwendung gemäß Anspruch 3, wobei die Verbindung der Formel (I) (1S,5R)-1-[2-Fluor-4-(trifluormethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexan oder ein pharmazeutisch verträgliches Salz oder Solvat davon ist.

7. Eine Verbindung der Formel (I) wie in Anspruch 1 definiert oder ein pharmazeutisch verträgliches Salz oder Solvat davon, zur Verwendung bei der Behandlung einer somatoformen Störung, wie einer körperdysmorphen Störung oder Hyperchondriasis, Bulimie, Anorexie, Binge Eating, Paraphilie und nicht paraphilischen sexuellen Abhängigkeiten, Sydenham-Chorea, Torticollis und vorzeitiger Ejakulation.

8. Eine Verbindung der Formel (I) wie in Anspruch 1 definiert oder ein pharmazeutisch verträgliches Salz oder Solvat davon zur Verwendung bei der Behandlung von Binge Eating.

9. Eine Verbindung der Formel (I) wie in Anspruch 1 definiert oder ein pharmazeutisch verträgliches Salz oder Solvat davon zur Verwendung bei der Behandlung von vorzeitiger Ejakulation.

10. (1S,SR)-1-[2-Fluor-4-(trifluormethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexan oder ein pharmazeutisch verträgliches Salz oder Solvat davon zur Verwendung bei der Behandlung einer somatoformen Störung, wie einer körperdysmorphen Störung oder Hyperchondriasis, Bulimie, Anorexie, Binge Eating, Paraphilie und nicht paraphilischen sexuellen Abhängigkeiten, Sydenham-Chorea, Torticollis und vorzeitiger Ejakulation.

11. (1S,5R)-1-[2-Fluor-4-(trifluormethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexan oder ein pharmazeutisch verträgliches Salz oder Solvat davon zur Verwendung bei der Behandlung von Binge Eating.

12. (1S,5R)-1-[2-Fluor-4-(trifluormethyl)phenyl]-3-(3-{[4-methyl-5-(4-methyl-1,3-oxazol-5-yl)-4H-1,2,4-triazol-3-yl]thio}propyl)-3-azabicyclo[3.1.0]hexan oder ein pharmazeutisch verträgliches Salz oder Solvat davon zur Verwendung bei der Behandlung von vorzeitiger Ejakulation.

## Revendications

1. Utilisation d'un composé de formule (I) ou d'un de ses sels ou produits de solvatation pharmaceutiquement acceptables : 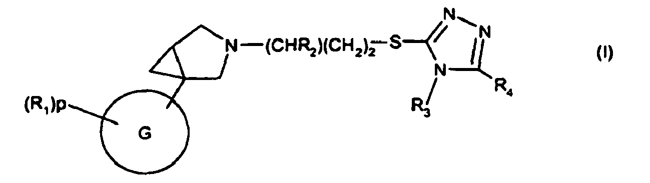 formule dans laquelle :
G est choisi dans un groupe consistant en des groupes : phényle, pyridyle, benzothiazolyle et indazolyle ;
p représente un nombre entier de 0 à 5 ;
R₁ est choisi indépendamment dans un groupe consistant en des groupes : halogéno, hydroxyle, cyano, alkyle en C₁ à C₄, halogénalkyle en C₁ à C₄, alkoxy en C₁ à C₄, halogénalkoxy en C₁ à C₄ et alcanoyle en C₁ à C₄ ; ou bien correspond à un groupe R₅ ;
R₂ représente un atome d'hydrogène ou un groupe alkyle en C₁ à C₄ ;
R₃ représente un groupe alkyle en C₁ à C₄ ;
R₄ représente un atome d'hydrogène, ou un groupe phényle, un groupe hétérocyclyle, un groupe hétéroaromatique penta- ou hexagonal ou un groupe bicyclique octo- à undécagonal, n'importe lequel de ces groupes étant facultativement substitué avec 1, 2, 3 ou 4 substituants choisis dans le groupe consistant en des groupes : halogéno, cyano, alkyle en C₁ à C₄, halogénalkyle en C₁ à C₄, alkoxy en C₁ à C₄ et alcanoyle en C₁ à C₄ ;
R₅ représente un groupement choisi dans le groupe consistant en des groupements : isoxazolyle, -CH₂-N-pyrrolyle, 1,1-dioxydo-2-isothiazolidinyle, thiényle, thiazolyle, pyridyle et 2-pyrrolidinonyle, et un tel groupe est facultativement substitué avec un ou deux substituants choisis entre des substituants : halogéno, cyano,
alkyle en C₁ à C₄, halogénalkyle en C₁ à C₄, alkoxy en C₁ à C₄ et alcanoyle en C₁ à C₄ ;
et, lorsque R₁ représente un atome de chlore et p est égal à 1, ce groupe R₁ n'est pas présent en position ortho par rapport à la liaison de réunion au reste de la molécule ; et, lorsque R₁ correspond à R₅, p est égal à 1 ;
dans la préparation d'un médicament destiné au traitement d'un trouble somatoforme tel qu'un trouble dysmorphique corporel ou l'hyperchondriase, la boulimie mentale, l'anorexie mentale, l'alimentation avec bombance, la paraphilie et des addictions sexuelles non paraphiliques, la chorée de Sydeham et le torticolis ; et dans la préparation d'un médicament destiné au traitement de l'éjaculation précoce.

2. Utilisation d'un composé de formule (I) suivant la revendication 1 dans la préparation d'un médicament destiné au traitement de l'alimentation avec bombance.

3. Utilisation d'un composé de formule (I) suivant la revendication 1 dans la préparation d'un médicament destiné au traitement de l'éjaculation précoce.

4. Utilisation suivant la revendication 1, dans laquelle le composé de formule (I) est le (1S,5R)-1-[2-fluoro-4-(trifluorométhyl)phényl]-3-(3-{[4-méthyl-5-(4-méthyl-(1,3-oxazole-5-yl)-4*H-*1,2,4-triazole-3-yl]thio}propyl)-3-azabicyclo-[3.1.0]hexane ou un de ses sels ou produits de solvatation pharmaceutiquement acceptables.

5. Utilisation suivant la revendication 2, dans laquelle le composé de formule (I) est le (1S,5R)-1-[2-fluoro-4-(trifluorométhyl)phényl]-3-(3-{[4-méthyl-5-(4-méthyl-(1,3-oxazole-5-yl)-4*H-*1,2,4-triazole-3-yl]thio}propyl)-3-azabicyclo-[3.1.0]hexane ou un de ses sels ou produits de solvatation pharmaceutiquement acceptables.

6. Utilisation suivant la revendication 3, dans laquelle le composé de formule (I) est le (1S,5R)-1-[2-fluoro-4-(trifluorométhyl)phényl]-3-(3-{[4-méthyl-5-(4-méthyl-(1,3-oxazole-5-yl)-4*H-*1,2,4-triazole-3-yl]thio}propyl)-3-azabicyclo-[3.1.0]hexane ou un de ses sels ou produits de solvatation pharmaceutiquement acceptables.

7. Composé de formule (I) suivant la revendication 1 ou un de ses sels ou produits de solvatation pharmaceutiquement acceptables, destiné à être utilisé dans le traitement d'un trouble somatoforme tel que le trouble dysmorphique corporel ou l'hyperchondriase, la boulimie mentale, l'anorexie mentale, l'alimentation avec bombance, la paraphilie et des addictions sexuelles non paraphiliques, la chorée de Sydeham et le torticolis ; et de l'éjaculation précoce.

8. Composé de formule (I) suivant la revendication 1 ou un de ses sels ou produits de solvatation pharmaceutiquement acceptables, destiné à être utilisé dans le traitement de l'alimentation avec bombance.

9. Composé de formule (I) suivant la revendication 1 ou un de ses sels ou produits de solvatation pharmaceutiquement acceptables, destiné à être utilisé dans le traitement de l'éjaculation précoce.

10. (1S,5R)-1-[2-fluoro-4-(trifluorométhyl)phényl]-3-(3-{[4-méthyl-5-(4-méthyl-(1,3-oxazole-5-yl)-4*H*-1,2,4-triazole-3-yl]thio}propyl)-3-azabicyclo-[3.1.0]hexane ou un de ses sels ou produits de solvatation pharmaceutiquement acceptables, destiné à être utilisé dans le traitement d'un trouble somatoforme tel que le trouble dysmorphique corporel ou l'hyperchondriase, la boulimie mentale, l'anorexie mentale, l'alimentation avec bombance, la paraphilie et des addictions sexuelles non paraphiliques, la chorée de Sydeham et le torticolis ; et de l'éjaculation précoce.

11. (1S,5R)-1-[2-fluoro-4-(trifluorométhyl)phényl]-3-(3-{[4-méthyl-5-(4-méthyl-(1,3-oxazole-5-yl)-4*H*-1,2,4-triazole-3-yl]thio}propyl)-3-azabicyclo-[3.1.0]hexane ou un de ses sels ou produits de solvatation pharmaceutiquement acceptables, destiné à être utilisé dans le traitement de l'alimentation avec bombance.

12. (1S, 5R)-1-[2-fluoro-4-(trifluorométhyl)phényl]-3-(3-{[4-méthyl-5-(4-méthyl-(1,3-oxazole-5-yl)-4*H*-1,2,4-triazole-3-yl]thio}propyl)-3-azabicyclo-[3.1.0]hexane ou un de ses sels ou produits de solvatation pharmaceutiquement acceptables, destiné à être utilisé dans le traitement de l'éjaculation précoce.
